# EUROPEAN PATENT SPECIFICATION

(11) **EP 3 607 084 B1**
(45) Date of publication and mention of the grant of the patent: **01.05.2024**
(21) Application number: 18780814.2
(22) Date of filing: 06.04.2018
(51) Int. Cl.: C12Q 1/18, C12Q 1/02, C12Q 1/06, G01N 15/14

(54) **A METHOD FOR TESTING ANTIMICROBIAL SUSCEPTIBILITY**
VERFAHREN ZUM TESTEN DER ANTIMIKROBIELLEN EMPFINDLICHKEIT
PROCÉDÉ DE TEST DE LA SENSIBILITÉ ANTIMICROBIENNE

(30) Priority: 07.04.2017 AU 2017901295; 24.08.2017 AU 2017903423
(43) Date of publication of application: 12.02.2020
(73) Proprietor: The University Of Western Australia, Nedlands, WA 6009 (AU)
(72) Inventor: INGLIS, Timothy John Jay, Attadale Western Australia 6156 (AU); MULRONEY, Kieran, Coodanup Western Australia 6210 (AU); HALL, Jarrad, Rivervale Western Australia 6103 (AU)
(74) Representative: AWA Sweden AB
(86) International application number: PCT/AU2018/050318
(87) International publication number: WO 2018/184073

(56) References cited:
- WO-A1-2012/164547
- WO-A1-2014/037883
- WO-A1-2015/111032
- WO-A1-2018/007504
- WO-A2-2013/003498
- US-A1- 2009 158 823
- Jarrad Michael Hall: "Molecular biology and epidemiology of multi-resistant Klebsiella pneumoniae in Western Australia THESIS - DOCTOR OF PHILOSOPHY - HALL Jarrad Michael - 2016", , 1 January 2016 (2016-01-01), XP055758341, Retrieved from the Internet: URL:https://research-repository.uwa.edu.au /files/14750178/THESIS_DOCTOR_OF_PHILOSOPH Y_HALL_Jarrad_Michael_2016.pdf
- HUANG, T-H. et al.: "Rapid Cytometric Antibiotic Susceptibility Testing Utilizing Adaptive Multidimensional Statistical Metrics", Analytical Chemistry, vol. 87, 13 January 2015 (2015-01-13), pages 1941-1949, XP055320068,
- SAINT-RUF, C. et al.: "Antibiotic Susceptibility Testing of the Gram-Negative Bacteria Based on Flow Cytometry", Frontiers in Microbiology, vol. 7 26 July 2016 (2016-07-26), XP055540198, Retrieved from the Internet: URL:https://www.frontiersin.org/articles/1 0.3389/fmicb.20 16.0 1121/full [retrieved on 2018-02-06]
- CHAU, F. et al.: "Flow Cytometry as a Tool to Determine the Effects of Cell Wall- Active Antibiotics on Vancomycin-Susceptible and -Resistant Enterococcus faecalis Strains", Antimicrobial Agents and Chemotherapy, vol. 55, no. 1 2011, pages 395-398, XP055439991, Retrieved from the Internet: URL:DOI:10.1128/AAC.00970-10
- OTTO, C.C. et al.: "Effects of Antibacterial Mineral Leachates on the Cellular Ultrastructure, Morphology, and Membrane Integrity of Escherichia coli and Methicillin-Resistant Staphylococcus aureus", Annals of Clinical Microbiology and Antimicrobials, vol. 9, no. 26 16 September 2010 (2010-09-16), XP055540211, Retrieved from the Internet: URL:https://ann-clinmicrob.biomedcentral.e om/articles/1 0. 1 186/1476-0711-9-26 doi: 10.1186/1476-0711-9-26 [retrieved on 2018-02-06]
- PAULANDER, W. et al.: "Bactericidal Antibiotics Increase Hydroxyphenyl Fluorescein Signal by Altering Cell Morphology", PLOS One, vol. 9 19 March 2014 (2014-03-19), XP055540218, Retrieved from the Internet: URL:https://doi.org/10.1371/journal.pone.0 092231 [retrieved on 2018-05-11]
- BRAGA, P.C. et al.: "Flow Cytometric Assessment of Susceptibilities of Streptococcus pyogenes to Erythromycin and Rokitamycin", Antimicrobial Agents and Chemotherapy, vol. 47, no. 1 January 2003 (2003-01), pages 408-412, XP055540225, Retrieved from the Internet: URL:doi: 10.1128/AAC.47.1.408-412.2003
- MOJSOSKA, B. et al.: "Peptoids Successfully Inhibit the Growth of Gram Negative E. coli Causing Substantial Membrane Damage", Scientific Reports, vol. 7 14 February 2017 (2017-02-14), XP055540231, Retrieved from the Internet: URL:https://www. nature .com/articles/srep42332 [retrieved on 2018-04-12]
- WENISCH, C. et al.: "Antifungal Susceptibility Testing of Fluconazole by Flow Cytometry Correlates with Clinical Outcome", Journal of Clinical Microbiology, vol. 39, no. 7 July 2001 (2001-07), pages 2458-2462, XP055540237, Retrieved from the Internet: URL:DOI: 10.1128/JCM.39.7.2458-2462.2001
- WALBERG, M. et al.: "Rapid Assessment of Ceftazidime, Ciprafloxacin, and Gentamicin Susceptibility in Exponentially-Growing E. coli Cells by Means of Flow Cytometry", Cytometry, vol. 27, no. 2 1 February 1997 (1997-02-01), pages 169-178, XP055540243, Retrieved from the Internet: URL:doi:10.1002/(SICI)1097-0320(19970201)2 7:2169:
- MASON, D.J. et al.: "Rapid Estimation of Bacterial Antibiotic Susceptibility with Flow Cytometry", Journal of Microscopy, vol. 176, no. Pt.1 October 1994 (1994-10), pages 8-16, XP055540252, Retrieved from the Internet: URL:https://doi.org/10.1111/j.1365-2818.19 94.tb03494.x
- NASEER, U.: "EUPHEM Report: Sumary of work activities", European Centre for Disease Prevention and Control, 7 November 2016 (2016-11-07), page 10, XP055540305, Retrieved from the Internet: URL:https://ecdc. europa . eu/sites/portal/files/media/en/epiet/who-w e- are/Documents/Portfolios%20C20t4/Summary%2 0of%20work%20activities_%20Uma er%20Naseer%20 _ 2016.pdf [retrieved on 2018-05-04]
- MULRONEY, K.T. et al.: "Rapid Susceptibility Profiling o Carbapenem-Resistant Klebsiella pneumoniae", Scientific Reports, vol. 7, no. 1 15 May 2017 (2017-05-15), pages 1-13, XP055540312, Retrieved from the Internet: URL:https://www. nature .com/articles/s 41598-017 -02009-3 doi: 10.1038/s41598-017-02009-3 [retrieved on 2018-01-29]

## Description

### FIELD OF THE INVENTION

The present invention relates to methods for the qualitative and quantitative determination of the susceptibility of microbial organisms such as bacteria to antimicrobial agents such as antibiotics.

### BACKGROUND

A review on Antimicrobial Resistance (AMR) estimated that 700,000 people die from infections due to resistant organisms every year, and that by 2050 AMR will surpass cancer as a cause of death [The Review on Antimicrobial Resistance. (2016). Tackling drug-resistant infections globally: Final report and recommendations (ed. O'Neil, J.) 1-72 (HM Government 2016)]. The World Health Organization (WHO) recognises AMR as a serious threat to global health [World Health Organization. Antimicrobial resistance. Factsheet no. 194. http://www.who.int/mediacentre/factsheets/fs194/en/HO AMR challenge], and singled out the emergence of carbapenem-resistant *Klebsiella* as its leading priority in the first WHO global report on AMR in 2014 [World Health Organization. WHO's first global report on antibiotic resistance reveals serious, worldwide threat to public. World Health Organisation Media Centre http://www.who.int/mediacentre/news/releases/2014/amr-report/en/ (2014)].

There are many types of antimicrobial agents. Antibiotics make up an important class of antimicrobial agents. There are also different types of antibiotics. Beta-lactam antibiotics form one example of antibiotic class. For example, the carbapenems belong to a broad-spectrum class of antibiotics having antibacterial activity against many aerobic and anaerobic Gram-positive and Gram-negative organisms. This class of beta-lactam antibiotics is particularly useful in treating bacterial infections due to their resistance to beta-lactamase enzymes (also known as penicillinases and cephalosporinases) produced by bacteria. By way of example of rise in AMR, a number of bacteria have developed an acquired form of resistance to carbapenem antibiotics.

For example, *Klebsiella* species are the most prominent carbapenem-resistant Enterobacteriaceae (CRE) and cause an excess hospital mortality of 27% in patients with septicemia and pneumonia [Hauck, C. et al., Spectrum of excess mortality due to carbapenem-resistant Klebsiella pneumoniae infections. Clin. Microbiol. Infect. 22, 513-519 (2016)]. For example, the Indian Ocean Rim region has become one of the main foci of emerging carbapenemases, and has seen successive waves of diverse forms of carbapenem-resistant *K*. *pneumoniae* [Hall, J.M. et al. Molecular mechanisms of β-lactam resistance in carbapenemase-producing Klebsiella pneumoniae from Sri Lanka. J. Med. Microbiol. 63, 1087-92 (2014); and Hall, J. M., Ingram, P. R., O'Reilly, L. C., & Inglis, T. J. J. Temporal flux in beta-lactam resistance among Klebsiella pneumoniae in Western Australia. J. Med. Microbiol. 65, 429-437 (2016)].

Due to the growing spread of AMR and its impact on patient safety, *e.g.,* as exemplified by the expanding global distribution of multi-resistant *Klebsiella pneumoniae* and its impact on safety of patients infected with *Klebsiella pneumoniae,* there is an increased need for a faster antimicrobial susceptibility testing system to guide the use of antimicrobial compounds such as antibiotics in patient treatment. Thus, faster antimicrobial susceptibility test (AST) methods are needed to stop inappropriate use of antimicrobial agents such as antibiotics, and therefore combat or halt the rise of AMR.

Notwithstanding the need in the art for fast antimicrobial susceptibility testing system, current laboratory techniques for determining susceptibility of microorganisms to antimicrobial agents are time-consuming because they rely heavily on culture-dependent methods to differentiate between resistance and susceptibility following the initial isolation of the microorganisms (such as bacteria) from a clinical specimen.

For example, detection of multidrug-resistant bacteria currently relies on primary isolation of the infecting bacteria from a clinical specimen followed by largely culture-dependent AST procedures. This approach delays the selection and commencement of effective targeted treatment, infection prevention and infection control measures by at least about 24 to 72 hours generally associated with isolation and culturing of the bacteria from the clinical specimen. Because of such delay in the selection and commencement of effective targeted treatment, inappropriate broad spectrum antimicrobials are used in the absence of empirical laboratory results.

Current rapid non-culture-based screening methods for susceptibility to antimicrobial agents such as mechanism-specific PCR assays and the widely used Carba-NP test, can be unreliable [Goire N. et al. The implications of endemic IMP-4 carbapenemase for clinical laboratory susceptibility testing. J. Microbiol. Methods. 124, 10-2 (2016)], which diminishes their value for predicting carbapenem susceptibility and thus diminishes their utility for the prescribing physician. For example, with these tests while positive results indicate likely carbapenem resistance, a negative result does not reliably predict a carbapenem-susceptible phenotype.

Minimum Inhibitory Concentration (MIC) determination by broth microdilution (BMD) is the internationally recognized standard for AST (ISO 200776-1, 2006) (ISO 20776-1:2006. *Clinical laboratory testing and in vitro diagnostic test systems* -- *Susceptibility testing of infectious agents and evaluation of performance of antimicrobial susceptibility test devices* -- Part 1: Reference method for testing the in vitro activity of antimicrobial agents against rapidly growing aerobic bacteria involved in infectious diseases]. A categorical classification (susceptible, intermediate or resistant - SIR, or susceptible/non-susceptible - SINS) can be made by comparing the MIC to species-specific breakpoints for the microorganism in question, using breakpoints issued either by the European Committee on Antimicrobial Susceptibility Testing (EUCAST) or the Clinical & Laboratory Standards Institute (CLSI). All current methods for AST are validated against BMD before introduction into clinical practice [Matuschek, E., Brown, D. F. J., Kahlmeter, G. Development of the EUCAST disk diffusion antimicrobial susceptibility testing method and its implementation in routine microbiology laboratories. Clin. Microbiol. Infect. 20, 0255-66 (2014)].

For example, both the EUCAST disk diffusion method, and the SIR categorization achieved by BMD method, require a secondary culture step which adds 16-20 hours to test completion times. Again, this delays the selection and commencement of effective targeted treatment and as a result requires physicians to prescribe broad spectrum antimicrobial agents such as antibiotics prior to empirical laboratory results.

Flow cytometry has been considered a candidate method for delivering rapid AST [Martinez, O. V., Gratzner, H. G., Malinin, T. I., Ingram, M. The effect of some β-lactam antibiotics on Escherichia coli studied by flow cytometry. Cytometry. 3, 129-1331 (1982)]. However, early flow cytometry analyses of bacteria were limited by their low resolution to studies of cellular aggregation. The introduction of bacterial viability dyes, improved flow cytometer resolution, and increased sophistication of multi-parameter analysis prompted renewed attempts to establish a method for flow-assisted antimicrobial susceptibility analysis [Gant, V. A., Warnes, G., Phillips, I., Savidge, G. F. The application of flow cytometry to the study of bacterial responses to antibiotics. J. Med. Microbiol. 39, 147-154 (1993); Durodie, J. et al. Rapid detection of antimicrobial activity using flow Cytometry. Cytometry 21, 374-377 (1995); Deere, D., Porter J., Edwards, C., Pickup, R. Evaluation of the suitability of bis-(1,3-dibutylbarbituric acid) trimethineoxonol, (diBA-C4(3)-), for the flow cytometric assessment of bacterial viability. FEMS Microbiol. Lett. 130, 165-9 (1995); Mason, D. J. et al. Antibacterial action of ciprofloxacin. Antimicrob. Agents Chemother. 39, 2752-8 (1995); Novo, D. J., Perlmutter, N. G., Hunt, R. H., Shapiro H. M. Multiparameter flow cytometric analysis of antibiotic effects on membrane potential, membrane permeability, and bacterial counts of Staphylococcus aureus and Micrococcus luteus. Antimicrob. Agents Chemother. 44, 827-34 (2000)].

All the above studies employed conventional hydrodynamic flow cytometers. Notwithstanding that these studies produced a catalogue of complex interactions between membrane-permeable dyes and bacteria during sub-lethal damage, the resolution limits of even the best of previously available hydrodynamic flow cytometers still placed significant constraints on previous attempts for devising any Flow Cytometer-Assisted Susceptibility Test (FAST) methods. As such, to date there has been little progress in making FAST methods widely accessible for AST.

Accordingly, the limited resolution of existing hydrodynamic flow cytometers, as well as the technical demands of these non-standard flow cytometry procedures and the need for validation against existing standard methods, has hindered development of any effective method of flow cytometry-assisted susceptibility testing of antimicrobial agents for microorganisms, which for example can be implemented rapidly at the point of care *e.g.,* in hospitals.

Proceeding on this basis, there remains a need in the art for faster methods for accurate determination of susceptibility of unicellular or multicellular microorganisms to antimicrobial agents including those methods that provide accurate and fast qualitative determination of susceptibility and/or fast quantitative determination of susceptibility such as fast determination of the MIC of an antimicrobial agent to a specific microorganism found in a clinical specimen taken from a subject having or suspected of having an infection with the microorganism.

### SUMMARY OF INVENTION

### General

In the work leading to the present invention, the inventors sought to develop a method employing acoustic flow cytometry (AFC) that leads to rapid qualitative determination from the AFC output data of susceptibility or non-susceptibility to one or more antimicrobial agent(s) of a unicellular or multicellular microorganism such as bacteria, fungus or yeast, preferably a bacteria, that may be present in and/or infecting a subject or suspect of being present in and/or infecting the subject.

In the work leading to the present invention the inventors also sought to utilize the method employing AFC for rapid quantitative determination from the AFC output data the minimal inhibitory concentration(s) of the one or more antimicrobial agent(s) to which the microorganism that may be present in and/or infecting a subject or suspect of being present in and/or infecting the subject is susceptible.

The inventors speculated that such AFC-assisted susceptibility testing method for determining qualitatively and/or quantitatively the susceptibility of a microorganism (such as a bacteria, fungus or yeast) in a clinical specimen obtained from a subject having or suspected of having an infection with the microorganism, would largely circumvent the need to rely on initial isolation of the infecting microorganism from the clinical specimen and the subsequent lengthy culture-dependent AST procedures employed by current standard practice susceptibility testing methods which are currently in place in clinical and pathology laboratories including those in place in hospitals everywhere.

The inventors reasoned that such AFC-assisted susceptibility testing method for determining qualitatively and/or quantitatively susceptibility of a microorganism to one or more antimicrobial agent(s), would therefore provide physician with a valuable tool for very rapid qualitative determination of the susceptibility of the microorganism to one or more antimicrobial agent(s) and/or very rapid quantitation of the MIC of the antimicrobial agent(s) to which the microorganism is susceptible, thereby ensuring selection and commencement of effective targeted treatment, infection prevention and infection control measures, within only few short hours such as within about 1 to 6 hours and or even within about 1 to 3 hours from collection of clinical specimen. The inventors reasoned that such AFC-assisted susceptibility testing method would provide physicians with the relevant empirical laboratory results within only a short time from specimen collection and avoid the need of physicians to treat subjects with inappropriate antimicrobial agents such as broad spectrum antibiotics currently used in absence of such empirical results. The inventors reasoned that such AFC-assisted susceptibility testing method would therefore contribute to reducing emergence of further AMR strains.

Accordingly, the inventors reason that such AFC-assisted susceptibility testing method will be valuable in very fast determination of the susceptibility of a microorganism from a clinical specimen to tested antimicrobial agents and/or very fast determination of the MIC of the antimicrobial agent(s) to which the microorganism is susceptible. Such method will facilitate fast delivery of effective targeted treatment to the subject *e.g.,* within only a few hours of specimen collection. The inventors reasoned that such method would be particularly useful for rapid treatment of infections such as with multi-drug resistant bacteria e.g., in hospital environment or an emergency room where rapid clinical intervention is required to avoid progression of infection and or onset of clinical complications associated with progression of the infection.

As exemplified herein, the present inventors have demonstrated an AFC method capable of rapidly determining qualitatively susceptibility of a microorganism (*e.g.,* bacteria) to an antimicrobial agent (e.g., antibiotic) by assigning susceptibility categories, such as within about 1 hour from initial microbial sample handling. As exemplified herein the present inventors have also demonstrated that the AFC method is further capable of rapidly determining MICs of the antimicrobial agents (e.g., antibiotics) tested for which the microorganism (*e.g.,* bacteria) was shown to be susceptible, whereby the MICs determination was achieved *e.g.,* within about 3 hours from initial microbial sample handling *e.g.,* from initial sample collection from the subject and/or first handing of the sample to incubate cells in and/or from the sample in a culture medium. Therefore as exemplified herein, the present inventors have demonstrated a new AFC-assisted susceptibility testing method and its utility in very rapid assigning of susceptibility categories to antimicrobial agent(s) for a microorganism in a clinical specimen and/or very fast determination of the MIC of the antimicrobial agent(s) to which the microorganism is susceptible.

As also exemplified herein, the inventors have also demonstrated that the AFC-assisted susceptibility testing method can be performed by (i) culturing in a culture medium cells of a microorganism from a sample obtained from a subject having or suspected of having an infection with said microorganism, and/or (ii) directly culturing in a culture medium a sample obtained from the subject which comprises the cells of the microorganism e.g., without first separating cells from the subject sample before culturing the sample in the culture medium, so as to produce an actively dividing culture of the cells of the microorganism for use in the method of the invention described herein.

Specifically, as exemplified herein, the present inventors demonstrated an AFC method capable of rapidly determining carbapenem MICs and assigning susceptibility categories. Furthermore, the inventors demonstrated successful efficacy of this method by comparing the MIC results obtained by this method against those MIC results obtained by broth microdilution in a blinded, prospective validation using a collection of carbapenem-susceptible and carbapenem-resistant *K. pneumoniae* and *K. oxytoca* isolates, including internationally dominant ESBL- and carbapenemase-producing isolates. It will be understood that the term "ESBL" as used herein throughout refers to "Extended Spectrum Beta Lactamase", and encompass two specific classes of antimicrobial resistance mechanisms being ESBL and carbapenemase. As demonstrated herein the collection of microbial strains assayed by the present inventors comprised representatives from the subclasses of these resistance mechanisms that are most prevalent in the world.

More specifically, unlike current laboratory ASTs which rely on time-consuming and lengthy culture-dependent methods for differentiation of resistance and susceptibility after initial isolation of microorganism e.g., bacteria from a clinical specimen, as exemplified herein the present inventors demonstrate an acoustic flow cytometry workflow to rapidly determine carbapenem susceptibility from bacterial cell characteristics in an international *K. pneumoniae* isolate collection (n= 48), expressing a representative range of carbapenemases. The acoustic flow cytometry-assisted susceptibility test (FAST) method combines both (i) rapid qualitative susceptible/non-susceptible classification and (ii) rapid quantitative MIC measurement in a test process that can be completed shortly after receipt of a primary isolate or a clinical sample (for example 54 and 158 minutes respectively). The qualitative FAST results and FAST-derived MIC (MIC_{FAST}) correspond closely with broth microdilution MIC (MIC_{BMD}, Matthew's correlation coefficient 0.887), and align with the international AST standard (ISO 200776-1; 2006). Accordingly, the results exemplified herein demonstrate that the acoustic flow cytometry method of the present invention can be applied to rapid determination of antimicrobial susceptibility in a wider range of microorganisms including Gram negative and Gram positive bacteria.

### Specific examples of the invention

The invention is set out in the appended set of claims.

Accordingly, in a first broad aspect of the present invention there is provided a method of determining the susceptibility of a unicellular or multicellular microorganism to an antimicrobial agent by acoustic flow cytometry. The method comprises the following steps: (i) incubating in a culture medium (a) cells of the microorganism from a sample obtained from a subject having or suspected of having an infection with said microorganism, and/or (b) a sample obtained from the subject having or suspected of having an infection with said microorganism, wherein said sample comprises cells of said microorganism, wherein said incubating is for a time and under conditions sufficient for said cells to actively divide to thereby obtain an actively dividing culture of said cells of said microorganism; (ii) exposing cells of the microorganism in and/or from the actively dividing cell culture obtained in step (i) to an antimicrobial agent for a time and under conditions sufficient for said cells of the microorganism to actively divide; (iii) labelling cells of the microorganism exposed to the antimicrobial agent in step (ii) with a nucleic acid binding fluorescent compound; (iv) measuring effect of said antimicrobial agent on cellular morphology of said cells of the microorganism by acoustic flow cytometry; and (v) determining susceptibility of the microorganism to the antimicrobial agent from the acoustic flow cytometry data output, wherein step (v) comprises (A) determining susceptibility of the microorganism to the antimicrobial agent qualitatively by determining from the acoustic flow cytometry data output that said microorganism is either susceptible or not susceptible to said antimicrobial agent; and (B) determining susceptibility of the microorganism to the antimicrobial agent quantitatively by determining from the acoustic flow cytometry data output the minimal inhibitory concentration (MIC) of said antimicrobial agent to which the microorganism is susceptible.

In one example, the method according to this embodiment comprises obtaining a sample comprising cells of the microorganism from a subject having or suspected of having an infection with the microorganism.

Preferably, the nucleic acid binding fluorescent compound is cell membrane permeable, natively fluorescent within the cells of the microorganism, has enhanced fluorescence upon binding to a nucleic acid, and/or is photostable. For example, the nucleic acid binding fluorescent compound permeatese the cell membrane of the cells of the microorganism and is fluorescent when bound to nucleic acids within said cells of said microorganism. Preferably the nucleic acid binding fluorescent compound is freely cell membrane permeable.

Preferably, the nucleic acid binding fluorescent compound binds to cytoplasmic, mitochondrial and nuclear nucleic acids in the cells of the microorganism. For example, the nucleic acid binding fluorescent compound binds to deoxyribonucleic acid (DNA) and/or ribonucleic acid (RNA) in the cells of the microorganism.

In one example, the nucleic acid binding fluorescent compound comprises a nucleic acid binding moiety and a fluorescent tag moiety. However, in another example, nucleic acid binding fluorescent compounds suitable for use in the present invention may be a fluorophore compound not having separate nucleic acid binding moiety and fluorescent tag moiety. For example, the nucleic acid binding fluorescent compound may be a nucleic acid intercalating fluorophore dye.

In one example, the nucleic acid binding fluorescent compound is a flurophore dye selected from the commercially available Quant-iT^{™} range dyes (e.g., ThermoFisher Scientific), and/or from the commercially available membrane permeable SYBR^{™} range dyes (e.g., ThermoFisher Scientific) and/or from the commercially available Hoechst range dyes (e.g., ThermoFisher Scientific; or Invitrogen) including but not limited to Hoechst 33258 (pentahydrate bis-benzimide), Hoechst 33342 [also known as [2-(4-ethoxyphenyl)-6-[6-(4-methylpiperazin-1-yl)-1H-benzimidazol-2-yl]-1H-benzimidazole or trihydrochloride trihydrate], Hoechst 34580 [also known as N,N-dimethyl-4-[6-[6-(4-methylpiperazin-1-yl)-1H-benzimidazol-2-yl]-1H-benzimidazol-2-yl]aniline], or nuclear yellow Hoechst S769121 and/or diaminophenylindole [also known as 4',6-diamidino-2-phenylindole or 2-(4-carbamimidoylphenyl)-1H-indole-6-carboximidamide or DAPI] and membrane-permeable DAPI derivatives (e.g., Biotium or ThermoFisher Scientific) and/or propidium iodide (PI) (e.g., ThermoFisher Scientific or Stemcell Technologies), and/or ethidium bromide (EB or EtBr) (e.g., Sigma-Aldrich, Merck) and/or Hexidium iodide (e.g., ThermoFisher Scientific) and/or acridine orange [also known as (3-N,3-N,6-N,6-N-tetramethylacridine-3,6-diamine or AO] (e.g., ThermoFisher Scientific) and/or DRAQ5^{™} and DRAQ7^{™} range dyes (e.g., Biostatus, BioLegend, or Beckman Coulter, Inc) and/or SYTOX^{™} (also known as SYTO Green) range dyes (e.g., ThermoFisher Scientific) and/or SYTO ^{™} range dyes such as SYTO 9, SYTO 11, SYTO 12, SYTO 13, SYTO 14, SYTO 15, SYTO 16, SYTO 18, SYTO 20, SYTO 21, SYTO 22, SYTO 23, SYTO 24, SYTO 25, SYTO BC (e.g., ThermoFisher Scientific).

In one example, the nucleic acid binding fluorescent compound is a flurophore dye selected from the group consisting of SYTO 9, SYTO 11, SYTO 12, SYTO 13, SYTO 14, SYTO 15, SYTO 16, SYTO 18, SYTO 20, SYTO 21, SYTO 22, SYTO 23, SYTO 24, SYTO 25, SYTO BC, Hoechst 33342, propidium iodine (PI), DAPI and any combination thereof. For example, the nucleic acid binding fluorescent compound is a flurophore dye selected from the group consisting of SYTO 9, Hoechst 33342, and a combination of SYTO 9 and propidium iodine (PI).

Preferably, the nucleic acid binding fluorescent compound is SYTO 9 dye.

In one example of a method according to this embodiment, the step of labelling cells of the microorganism exposed to the antimicrobial agent in step (iii) with the nucleic acid binding fluorescent compound, comprises staining the cells of the microorganism with the nucleic acid binding fluorescent compound to discriminate between bacterial events and non-bacterial debris when measuring effect of the antimicrobial agent on cellular morphology of the cells of the microorganism by acoustic flow cytometry in step (iv).

In another example, the labelling the cells of the microorganism exposed to the antimicrobial agent in step (ii) with the nucleic acid binding fluorescent compound results in arrest in cell division of intact and/or replicating cells of the microorganism. For example, use of SYTO 9 and other DNA other DNA intercalating dyes, may arrest cell division by blocking microbial cel DNA synthesis and so may stop cell division before the increase in cytoplasmic volume.

In one example of a method according to this embodiment of the invention, the microorganism is susceptible to the antimicrobial agent. According to this example, measuring the effect of the antimicrobial agent on the cellular morphology of cells of the microorganism by acoustic flow cytometry may comprise measuring effect of the antimicrobial agent on the cellular morphology of the actively dividing cells of the microorganism derived from the subject as the cells become compromised by the antimicrobial agent and before cells lysis and/or complete cell breakdown following exposure of the actively dividing cells of the microorganism to the antimicrobial agent.

Preferably, the step of measuring the effect of the antimicrobial agent on the cellular morphology of cells of the microorganism by acoustic flow cytometry comprises measuring by acoustic flow cytometry the effect of the antimicrobial agent on (i) the amount nucleic acid content in said cells of said microorganism, and/or (ii) the cytoplasmic volume of the cells of the microorganism.

Also preferably, the step of measuring the effect of the antimicrobial agent on the cellular morphology of cells of the microorganism by acoustic flow cytometry comprises measuring a change in the cellular morphology of actively dividing cells of said microorganism as determined by acoustic flow cytometry following exposure of said cells of the microorganism to a concentration of said antimicrobial agent, relative to the cellular morphology of actively dividing cells of the microorganism in and/or from the sample obtained from the subject as determined by acoustic flow cytometry absent exposure of said cells to said antimicrobial agent.

It would be understood that any change can be measured down to the limit of resolution of the acoustic cytometer. It will also be understood that it is the magnitude of this shift that may be considered when making determinations for the inhibitory concentration.

For example, measuring a change in the cellular morphology of actively dividing cells of the microorganism following exposure of the cells of the microorganism to a concentration of the antimicrobial agent relative to the cellular morphology of actively dividing cells of the microorganism in and/or from the sample obtained from the subject absent exposure of said cells to said antimicrobial agent, comprises measuring a shift in the forward scatter and/or a change in fluorescence intensity on bi-axial plots of the acoustic flow cytometry output between: acoustic flow cytometry output data measured for cells of the microorganism following exposure of those cells to a concentration of the antimicrobial agent, relative to acoustic flow cytometry output data measured for cells of said microorganism absent exposure of the cells to the antimicrobial agent.

In one example, measuring the effect of the antimicrobial agent on the cellular morphology of cells of the microorganism by acoustic flow cytometry comprises measuring a change in the size of actively dividing cells of the microorganism following exposure of the cells to a concentration of the antimicrobial agent. For example, the method of the present invention may comprise measuring a change in the size of actively dividing cells of the microorganism as determined by acoustic flow cytometry following exposure of the cells to a concentration of the antimicrobial agent, relative to the size of actively dividing cells of the microorganism in and/or from the sample obtained from the subject as determined by acoustic flow cytometry absent exposure of the cells to the antimicrobial agent.

In one such example, measuring a change in the size of actively dividing cells of the microorganism comprises, measuring or determining a shift in the forward scatter and/or a change in fluorescence intensity on bi-axial plots of the acoustic flow cytometry output between the acoustic flow cytometry output data observed or measured for cells of the microorganism following exposure of the cells to the concentration of the antimicrobial agent, relative to the acoustic flow cytometry output data measured for cells of the microorganism in absence of exposure of the cells to the antimicrobial agent, wherein the shift in the forward scatter plot represents a change in the size of the actively dividing cells exposed to the antimicrobial agent relative to the size of actively cells of the microorganism in absence of exposure to the antimicrobial agent.

In another example, measuring a change in the size of actively dividing cells comprises measuring an increase in the size of actively dividing cells of the microorganism in and/or from the sample obtained from the subject as determined by acoustic flow cytometry following exposure of actively dividing cells of the microorganism to a concentration of the antimicrobial agent, relative to the size of actively dividing cells of the microorganism in and/or from the sample obtained from the subject as determined by acoustic flow cytometry in absence of exposure of the cells to the antimicrobial agent. In one such example, the method comprises measuring or determining a shift in the forward scatter and/or a change in fluorescence intensity on bi-axial plots of the acoustic flow cytometry output between the acoustic flow cytometry output data observed or measured for cells of the microorganism following exposure of the cells to the concentration of the antimicrobial agent, relative to the acoustic flow cytometry output data measured for cells of the microorganism in absence of exposure of the cells to the antimicrobial agent, wherein the shift in the forward scatter plot represents an increase in the size of the actively dividing cells exposed to the antimicrobial agent relative to the size of actively dividing cells of the microorganism in absence of exposure to the antimicrobial agent.

Preferably, measuring an increase in the size of actively dividing cells of the microorganism comprises measuring an increase in (i) the nucleic acid content in said cells of said microorganism, and/or (ii) the cytoplasmic volume of the cells of the microorganism *i.e.,* following exposure of the cells to a concentration of the antimicrobial agent relative to the size of actively dividing cells of the microorganism obtained from the subject without exposure to the antimicrobial agent. For example, the increase in the cytoplasmic volume of the cells of the microorganism comprises cell elongation and/or cell swelling and/or ballooning.

Preferably, in the examples and embodiments described herein above, the concentration of the antimicrobial agent is an inhibitory concentration of said antimicrobial agent to which the cells of the microorganism obtained from the subject are susceptible.

According to the present invention, the method according to this first broad aspect may comprises a step of validating or recording a change in cellular morphology of the cells of the microorganism following exposure of said cells to a concentration of the antimicrobial agent relative to cellular morphology of the cells of the microorganism in and/or from the sample obtained from the subject absent exposure of the cells to said antimicrobial agent by observation of the cellular morphology of said cells using fluorescence microscopy such as digital fluorescence microscopy.

According to the present invention, the method according to the first broad aspect of the invention, determining susceptibility of the microorganism obtained from the subject to the antimicrobial agent from the acoustic flow cytometry data output comprises determining susceptibility of the microorganism to the antimicrobial agent qualitatively by determining from the acoustic flow cytometry data output that said microorganism is either susceptible or not susceptible to said antimicrobial agent. In addition, determining susceptibility of the microorganism obtained from the subject to the antimicrobial agent from the acoustic flow cytometry data output comprises determining susceptibility of the microorganism to the antimicrobial agent quantitatively by determining from the acoustic flow cytometry data output the minimal inhibitory concentration (MIC) of the antimicrobial agent to which the microorganism is susceptible.

The method of the present invention comprises (i) determining that the microorganism obtained from the subject is either susceptible or not susceptible to the antimicrobial agent, and (ii) when the microorganism is determined in (i) to be susceptible to the antimicrobial agent, the method further comprises determining the MIC of the antimicrobial agent to which the microorganism is susceptible.

For example, determining from the acoustic flow cytometry data output that the microorganism is either susceptible or not susceptible to the antimicrobial agent comprises measuring from the acoustic flow cytometry data output the effect the antimicrobial agent has on the cellular morphology of the actively dividing cells of said microorganism at different concentrations of the antimicrobial agent. In one such example, the method of the present invention further comprises determining that the microorganism is susceptible to the antimicrobial agent when the acoustic flow cytometry data output measures a change in the cellular morphology of actively dividing cells of the microorganism obtained from the subject following exposure of the actively dividing cells of the microorganism to a concentration of the antimicrobial agent which is equals to or less than a pre-determined concentration of said antimicrobial agent, relative to the cellular morphology of actively dividing cells of the microorganism in and/or from the sample obtained from the subject absent exposure of those cells to said antimicrobial agent. In an alternative example, the method of the present invention further comprises determining that the microorganism is not susceptible to said antimicrobial agent when (i) the acoustic flow cytometry data output fails to measure a change in the cellular morphology of actively dividing cells of the microorganism exposure of the actively dividing cells of the microorganism obtained from the subject to a concentration of said antimicrobial agent which is equals to or less than said predetermined concentration of said antimicrobial agent and/or (ii) the acoustic flow cytometry data output measures a change in the cellular morphology of actively dividing cells of the microorganism following exposure of said actively dividing cells of said microorganism obtained from the subject to a concentration of said antimicrobial agent which is greater than said predetermined concentration of said antimicrobial agent.

For example, the acoustic flow cytometry data output measures a change in the cellular morphology of actively dividing cells of the microorganism following exposure of the cells of the microorganism from the subject to the concentration of said antimicrobial agent, by the acoustic flow cytometry data output measuring a shift in the forward scatter and/or a change in fluorescence intensity on bi-axial plots of the acoustic flow cytometry output between (i) the acoustic flow cytometry output data measured for the actively dividing cells of said microorganism following exposure of said cells to a concentration of said antimicrobial agent, and (ii) the acoustic flow cytometry output data measured for said actively dividing cells of said microorganism in and/or from the sample obtained from the subject absent exposure of said cells to said antimicrobial agent.

Preferably, a change in the cellular morphology of actively dividing cells of the microorganism comprises an increase in the size of the actively dividing cells of the microorganism. For example, the increase change in the size of the actively dividing cells of the microorganism comprises (i) an increase in the amount of nucleic acid content in said actively dividing cells of said microorganism, and/or (ii) an increase in the cytoplasmic volume of said actively dividing cells of said microorganism optionally as determined by cell elongation and/or cell swelling and/or ballooning.

Also preferably, the pre-determined concentration of the antimicrobial agent detailed in the examples herein above, is an internationally recognised clinical susceptibility breakpoint concentration or an internationally recognised MIC of said antimicrobial agent for said microorganism in the sample obtained from the or a species to which said microorganism belongs. Accordingly, it will be understood that the term "MIC" as used herein throughout, refers to the minimum inhibitory concentration of an antimictrobial agent which is the concentration of a antimicrobial agent that required to inhibit the growth of cells of a specific microbial isolate. For example, the pre-determined concentration of the antimicrobial agent is a clinical susceptibility breakpoint concentration for the antimicrobial agent as determined or issued by an internationally recognised antimicrobial susceptibility testing authority such as the European Committee on Antimicrobial Susceptibility Testing (EUCAST) (as can be found e.g., at http://www.eucast.org/) or the Clinical & Laboratory Standards Institute (CLSI) (as can be found e.g., at http://clsi.org/) in respect of the microorganism obtained from the patient or for a species to which said microorganism belongs. In the absence of a determined or issued clinical breakpoint, a concentration can be determined from epidemiological data to represent a concentration sufficiently low as to ensure clinical treatment success should the determined MIC fall below said concentration.

In one embodiment of the method according to this first broad aspect of the invention, the method comprises measuring from the acoustic flow cytometry data output the effect the antimicrobial agent has on the cellular morphology of the actively dividing cells of the microorganism obtained from subject at different concentrations of the antimicrobial agent, for example at concentrations of the antimicrobial agent comprising 0 mg/L, 0.25 mg/L, 1 mg/L, 2 mg/L, 4 mg/L and 16 mg/L.

In one embodiment of the method according to the first broad aspect of the present invention, determination from the acoustic flow cytometry data output that the microorganism obtained from the subject is either susceptible or not susceptible to the one or more antimicrobial agent(s) tested by this method is achieved within about 1 to 3 hours from the time of obtaining the sample comprising cells of the microorganism from the subject; and/or within about 1 to 3 hours from the time of first incubating the cells of the microorganism from said sample in the culture medium to obtain the actively dividing culture of said cells of said microorganism used for exposing actively dividing cells of the microorganism to the antimicrobial agent; and/or within about 1 to 3 hours from the time of first incubating the sample obtained from the subject which comprises the cells of the microorganism in the culture medium to obtain the actively dividing culture of said cells of said microorganism used for exposing actively dividing cells of the microorganism to the antimicrobial agent.

In one preferred embodiment of the method according to the first broad aspect described herein the actively dividing microbial cells which are exposed to the test antimicrobial agent in a method according to any broad aspect of the invention described herein are present at a concentration in the range of about 5.0 × 10¹ to about 5.0 × 10⁹ microbial cells / mL, and more preferably at a concentration of about 5.0 × 10² to about 5.0 × 10⁸ microbial cells / mL, and most preferably at a concentration of about 5.0 × 10³ to about 5.0 × 10⁷ microbial cells / mL. Accordingly, in one example, the step of exposing cells of the microorganism to in and/or from the actively dividing cell culture obtained in step (i) to an antimicrobial agent for a time and under conditions sufficient for the cells of the microorganism to actively divide, comprises exposing to the antimicrobial agent an amount of microbial cells at a concentration in the range of about 5.0 × 10¹ to about 5.0 × 10⁹ microbial cells / mL, preferably at a concentration of about 5.0 × 10² to about 5.0 × 10⁸ microbial cells / mL, and more preferably at a concentration of about 5.0 × 10³ to about 5.0 × 10⁷ microbial cells / mL. In one such example, the concentration of the actively dividing microbial cells being exposed to the antimicrobial agent is about 5.0 × 10⁴ to about 5.0 × 10⁷ microbial cells / mL, or about 5.0 × 10⁵ to about 5.0 × 10⁷ cells / mL, or about 5.0 × 10⁶ to about 5.0 × 10⁷ cells / mL. In another example, the concentration of the actively dividing microbial cells being exposed to the antimicrobial agent is about 5.0 × 10³ cells / mL or about 5.0 × 10⁴ cells / mL or about 5.0 × 10⁴ cells / mL, or about 5.0 × 10⁵ cells / mL, or about 5.0 × 10⁶ cells / mL, or about 5.0 × 10⁷ cells / mL or about 5.0 × 10⁸ cells / mL. In some preferred examples the concentration of the actively dividing microbial cells being exposed to the antimicrobial agent is about 5.0 × 10⁵ cells / mL.

It is preferred that the actively dividing microbial cells which are exposed to the test antimicrobial agent are present at the above mentioned concentrations when determining susceptibility of the microorganism to an antimicrobial agent. Alternatively or in addition, it is preferred that the actively dividing microbial cells which are exposed to the test antimicrobial agent are present at the above mentioned concentrations when determining the MIC of the microbial agent to which the microorganism has been shown to be susceptible by the method of this aspect.

Accordingly, in some examples according to this embodiment the method according to the first broad aspect described herein, comprises a step of incubating in the culture medium (a) cells of the microorganism from a sample obtained from a subject having or suspected of having an infection with said microorganism, and/or (b) a sample obtained from the subject having or suspected of having an infection with said microorganism, wherein said sample comprises cells of said microorganism, wherein the incubating is for a time and under conditions sufficient for said cells to actively divide to thereby obtain an actively dividing culture of said cells of said microorganism comprising actively dividing microbial cells at a concentration in the range of about 5.0 × 10¹ to about 5.0 × 10⁹ microbial cells / mL, preferably at a concentration of about 5.0 × 10² to about 5.0 × 10⁸ microbial cells / mL, and more preferably at a concentration of about 5.0 × 10³ to about 5.0 × 10⁷ microbial cells / mL. In one such example, it is preferred that the actively dividing culture comprises a concentration of actively dividing microbial cells of about 5.0 × 10⁴ to about 5.0 × 10⁷ microbial cells / mL, or about 5.0 × 10⁵ to about 5.0 × 10⁷ cells / mL, or about 5.0 × 10⁶ to about 5.0 × 10⁷ cells / mL. For example, the actively dividing culture comprises a concentration of actively dividing microbial cells of about .0 × 10³ cells / mL, or 5.0 × 10⁴ cells / mL, or about 5.0 × 10⁵ cells / mL, or about 5.0 × 10⁶ cells / mL, or about 5.0 × 10⁷ cells / mL, or about 5.0 × 10⁸ cells / mL.. In some preferred examples the concentration of the actively dividing microbial cells being exposed to the antimicrobial agent is about 5.0 × 10⁵ cells / mL.

In alternative examples, to acquire the actively dividing microbial cells from the actively dividing culture of said cells of said microorganism is concreted to achieve the above mentioned concentration of actively dividing microbial cells prior to exposing cells of the microorganism in and/or from the actively dividing cell culture to the antimicrobial agent.

It is desirable that the actively dividing microbial cells which are exposed to the test antimicrobial agent are present at the above mentioned concentrations for determining susceptibility of the a microorganism to an antimicrobial agent by acoustic flow cytometry using the method of any broad aspect described herein. Alternatively, or in addition, it is desirable that the actively dividing microbial cells which are exposed to the test antimicrobial agent are present at the above mentioned concentrations when determining the MIC of the microbial agent to which the microorganism has been determined to be susceptible. In some such examples, the microbial cells may be bacteria.

In another embodiment of the method according to the first broad aspect of the present invention, the method further comprises:
(a) producing an unexposed control cell sample by incubating in a culture medium cells of the microorganism from the sample obtained from the subject for a time and under conditions sufficient for the cells to actively divide to obtain an actively dividing culture of the cells of the microorganism, and labelling the cells in or form said actively dividing culture with the nucleic acid binding fluorescent compound in the absence of a prior exposure of the cells to the antimicrobial agent; and
(b) measuring by acoustic flow cytometry the cellular morphology and/or amount of the cells of the microorganism in the unexposed control sample.

In one example, the method according to this embodiment further comprises measuring by acoustic flow cytometry the cellular morphology and/or amount of the cells of the microorganism from the sample obtained from the subject following exposure of those cells to a concentration of the antimicrobial agent, and optionally comparing the cellular morphology and/or amount of cells of the microorganism after exposure to the antimicrobial agent with the cellular morphology and/or amount of cells measured by acoustic flow cytometry for the unexposed control sample.

Referring to those preferred embodiments of the method of the present invention where determining susceptibility of the microorganism obtained from the subject to the antimicrobial agent from the acoustic flow cytometry data output comprises quantitatively determining susceptibility of the microorganism to the antimicrobial agent from the acoustic flow cytometry data output by measuring or determining the MIC of the antimicrobial agent to which the microorganism is susceptible, in one example of such embodiments the step of measuring or determining from the acoustic flow cytometry data output the MIC of the antimicrobial agent to which the microorganism is susceptible, comprises:
(a) incubating in a culture medium cells of the microorganism from the sample obtained from the subject and/or incubating in a culture medium a sample obtained from the subject having or suspected of having an infection with said microorganism wherein said sample comprises cells of the microorganism, for a time and under conditions sufficient for the cells to actively divide to thereby obtain an actively dividing culture of the cells of the microorganism, and labelling the cells of the microorganism in and/or from this actively dividing culture with the nucleic acid binding fluorescent compound in absence of any prior exposure to the antimicrobial agent, to thereby produce an unexposed control sample;
(b) measuring by acoustic flow cytometry the cellular morphology and amount of said cells of said microorganism in the unexposed control sample;
(c) performing steps comprising
   (i) incubating in a culture medium cells of the microorganism from a sample obtained from a subject having or suspected of having an infection with the microorganism, and/or incubating in the culture medium a sample obtained from the subject having or suspected of having an infection with the microorganism wherein the sample comprises cells of the microorganism, wherein the incubating of the cells of the microorganism in and/or from the subject sample is for a time and under conditions sufficient for the cells to actively divide to thereby obtain an actively dividing culture of the cells of the microorganism;
   (ii) exposing cells of the microorganism in and/or from the actively dividing cell culture obtained in step (i) to the antimicrobial agent for a time and under conditions sufficient for the cells of the microorganism to actively divide;
   (iii) labelling the cells of the microorganism exposed to the antimicrobial agent in step (ii) with the nucleic acid binding fluorescent compound;
   (iv) measuring the effect of the antimicrobial agent on the cellular morphology of the cells of the microorganism by acoustic flow cytometry; and
(d) determining from the acoustic flow cytometry data output the minimal concentration of the antimicrobial agent for which the amount or proportion of the cells of the microorganism exposed to the antimicrobial agent having a cellular morphology of non-antimicrobial agent-exposed cells in the unexposed control sample, equals to or less than a predetermined amount or proportion of the cells of the microorganism in the unexposed control sample measured by the acoustic flow cytometry to exhibit said cellular morphology of non-antimicrobial agent-exposed cells.

Preferably, step (c) (ii) of this method comprises exposing the cells of the microorganism in and/or from the actively dividing cell culture obtained in step (i) to a concentration of the antimicrobial agent for a time and under conditions sufficient for the cells of the microorganism to actively divide, wherein the cells of the microorganism which are being exposed to the antimicrobial agent are present at a concentration of about 5.0 × 10¹ to about 5.0 × 10⁹ microbial cells / mL, preferably at a concentration of about 5.0 × 10² to about 5.0 × 10⁸ microbial cells / mL, and more preferably at a concentration of about 5.0 × 10³ to about 5.0 × 10⁷ microbial cells / mL. For example, the method comprises exposing cells of the microorganism in and/or from the actively dividing cell culture to the antimicrobial agent at a concentration of about 5.0 × 10⁴ to about 5.0 × 10⁷ microbial cells / mL, or about 5.0 × 10⁵ to about 5.0 × 10⁷ cells / mL, or about 5.0 × 10⁶ to about 5.0 × 10⁷ cells / mL. In one such example, the concentration of the microbial cells being exposed to the antimicrobial agent is about 5.0 × 10⁴ cells / mL, or about 5.0 × 10⁵ cells / mL, or about 5.0 × 10⁶ cells / mL, or about 5.0 × 10⁷ cells / mL. In some examples the concentration of microbial cells being exposed to the antimicrobial agent is about 5.0 × 10⁵ cells / mL.

The method in step (c) above comprises preparing an antimicrobial agent exposed sample by
(i) incubating in a culture medium cells of the microorganism from a sample obtained from the subject having or suspected of having an infection with said microorganism, and/or incubating in a culture medium a sample obtained from the subject having or suspected of having an infection with said microorganism wherein said sample comprises cells of the microorganism, wherein the incubating is for a time and under conditions sufficient for the cells of said microorganism to actively divide to thereby obtain an actively dividing culture of the cells of said microorganism for the subject;
(ii) exposing cells of the microorganism in and/or from the actively dividing cell culture obtained in step (i) to a concentration of the antimicrobial agent for a time and under conditions sufficient for the cells of the microorganism to actively divide; and
(iii) labelling cells of the microorganism exposed to the antimicrobial agent in step (ii) with the nucleic acid binding fluorescent compound.

Preferably, step (c) (ii) of this method comprises exposing the cells of the microorganism in and/or from the actively dividing cell culture obtained in step (i) to a concentration of the antimicrobial agent for a time and under conditions sufficient for the cells of the microorganism to actively divide, wherein the cells of the microorganism which are being exposed to the antimicrobial agent are present at a concentration of about 5.0 × 10¹ to about 5.0 × 10⁹ microbial cells / mL, preferably at a concentration of about 5.0 × 10² to about 5.0 × 10⁸ microbial cells / mL, and more preferably at a concentration of about 5.0 × 10³ to about 5.0 × 10⁷ microbial cells / mL, as described above.

In one such example, the method further comprises measuring by acoustic flow cytometry the cellular morphology and amount of the cells of the microorganism in the antimicrobial agent exposed sample, for example, by measuring the amount or proportion of cells in said antimicrobial agent exposed sample that have the same cellular morphology of non-antimicrobial agent-exposed cells in the unexposed control sample.

Preferably, the method further comprises measuring or determining that the amount or proportion of the cells in the antimicrobial agent exposed sample that have the same cellular morphology of non-antimicrobial agent-exposed cells of the microorganism in the unexposed control sample is equal to or less than the pre-determined amount or proportion of the cells in the unexposed control sample measured (by the acoustic flow cytometry) to exhibit said cellular morphology of non-antimicrobial agent-exposed cells.

In one example according to the above-described embodiments, the method may comprise in step (c), exposing the actively dividing cells of the microorganism in and/or from the actively dividing cell culture of the microorganism obtained in step (c)(i) to different concentrations of the antimicrobial agent. The method may further comprise in step (d) measuring for each of the concentrations of the antimicrobial agent the amount or proportion of cells of the microorganism exposed to the antimicrobial agent that have or exhibit a cellular morphology of non-antimicrobial agent-exposed cells of the unexposed control sample. The method may also further comprise identifying from the lowest concentration of the antimicrobial agent in which the amount or proportion of the cells of the microorganism exposed to the antimicrobial agent having the same cellular morphology of non-antimicrobial agent-exposed cells, equals to or less than the pre-determined amount or proportion (i.e., of the cells in the unexposed control sample measured by the acoustic flow cytometry to exhibit the cellular morphology of non-antimicrobial agent-exposed cells).

In one such example the different concentrations of the antimicrobial agent comprise a 1:2 dilution series ranging from about 2560 mg/ml to about 2.5 mg/ml of said antimicrobial agent.

Preferably, the same standardised inoculum density of the cells of the microorganism obtained from the subject is used to prepare the actively dividing culture from which the unexposed control sample is produced and the actively dividing culture for exposing actively dividing cells of the microorganism to the antimicrobial agent. For example, the same standardised inoculum density of the cells of the microorganism obtained from the subject is used to prepare the actively dividing culture from which the unexposed control sample is produced and the actively dividing culture for exposing actively dividing cells of the microorganism to each of the different concentrations of the antimicrobial agent.

In one example, the method comprises preparing (i) a series of at least three samples of the unexposed control, and (ii) a series of at least three samples for cells of the microorganism exposed to each tested concentration of the antimicrobial agent, and then measuring by acoustic flow cytometry the amount and cellular morphology of cells in each of the at least three samples of the unexposed control and in each of the three sample of cells of the microorganism exposed to any tested concentration of the antimicrobial agent. According to this example, the method further comprises determining from the acoustic flow cytometry data output the minimal test concentration of the antimicrobial agent for which two or more of the at least three samples exposed to the antimicrobial agent comprise an amount or proportion of cells of the microorganism having a cellular morphology of non-antimicrobial agent-exposed cells in the unexposed control sample which is equal to or less than a pre-determined amount or proportion of the cells in each of the at least three samples of the unexposed control measured by the acoustic flow cytometry to exhibit said cellular morphology of non-antimicrobial agent-exposed cells.

In some preferred embodiments, the method comprises standardising the acoustic flow cytometry measurements by defining a rational gate of unexposed cellular morphology of the cells of the microorganism on acoustic flow cytometry forward scatter (FSC) and fluorescence biaxial plots that bounds acoustic flow cytometry events corresponding to all cells in an unexposed control sample having a cellular morphology of non-antimicrobial agent-exposed cells of said microorganism, excluding aggregate and co-incident events at a 10% threshold. In some preferred examples, the data collected from the acoustic flow cytometer from an antimicrobial unexposed control sample is used to define the distribution of recorded parameters for antimicrobial unaffected cells. For example, this may be performed by using any contouring or clustering algorithm to identify the distribution of parameters of the observed unexposed population. An ideal threshold for a contouring or clustering algorithm is 10% similarity.

For example, the method may comprise determining from the acoustic flow cytometry data output the minimal concentration of the antimicrobial agent for which the amount or proportion of events corresponding to cells of the microorganism exposed to the antimicrobial agent falling within the rational gate of unexposed cellular morphology, is equal to or less than a predetermined amount or proportion of the events corresponding to all cells in the unexposed control sample having a cellular morphology of non-antimicrobial agent-exposed cells of said microorganism, excluding aggregate and co-incident events at a 10% threshold.

Preferably, the pre-determined amount or proportion of the cells in the unexposed control sample measured by the acoustic flow cytometry to exhibit the cellular morphology of non-antimicrobial agent-exposed cells, is determined by reference to known minimal inhibitory concentrations (MICs) of the anti-microbial agent to a panel of known isolates of the microorganism. For example, the pre-determined amount is the known amount or proportion of actively dividing cells of the known panel of isolates of the microorganism that exhibit the cellular morphology of non-antimicrobial agent-exposed cells of the microorganism at the MIC of the antimicrobial agent to the panel of the known isolates.

In one such example, the predetermined amount has been previously determined by performing electron microscopy and/or flow cytometry analysis of the cellular morphology of actively dividing cells of the known panel of isolates at the known minimal inhibitory concentrations of said anti-microbial agent to said panel of the known isolates.

Alternatively, or in addition, the method may further comprise measuring or determining the amount or proportion of actively dividing cells of the known panel of isolates of the microorganism that exhibit the cellular morphology of non-antimicrobial agent-exposed cells of the microorganism at the MIC of said anti-microbial agent to said panel of the known isolates. Optionally wherein the measuring or determining is performed by electron microscopy and/or flow cytometry analysis.

Preferably, the panel of the known isolates of the microorganism comprises at least three different known isolates, wherein at least one of said isolates is known to be susceptible to said antimicrobial drug, and wherein at one of the said isolates is known to be non-susceptible (e.g., resistant) to the antimicrobial agent, and wherein the minimal inhibitory concentrations of the isolates in said panel which are known to be susceptible to the anti-microbial agent across the range concentrations of the anti-microbial agent tested in respect of actively dividing cells of said microorganism obtained from the patient. Preferably, the at least three known isolates represent known isolates obtained from different geographical locations.

In one example, the panel of the known isolates comprises at least about 10 different known isolates of the microorganism, optionally wherein those know isolates are originated from different geographical locations. In one such example, the panel comprises at least about 10 to about at least about 50 different known isolates of said microorganism optionally obtained from different geographical locations. In one particular example, the microorganism obtained from the patient is the bacteria *Klebsiella pneumoniae,* and the panel of known isolates of said microorganism comprises the 48 *Klebsiella pneumoniae* known isolates listed in Table 1 and/or Table 2.

All bacterial isolates listed in Tables 1 and 2 have been known in the art. For example, the isolate ATCC 700603 is a previously deposited (with ATCC) strain of *K. pneumoniae* ESBL and may be employed in the method according to any aspect of the invention described hereof as positive control. The bacterial isolate ATCC BAA1706 was also previously deposited (with ATCC) strain of *K. pneumoniae* and may be used in the method according to any aspect of the present invention as a wild type control strain of *K. pneumoniae.* The isolate ATCC BAA1705 was also previously deposited (with ATCC) strain of *K. pneumoniae* KPC and is known to be producing carbapenemase. As such the ATCC BAA1705 stain may be used in the method of the present invention as a carbapenemase- producing control isolate. The remaining *Klebsiella* strains listed in Tables 1 and 2 have been previously described *e.g.,* in Hall, J.M. et al. Molecular mechanisms of β-lactam resistance in carbapenemase-producing Klebsiella pneumoniae from Sri Lanka. J. Med. Microbiol. 63, 1087-92 (2014); and Hall, J. M., Ingram, P. R., O'Reilly, L. C., & Inglis, T. J. J. Temporal flux in beta-lactam resistance among Klebsiella pneumoniae in Western Australia. J. Med. Microbiol. 65, 429-437 (2016).

In one example, the known MICs of the anti-microbial agent to the panel of the known isolates, are clinical susceptibility breakpoint concentrations or known MICs for the antimicrobial agent as determined or issued by an internationally recognised antimicrobial susceptibility testing authority such as the EUCAST or CLSI for said microorganism. Alternatively, or in addition, the known MICs of the anti-microbial agent to the panel of the known isolates of the microorganism, have been previously determined *e.g.,* by a broth microdilution (BMD) assay and/or by a flow cytometry method. Alternatively, or in addition, the method of according to any broad aspect of the invention described hereof comprises measuring the MICs of the antimicrobial agent to the panel of the known isolates *e.g.,* by performing BMD assay and/or by a flow cytometry method.

Preferably, the pre-determined amount or proportion of the cells in the unexposed control sample measured by the acoustic flow cytometry to exhibit said cellular morphology of non-antimicrobial agent-exposed cells, is about 1% to about 50% of total cells in the unexposed control sample, more preferably, about 1% or about 5 % or about 10% or about 15% or about 20% or about 25% or about 30% or about 35% or about 40% or about 45% or about 50% of total the cells in the said unexposed control sample.

In one example, determining susceptibility of the microorganism to the antimicrobial agent quantitatively by measuring from the acoustic flow cytometry data output the minimal inhibitory concentration of said antimicrobial agent to which the microorganism is susceptible, is achieved within about 3 to 6 hours, preferably within about 3 hours, from the time of obtaining the sample comprising cells of the microorganism from the subject; and/or within about 3 to 6 hours, preferably within about 3 hours, from the time of first incubating the cells of the microorganism from said sample in the culture medium to obtain the actively dividing culture of said cells of said microorganism; and/or within about 1 to 6 hours, preferably within about 3 hours, or within about 2 hours, or within about 1 hour, from the time of first incubating the sample obtained from the subject which comprises cells of the microorganism in the culture medium to obtain the actively dividing culture of said cells of said microorganism.

In another example, the method may further comprise determining appearance of susceptibility-associated signature for one or more of the isolates in the panel of the known isolates, and measuring by acoustic flow cytometry the amount or proportion of the cells of the microorganism exposed to the antimicrobial agent having a cellular morphology of non-antimicrobial agent-exposed cells not resembling morphological characteristics of the appearance of susceptibility-associated signature of the one or more known isolates.

In some preferred examples, the step of measuring by acoustic flow cytometry the cellular morphology and amount of the cells of the microorganism in the unexposed control sample, may comprise measuring the cell size (as determined by cell cytoplasmic volume) and the amount of cellular nucleic acid in the cells of the microorganism in the unexposed control sample. In such preferred examples, the method of the present invention may further comprise determining from the acoustic flow cytometry data output the minimal concentration of the antimicrobial agent for which the amount or proportion of the cells of the microorganism exposed to said antimicrobial agent that have a cellular morphology of non-antimicrobial agent-exposed cells in the unexposed control sample, by determining or measuring the amount or proportion of the cells of the microorganism exposed to the antimicrobial agent that have the same amount of cellular nucleic acid as in the cells of the unexposed control sample and/or do not show an increase in cell size as determined by cell cytoplasmic volume relative to the cells of the unexposed control sample.

The method according to the present invention further comprises incubating in a culture medium cells of the microorganism from the sample obtained from the subject and/or incubating in the culture medium the sample obtained from the subject wherein the sample comprises the cells of the microorganism, for a time and under conditions sufficient for said cells to actively divide to obtain an actively dividing culture of said cells of said microorganism. In these preferred examples, the method further comprises labelling said cells of said microorganism in or from said actively dividing culture with the nucleic acid binding fluorescent compound absent prior exposure to the antimicrobial agent, to thereby produce an unexposed control sample; and measuring by acoustic flow cytometry the cellular morphology and amount of said cells of said microorganism in the unexposed control sample.

It is to be understood that in the method according to the first broad aspect of the present invention, the sample obtained from the subject is a biological sample comprising cells of the microorganism infecting the subject or suspect of infecting the subject.

In one example, the sample is a biological sample selected from a throat swab sample, a sputum sample, urine sample, blood sample, a joint fluid sample, a continuous ambulatory peritoneal dialysis (CAPD) fluid sample, a buccal swab sample, a tissue biopsy, a surgical drain fluid sample, a stool sample, a cerebrospinal fluid sample, ascitic fluid sample, pleural fluid sample, a blood sample such as blood plasma and/or blood serum sample, a wound swab, or wound mucus sample, a nasal swab sample, a rectal swab sample, a urethral swab sample, a skin sample or skin swab sample, a genital swab sample, pus sample, saliva sample, rheum sample, serous fluid sample, vaginal secretion sample, vomit sample, sweat, tears, mucus sample, pericardial fluid sample, peritoneal fluid sample, sebum sample, smegma sample, gastric juice sample, a bile sample, chyle sample, chyme sample, endolymph and /or perilymph fluid sample, lymph fluid sample, Interstitial fluid sample, breast milk, perioperative surgical field swab sample, any sample or swab taken to determine presence of a multi-drug resistant organism, and any combination thereof. Alternatively or in addition, the sample is a food sample such as that for tracking antimicrobial resistance in foods both uncooked and cocked or ready to eat with which the subject having or suspected of having an infection with a microorganism, may have come in contact with.

In preferred embodiments of the method according to the first broad aspect of the present invention, the culture medium is a culture medium that supports growth and replication of the microorganism to thereby produce the actively dividing cell culture of said microorganisms. Preferably, the culture medium is sterile and/or free of microbial contaminants prior to inoculation of the culture medium with the cells of the microorganism obtained from the subject. Also preferably the culture medium is capable of supporting growth and replication of Gram positive and Gram negative bacteria.

In one example, the culture medium is a sterile and/or culture medium selected from Mueller-Hinton Broth (MHB), Brain Heart Infusion Broth (BHIB), Lysogeny Broth, Nutrient Broth, Oxoid Iso-Sensitest Medium, and Trypticase Soy Broth.

A suitable MBH culture medium may comprise about 300.0g beef extract (dehydrated infusion from), about 17.5g casein hydrolysate, and about 1.5g starch, e.g., reconstituted in about 1L of water, e.g., and pH may be adjusted to about pH 7.3 ± 0.1 as measured at about 25°C.

A suitable BHIB culture medium may comprise about 12.5g brain infusion solids, about 5.0g beef heart infusion solids, about 10.0g proteose peptone, about 2.0g glucose, about 5.0g sodium chloride, about 2.5g disodium phosphate, e.g., reconstituted in about 1L of water, and pH may be adjusted to about pH 7.4 ± 0.2 as measured at about 25°C.

A suitable Lysogeny Broth culture medium may comprise about 10g peptone such as peptone 140, about 5g yeast extract, about 5g sodium chloride, and reconstituted in about 1L of water.

A suitable Nutrient Broth culture medium may comprise about 1.0g 'Lab-Lemco' meat extract powder (e.g., Thermo Scientific; Oxoid Limited), about 2.0g yeast extract, about 5.0g peptone, about 5.0g sodium chloride, and may be reconstituted in about 1L of water, and pH may be adjusted to abut pH 7.4 ± 0.2 as measured e.g., at about 25°C.

A suitable Oxoid Iso-Sensitest Medium culture medium may comprise about 11g hydrolysed casein, about 3.0g peptones, about 2.0g glucose, about 3.0g sodium chloride, about 1.0g soluble starch, about 2.0g disodium hydrogen phosphate, about 1.0g sodium acetate, about 0.2g magnesium glycerophosphate, about 0.1g calcium gluconate, about 0.001 cobaltous sulphate, about 0.001 cupric sulphate, about 0.001 zinc sulphate, about 0.001g ferrous sulphate, about 0.002 mangous chloride, about 0.001g menadione, about 0.001g cyanocobalamin, about 0.02g L-cysteine hydrochloride, about 0.02g L-tryptophan, about 0.003g pyridoxine, about 0.003g pantothenate, about 0.003g nicotinamide, about 0.0003g biotin, about 0.00004g thiamine, about 0.01g adenine, about 0.01g guanine, about 0.01g xanthine, about 0.01g uracil, and may be reconstituted e.g., in about 1L of water, and pH may be adjusted to e.g., about pH 7.4 ± 0.2 as measured at about 25°C.

A suitable Trypticase Soy Broth culture medium may comprise about 17g tryptone (e.g., from pancreatic digest of casein), about 3.0g soytone (e.g., from peptic digest of soybean), about 2.5g glucose (e.g., dextrose), about 5.0g sodium chloride, about 2.5g dipotassium phosphate, e.g., reconstituted in about 1L of water.

In one preferred example, the method according to this first broad aspect, comprises incubating in the culture medium the cells of the microorganism in and/or from the subject sample for a time and under conditions sufficient for the cells to actively divide, preferably, for a time and under conditions sufficient for about 1 to about 5 cells divisions to occur, and more preferably for a time and under conditions sufficient for the cells to undergo about 1 to about 3 cell divisions. For example, the microorganism may be a bacteria and the method may comprise incubating cells of the microorganism in and/or from the subject sample in a culture medium for a period of at least about 20 minutes to at least about 3 hours, preferably for about 20 minutes or about 30 minutes or about 40 minutes or about 50 minutes or about 60 minutes or about 70 minutes or about 80 minutes or about 90 minutes or about 100 minutes or about 110 minutes or about 120 minutes or about 130 minutes, or about 140 minutes, or about 150 minutes, or about 160 minutes, or about 170 minutes, or about 180 minutes, or about 190 minutes, or about 200 minutes, or about 210 minutes, or about 220 minutes, or about 230 minutes, or about 240 minutes, and more preferably, preferably about 30 minutes, or about 1 hour or about 1.5 hours. Alternatively or in addition, the method comprises incubating the cells of the microorganism in and/or from the subject sample in the culture medium under temperature conditions from about 25°C to about 42°C, preferably about 37°C and with or without aeration.

In another example, the method according to this first broad aspect comprises incubating cells of the microorganism in the presence of the antimicrobial agent for about 20 min to about 3 hours. For example, the microorganism may be a bacteria and the method may comprise incubating cells of the microorganism in the presence of the antimicrobial agent for a period of at least about 20 minutes to at least about 3 hours, preferably for about 20 minutes or about 30 minutes or about 40 minutes or about 50 minutes or about 60 minutes or about 70 minutes or about 80 minutes or about 90 minutes or about 100 minutes or about 110 minutes or about 120 minutes or about 130 minutes, or about 140 minutes, or about 150 minutes, or about 160 minutes, or about 170 minutes, or about 180 minutes, or about 190 minutes, or about 200 minutes, or about 210 minutes, or about 220 minutes, or about 230 minutes, or about 240 minutes, and more preferably, preferably about 30 minutes, or about 1 hour or about 1.5 hours. Alternatively or in addition, the method may comprise incubating the cells of the microorganism in the presence of the antimicrobial agent under temperature conditions from about 25°C to about 42°C, preferably about 37°C and with or without aeration.

In one example, the method comprises exposing the actively dividing cells of the microorganism to a range of different concentrations of the antimicrobial agent such as 0 mg/L, 0.25 mg/L, 1 mg/L, 2 mg/L, 4 mg/L and 16 mg/L. Alternatively, or in addition, the range of different concentrations of the antimicrobial agent may comprise 1:2 dilution series ranging from about 2560 mg/ml to about 2.5 mg/ml of said antimicrobial agent.

In another example, the method comprises exposing the actively dividing cells of the microorganism to a range of different antimicrobial agents.

In another example, the method comprises exposing the actively dividing cells of the microorganism to a range of different antimicrobial agents.

Preferably, in the method according to the first broad aspect of the present invention described above and according to any subsequent aspect of the invention described hereof, the unicellular or multicellular microorganism is selected from a bacteria, a fungus or a yeast.

In one preferred example, the microorganism is a unicellular microorganism such as a bacteria. For example, the bacteria can be a Gram negative or a Gram positive bacteria. The bacteria may be aerobic or anaerobic or facultative anaerobe or facultative aerobe or obligate aerobe or obligate anaerobe and/or may be any pathogenic or non-pathogenic bacteria.

In one example, the bacteria is selected from bacteria belonging to one or more of the following bacterial groups, genera and/or species: *Enterobacter,* Enterobacteriaceae, *Campylobacter, Klebsiella, Pseudomonas, Acinetobacter, Burkholderia, Clostridium, Stenotrophomonas, Serratia, Haemophilus, Neisseria, Burkholderia, Staphylococcus, Streptococcus, Clostridium, Bacillus, Bacteroides, Listeria, Mycobacterium, Salmonella, Brucella, Bordetella, Borrelia, Nocardia, Francisella, Legionella, Yersinia, Leptospira, Borrelia, Shigella, Shewanella, Campylobacter, Chlamydia, Clostridium, Corynebacterium, Escherichia, Haemophilus, Helicobacter, Leptospira, Mycoplasma, Rickettsia, Treponema,* and *Vibrio.*

For example, the bacteria is selected from any one or more of the following: Acinetobacter baumanii, Bacillus anthracis, Bacillus cereus, Bordetella pertussis, Borrelia burgdorferi, Brucella abortus, Brucella canis, Brucella melitensis, Brucella suis, Burkholderia pseudomallei, Burkholderia cepacia, Burkholderia cenocepacia, Burkholdria thailandesis, Campylobacter coli, Campylobacter jejuni, Chlamydia pneumoniae, Chlamydia psittaci, Chlamydia trachomatis, Clostridium botulinum, Clostridium difficile, Clostridium perfringens, Clostridium tetani, Corynebacterium diphtheriae, Enterobacter cloacae, Enterobacter aerogenes, Enterococcus faecalis, Enterococcus faecium, Escherichia coli, Enterotoxigenic Escherichia coli (ETEC), Enteropathogenic E. coli, E. coli (O157:H7), Francisella tularensis, Haemophilus influenza, Haemophilus parainfluenzae, Klebsiella pneumoniae, Klebsiella oxytoca, Helicobacter pylori, Legionella pneumophila, Legionella longbeachae, Leptospira interrogans, Leptospira Santarosai, Listeria monocytogenes, Mycobacterium avium, Mycobacterium intracellulare, Mycobacterium kansasii, Mycobacterium tuberculosis, Mycoplasma pneumoniae, Neisseria gonorrhoeae, Neisseria meningitidis, Pseudomonas aeruginosa, Rickettsia rickettsiae, Salmonella enterica var. Typhi, Salmonella enterica var. Typhimurium, Serratia marcescens, Shewanella oneidensis, Shigella sonnei, Shigella dysenteriae, Shigella boydii, Shigella flexneri, Staphylococcus aureus, Staphylococcus epidermidis, Staphylococcus lugdunensis, Staphylococcus saprophyticus, Stenotrophomonas maltophila, Streptococcus agalactiae, Streptococcus pneumoniae, Streptococcus pyogenes, Streptococcus anginosus-constellatusintermedius, Streptococcus mitis, Vibrio cholera, Yersinia pseudotuberculosis and Yersinia pestis.

In one example, the bacteria belong to species included in the *Enterobacteriaceae,* such as the *Klebsiella* species, or to other groups of bacteria including the *Pseudomonas* species or the *Acinetobacter* species

For example, the bacteria are a strain of *Klebsiella pneumoniae* or *Pseudomonas aeruginosa.*

In another preferred example, the microorganism is a yeast. For example, the yeast is selected from a yeast belonging to one or more of the following species of yeast: *Candida, Saccharomyces,* and *Cryptococcus.*

For example, the yeast is selected from any one or more of the following: Candida albicans, Candida krusei, Candida tropica, Torulopsis glabrata, Cryptococcus neoformans.

In another preferred example, the microorganism is a fungus. For example, the fungus is selected from one or more of the following genus or species: *Aspergillus, Candida, Histoplasma, Pneumocystis,* and *Stachybotrys.*

For example, the fungus is selected from any one or more of the following: Aspergillus fumigatus, Aspergillus flavus, Candida albicans, Cryptococcus neoformans, Cryptococcus gattii, Histoplasma capsulatum, Pneumocystis jirovecii (also known as Pneumocystis carinii), and Stachybotrys chartarum.

Preferably, in the method according to the first broad aspect of the present invention described above and according to any subsequent aspect of the invention described hereof, the antimicrobial agent is or comprises an anti-bacterial agent and/or anti-fungal agent and/or anti-yeast agent. The antimicrobial agent may have biostatic and/or microbicidal effect on the unicellular and/or multicellular microorganisms of the present invention. Accordingly, the antimicrobial agent according to any aspect, embodiment and example described hereof may inhibit growth and/or kill actively dividing cells of the microorganism assayed in the method of the present invention. In one example, the antimicrobial agent may be any compound such as a chemical compound or a pharmaceutical composition such as a drug. In another example the antimicrobial agent is a composition comprising a compound such as a chemical compound or a pharmaceutical or a drug. In one such example the antimicrobial agent may be a composition comprising two or more compounds such as chemical compounds, pharmaceuticals and/or drugs. Similarly, the antimicrobial agent may be synthetic and/or chemically-produced and/or naturally occurring and/or derived whether directly or indirectly from an organism or a plant or an animal. In another example, the antimicrobial agent may comprise or consist of a physical and/or chemical phenomenon or stress applied to the cells of the microorganisms described according to broad aspect, embodiment of example hereof. According to this example, the antimicrobial agent may comprise or consist of one or more physical and/or chemical phenomenon or stress applied to actively dividing cells of a microorganism selected from heat stress, ultraviolet light, blue light, radiation such as electromagnetic radiation, microwave radiation, radiation with X-rays or gamma rays, particle radiation such as alpha, beta or neurons particle radiation, and/or oxidative stress. Preferably, the antimicrobial agent is or comprises an anti-bacterial agent such as a bactericidal agent and/or bacteriostatic agent.

For example, the antimicrobial agent may be or comprise any one of more of the following: amino-acids or amino acid derivatives, fatty acids or fatty acid derivatives, antimicrobial peptides or peptide derivatives, aminoglycosides, aureolic acids, aziridines, benzenoids, Benzimidazoles, coumarin-glycosides, diphenyl ether derivatives, epipolythiodioxopiperazines, glucosamines, glycopeptides, Imidazoles, indol derivatives, macrolactam, macrolides, nucleosides, beta-Lactams such as penicillins, cephalosporins, monobactams and/or carbapenems, peptidyl nucleosides, phenicoles, polyenes, pyridines and pyrimidines, quinolones and fluoroquinolones, statins, steroids, sulfonamides, taxoides, tetracyclines, metal alloys such as copper alloys.

In one particularly preferred example, the antimicrobial agent is or comprises an antibiotic. For example, the antibiotic is an anti-bacterial and/or anti-fungal and/or anti-yeast antibiotic.

In one example, the antimicrobial agent is or comprises a beta lactam antibiotic such as penicillins, cephalosporins, monobactams and/or carbapenems.

For example the antimicrobial agent is or comprises a penicillin antibiotic selected from one or more of penicillin G such as penicillin G potassium salt or penicillin G sodium salt, ampicillin such as ampicillin anhydrous, ampicillin trihydrate and/or ampicillin sodium salt, amoxicillin, ticarcillin piperacillin, (+)-6-Aminopenicillanic acid (6-APA), azlocillin such as azlocillin sodium salt, carbenicillin such as carbenicillin disodium salt, cloxacillin such as cloxacillin sodium salt (e.g., cloxacillin sodium salt monohydrate), dicloxacillin such as dicloxacillin sodium salt monohydrate, flucloxacillin, mezlocillin, methicillin, nafcillin such as nafcillin sodium salt monohydrate, oxacillin such as oxacillin sodium salt, D-penicillamine, penicillin V such as penicillin V potassium salt, phenoxymethylpenicillinic acid such as phenoxymethylpenicillinic acid potassium salt, piperacillin such as piperacillin sodium salt, temocillin, and ticarcillin such as ticarcillin disodium salt. Each of these penicillins is commercially available *e.g.,* from Sigma-Aldrich.

In one such example, the antimicrobial agent which is or comprises a penicillin antibiotic provides antimicrobial effect such as antibacterial effect on actively dividing cells of the microorganism (e.g., bacteria) by disrupting synthesis of the peptidoglycan layer of the cell walls of the microorganism.

In another example, the antimicrobial agent is or comprises a cephalosporin antibiotic selected from one or more of cephalothin, cefalexin, cefaclor, cefixime such as cefixime trihydrate, cefmetazole such as cefmetazole sodium salt, cefoperazone such as cefoperazone sodium salt, sodium salt, cefotaxime such as cefotaxime sodium salt, cefuroxime, cefprozil, cefoxitin, ceftriaxone, ceftazidime, cefpodoxime, cefepime, 7-Aminocephalosporanic acid (7-ACA), 7-Aminodesacetoxycephalosporanic acid (7-ADCA), cefotiam such as cefotiam dihydrochloride hydrate, ceftazidime such as ceftazidime hydrate, cefsulodin such as cefsulodin sodium salt hydrate, ceftibuten such as ceftibuten hydrate, ceftriaxone such as ceftriaxone disodium salt hemi(heptahydrate), cephalexin such as cephalexin hydrate, cephalomannine, cephalothin such cephalothin as sodium salt, cephradine, imipenem e.g., imipenem monohydrate, moxalactam such as moxalactam sodium salt, and tazobactam sodium salt. Each of these cephalosporins is commercially available *e.g.,* from Sigma-Aldrich.

In another example, the antimicrobial agent is or comprises a first generation cephalosporin antibiotic selected from: cefadroxil, cefazolin, cefalotin or cefalothin, and cephalexin.

In another example, the antimicrobial agent is or comprises a second generation cephalosporin antibiotic selected from: cefaclor, cefamandole, cefoxitin, cefprozil, and cefuroxime.

In another example, the antimicrobial agent is or comprises a third generation cephalosporin antibiotic selected from: cefixime, cefdinir, cefditoren, cefotaxime, cefoperazone, cefotaxime, cefpodoxime, ceftibuten, ceftizoxime, and ceftriaxone.

In another example, the antimicrobial agent is or comprises a fourth generation cephalosporin antibiotic such as cefepime.

In another example, the antimicrobial agent is or comprises a fifth generation cephalosporin antibiotic such as ceftaroline fosamil and/or ceftobiprolel.

In one example, according to any example or embodiment or aspect described hereof involving an antimicrobial agent which is or comprises one of more cephalosporin (whether first, second, third, fourth and/or fifth generation cephalosporin antibiotic), the antimicrobial agent provides antimicrobial effect such as antibacterial effect on actively dividing cells of the microorganism (e.g., bacteria) by disrupting synthesis of the peptidoglycan layer of the cell walls of the microorganism.

In another example, the antimicrobial agent is or comprises a monobactam antibiotic such as aztreonam, which is commercially available *e.g.,* from Sigma-Aldrich.

In one such example, the antimicrobial agent comprising a monobactam antibiotic such as aztreonam provides antimicrobial effect such as antibacterial effect on actively dividing cells of the microorganism (e.g., bacteria) by disrupting synthesis of the peptidoglycan layer of the cell walls of the microorganism.

In another example, the antimicrobial agent is or comprises a carbapenem antibiotic selected from one or more of imipenem or cilastatin, meropenem, ertapenem and doripenem. Each of these exemplary carbapenem antibiotic is commercially available *e.g.,* from Sigma-Aldrich. In one example, the antimicrobial agent which is or comprises a carbapenem antibiotic provides antimicrobial effect such as antibacterial effect by inhibiting cell synthesis in actively dividing cells of the microorganism.

In another example, the antimicrobial agent is or comprises an amino acid or amino acid derivative. For example, the antimicrobial agent is or comprises an amino acid derivative selected from one or more of: L-alanyl-L-1-aminoethylphosphonic acid, azaserine (O-diazoacetyl-L-serine), bestatin hydrochloride (N-[(2S,3R)-3-Amino-2-hydroxy-4-phenylbutyryl]-L-leucine hydrochloride), D-cycloserine [(*R*)-4-Amino-3-isoxazolidone, 4-Amino-3-isoxazolidinone], and 6-Diazo-5-oxo-L-norleucine [(S)-2-Amino-6-diazo-5-oxocaproic acid, or DON]. Each of these amino acids or derivatives is commercially available *e.g.,* from Sigma-Aldrich.

In another example, the antimicrobial agent is or comprises an aminoglycoside. For example, the antimicrobial agent is or comprises an aminoglycoside selected from one or more of: gentamicin, tobramycin, amikacin, daunorubicin such as hydrochloride, dihydrostreptomycin sesquisulfate, antibiotic G418 such as antibiotic G418 disulfate salt (C₂₀H₄₀N₄O₁₀ · 2H₂SO₄) (e.g., Sigma-Aldrich), gentamicin, hygromycin B (e.g., from *Streptomyces hygroscopicus* lyophilized powder) (e.g., Sigma-Aldrich), Kanamycin, neomycin such as neomycin trisulfate salt hydrate, netilmicin such as netilmicin sulfate salt, paromomycin such as paromomycin sulfate salt, ribostamycin such as ribostamycin sulfate salt, sisomicin such as sisomicin sulfate salt, spectinomycin such as spectinomycin dihydrochloride pentahydrate, streptomycin such as streptomycin sulfate salt, and tobramycin aminoglycoside antibiotic such as tobramycin sulfate salt (aminoglycoside antibiotic), paromomycin, each of which is commercially available *e.g.,* from Sigma-Aldrich.

In one such example, the antimicrobial agent is or comprises an aminoglycoside antibiotic selected from: amikacin, gentamicin, kanamycin, neomycin, netilmicin, tobramycin, streptomycin, and spectinomycin.

In one example, the antimicrobial agent which is or comprises an aminoglycoside provides antimicrobial effect such as antibacterial effect by binding to the microbial (e.g., bacterial) 30S ribosomal subunit and/or the 50S subunit, thereby inhibiting the translocation of the peptidyltRNA from the A-site to the P-site and/or causing misreading of mRNA, leaving the microorganism cells (e.g., bacterium cells) unable to synthesize proteins vital to its growth.

In another example, the antimicrobial agent is or comprises an ansamycin antibiotic selected from: geldanamycin, herbimycin, and rifaximin.

In another example, the antimicrobial agent is or comprises a macrolide. For example, the macrolide is or comprises a compound selected from: anhydroerythromycin A (also known as erythromycin anhydride), apoptolidin A, azithromycin, dafilomycin A1 *e.g*., from *Streptomyces griseus,* bafilomycin B1 *e.g.,* from *Streptomyces sp.,* borrelidin *e.g.,* from *Streptomyces sp.* (such as *Streptomyces parvulus*), clarithromycin, clindamycin such as clindamycin hydrochloride lincosamide antibiotic or clindamycin 2-phosphate aminoglycoside antibiotic, concanamycin A *e.g*., from *Streptomyces sp.,* dirithromycin, dirithromycin, erythromycin such as erythromycin estolate or erythromycin ethyl succinate or erythromycin stearate, filipin complex *e.g.,* from *Streptomyces filipinensis,* Josamycin, lincomycin such as lincomycin hydrochloride, mepartricin, nonactin *e.g.,* from *Streptomyces sp.,* (such as *Streptomyces griseus*), oligomycin *e.g.,* from *Streptomyces diastatochromogenes,* oligomycin A, pimaricin *e.g., Streptomyces chattanoogensis,* rapamycin *e.g*., from *Streptomyces hygroscopicus* such as rapamycin sodium salt, rifapentine, rifaximin, roxithromycin, spiramycin *e.g*., from *Streptomyces* sp. such as spiramycin adipate, tiamulin, telithromycin, troleandomycin, tylosin such as tylosin tartrate, virginiamycin S1 (also known as Antibiotic 899 or Staphylomycin S), and virginiamycin M1 (also known as mikamycin A or staphylomycin). Each of these macrolides is commercially available *e.g*., from Sigma-Aldrich.

For example, the macrolide is or comprises a compound selected from: azithromycin, clarithromycin, dirithromycin, erythromycin, roxithromycin, troleandomycin, telithromycin, spiramycin.

In one such example, the antimicrobial agent which is or comprises one or more macrolide(s), provides antimicrobial effect such as antibacterial effect by inhibiting microbial (e.g., bacterial) protein biosynthesis by binding reversibly to the subunit 50S of the microbial ribosome, thereby inhibiting translocation of peptidyl tRNA.

In another example, the antimicrobial agent is or comprises a tetracycline antibiotic such as a tetracycline selected from any one or more of: chlortetracycline such as chlortetracycline hydrochloride, demeclocycline such as demeclocycline hydrochloride, doxorubicin such as doxorubicin hydrochloride, doxycycline such as doxycycline hyclate, duramycin *e.g.,* from *Streptoverticillium cinnamoneus,* meclocycline such as meclocycline sulfosalicylate salt, minocycline such as minocycline hydrochloride, oxytetracycline such as oxytetracycline dehydrate or oxytetracycline hydrochloride, and tetracycline such as tetracycline hydrochloride. Each of these tetracyclines is commercially available *e.g.,* from Sigma-Aldrich.

In one such example, the antimicrobial agent which is or comprises a tetracycline antibiotic provides antimicrobial effect such as antibacterial effect by inhibiting binding of microbial aminoacyl-tRNA to the mRNA-ribosome complex *e.g.,* in actively dividing cells of the microorganism, for example by binding to the 30S ribosomal subunit in the mRNA translation complex of the microbial cells.

In another example, the antimicrobial agent is or comprises a polymixin antibiotic such as polymixin B and/or colistin, each of which is commercially available *e.g.,* from Sigma-Aldrich.

In another example, the antimicrobial agent is or comprises a lincosamide such as clindamycin and/or lincomycin, each of which is commercially available *e.g.,* from Sigma-Aldrich. For example, the antimicrobial agent which is or comprises the lincosamide(s) provides antimicrobial effect such as antibacterial effect by binding to the microbial (e.g., bacterial) 50S subunit of (e.g., bacterial) ribosomal RNA, thereby inhibiting protein synthesis in the actively dividing cells of the microorganism.

In another example, the antimicrobial agent is or comprises a nitrofuran such as furazolidone and/or nitrofurantoin, each of which is commercially available *e.g.,* from Sigma-Aldrich.

In another example, the antimicrobial agent is or comprises an oxazolidinone such as linezolid (e.g., Sigma-Aldrich) and/or posizolid (e.g., Sigma-Aldrich) and/or radezolid and/or torezolid.

In one such example, the antimicrobial agent which is or comprises an oxazolidinone provides antimicrobial effect such as antibacterial effect by inhibiting protein initiation of protein synthesis e.g., in actively dividing cells of the microorganism.

In another example, the antimicrobial agent is or comprises a peptide or polypeptide or peptide derivative or polypeptide derivative. For example, the peptide or peptide derivative is selected from one or more of: actinomycin (actinomycin C1), actinonin, amastatin hydrochloride hydrate, 7-aminoactinomycin D (7-AAD), antimycin A (e.g., from *Streptomyces* sp.), antimycin A₂, antipain such as antipain dihydrochloride, bacitracin such as bacitracin zinc salt (e.g., from *Bacillus licheniformis*), bactenecin, BD-3 human recombinant (e.g., Sigma-Aldrich), Beta D-4 human recombinant (e.g., Sigma-Aldrich), carbenicillin such as carbenicillin disodium salt, cecropin A, cecropin B, cecropin P1 procine, cinnamycin *e.g.,* from *Streptomyces cinnamoneus,* colistin such colistin as sulfate salt or colistin sodium methanesulfonate, cyclosporin A e.g., from *tolypocladium inflatum,* β-Defensin 1 human (e.g., Sigma-Aldrich), β-Defensin 2 human recombinant (e.g., Sigma-Aldrich), defensin HNP-1 human (e.g., Sigma-Aldrich), defensin HNP-2 human (e.g., Sigma-Aldrich), Elafin human (e.g., recombinant, expressed in *Saccharomyces cerevisiae*) (e.g., Sigma-Aldrich), fengycin (e.g., Sigma-Aldrich), gramicidin polypeptide e.g., from *Bacillus aneurinolyticus* such as gramicidin A polypeptide or gramicidin C polypeptide, Kendomycin *e.g.,* from *Streptomyces violaceoruber,* lactoferricin B (fragment 4-14 trifluoroacetate salt) (e.g., Sigma-Aldrich), LL-37 (human) trifluoroacetate salt (e.g., Sigma-Aldrich), lysostaphin *e.g.,* from *Staphylococcus staphylolyticus,* magainin I, magainin II, melittin *e.g.,* from honey bee venom, mycosubtilin, nisin e.g., *from Lactococcus lactis,* NP-1 human recombinant (e.g., Sigma-Aldrich), pediocin *e.g.,* from *Pediococcus acidilactici,* PGLa, Polymyxin B, Surfactin *e.g.,* from Bacillus subtilis, thiostrepton *e.g.,* from *Streptomyces azureus,* and valinomycin. All the above mentioned peptide or peptide derivatives are commercially available *e.g.,* from Sigma-Aldrich.

In one such example, the antimicrobial agent is or comprises the bacitracin polypeptide. For example, such antimicrobial agent provides antimicrobial activity such as antibacterial activity by inhibiting the microbial isoprenyl pyrophosphate (a molecule that carries the building blocks of the peptidoglycan microbial *e.g.,* bacterial cell wall outside of the inner membrane of the cells) such as in actively dividing cells of the microorganism.

In another such example, the antimicrobial agent is or comprises the colistin polypeptide or the polymyxin B polypeptide. For example, such antimicrobial agent provides antimicrobial activity such as anti-bacterial activity by interacting with a gram-negative bacterial outer membrane and cytoplasmic membrane, and displacing bacterial counterions, thereby destabilizing the bacterial outer membrane. Alternatively, or in addition, such antimicrobial agent may function as a detergent against the cytoplasmic membrane *e.g.,* of actively dividing cell of the microorganism thereby altering their cellular permeability.

In another example, the antimicrobial agent is or comprises a glycopeptide. For example, the glycopeptide is selected from one or more of: vancomycin, teicoplanin, bleomycin such as bleomycin sulfate *e.g.,* from *Streptomyces verticillus,* phleomycin *e.g.,* from *Streptomyces verticillus,* ramoplanin, ristomycin *e.g.,* ristomycin monosulfate, vancomycin such as vancomycin hydrochloride *e.g.,* from *Streptomyces orientalis,* telavancin, dalbavancin, and oritavancin. All the above mentioned glycopeptides are commercially available *e.g.,* from Sigma-Aldrich. In one such example, the antimicrobial agent is or comprises a glycopeptide selected from teicoplanin, vancomycin, telavancin, dalbavancin and/or oritavancin. For example, the antimicrobial agent which is or comprises a glycopeptide provides antimicrobial effect such as antibacterial effect by binding to the microbial (e.g., bacterial) Inhibits peptidoglycan synthesis *e.g.,* of actively dividing cells of the microorganism.

In another example, the antimicrobial agent is or comprises a nucleoside. For example, the nucleoside is selected from: Adenine 9-β-D-arabinofuranoside, 5-Azacytidine, blasticidine S hydrochloride, cordycepin *e.g.,* from *Cordyceps militaris,* 5-Fluorocytosine nucleoside analog, formycin A *e.g.,* from *Streptomyces kaniharaensis,* ganciclovir, ribavirin [1-β-D-Ribofuranosyl-1,2,4-triazole-3-carboxamide], sangivamycin such as sangivamycin hydrate *e.g.,* from *Streptomyces rimosus,* sinefungin [5'-Deoxy-5'-(1,4-diamino-4-carboxybutyl)adenosine], tubercidin *e.g.,* from *Streptomyces tubercidicus,* tunicamycin e.g., from *Streptomyces sp.,* and tunicamycin C₂ homolog. Each one of the above mentioned nucleosides is commercially available *e.g.,* from Sigma-Aldrich.

In another example, the antimicrobial agent is or comprises a phenicole compound. For example, the phenicole compound is selected from: chloramphenicol such as chloramphenicol succinate sodium salt and/or thiamphenicol [D-threo-2,2-Dichloro-N-(β-hydroxy-α-[hydroxymethyl]-4-[methylsulfonyl]phenethyl)acetamide], each of which is commercially available *e.g.,* from Sigma-Aldrich.

In another example, the antimicrobial agent is or comprises a polyene compound. For example, the polyene compound is selected from: Amphotericin B *e.g.,* form *Streptomyces* sp., kirromycin (also known as antibiotic Myc-8003 or mocimycin) *e.g.,* from *Streptomyces collinus,* and nystatin (also known as fungicidin or mycostatin), each of which is commercially available *e.g.,* from Sigma-Aldrich.

In another example, the antimicrobial agent is or comprises a polyether compound. For example, the polyether compound is selected from: monensin (also known as monensin A) such as monensin sodium salt or monensin sodium salt hydrate, nigericin (also known as antibiotic K178, antibiotic X464, azalomycin M, helexin C, polyetherin A) such as nigericin sodium salt, and Salinomycin *e.g.,* from *Streptomyces albus,* each of which is commercially available *e.g.,* from Sigma-Aldrich.

In another example, the antimicrobial agent is or comprises a pyridines and/or pyrimidine compound. For example, the pyridines and/or pyrimidine compound is selected from: fusaric acid [5-Butylpyridine-2-carboxylic acid] *e.g.,* from *Gibberella fujikuroi,* isoniazid [4-Pyridinecarboxylic acid hydrazide], prothionamide [2-propyl-4-pyridinecarbothioamide], tioconazole [1-[2-[(2-chloro-3-thienyl)methoxy]-2-(2,4-dichlorophenyl)ethyl]-1H-imidazole], trimethoprim [2,4-Diamino-5-(3,4,5-trimethoxybenzyl)pyrimidine] such as trimethoprim lactate salt. Each of these exemplified pyridines and/or pyrimidine compound is commercially available *e.g.,* from Sigma-Aldrich.

In another example, the antimicrobial agent is or comprises a quinolone compound and/or a fluoroquinolone compound. For example, the quinolone compound and/or a fluoroquinolone compound is/are selected from: cinoxacin [1-ethyl-1,4-dihydro-4-oxo-[1,3]dioxolo[4,5-g]cinnoline-3-carboxylic acid], ciprofloxacin [1-Cyclopropyl-6-fluoro-4-oxo-7-(piperazin-1-yl)-1,4-dihydroquinoline-3-carboxylic acid], difloxacin hydrochloride [6-Fluoro-1-(4-fluorophenyl)-1,4-dihydro-7-(4-methylpiperazino)-4-oxo-3-quinolinecarboxylic acid hydrochloride], enoxacin, enrofloxacin (also known as baytril), flumequine, 8-Hydroxyquinoline, nadifloxacin [9-fluoro-6,7-dihydro-8-(4-hydroxy-1-piperidinyl)-5-methyl-1-oxo-1H,5H-benzo[ij]quinolizine-2-carboxylic acid], nalidixic acid [4-Dihydro-1-ethyl-7-methyl-1,8-naphthyridin-4-one-3-carboxylic acid] such as nalidixic acid sodium salt, ofloxacin fluoroquinolone antibiotic (also known as fluoroquinolone antibiotic), oxolinic acid quinolone antibiotic (also known as quinolone antibiotic) [5,8-Dihydro-5-ethyl-8-oxo-1,3-dioxolo[4,5-g]quinoline-7-carboxylic acid], pefloxacin mesylate dehydrate [3-Quinolinecarboxylic acid, 1-ethyl-6-fluoro-1,4-dihydro-7-(4-methyl-1-piperazinyl)-4-oxo-, monomethanesulfonate], pipemidic acid [8-ethyl-5,8-dihydro-5-oxo-2-(1-piperazinyl)pyrido(2,3-d)pyrimidine-6-carboxylic acid], 8-quinolinol [8-Hydroxyquinoline] such as 8-quinolinol hemisulfate salt, levofloxacin, gatifloxacin, gemifloxacin, lomefloxacin, moxifloxacin, nalidixic acid, and norfloxacin. Each of these exemplified quinolones and fluoroquinolones is commercially available *e.g.,* from Sigma-Aldrich.

In one such example, the antimicrobial agent which is or comprises quinolone compound and/or a fluoroquinolone compound provides antimicrobial effect such as antibacterial effect by inhibiting the microbial (e.g., bacterial) DNA gyrase and/or the topoisomerase IV enzyme, thereby inhibiting DNA replication and transcription *e.g.,* in actively dividing cells of the microorganism.

In another example, the antimicrobial agent is or comprises a sulfonamide compound. For example, the sulfonamide compound is selected from: succinylsulfathiazole, sulfabenzamide [N-(4-aminobenzenesulfonyl)benzamide], sulfacetamide [N-(4-Aminobenzenesulfonyl)acetamide] such as sulfacetamide sodium salt monohydrate, sulfadiazine [4-Amino-N-(2-pyrimidinyl)benzenesulfonamide], sulfadimethoxine [4-Amino-N-(2,6-dimethoxy-4-pyrimidinyl)benzenesulfonamide], sulfadoxin [4-Amino-N-(5,6-dimethoxy-4-pyrimidinyl)benzenesulfonamide], silver sulfadiazine, sulfaguanidine [-amino-N-(aminoiminomethyl)benzenesulfonamide], sulfameter [5-Methoxysulfadiazine], sulfamethazine [4,6-Dimethylsulfadiazine], sulfamonomethoxine [4-methoxy-6-sulfanilamidopyrimidine], sulfanilamide [*p*-aminobenzenesulfonamide], sulfaquinoxaline *e.g.,* as sodium salt, sulfasalazine [2-hydroxy-5-{{4-[(2-pyridinylamino)sulfonyl]phenyl}azo}benzoic acid], sulfathiazole e.g., as sodium salt [4-amino-N-(2-thiazolyl)benzenesulfonamide sodium salt], sulfamethoxazole, mafenide, sulfamethizole, sulfanilamide, trimethoprim, trimethoprim-sulfamethoxazole (cotrimoxazole; also known as TMP-SMX) and sulfonamidochrysoidine. Each of these exemplified sulfonamides is commercially available *e.g.,* from Sigma-Aldrich.

In one such example, the antimicrobial agent which is or comprises a sulfonamide compound provides antimicrobial effect such as antibacterial effect by inhibiting folate synthesis *e.g.,* in actively dividing cells of the microorganism. According to this example, the sulfonamide compound may be a competitive inhibitor of the microbial enzyme dihydropteroate synthetase (DHPS). According to this example, in the absence of the antimicrobial agent, the microbial DHPS may generally catalyze the conversion of PABA (para-aminobenzoate) to dihydropteroate, a step in folate synthesis.

Without being bound by any particular theory or specific mode of action, the inventors reason that folate may be necessary for microbial cells to synthesize nucleic acids (such as microbial DNA and/or RNA). Accordingly, the inventors speculate that antimicrobial agents comprising a sulfonamide compound and prevent synthesis microbial nuclei acids *e.g.,* in actively dividing cells of the microorganism.

In another example, the antimicrobial agent is or comprises a statin such as mevastatin (also known as compactin or ML-236B), which is commercially available *e.g.,* from Sigma-Aldrich.

In another example, the antimicrobial agent is or comprises a steroid such as fusidic acid sodium salt (also known as fucidin) which is commercially available *e.g.,* from Sigma-Aldrich.

In another example, the antimicrobial agent is or comprises a taxoide compound such as paclitaxel *e.g.,* from *Taxus brevifolia* which is commercially available *e.g.,* from Sigma-Aldrich.

Other exemplary antimicrobial agents include antimicrobial agents comprising any one or more of the following: anisomycin [(2R,3S,4S)-2-(4-Methoxybenzyl)-3,4-pyrrolidinediol-3-acetate] *e.g.,* from *Streptomyces griseolus,* cycloheximide [3-[2-(3,5-Dimethyl-2-oxocyclohexyl)-2-hydroxyethyl]glutarimide] (also known as actidione or naramycin A), cytochalasin D (also known as Zygosporin A) *e.g.,* from *Zygosporium mansonii,* cytochalasin B (also known as Phomin) *e.g.,* from *Drechslera dematioidea,* emetine dihydrochloride hydrate, ionomycin such ionomycin as calcium salt *e.g.,* from *Streptomyces conglobatus,* and Piericidin A (also known as Shaoguanmycin B) *e.g.,* from *Streptomyces mobaraensis,* each of which is commercially available *e.g.,* from Sigma-Aldrich.

In another example, the antimicrobial agent is or comprises a peptidyl nucleoside. For example, the peptidyl nucleoside is any one or more of: nikkomycin Z *e.g.,* from *Streptomyces tendae,* nourseothricin sulfate and puromycin dihydrochloride e.g., from *Streptomyces alboniger.* All the above mentioned peptidyl nucleoside are commercially available *e.g.,* from Sigma-Aldrich.

In another example, the antimicrobial agent is a aureolic acid such as chromomycin A3 *e.g.,* from *Streptomyces griseus* and/or mithramycin A *e.g.,* from *Streptomyces plicatus,* each of which readily commercially available *e.g.,* form Sigma-Aldrich.

In another example, the antimicrobial agent is or comprises an aziridine compound such as mitomycin C *e.g.,* from *Streptomyces caespitosus* and/or Thio-TEPA [N,N',N"-Triethylenethiophosphoramide, also known as Tris(aziridinyl)phosphine sulfide], each of which is commercially available *e.g.,* from Sigma-Aldrich.

In another example, the antimicrobial agent is or comprises a benzenoid compound such as 17-(Allylamino)-17-demethoxygeldanamycin, 17-Dimethylaminoethylamino-17-demethoxygeldanamycin, geldanamycin *e.g.,* from *Streptomyces hygroscopicus,* and/or herbimycin A *e.g.,* from *Streptomyces hygroscopicus,* each of which is commercially available *e.g.,* from Sigma-Aldrich.

In another example, the antimicrobial agent is or comprises a Benzimidazole compound such as albendazole [also known as Methyl 5-(propylthio)-2-benzimidazolecarbamate] and/or ricobendazole [also known as Albendazole oxide, or Methyl [5-(propane-1-sulfinyl)-1H-benzoimidazol-2-yl]-carbamate], each of which is commercially available *e.g.,* from Sigma-Aldrich.

In another example, the antimicrobial agent is or comprises a coumarin-glycoside compound such as coumermycin A1 and/or novobiocin *e.g.,* novobiocin sodium salt, each of which is commercially available *e.g.,* from Sigma-Aldrich.

In another example, the antimicrobial agent is or comprises a diphenyl ether derivative such as irgasan [also known as 5-Chloro-2-(2,4-dichlorophenoxy)phenol] which is commercially available *e.g.,* from Sigma-Aldrich.

In another example, the antimicrobial agent is or comprises an epipolythiodioxopiperazine such as gliotoxin from *Gliocladium fimbriatum* which is commercially available *e.g.,* from Sigma-Aldrich.

In another example, the antimicrobial agent is or comprises a fatty acid or a fatty acid derivative such as cerulenin [(2R,3S,E,E)-2,3-Epoxy-4-oxo-7,10-dodecadienamide] *e.g.,* from *Cephalosporium caerulens* which is commercially available *e.g.,* from Sigma-Aldrich.

In another example, the antimicrobial agent is or comprises a glucosamine such as 1-deoxymannojirimycin hydrochloride [1,5-Dideoxy-1,5-imino-D-mannitol hydrochloride] and/or N-methyl-1-deoxynojirimycin [1,5-ddeoxy-1,5-imino-1-methyl-D-sorbitol], each of which is commercially available *e.g.,* from Sigma-Aldrich.

In another example, the antimicrobial agent is or comprises an imidazole compound. For example, the imidazole compound is selected from: clotrimazole, econazole such as econazole nitrate salt, ketoconazole, metronidazole, miconazole, such as (±)-miconazole nitrate salt, sulconazole such as sulconazole nitrate salt, and thiabendazole, each of which is commercially available *e.g.,* from Sigma-Aldrich.

In another example, the antimicrobial agent is or comprises an indol derivative such as fumitremorgin C *e.g.,* from *Neosartorya fischeri* and/or staurosporine *e.g.,* from *Streptomyces sp*., each of which is commercially available *e.g.,* from Sigma-Aldrich.

In another example, the antimicrobial agent is or comprises a macrolactam such as ascomycin *e.g.,* from *Streptomyces hygroscopicus* var. *ascomyceticus* which is commercially available *e.g.,* from Sigma-Aldrich.

In one particularly preferred example, the antimicrobial agent is or comprises an antibiotic. For example, the antibiotic may be a beta-lactam antibiotic such as penicillin antibiotic, a cephalosporin antibiotic, a monobactam antibiotic and/or a carbapenem antibiotic. For example, the antimicrobial agent is or comprises a cephalosporin antibiotic and/or a carbapenem antibiotic. The antimicrobial agent may be or comprises a cephalosporin antibiotic. Alternatively, the antimicrobial agent may be or comprises a carbapenem antibiotic

In one example, the beta-lactam antibiotic is a carbapenem antibiotic selected from meropenem, imipenem and ertapenem.

In another example, the beta-lactam antibiotic is a cephalosporin antibiotic selected from ceftazidime, cefepime and ceftriaxone.

In another example, the antimicrobial agent may comprise or consist of a physical and/or chemical phenomenon or stress applied to the cells of the microorganisms described according to broad aspect, embodiment of example hereof. For example, the antimicrobial agent may comprise or consist of one or more physical and/or chemical phenomenon or stress applied to actively dividing cells of a microorganism selected from heat stress, ultraviolet light, blue light, radiation such as electromagnetic radiation, microwave radiation, radiation with X-rays or gamma rays, particle radiation such as alpha, beta or neurons particle radiation, and/or oxidative stress.

In one preferred example, the microorganism present in the sample obtained from the subject is a bacteria belonging to the *Klebsiella* species and/or the cells of the microorganism present in the sample obtained from the subject are cells of bacteria belonging to the *Klebsiella* species, and the antimicrobial agent assayed in the method according to any broad aspect of the invention described hereof comprises a beta-lactam antibiotic. In one such preferred example, the microorganism is a strain of *K. pneumoniae* or *K. oxytoca* and/or the cells of the microorganism are cells of a strain of *K. pneumoniae* or *K. oxytoca,* and the antimicrobial agent comprises a carbapenem antibiotic such as meropenem and/or imipenem and/or ertapenem. In an alternative preferred example, the microorganism is a strain of *K. pneumoniae* and/or the cells of the microorganism are cells of a strain of *K. pneumoniae,* and the antimicrobial agent comprises a cephalosporin antibiotic such as ceftriaxone.

In an alternative preferred example, the microorganism present in the sample obtained from the subject is a bacteria belonging to the *Staphylococcus* species and/or the cells of the microorganism present in the sample obtained from the subject are cells of bacteria belonging to the *Staphylococcus* species, and the antimicrobial agent assayed in the method according to any broad aspect of the invention described hereof comprises a beta-lactam antibiotic. In one such preferred example, the microorganism is a strain of *Staphylococcus aureus* and/or the cells of the microorganism are cells of a strain of *Staphylococcus aureus,* and the antimicrobial agent comprises a cephalosporin antibiotic such as cefoxitin. In an alternative preferred example, the microorganism is a strain of *Staphylococcus aureus* and/or the cells of the microorganism are cells of a strain of *Staphylococcus aureus,* and the antimicrobial agent comprises a penicillin antibiotic such as oxacillin.

In one example, the method according to the first broad aspect of the invention further comprises recovering, isolating and/or purifying the microorganism from the sample obtained from the subject.

In yet another example, the method according to the first broad aspect of the invention further comprises further comprising culturing the cells of the microorganism in and/or from the sample obtained from the subject for the purpose of identifying (e.g., identifying the species or genus of) the microorganism obtained from the subject.

Of course, in an alternative preferred example, the method does not comprise identifying the microorganism or culturing the cells of the microorganisms in and/or from the sample obtained from the subject to identify the microorganism (e.g., to identifying the species or genus of the microorganism).

Preferably, the subject is an animal or a human subject. In one example, the subject having or suspected of having an infection with a microorganism may present one or more symptoms indicative or suggestive to a clinician such as a medical doctor or a nurse of an infection with one or more microorganisms. By way of a non-limiting example, a subject having or suspected of having an infection with a microorganism may suffer from or present one or more symptoms selected from elevated body temperature such as greater than 38°C (or 100.4°F), muscle weakness, organ or site specific pain, tissue necrosis or putrefaction, excessive drowsiness or sleepiness, muscle weakness, excessive pre-nasal or post-nasal mucous production, diarrhea, abdominal pain, coughing and/or sneezing, non-specific joint and muscle pain, and/or an organ specific inflammation such as cellulitis, folliculitis, impetigo, otitis or otitis media, inflammation of the urinary tract, gastroenteritis and/or meningitis. Alternatively, or in addition, the subject having or suspected of having an infection with a microorganism may present one or more symptoms indicative or suggestive to a clinician such as a medical doctor or a nurse and/or one or more symptoms indicative or suggestive of sepsis such as one or more symptoms selected from elevated body temperature greater than 38°C (100.4°F) or a lower body temperature of less than 36°C (96.8°F), increased heart rate (e.g., of about more than 90 beats per minute), irregular or changing heart rhythm, shortness of breath or increased breathing rate (e.g., breathing rate greater than 20 breaths per minute), difficulty or pain associated with breathing, altered mental state such as disorientation, confusion, unprovoked aggression, decreased urine production, abnormal blood clotting, evidence of microbial infection presenting at multiple body sites, a drop in blood pressure (e.g., relative to individual patient norm), weakness or extreme weakness, unconsciousness, and/or death.

In a second broad aspect, the present invention provides a method of determining susceptibility of a unicellular or multicellular microorganism to a plurality of antimicrobial agents. The method comprises performing the method according to the first broad aspect wherein the method comprises exposing the actively dividing cells of the microorganism in and/or from the sample obtained from the subject to one or more representative antimicrobial agent(s) of a first family or panel of a plurality of antimicrobial agents, to thereby determine susceptibility of the cells of the microorganism to antimicrobial agents belonging to this first family or panel of antimicrobial agents.

In one example, when the cells of the microorganism are determined to be susceptible to the one or more representative antimicrobial agent(s) of the first family or panel of the plurality of antimicrobial agents, the method further comprises performing the method according to the first broad aspect by exposing the actively dividing cells of the microorganism in and/or from the sample obtained from the subject to a one or more additional antimicrobial agents belonging to the first family or panel of antimicrobial agents, to thereby determine susceptibility of the cells of the microorganism for the subject to said additional antimicrobial agents.

In another example, the method according to this second aspect may further comprise performing the method according to the first aspect, wherein the method comprises exposing the actively dividing cells of the microorganism in and/or from the sample obtained from the subject to
(i) one or more representative antimicrobial agent(s) of a second family or panel of a plurality of antimicrobial agents, and/or
(ii) one or more representative antimicrobial agent(s) of a third family or panel of a plurality of antimicrobial agents, and/or
(iii) one or more representative antimicrobial agent(s) of a fourth family or panel of a plurality of antimicrobial agents, and/or
(iv) to one or more representative antimicrobial agent(s) of any additional further family or panel of a plurality of antimicrobial agents,
to thereby determine susceptibility of the cells of the microorganism to antimicrobial agents belonging to said second and/or third and/or fourth and/or any further additional family or panel of a plurality of antimicrobial agents.

In one such example, when the cells of the microorganism are determined to be susceptible to the one or more representative antimicrobial agent(s) of the second and/or third and/or fourth and/or further additional family or panel of the plurality of antimicrobial agents, then said method further comprises performing the method according to the first broad aspect by exposing the actively cells of the microorganism from the sample obtained in and/or from the subject to a one or more additional antimicrobial agents belonging to the second and/or third and/or fourth and/or any additional further family or panel of the plurality of antimicrobial agents, to thereby determine susceptibility of said cells of said microorganism to the additional antimicrobial agents.

In a third broad aspect, described but not claimed, the present invention provides a method of treating a subject having or suspect of having an infection with a unicellular or multicellular microorganism. The method comprises determining susceptibility of the microorganism in said subject to any one or more antimicrobial agent(s) by performing the method according to the first or second broad aspects of the invention. The method further comprises identifying one or more antimicrobial agent(s) to which the microorganism has been determined to be susceptible, and administering to the subject a therapeutically effective amount of the one or more antimicrobial agent(s).

In a fourth broad aspect, described but not claimed, the present invention provides use of a therapeutically effective amount of any one or more antimicrobial agent(s) in the preparation of a medicament for the treatment of a subject having or suspect of having an infection with a unicellular or multicellular microorganism, wherein the microorganism has been determined to be susceptible to the one or more antimicrobial agent(s) by performing the method according to the first or second broad aspects.

In a fifth broad aspect, described but not claimed, the present invention provides use of a therapeutically effective amount of any one or more antimicrobial agent(s) in the treatment of a subject having or suspect of having an infection with a unicellular or multicellular microorganism, wherein the microorganism has been determined to be susceptible to the one or more antimicrobial agent(s) by performing the method according to the first or second broad aspects.

In one example according to any broad aspect, embodiment and/or example described hereof, the method of the susceptibility of a unicellular or multicellular microorganism to an antimicrobial agent by acoustic flow cytometry is performed using an acoustic flow cytometer. In one preferred example, the acoustic flow cytometer is optimised for resolution of small particles, and/or comprises a laser and detector installed capable of measuring the fluorescent dye being used in the method, and/or be capable of resolving particles of about 800 nm and larger, and/or capable of accurate volumetric delivery or, in other words, be capable of accurately measuring the dispensed volume of a sample *e.g.,* a cell sample being analyzed. In one such example, when the method employs a nucleic acid binding fluorescent compound such as SYTO 9, the cytometer comprises a laser and detector combination comprising a 488nm blue laser with a 530/30 nm filter on the photomultiplier tube, for measuring the fluorescent compound being used.

For example, the acoustic flow cytometer employed in the method according to any aspect, embodiment or exampled described hereof, may be Attune acoustic flow cytometer (*e.g.,* Thermo Fisher Scientific) or Attune NxT acoustic flow cytometer (*e.g.,* Thermo Fisher Scientific).

In one example of the method according to any aspect, embodiment or example described throughout herein, the sample obtained from the subject is a blood sample comprising the cells of the microorganism.

In one example of the method according to any aspect according to any aspect, embodiment or example described throughout herein, the sample obtained from the subject is a blood sample.

In one example of the method according to any aspect according to any aspect, embodiment or example described throughout herein, determining susceptibility of the microorganism to the antimicrobial agent by performing said method is achieved within about 1 to about 3 hours or within about 1 hour or within about 2 hours or within about 3 hours from the time of obtaining the sample from the subject and/or from the time of first incubating the sample obtained from the subject in the culture medium.

In some examples of the method according to any aspect, embodiment or example described throughout herein, the sample obtained from the subject is a blood sample comprising the cells of the microorganism.

In some examples of the method according to any aspect, embodiment or example described throughout herein, the method comprises incubating in the culture medium a sample obtained from the subject having or suspected of having an infection with said microorganism, wherein the sample comprises cells of the microorganism, and wherein the sample is obtained from the subject is incubated directly in said culture medium without first isolating and/or removing said cells of said microorganism from said sample prior to said incubation of said sample in said culture medium. For example, the sample obtained from the subject is a blood sample. Also, for example, determining susceptibility of the microorganism to the antimicrobial agent by performing said method is achieved within about 1 to about 3 hours or within about 1 hour or within about 2 hours or within about 3 hours from the time of obtaining the sample from the subject and/or from the time of first incubating the sample obtained from the subject in the culture medium.

In some examples of the method according to any aspect, embodiment or example described throughout herein, the microorganism is *Burkholdria thailandesis* or *Burkholderia pseudomallei,* and optionally the antimicrobial agent is a carbapenem antibiotic such as meropenem.

In other examples of the method according to any aspect, embodiment or example described throughout herein, the microorganism is *Staphylococcus aureus,* and optionally the antimicrobial agent is a carbapenem antibiotic. For example, the carbapenem antibiotics may be oxacillin and/or cefoxitin. In one preferred example, the *Staphylococcus aureus* is methicillin resistant. Alternatively, the *Staphylococcus aureus* is methicillin susceptible to methicillin.

The method as described herein is adapted for high-throughput qualitative and/or quantitative screening for susceptibility of microorganisms in or form a clinical sample to one or more antimicrobial agents simultaneously. In one example, the method is adapted for simultaneous high-throughput screening of susceptibility of multiple sample of microorganisms obtained from different clinical samples and/or for multiple antimicrobial agents. The high-throughput screening method may be employed to rapidly predict qualitatively susceptibility of one or plurality of microorganisms to one or plurality of antimicrobial agents within about 1 to 3, preferably with about 2 hours and more preferably within about 1 hour from the time of obtaining the sample comprising cells of the microorganism from the subject and/or from the time of first incubating cells of the microorganism in a culture medium to obtain actively dividing culture of the microorganism. Alternatively, or in addition, the rapid high throughput method according to this embodiment may be employed to rapidly predict quantitatively susceptibility of one or plurality of microorganisms to one or plurality of antimicrobial agents by determining MIC FAST values within about 2 to 6 hours, preferably within about 2 to 5 hours, or within about 2 to about 4 hours or about 3 to about 4 hours or about 2 to about 3 hours, and more preferably about 3 hours from the time of obtaining the sample comprising cells of the microorganism from the subject, and/or from the time of first incubating cells of the microorganism in a culture medium to obtain actively dividing culture of the microorganism.

In one such example, the high-throughput method utilized at least one 96 well plate for screening determine qualitatively and/or quantitatively of one or more microorganisms to one or more antimicrobial agents. In one example, the high-throughput method used to determine qualitatively and/or quantitatively susceptibility of at least 13 different microorganisms obtained from different clinical samples to one or more antimicrobial agent(s), in a single 96 well plate.

In another example, using a single 96 well plate, the high-throughput method can be used to simultaneously determine qualitatively and/or quantitatively (by determining MIC FAST values) susceptibility of a single microorganism (obtained from one clinical sample such as a blood sample) to at least 13 different antimicrobial agents.

In another example, using a single 96 well plate, the high-throughput method can be used to simultaneously determine qualitatively and/or quantitatively (by determining MIC FAST values) susceptibility of two or more microorganisms (obtained from two or more clinical samples to at least four different antimicrobial agents for example tested at different concentrations of each antimicrobial agent. For example, the different concentrations of the antimicrobial agent assayed may be a serial dilution comprising one or more of the following concentrations 0.25 mg/L, 0.5 mg/L, 1.0 mg/L, 2.0 mg/L, 4.0 mg/L, 8.0 mg/L, 16.0 mg/L, 32.0 mg/L, 64.0 mg/L, 128.0 mg/L, and 256.0 mg/L.

In another such example, using a single 96 well plate, the high-throughput method may be used to simultaneously determine qualitatively and/or quantitatively (by determining MIC FAST values) susceptibility of two or more microorganisms (obtained from two or more clinical samples) to at least eight different antimicrobial agents for example tested at different concentrations of each antimicrobial agent e.g., selected from 0.25 mg/L, 0.5 mg/L, 1.0 mg/L, 2.0 mg/L, 4.0 mg/L, 8.0 mg/L, 16.0 mg/L, 32.0 mg/L, 64.0 mg/L, 128.0 mg/L, and/or 256.0 mg/L.

In yet a further such example, using a single 96 well plate, the high-throughput method may be used to simultaneously determine qualitatively and/or quantitatively (by determining MIC FAST values) susceptibility at least one microorganism (obtained from a single clinical sample) to at least 13 different antimicrobial agents for example tested at different concentrations e.g., selected from 0.25 mg/L, 0.5 mg/L, 1.0 mg/L, 2.0 mg/L, 4.0 mg/L, 8.0 mg/L, 16.0 mg/L, and 32.0 mg/L.

The person skilled in the art would recognize that other permutations of number of microorganisms and antimicrobial agents other than those described herein can be tested in the high-throughput method of the invention without affecting efficacy of the method to achieve rapid determination of susceptibility to antimicrobial agents tested.

In other examples of the method, the microorganism is *Klebsiella pneumoniae,* and optionally the antimicrobial agent is piperacillin and/or tazobactam. For example, the antimicrobial agent comprises a combination of both piperacillin and tazobactam antibiotics. For example, the carbapenem antibiotics may be oxacillin and/or cefoxitin. In one preferred example, the *Staphylococcus aureus* is methicillin resistant. Alternatively, the *Staphylococcus aureus* is susceptible to methicillin.

### BRIEF DESCRIPTION OF THE DRAWINGS

Further features of the present invention are more fully described in the following description of several non-limiting embodiments thereof. This description is included solely for the purposes of exemplifying the present invention. It should not be understood as a restriction on the broad summary, disclosure or description of the invention as set out above. The description will be made with reference to the accompanying drawings in which:

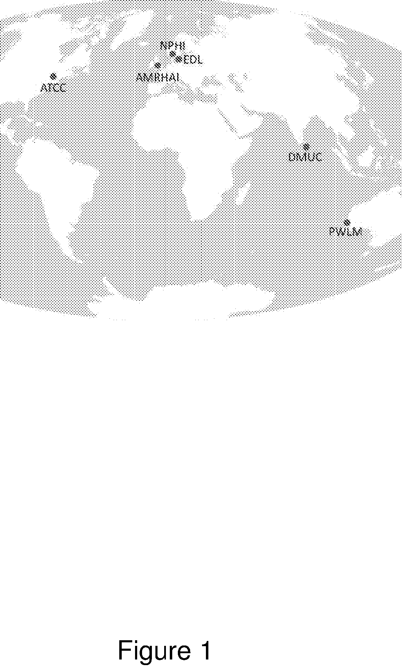

**Figure 1** is a pictorial representation showing an international collection of a panel of *Klebsiella* spp. isolates assembled in the work leading to the present invention. "ATCC" denotes isolates obtained from the American Type Culture Collection; "AMRHAI" denotes isolates obtained from the Antimicrobial Resistance and Hospital Associated Infection laboratory; "NPHI" denotes isolates obtained from the Norwegian Public Health Institute; "EDL" denotes isolates obtained from the EUCAST Development Laboratory; "DMUC" denotes isolates obtained from the Department of Microbiology, University of Colombo; and "PWLM" denotes isolates obtained from the PathWest laboratory Medicine.

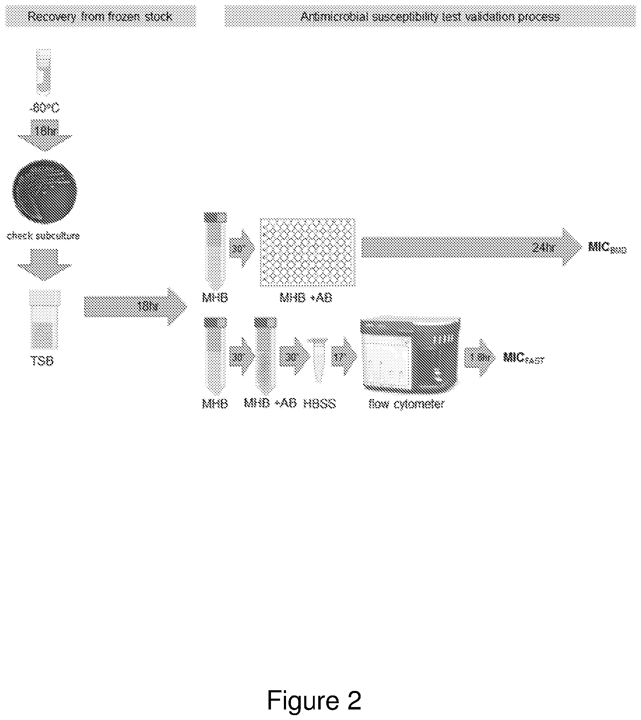

**Figure 2** is a schematic diagram of acoustic flow cytometry-assisted susceptibility test method validation.

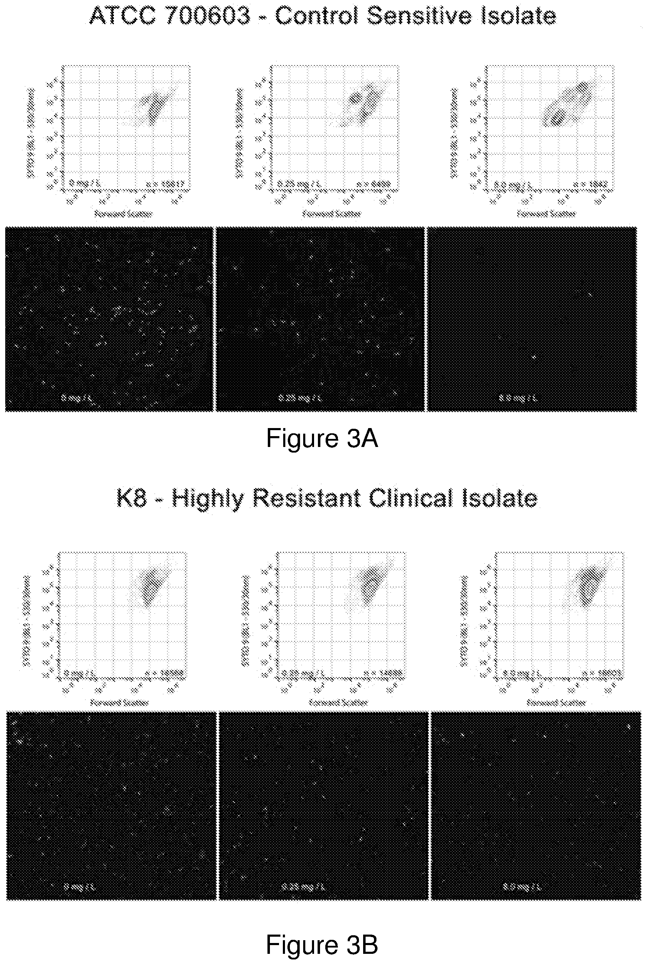

**Figure 3** is graphical (biaxial AFC plot) representation demonstrating that susceptibility to meropenem can be identified by AFC by observing a susceptibility-associated signature (SAS). **Panel (A)** shows that exposure of the *K. pneumoniae* susceptible type strain (ATCC 700603) produced an increase in forward scatter, SYTO 9 fluorescence, reduced overall event numbers, and formed a new contouring focus at the isolate's MIC (0.25 mg/L). At 32 times the MIC, a total of four contouring foci were observed, with an overall shift towards low forward scatter, low fluorescent debris. The progression of these features, when observed in combination, constitutes the susceptibility-associated signature. Colouring on biaxial plots indicates separate clustering foci. Accompanying the biaxial AFC plots are fluorescence micrographs (acquired at 60x magnification) showing reduced overall cell numbers and increased aberrant cell morphotypes as meropenem concentration increases. **Panel (B)** demonstrates that exposure of highly resistant clinical *K. pneumoniae* strain K8 to meropenem shows an absence of susceptibility-associated signature across clinically relevant meropenem concentrations by AFC bi-axial plot, and an absence of aberrant cell morphotypes by fluorescence microscopy.

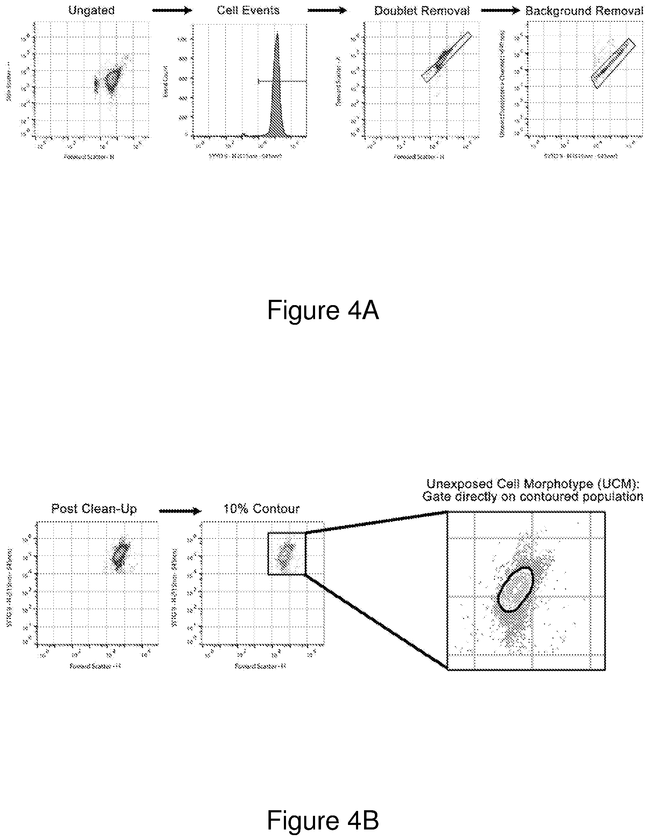

**Figure 4** is a graphical (biaxial AFC plot) representation demonstrating a standardised gating strategy applied to raw data of bacterial events to *inter alia* produce a consistent output of qualitative and/or quantitative susceptibility measurements by the method of the present invention. The strategy defines a rational gate of unexposed cell morphotype (UCM) of the test microorganism that bounds all contoured bacteria events on a bivariate contour plot of FSC-H vs BL1-H at the 10% threshold. The defined UCM gate on the bivariate plot can then be applied consistently to all samples across the antimicrobial agent dilution series which assist in FAST MIC measurement or determination. **Panel (A)** shows that collected events were gated to includes only those events with a SYTO 9 (BL1 - 530/30nm) fluorescence of 10⁴ arbitrary fluorescence units or higher. Doublets were removed via a FSC-A vs FSC-H plot, and background was removed by plotting specific SYTO 9 fluorescence (BL1 - 530/30) against an unused channel (BL3 - 640 LP). **Panel (B)** shows that in the antibiotic unexposed sample, 10% nearest-neighbour contouring was applied, and a gate (referred to as Unexposed Cell Morphotype) was set to include all clustered events. This gate was then applied to all samples across the antibiotic dilution series.

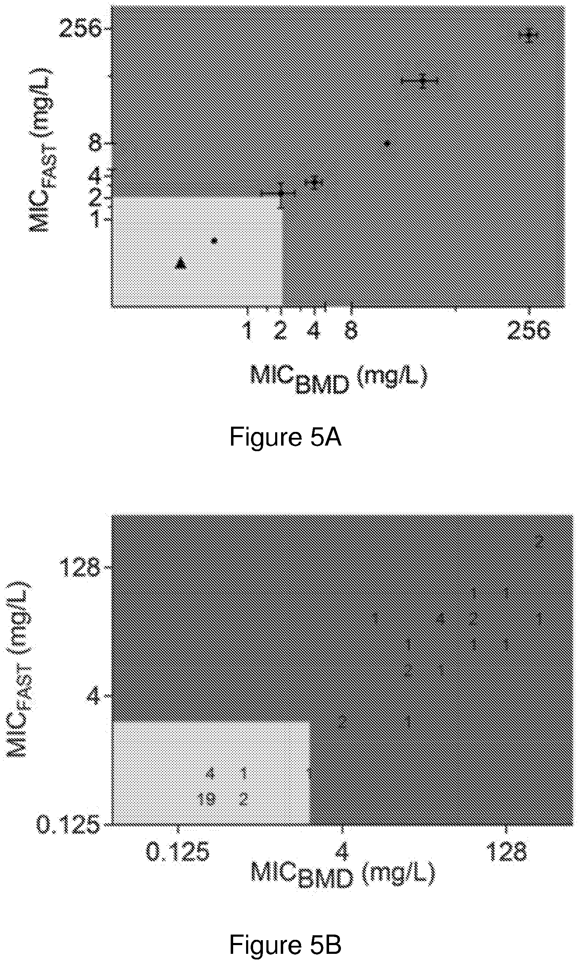

**Figure 5** is a graphical representation shows that acoustic flow cytometry-assisted susceptibility test method of the invention accurately predicted MIC_{BMD} across the collection of *K*. *pneumoniae* isolates. Coloured (differently shaded) regions represent the qualitative susceptible (green or light shaded)/non-susceptible (red or dark shaded) determination. **Panel (A)** shows qualitative susceptible / non-susceptible determination across the initial 10 isolates tested (shown as mean and error bars represent SEM of biological replicates), a strong positive correlation was observed (r = 0.899, p < 0.0001), ▲ represents four isolates, all with perfectly concordant MIC_{BMD} and MIC_{FAST} coordinates. **Panel (B)** shows qualitative susceptible / non-susceptible determination across the full collection of 48 isolates. Numerals indicate the numbers of isolates occupying the same MIC_{BMD}, MIC_{FAST} coordinates. Across the full collection of isolates, a strong positive correlation was observed (Matthew's correlation co-efficient = 0.918).

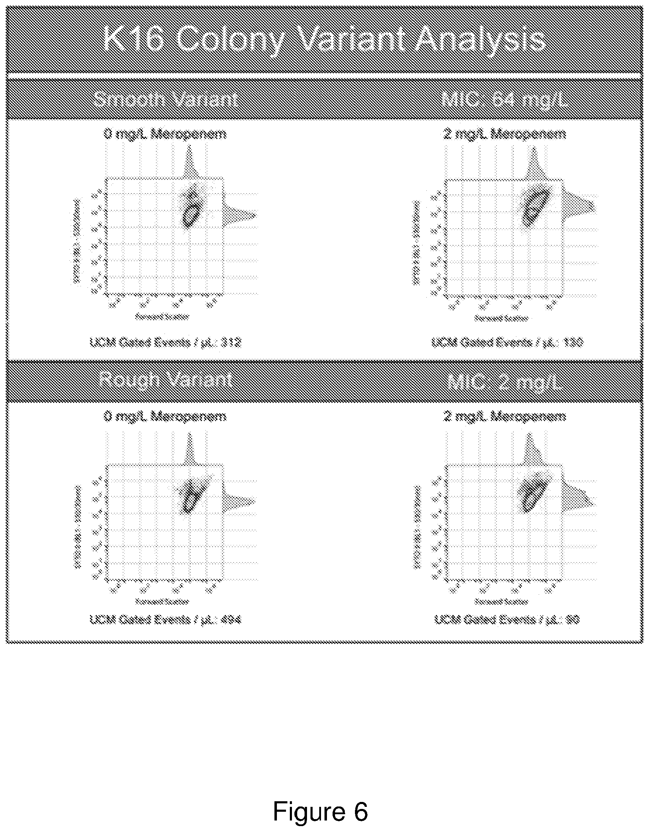

**Figure 6** is a graphical (biaxial AFC plot) representation showing differences in MIC_{FAST} that were observed between colony variants of *K. pneumoniae* isolate K16. When subculturing IMP-4 producing *K. pneumoniae* isolate K16, a rough and smooth colony variant was observed. Each was subcultured onto 5% horse blood agar and subjected to acoustic flow cytometry-assisted susceptibility test independently. The smooth colony variant produced an MIC_{FAST} of 2 mg/L. The rough colony produced an MIC_{FAST} of 64 mg/L and, at 2 mg/L, was observed by AFC to contain a population with consistent with a non-susceptible phenotype. Both MIC_{FAST} results were concordant (i.e., within the resolution of the BMD assay) with the MIC_{BMD} results.

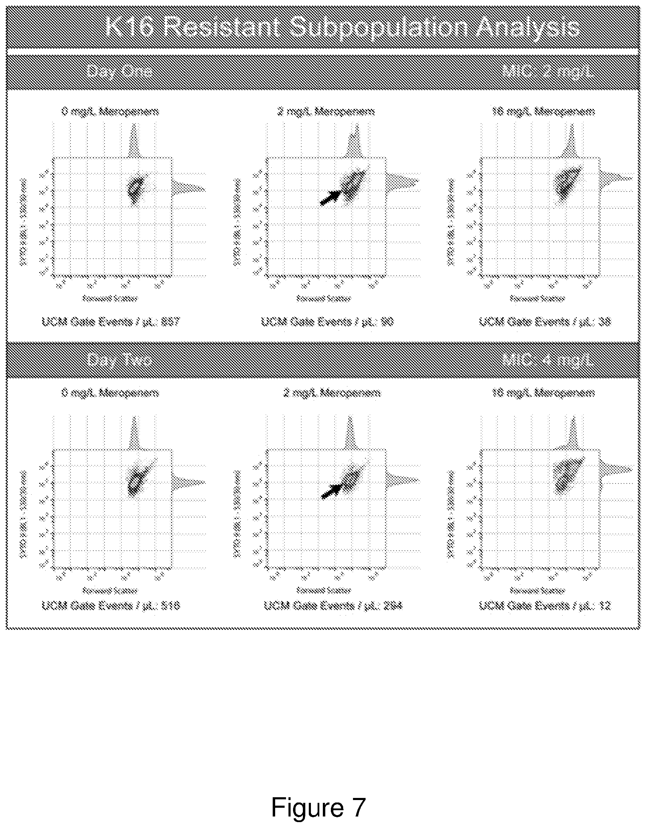

**Figure 7** is a graphical (biaxial bi-axial FSC/BL1 AFC plot) representation demonstrating that resistant sub-populations were observed in *K. pneumoniae* isolate K16 across two days of selective passage and acoustic flow cytometry-assisted susceptibility test. **Day one** - IMP-4 producing *K. pneumoniae* isolate K16 was found, at 2 mg/L meropenem, to contain a minority population of cells with a phenotype consistent with unexposed cells (remaining within the Unexposed Cell Morphotype gate - indicated by arrow). This subpopulation persisted, at a diminished frequency, at 16 mg/L meropenem while the majority of cells display a compromised phenotype (shifted outside the gate). **Day 2** - The 2mg/L culture of K16 from Day 1 was subcultured and subjected to acoustic flow cytometry-assisted susceptibility test on the following day. The isolate displaying an increased MIC (4 mg/L) delayed progression to the emergence susceptibility-associated signature, with most events consistent with a non-susceptibility phenotype at 2 mg/L. Most events at 16 mg/L were consistent with a susceptible phenotype, however a small subpopulation remained inside the Unexposed Cell Morphotype gate.

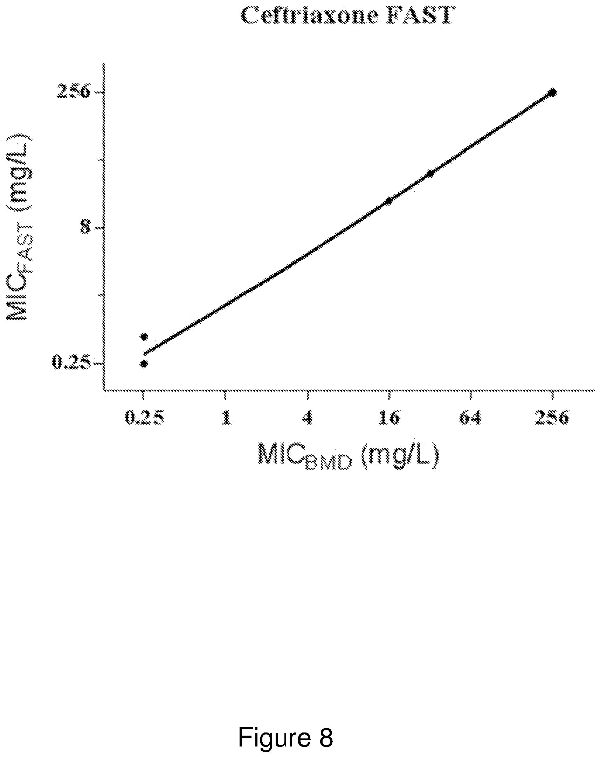

**Figure 8** is a graphical representation showing correlation of results susceptibility and MIC values determined using the acoustic flow cytometry assisted method of the present invention (MIC_{FAST}) and MIC_{BMD} for ceftriaxone (CRO) antibiotic across collection of 14 *K. pneumoniae* isolates. The acoustic flow cytometry-assisted susceptibility test method of the invention accurately predicted MIC_{BMD} across the collection of 14 *K. pneumoniae* isolates for ceftriaxone. Fourteen *K. pneumoniae* isolates from Table 2 (including the 10 isolates shown in Table 1) were exposed to ceftriaxone at different concentrations (as per the acoustic flow cytometry assisted method of the present invention) and corresponding MIC_{BMD} and MIC_{FAST} results were correlated as shown. Regression line y = 0.9997± 0.0001488 x + 0.06866 ± 0.03223, R² = 1. Data point at 256 represents 10 isolates.

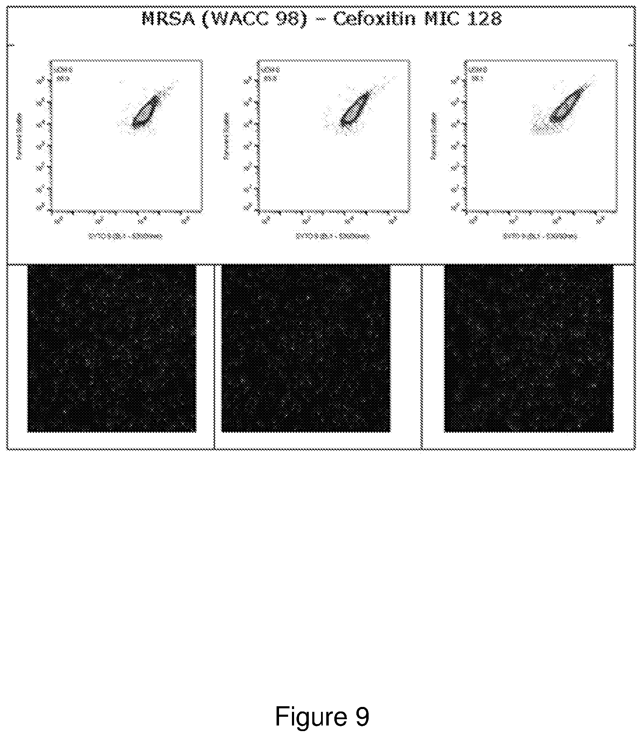

**Figure 9** is graphical (biaxial AFC plot) representation demonstrating that susceptibility to cefoxitin of the *Staphylococcus aureus* stain WACC 98 can be identified by AFC by observing a susceptibility-associated signature at MIC dilution 128 (Panel A), and corresponding cell morphology by fluorescence microscopy (Panel B).

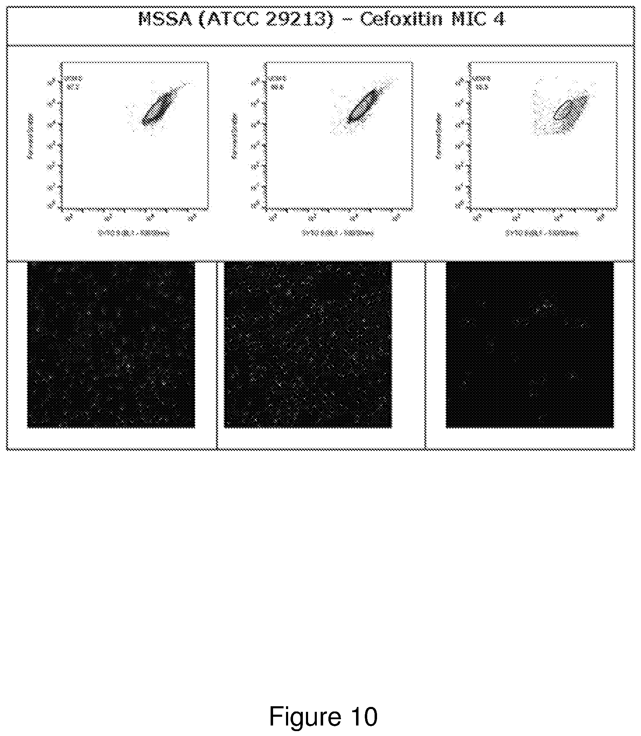

**Figure 10** is graphical (biaxial AFC plot) representation demonstrating that susceptibility to cefoxitin of the *Staphylococcus aureus* stain ATCC 29213 can be identified by AFC by observing a susceptibility-associated signature at MIC dilution 4 (Panel A), and corresponding cell morphology by fluorescence microscopy (Panel B).

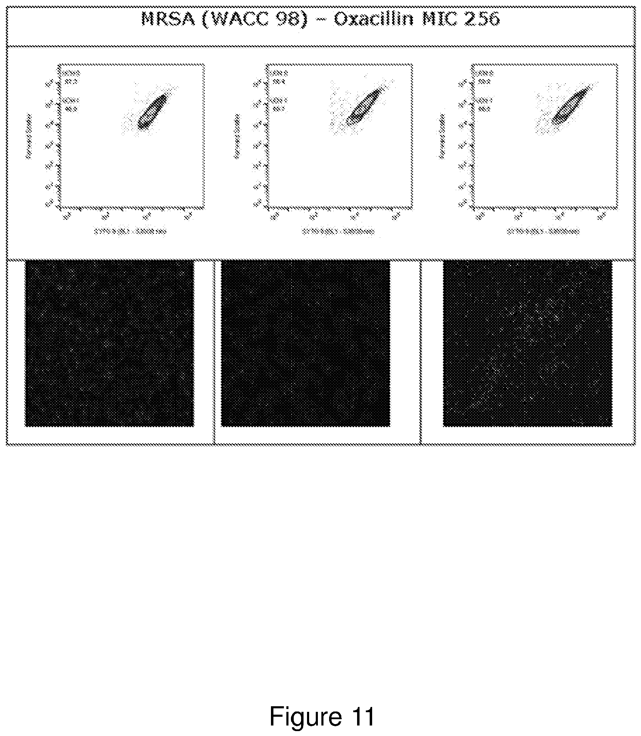

**Figure 11** is graphical (biaxial AFC plot) representation demonstrating that susceptibility to oxacillin of the *Staphylococcus aureus* stain WACC 98 can be identified by AFC by observing a susceptibility-associated signature at MIC dilution 256 (Panel A), and corresponding cell morphology by fluorescence microscopy (Panel B).

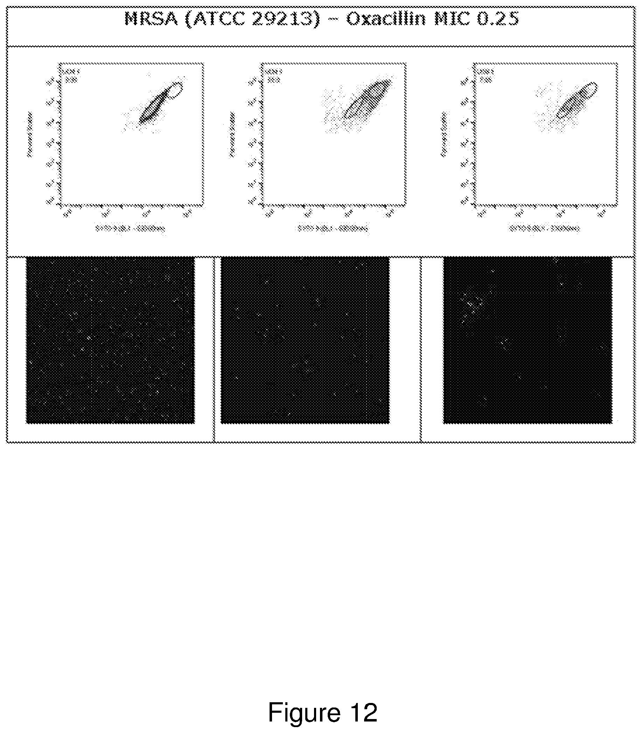

**Figure 12** is graphical (biaxial AFC plot) representation demonstrating that susceptibility to oxacillin of the *Staphylococcus aureus* stain ATCC 29213 can be identified by AFC by observing a susceptibility-associated signature at MIC dilution 0.25 (Panel A), and corresponding cell morphology by fluorescence microscopy (Panel B).

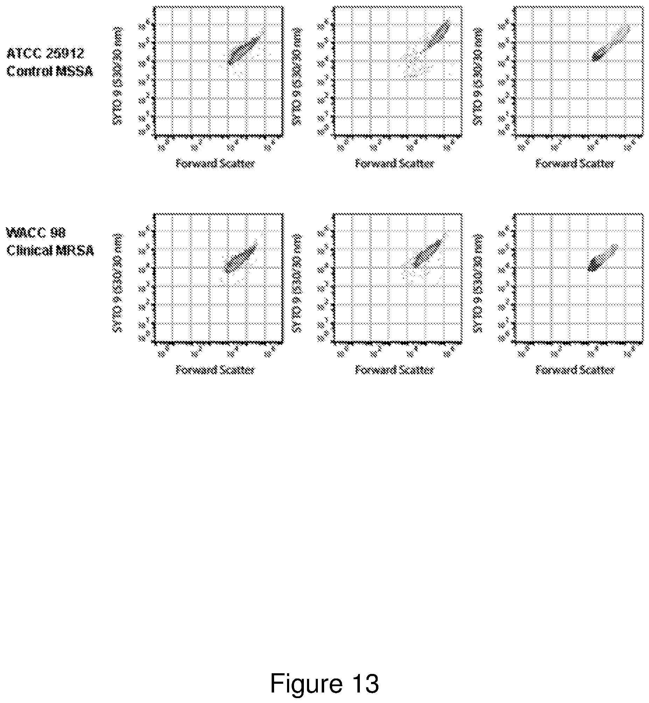

**Figure 13** is graphical representation showing induction of resistance by oxacillin in clinical methicillin resistant *S*. *aureus* (MRSA) WACC 98 isolate compared with control methicillin sensitive *S*. *aureus* (MSSA) isolate ATCC 25912; bi-axial flow cytometry plots of forward scatter vs SYTO 9 fluorescence at 0 mg/L oxacillin (left) and 8 mg/L oxacillin (center). Using 10% nearest neighbour contour plots, the two isolates are compared in the final plot (right) - blue population is ATCC 25912, gold population is WACC 98.

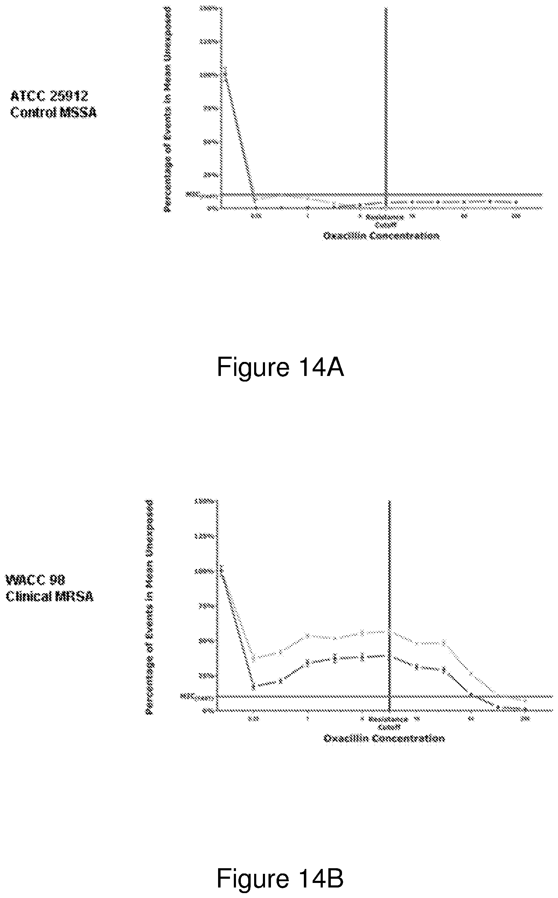

**Figure 14** provides graphical representations showing MIC_{FAST} results obtained by the AFC-assisted susceptibility testing method of the invention for control methicillin sensitive *S. aureus* (MSSA) isolate ATCC 25912 (panel A) and clinical methicillin resistant *S. aureus* (MRSA) WACC 98 isolate (panel B) following exposure to varying concentrations of oxacillin. In brief, this figure shows comparisons of events remaining in Unexposed Cell Morphotype Gate over a concentration range of oxacillin. A) Control methicillin susceptible S. aureus (ATCC 25912) shows a near complete reduction of events by 0.25 mg/L oxacillin, and remains below the 10% MIC_{FAST} threshold. Blue data points represent those uncorrected for oxacillin induction of resistance, gold represent post correction. B) Clinical methicillin resistant S. aureus (WACC 98) shows an immediate reduction in events at 0.25 mg/L of oxacillin, but remains high across the dilution series and above the 10% threshold for MIC_{FAST} at the EUCAST breakpoint for non-susceptibility (8 mg/L - indicated by the red line). Blue data points represent those uncorrected for oxacillin induction of resistance, gold represent post correction. In particular, notice the 128 mg/L data points - without correction, the event numbers drop below the MIC_{FAST} threshold, without they remain above (an accurate representation of biology).

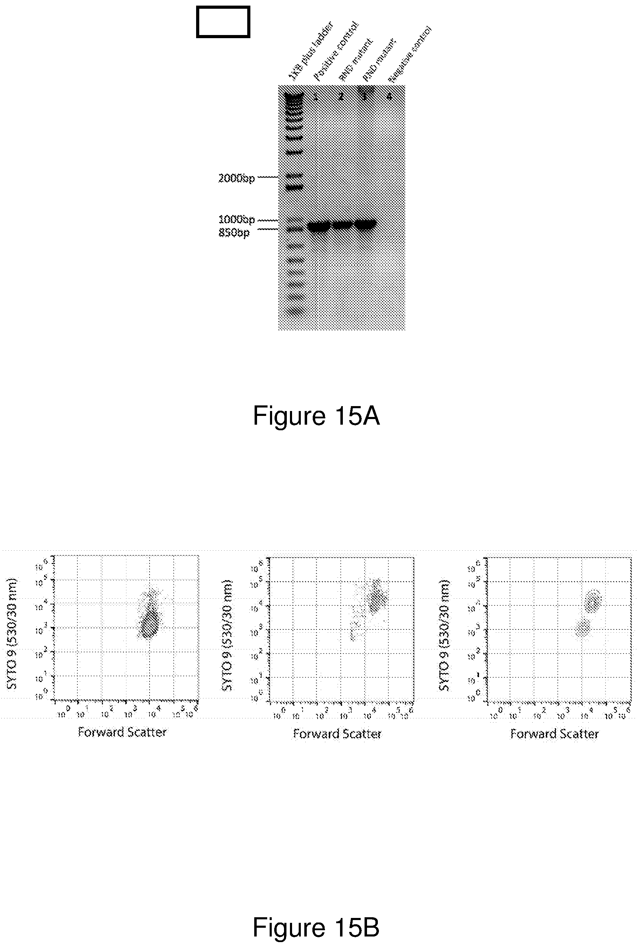

**Figure 15** provides a pictorial (panel A) and graphical (panel B) representations demonstrating applicability of the AFC-assisted susceptibility testing method of this invention for studying an efflux system (RND) mutant of *B*. *thailandensis* constructed as a surrogate model for *B. pseudomallei.* The RND efflux pump was deleted by allelic exchange to generate a *B.thailandensis ΔRND* mutant. Panel B bi-axial FSC/BL1 AFC plots show exposure of *B.thailandensis* to meropenem assayed by FAST: reference wildtype isolates of *B*. *thailandensis* was assayed to determine MIC_{FAST}. Samples across the dilution series displayed a susceptibility associated signature consistent with those observed in *Klebsiella pneumoniae* exposed to meropenem, with an increase in forward scatter and SYTO^{®} 9 fluorescence, and a decrease in overall cell numbers as the isolate approached MIC_{BMD}. MIC_{FAST} for *B*. *thailandensis* used the identical post-processing algorithms as for *K. pneumoniae.*

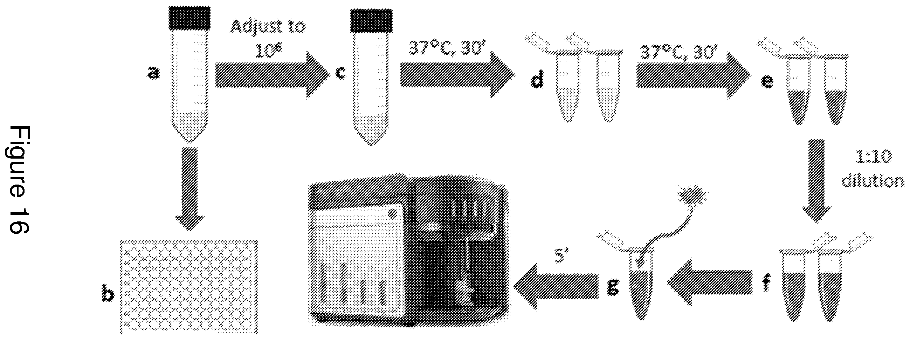

**Figure 16** provides a pictorial representation of TZP FAST work flow. Overnight cultures (a) were adjusted to cell density of 10⁶ cells/ml (c) and incubated. They were then exposed to TZP (d). Cells were centrifuged and re-suspended in HBSS (e). Samples were diluted (f) and stained with SYTO^{®}9 (g) before flow analysis. Each experiment was internally controlled by broth microdilution (b).

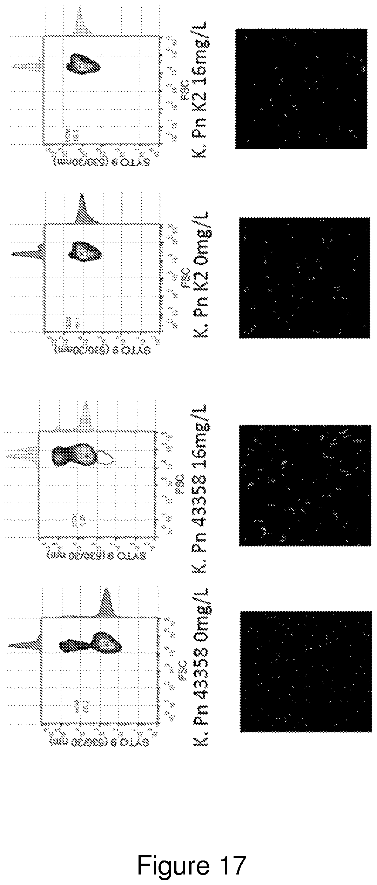

**Figure 17** provides a graphical (top panels) and pictorial (bottom panels) representation of comparison of TZP induced changes by forward scatter (top panels) and 540/40 nm fluorescence intensity (bottom panels) between a TZP susceptible *Klebsiella pneumoniae* isolate (43358) and a *Klebsiella pneumoniae* isolate resistant isolate (K2). Gross morphological change could be observed with epifluorescent microscopy in the TZP exposed susceptible isolates (false colour composite).

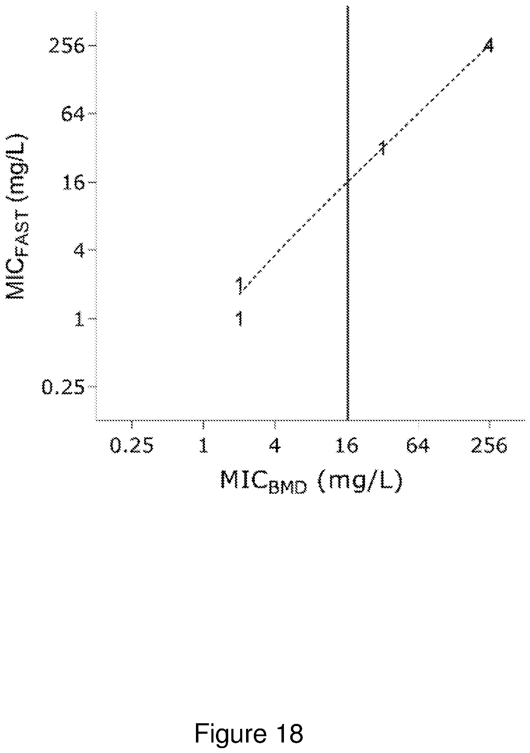

**Figure 18** provides a graphical representation showing a regression scatter for MIC_{FAST} and MIC_{BMD} results. The vertical (red) line indicates the EUCAST break point for *Enterobacteriaceae.*

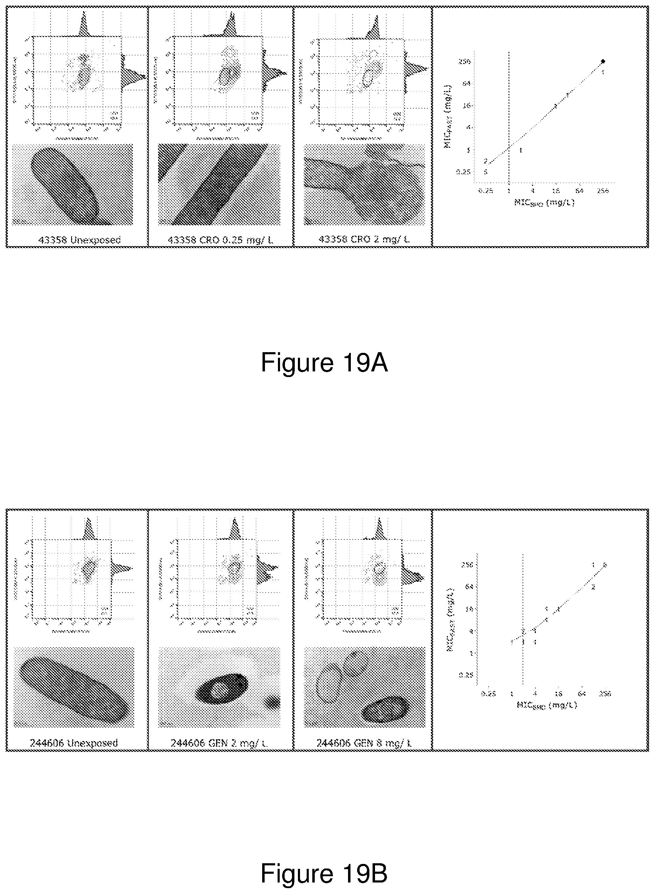

**Figure 19** provides graphical and pictorial representations showing rapid determination of susceptibly of bacteria to antimicrobial using the using the AFC-assisted susceptibility testing method of the invention. Panel A demonstrates that susceptibility to ceftriaxone can be identified by AFC by observing a susceptibility-associated signature. Exposure of the *K. pneumoniae* susceptible type strain (SSCC 43358) increased forward scatter, SYTO 9 fluorescence, reduced overall event numbers, and formed a new contouring focus at the isolate's MIC (0.25 mg/L) which further increased at 2 mg/L ceftriaxone. These changes were accompanied, evidenced with demonstrative transmission electron micrographs, with elongation of cells, distension of membrane and cell wall structures, and eventual cell lysis. Panel B demonstrates that susceptibility to gentamicin can be identified by AFC by observing a susceptibility-associated signature. Exposure of the *K. pneumoniae* susceptible type strain (isolate 244606) increased SYTO 9 fluorescence and formed a new contouring focus above the isolate's MIC (1 mg/L). These changes were accompanied, evidenced with demonstrative transmission electron micrographs, with increased cytoplasmic disarray, and vacuolation within the cytoplasm of the cells.

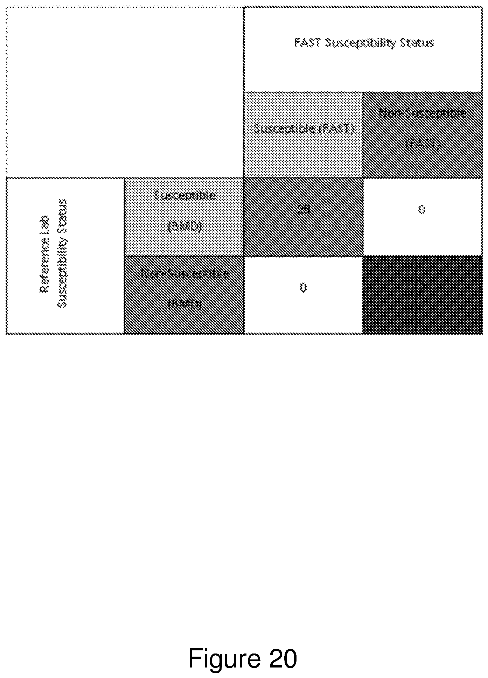

**Figure 20** provides a comparison of FAST and reference laboratory susceptibility category results for 10 isolates against 3 antibiotics (ceftriaxone, gentamicin, meropenem; 30 AST tests) showing perfect concordance of MIC_{FAST} and MIC_{BMD} susceptibility results obtained.

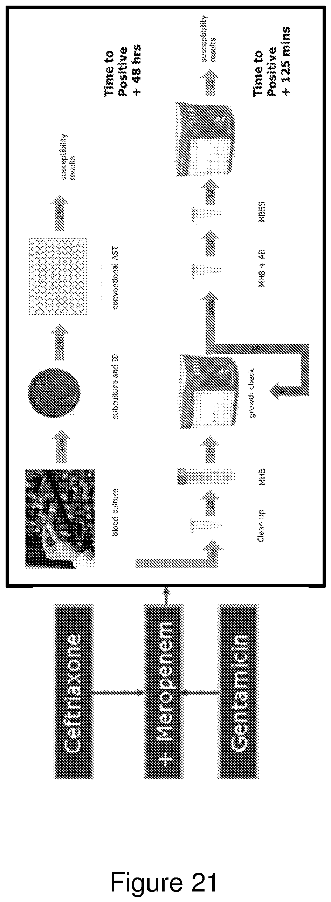

**Figure 21** provides a pictorial representation of FAST work flow for testing susceptibility of unidentified microorganisms directly from blood cultures, and comparing to the work flow of testing susceptibility of unidentified microorganisms directly from blood cultures using conventional methods such as micro broth dilution. As indicated positive susceptibility results may be obtained using the FAST method very rapidly within 125 minutes compared with more than 48 hours using known conventional techniques. Providing a time saving as high as 46 hours. In brief, positive blood culture bottles were retrieved once pathology services indicated a positive result, and an aliquot taken. Samples were processed in a clean-up protocol and inoculated into Mueller-Hinton broth (MHB) and incubated at 37 °C to ensure bacteria were actively dividing. Once active division was confirmed, one aliquot was subjected to traditional microbroth dilution to ascertain MIC_{BMD}. Another aliquot was exposed to meropenem, ceftriaxone or gentamicin for 30 minutes, harvested, stained with SYTO 9, and then assayed by AFC to ascertain MIC_{FAST}.

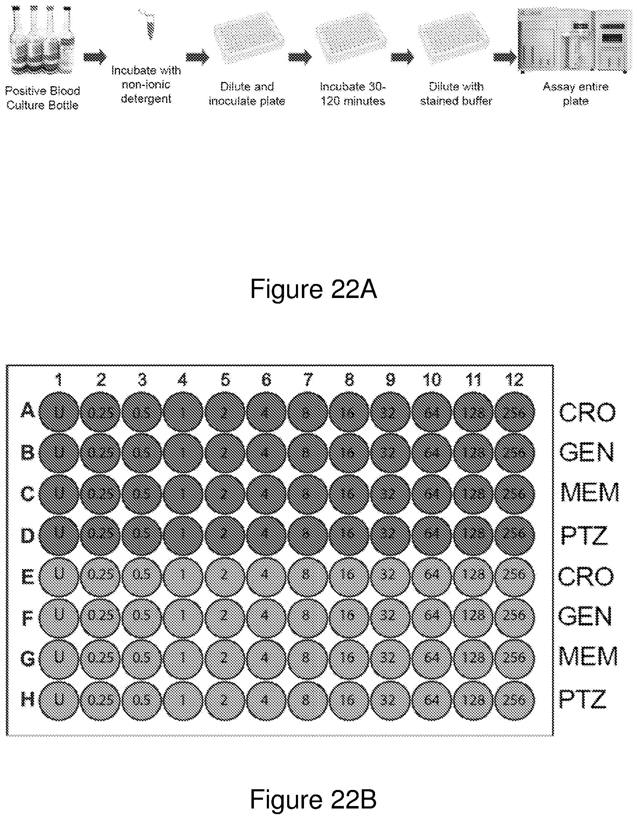

**Figure 22** provides a schematic and pictorial representation of the FAST method of the invention performed in a high throughput manner. Panel A) provides a schematic representation of an exemplary high-thought put process assaying simultaneously 96 test samples in a 96 well palate/tray. Two separate positive blood cultures collected in a bottle from separate subject having or suspect of having microbial infection was sampled, diluted with a nonionic detergent solution (0.001% Triton-X), and inoculated into a 96-well tray containing four antibiotics (CRO - ceftriaxone, GEN - gentamicin, MEM - meropenem, and PTZ - piperacillin-tazobactam) which were serially diluted to concentrations of 0.25 mg/L, 0.5 mg/L, 1.0 mg/L, 2.0 mg/L, 4.0 mg/L, 8.0 mg/L, 16.0 mg/L, 32.0 mg/L, 64.0 mg/L, 128.0 mg/L, 256.0 mg/L.the suspensions containing the microbial (in this case bacterial) cells from each blood sample and the antibiotics were incubated for 30-120 minutes, diluted with pre-SYTO 9 stained Hank's Balanced Salt Solution, and analysed with an acoustic flow cytometer equipped with a plate reader. Panel B) shown exemplary 96 well plate comprising the bacterial cells which were incubated in the above serial dilutions of the 4 antibiotics. Samples containing bacterial cells obtained from one positive-blood culture are shown in dark-shaded circles, and samples containing bacterial cells obtained from the other positive-blood culture are shown in light-shaded circles. By using full ranges of dilutions for the 4 antibiotics (CRO, GEN, MEM, and PTZ) four MIC FAST results (one MIC result for each antibiotic) for the 2 bacterial clinical isolates tested simultaneously. All MIC FAST results were returned in an average of approximately 2 to 3 hours from initial blood culture positive flag.

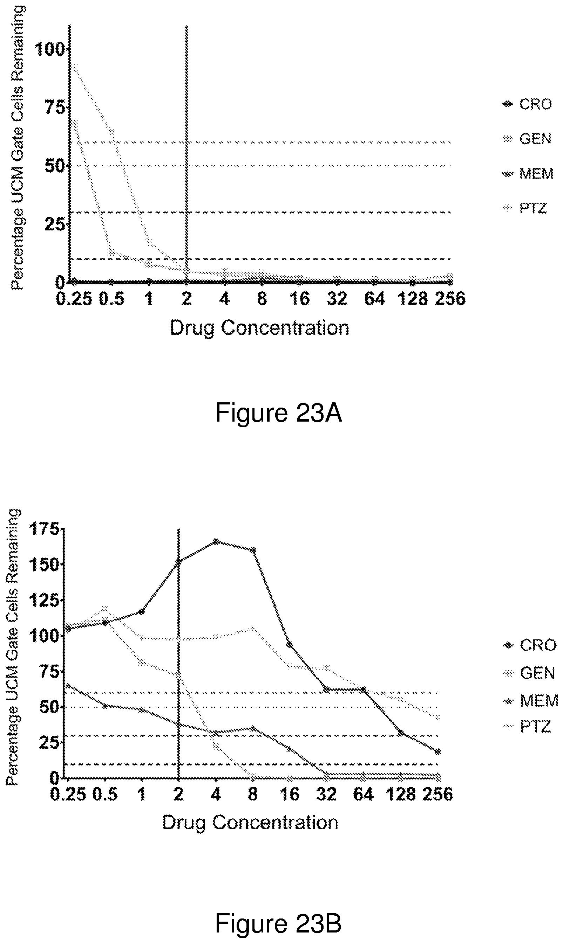

**Figure 23** provides a graphical representation showing that following the high throughput FAST blood culture assays e.g., with 4 antimicrobial agents (CRO - ceftriaxone, GEN - gentamicin, MEM - meropenem, and PTZ - piperacillin-tazobactam), numbers of cells per microliter that fall within the UCM gated region in each series can be plotted as a percentage of those found in the unexposed control. Panel A) shows a representation for a microbial isolate that previously been shown to be susceptible to all tested antimicrobials - it demonstrates a rapid drop in numbers at the lowest concentrations, with all antimicrobial agents falling below the thresholds of susceptibility (shaded matched, dashed lines) before reaching a concentration associated with a resistant phenotype (the vertical line). In a multiple-drug resistant isolate (panel B) this drop does not occur until concentrations of antimicrobial agents are above the concentration associated with a resistant phenotype (vertical line).

### DETAILED DESCRIPTION OF THE PREFERED EMBODIMENTS

### General

The invention is defined in the appended claims.

The invention described herein may include one or more range of values (e.g. size, displacement and field strength). A range of values will be understood to include all values within the range, including the values defining the range, and values adjacent to the range which lead to the same or substantially the same outcome as the values immediately adjacent to that value which defines the boundary to the range.

Definitions for selected terms used herein may be found within the detailed description of the invention and apply throughout. Unless otherwise defined, all other scientific and technical terms used herein have the same meaning as commonly understood to one of ordinary skill in the art to which the invention belongs.

Each example, embodiment and aspect described herein is to be applied *mutatis mutandis* to each and every other example, embodiment and aspect unless specifically stated otherwise.

### Definitions

Throughout the specification and claims, unless the context requires otherwise, the word "comprise" or variations such as "comprising" will be understood to imply the inclusion of a stated step or element or integer or group of steps or elements or integers but not the exclusion of any other step or element or integer or group of steps or elements or integers.

Throughout this specification, unless stated otherwise or the context requires otherwise, reference to a single step, composition or matter, group of steps or group of compositions of matter shall be taken to encompass one and a plurality (i.e., one or more) or those steps, compositions or matter, group of steps or group of compositions of matter. It must be noted that, as used herein and in the appended claims, the singular forms "a," "or," and "the" include plural references unless the context clearly dictates otherwise. Thus, for example, reference to "antimicrobial agent" or "microorganism" includes a plurality of such antimicrobial agents or microorganisms, and so forth.

As used herein throughout this specification the term "derivative" or "derived from" shall be taken to indicate that a specified integer may be obtained from a particular source albeit not necessarily from that source.

The term "acoustic flow cytometry" as used herein is intended to encompass any flow cytometry method or technique for interrogation of cells of microorganism such as bacterial cells, yeast cells and/or fungal cells *e.g.,* single cells of the microorganism, in an acoustically focused flowing suspension past a light source with photomultiplier tubes with photomultiplier tubes accepting transmitted and emitted light from each cell as it passes the light source. The acoustic flow cytometry method or technique encompassed the present invention preferably employ a focusing device (for example, but not limited to, a piezoelectric focusing device) to generate an acoustic standing wave that directs and aligns cells following past the light source. It will be understood that acoustic flow cytometry methods or techniques encompassed by the present invention are different and distinct from known hydrodynamic flow cytometry methods or techniques *inter alia* in the way a flowing suspension of the cells that flows passed the light source in the flow cytometer is compressed into a stream for interrogation at the light source. For example, conventional hydrodynamic flow cytometry methods or techniques generally employ a pressurized vortex of fluid to align cells into a single stream. It will be understood by those of skill in the art that generating a hydrodynamically pressurized vortex of fluid to direct and align cells is conceptually entirely different to an acoustic standing wave that directs and aligns cells following past the light source. Accordingly, it will be understood that the acoustic flow cytometry methods or techniques of the present invention and the acoustic flow cytometer devices encompassed by the present invention exclude conventional hydrodynamic flow cytometry techniques and devices employing hydrodynamic pressurized focusing of the cells of microorganism. It will be understood that the acoustic flow cytometry methods or techniques of the present invention and the acoustic flow cytometer devices encompassed by the present invention are those flow cytometry methods and devices which, among other things, involve interrogation of cells of microorganisms in an acoustically focused flowing suspension past a light source by generating (e.g., using a piezoelectric focusing device) an acoustic standing wave to direct and align the cells.

Preferably, the acoustic flow cytometer employed in the methods of the present invention is optimised for resolution of small particles, and/or comprises a laser and detector installed capable of measuring the fluorescent dye being used in the method, and/or be capable of resolving particles of about 800 nm and larger, and/or capable of accurate volumetric delivery or, in other words, be capable of accurately measuring the dispensed volume of a cell sample being analyzed. For example, the acoustic flow cytometer employed may be Attune acoustic flow cytometer (e.g., Thermo Fisher Scientific) or Attune NxT acoustic flow cytometer (e.g., Thermo Fisher Scientific). In examples, where the nucleic acid binding fluorescent compound used is SYTO 9 dye, the acoustic flow cytometer preferably may comprise a 488nm blue laser with a 530/30 nm filter on the photomultiplier tube for measuring fluorescence of cells assayed.

As used herein, the term "sample comprising cells of a microorganism" in the context of the present invention as far as it relates to a sample comprising cells of a microorganism obtained from a subject having or suspected of having an infection with a microorganism, is intended to encompass any clinical or biological sample obtained directly or indirectly from a subject such as a human having or suspect of having an infection with a microorganism. It will also be understood that the term "sample" as used herein also encompasses suspensions made from primary isolates including both pure and/or mixed cultures obtained directly or indirectly from a biological or clinical specimen of the subject. The sample may be sterile or non-sterile and may be obtained or derived from a sterile or non-sterile site in the subject. For example, a sample comprising cells of a microorganism obtained from a subject may be any clinical or biological sample comprising cells of a microorganism, may be selected from a throat swab sample, a sputum sample urine sample, blood sample, a joint fluid sample, a continuous ambulatory peritoneal dialysis (CAPD) fluid sample, a buccal swab sample, a tissue biopsy, a surgical drain fluid sample, a stool sample, a cerebrospinal fluid sample, ascitic fluid sample, pleural fluid sample, a blood sample such as blood plasma and/or blood serum sample, a wound swab, or wound mucus sample, a nasal swab sample, a rectal swab sample, a urethral swab sample, a skin sample or skin swab sample, a genital swab sample, pus sample, saliva sample, rheum sample, serous fluid sample, vaginal secretion sample, vomit sample, sweat, tears, mucus sample, pericardial fluid sample, peritoneal fluid sample, sebum sample, smegma sample, gastric juice sample, a bile sample, chyle sample, chyme sample, endolymph and /or perilymph fluid sample, lymph fluid sample, interstitial fluid sample, breast milk, perioperative surgical field swab sample, any sample or swab taken to determine presence of a multi-drug resistant organism, and any combination thereof. Alternatively or in addition, "sample comprising cells of a microorganism" in the context of the present invention also encompass food samples for tracking antimicrobial resistance in foods both uncooked and cocked or ready to eat with which the subject suspect having or suspected of having an infection with a microorganism, may have come in contact with.

As used herein, the term "antimicrobial agent" or "antimicrobial agents" is intended to encompass any agent, substance, compound or composition of matter having or capable of having an effect (whether directly or indirectly) on the growth and/or replication of a unicellular and/or multicellular microorganism such as bacteria, yeast and/or fungi. For example, the antimicrobial agent (s) encompassed within the scope of the present invention can act to inhibit growth and/or kill the microorganism preferably by any mode or mechanism of action. Accordingly, the antimicrobial agent(s) encompassed within the present invention may have biostatic and/or microbicide effect on the unicellular and/or multicellular microorganisms of the present invention. For example, the antimicrobial agent(s) of the present invention include bactericidal agents and/or bacteriostatic agents, such as antibiotic(s). It will be understood that the antimicrobial agent(s) of the present invention may not be limited by their manner of production or manufacture, and include agents, substances, compounds or composition of matters which are synthetic, chemically-produced, naturally occurring and/or derived whether directly or indirectly from an organism or animal preferably, living organism including microorganisms, plants and animals. For example, the antimicrobial agent may one or synthetic or naturally occurring compound(s) derived directly or indirectly from a bacteria, fungus, yeast, plant, insect, reptile or mammal. In one example, the antimicrobial agent may be any compound such as a chemical compound or a pharmaceutical composition such as a drug. In another example the antimicrobial agent is a composition comprising a compound such as a chemical compound or a pharmaceutical or a drug. In one such example the antimicrobial agent may be a composition comprising two or more compounds such as chemical compounds, pharmaceuticals and/or drugs. In another example, the antimicrobial agent may comprise or consist of a physical and/or chemical phenomenon or stress applied to the cells of the microorganisms described according to broad aspect, embodiment of example hereof. According to this example, the antimicrobial agent may comprise or consist of one or more physical and/or chemical phenomenon or stress applied to actively dividing cells of a microorganism selected from heat stress, ultraviolet light, blue light, radiation such as electromagnetic radiation, microwave radiation, radiation with X-rays or gamma rays, particle radiation such as alpha, beta or neurons particle radiation, and/or oxidative stress.

Non-limiting examples antimicrobial agent(s) encompassed within the scope of the present invention include amino-acids and amino acid derivatives, fatty acids and fatty acid derivatives, antimicrobial peptides and peptide derivatives, aminoglycosides, aureolic acids, aziridines, benzenoids, Benzimidazoles, coumarin-glycosides, diphenyl ether derivatives, epipolythiodioxopiperazines, glucosamines, glycopeptides, Imidazoles, indol derivatives, macrolactam, macrolides, nucleosides, beta-Lactams such as penicillins and cephalosporins, peptidyl nucleosides, phenicoles, polyenes, pyridines and pyrimidines, quinolones and fluoroquinolones, statins, steroids, sulfonamides, taxoides, tetracyclines, and metal alloys such as copper alloys.

It will also be understood that the antimicrobial agent(s) of the present invention may be formulated for administration to a subject in any form of administration, including but not limited to auricular, conjunctival, buccal, cutaneous, dental, electro-osmosis, endocervical, endosinusial, endotracheal, enteral, epidural, extra-amniotic, extracorporeal, hemodialysis, infiltration, interstitial, intra-abdominal, intra-amniotic, intra-arterial, intra-articular, intrabiliary, intrabronchial, intrabursal, intracardiac, intracartilaginous, intracaudal, intracavernous, intracavitary, intracerebral, intracisternal, intracisternal, intracisternal, intracorneal, intradermal, intradiscal, intraductal, intraduodenal, intradural, intraepidermal, epidermal, intraoesophageal, intragastric, intragingival, intraileal, intralesional, intraluminal, intralymphatic, intramedullary, intrameningeal, intramuscular, intraocular, intraovarian, intrapericardial, intrapleural, intraprostatic, intrapulmonary, intrasinal, intraspinal, intrasynovial, intratendinous, intratesticular, intrathecal, intrathoracic, intratubular, intratumour, intratympanic, intrauterine, intravascular, intravenous, intravenous bolus, by intravenous drip, intraventricular, intravesical, intravitreal, by iontophoresis, laryngeal, nasal, nasogastric, ophthalmic, oral, oropharyngeal, parenteral, percutaneous, periarticular, peridural, periodontal, rectal, inhalation, retrobulbar, subconjunctival subcutaneous, sublingual, submucosal, topical, transdermal, transmucosal, transplacental, transtracheal, transtympanic, ureteral, urethral, and vaginal.

As used herein the phrases "susceptibility of cells of the microorganism to antimicrobial agent(s)" or "susceptibility of the microorganism to antimicrobial agent(s)" or "sensitivity of a microorganism to antimicrobial agent(s)" or "sensitivity of a microorganism to antimicrobial agent(s)" will be understood to reflect the one or more antimicrobial agents to promote damage to, or compromise the integrity of, the cellular morphology of actively dividing cells of the microorganism when the actively dividing cells are exposed to, or cultured with an amount of the antimicrobial agent compared with the cellular morphology of actively dividing cells of the microorganism obtained from the subject when cultured or incubated in the absence of the antimicrobial agent. For example, the antimicrobial agent promotes damage to, or compromises the integrity of the cell wall of the microorganism which may ultimately lead to cells of the microorganism undergoing cell lysis and/or complete cell breakdown following exposure of actively dividing cells of the microorganism in or derived from a sample obtained from the subject to the antimicrobial agent. The "susceptibility of cells of the microorganism to the antimicrobial agent" or the "sensitivity of cells of the microorganism to the antimicrobial agent" can be assessed by measuring e.g., by acoustic flow cytometry the effect or degree of the effect of the antimicrobial agent on (i) the amount of nucleic acid content in, and/or (ii) cytoplasmic volume of, cells of the microorganism in or derived from the subject sample following co-culturing of actively dividing cells of the microorganism in and/or from the subject sample with an amount of the antimicrobial agent preferably before any cell lysis and/or complete cell breakdown takes place. The effect or degree of the effect of the antimicrobial agent on (i) the amount of nucleic acid content in, and/or (ii) cytoplasmic volume of, cells of the microorganism following exposure to the amount of the antimicrobial agent, can be determined by comparison to the (i) the amount of nucleic acid content in, and/or (ii) cytoplasmic volume of actively dividing cells of the microorganism when cultured in absence of the antimicrobial agent.

The term "nucleic acid binding fluorescent compound" as used herein is intended to encompass any florescent compound or composition of matter which is naturally freely cell membrane permeable or can be rendered prior to use in the present invention to be membrane permeable, and is capable of binding whether reversible or non-reversible to nucleic acids (such as RNA and DNA) in cells of microorganism assayed in the method of the present invention, including cytoplasmic and/or nuclear and/or mitochondrial nucleic in cells of the microorganism and be fluorescent within the cells of the microorganism when bound to cellular nucleic acids within the cells of the microorganism so as to enable detection of fluorescence signal or intensity by acoustic flow cytometry.

Suitable nucleic acid binding fluorescent compounds for use in the present invention will be known to the skilled artisan and include fluorescent nucleic acid binding dyes such as SYTO 9. Also, fluorescent nucleic acid binding dyes have been widely described in the art for example in Deere, D., Porter J., Edwards, C., Pickup, R. Evaluation of the suitability of bis-(1 ,3-dibutylbarbituric acid) trimethineoxonol, (diBA-C4(3)-), for the flow cytometric assessment of bacterial viability. FEMS Microbiol. Lett. 130, 165-9 (1995).

The term "freely permeable" when used herein in connection with a nucleic acid binding fluorescent compound of the present invention is intended to mean any nucleic acid fluorescent compound that is able to readily cross the cell membrane of the cells of the microorganism and enter the cells to bind cellular nucleic acids, substantially absent treatment of cells of the microorganism for the purpose of rendering the microorganism susceptible to permeability of the nucleic acid fluorescent compound through its cell membrane.

The term "microbial event " as used herein in the context of the present invention, will be understood to mean a packet of data measured by acoustic flow cytometry that was generated by a microbial (e.g., bacterial) cell. Specifically, it will be understood that when a particle such as microbial cell passes through the lasers of the cytometer, the photons collected from that event intersect a photomultipler tube, which converts the physical signal into an electrical signal, which software attached to the cytometer converts into digital data, which is registered as an "event". A distinction is made based on size parameters, and the presence of fluorescence parameters consistent with mcrobial nucleic acids bound to a nucleic acid binding fluorescent compound. These parameters may be established by measuring the microbial cell suspension before, and then after the nucleic acid binding fluorescent compound is added. It wil be understood that there will be a minimum of increase in arbitrary fluorescence units equal to the nucleic acid bound fluorescence enhancement of the dye used, in those events that contain nucleic acids such as DNA. For example, when using SYTO 9, events that contain nucleic acid such as DNA will be no less than 10 times brighter (in the 515-545nm channel) when the SYTO 9 is present compared to when it is not.

Similarly, the term "non-microbial debris" as used herein with reference of measuring events by acoustic flow cytometry, will be understood to mean a packet of data measured by acoustic flow cytometry that was generated by a microbial (e.g., bacterial) cell.

Other definitions for selected terms used herein may be found within the detailed description of the invention and apply throughout. Unless otherwise defined, all other scientific and technical terms used herein have the same meaning as commonly understood to one of ordinary skill in the art to which the invention belongs. The term "active agent" may mean one active agent, or may encompass two or more active agents.

### Specific

Applicants have developed a method for determining the susceptibility of microbial organisms to antimicrobial agents. In particular, the method is useful for determining the susceptibility of *Klebsiella* and other Gram negative bacteria to an antibacterial agent. In addition, the method is also useful in determining the Minimum Inhibitory Concentration (MIC) of bacteria to antimicrobial agents.

In the present invention, the applicants have developed an acoustic flow cytometry workflow to characterise population dynamics and measure the qualitative susceptibility to carbapenem antibiotics and to a cephalosporin antibiotic, and also measure the Minimum Inhibitory Concentration (MIC), in an internationally representative *K. pneumoniae* isolates collection. In addition, the applicants employed the acoustic flow cytometry workflow to characterise and measure the qualitative susceptibility to a penicillin antibiotic and to a cephalosporin antibiotic, and also to measure susceptibility quantitatively by measuring the MIC, in representative isolate collection in representative *Staphylococcus aureus* isolates collection. The acoustic flow cytometry-assisted susceptibility test (FAST) method of the present invention combines rapid qualitative S/NS categorization and quantitative MIC measurement in a test process that can be completed shortly after receipt of a primary isolate. The FAST qualitative susceptibility results and FAST-derived MIC correspond closely with broth microdilution MIC, are aligned to the ISO standard for AST (ISO 200776-1; 2006) and have potential applications for rapid determination of antimicrobial susceptibility in a wider range of microorganisms including wider range of Gram negative bacteria and/or Gram positive bacteria.

Accordingly, in one broad aspect of the present invention, there is provided a method of determining the susceptibility of a unicellular or multicellular microorganism to an antimicrobial agent by acoustic flow cytometry. The method comprises the following steps: (i) incubating in a culture medium (a) cells of the microorganism from a sample obtained from a subject having or suspected of having an infection with said microorganism, and/or (b) a sample obtained from the subject having or suspected of having an infection with said microorganism, wherein said sample comprises cells of said microorganism, wherein said incubating is for a time and under conditions sufficient for said cells to actively divide to thereby obtain an actively dividing culture of said cells of said microorganism; (ii) exposing cells of the microorganism in and/or from the actively dividing cell culture obtained in step (i) to an antimicrobial agent for a time and under conditions sufficient for said cells of the microorganism to actively divide; (iii) labelling cells of the microorganism exposed to the antimicrobial agent in step (ii) with a nucleic acid binding fluorescent compound; (iv) measuring effect of said antimicrobial agent on cellular morphology of said cells of the microorganism by acoustic flow cytometry; and (v) determining susceptibility of the microorganism to the antimicrobial agent from the acoustic flow cytometry data output, wherein step (v) comprises (A) determining susceptibility of the microorganism to the antimicrobial agent qualitatively by determining from the acoustic flow cytometry data output that said microorganism is either susceptible or not susceptible to said antimicrobial agent; and (B) determining susceptibility of the microorganism to the antimicrobial agent quantitatively by determining from the acoustic flow cytometry data output the minimal inhibitory concentration (MIC) of said antimicrobial agent to which the microorganism is susceptible.

For example, the method comprises obtaining a sample comprising cells of the microorganism from a subject having or suspected of having an infection with the microorganism.

In a second broad aspect of the present invention, there is provided a method of determining susceptibility of a unicellular or multicellular microorganism to a plurality of antimicrobial agents. The method comprises performing the method according to the first broad aspect wherein the method comprises exposing the actively dividing cells of the microorganism from the sample obtained from the subject to one or more representative antimicrobial agent(s) of a first family or panel of a plurality of antimicrobial agents, to thereby determine susceptibility of the cells of the microorganism to antimicrobial agents belonging to this first family or panel of antimicrobial agents.

In one embodiment of this second aspect, when the cells of the microorganism are determined to be susceptible to the one or more representative antimicrobial agent(s) of the first family or panel of the plurality of antimicrobial agents, the method may further comprise performing the method according to the first broad aspect by exposing the actively dividing cells of the microorganism to one or more additional antimicrobial agents belonging to the first family or panel of antimicrobial agents, to thereby determine susceptibility of the cells of the microorganism for the subject to said additional antimicrobial agents.

In a further embodiment, the method according to this second aspect may further comprise performing the method according to the first aspect, wherein the method comprises exposing the actively dividing cells of the microorganism to
(i) one or more representative antimicrobial agent(s) of a second family or panel of a plurality of antimicrobial agents, and/or
(ii) one or more representative antimicrobial agent(s) of a third family or panel of a plurality of antimicrobial agents, and/or
(iii) one or more representative antimicrobial agent(s) of a fourth family or panel of a plurality of antimicrobial agents, and/or
(iv) to one or more representative antimicrobial agent(s) of any additional further family or panel of a plurality of antimicrobial agents,
to thereby determine susceptibility of the cells of the microorganism to antimicrobial agents belonging to said second and/or third and/or fourth and/or any further additional family or panel of a plurality of antimicrobial agents.

In one example, when the cells of the microorganism are determined to be susceptible to the one or more representative antimicrobial agent(s) of the second and/or third and/or fourth and/or further additional family or panel of the plurality of antimicrobial agents, then said method further comprises performing the method according to the first broad aspect by exposing the actively dividing cells of the microorganism to one or more additional antimicrobial agents belonging to the second and/or third and/or fourth and/or any additional further family or panel of the plurality of antimicrobial agents, to thereby determine susceptibility of said cells of said microorganism to the additional antimicrobial agents.

One example of a method of according to the second aspect, comprises screening susceptibility to antimicrobial agents of Gram negative or Gram positive bacteria.

For example, screening susceptibility of bacteria to antimicrobial agents may be performed as broadly described in the example provided below e.g., in respect of screening susceptibility of Gram negative bacteria.

For example, antimicrobial chemotherapy ideally involves the selection of the most effective, narrow-spectrum antibiotic active against the microbe. These criteria may form the basis of guidelines classifying antimicrobial agents as either first-, second-, third-, and fourth-line. For example, first-line agents are the most narrow-spectrum with last-line agents preferably being reserved for difficult to treat isolates. The inventors have speculated that the emergence of antimicrobial resistance has made selecting antimicrobial treatments difficult. As a result, treatment often begins with second- or third-line agents. This may promotes increased resistance to second- and third-line agents, which may in turn promote use of fourth-line agents therefore promoting resistance to these in turn. A rapid panel indicating the effectiveness of early lines of antimicrobial agents is therefore suggested.

For example, the bulk of second- and third-line antimicrobial agents active against Gram-negative bacteria may belong to the extended-spectrum beta-lactam family (*e.g.,* ceftriaxone, ceftazidime, and cefepime). Ceftriaxone may be used in the laboratory as a screening agent to indicate the presence of resistance mechanisms which render all extended-spectrum beta-lactams ineffective (*e.g.,* as may be for Enterobacteriaceae). Ceftriaxone non-susceptibility *e.g.,* in Enterobacteriaceae may be determined when isolates are able grow in media containing *e.g.,* 2 mg/L of ceftriaxone. This growth may indicate the use of a carbapenem.

For example, carbapenem susceptibility may be tested by exposing isolates to meropenem at the suggested breakpoint (*e.g.,* 2 mg/L mentioned above or any other current EUCAST Enterobacteriaceae breakpoint). Where the isolates were resistant to a carbapenem, the addition of additional antimicrobial agents (e.g. colistin) may need to be considered. The panel may also test for colistin resistance *e.g.,* to enable a rapid selection of last-line agents.

In one such example, species, temporal, and location specific breakpoints may be used depending on the antimicrobial standard (EUCAST, CLSI, or other), subsequent revision of breakpoints, or organism detected.

For example, the above Gram negative susceptibility screening method may not be applicable to screening susceptibility of *Pseudomonas, Acinetobacter* species. In those cases, the antibiotic panel may need to incorporate an equivalent indicator beta-lactam like cefepime.

### Preferred concentration of actively dividing cells of a microorganism being exposed to an antimicrobial agent

In one example, the amount and/or type of the antimicrobial agent used for determining susceptibility of a microorganism may depend on the "inoculum effect" for that antimicrobial agent. As used herein the term "inoculum effect" will be understood to refer to the laboratory phenomenon whereby an increase in the minimal inhibitory concentration of an antimicrobial agent such as an antibiotic may be observed when the number of microorganisms exposed to the antimicrobial agent is increased. For example, in a laboratory environment the efficacy of the antimicrobial agent may depend on its final concentration in the test culture or suspension as a function of the total number or concertation of the microbial cells present in the test culture or suspension.

The method for determining susceptibility of a microorganism to an antimicrobial agent and/or determining the M IC of the microbial agent to which the microorganism is susceptible by acoustic flow cytometry according to any broad aspect described herein may be carried out using any concentration or amount of the actively dividing cells of a microorganism which are being exposed to an antimicrobial agent. Notwithstanding this, the present inventors have shown that, to account for the inoculum effect of the antimicrobial agent tested, it is preferred that actively dividing microbial cells which are exposed to the test antimicrobial agent in a method according to any broad aspect of the invention described herein are present at a concentration in the range of about 5.0 × 10¹ to about 5.0 × 10⁹ microbial cells / mL, and more preferably at a concentration of about 5.0 × 10² to about 5.0 × 10⁸ microbial cells / mL, and most preferably at a concentration of about 5.0 × 10³ to about 5.0 × 10⁷ microbial cells / mL. For example, the method according to any broad aspect of the invention described herein comprises exposing cells of the microorganism in and/or from the actively dividing cell culture to the antimicrobial agent, wherein the microbial cells being exposed to the antimicrobial agent are present at a concentration of about 5.0 × 10³ to about 5.0 × 10⁷ microbial cells / mL. In one example, the concentration of the actively dividing microbial cells being exposed to the antimicrobial agent is about 5.0 × 10⁴ to about 5.0 × 10⁷ microbial cells / mL, or about 5.0 × 10⁵ to about 5.0 × 10⁷ cells / mL, or about 5.0 × 10⁶ to about 5.0 × 10⁷ cells / mL. In one such example, the concentration of the actively dividing microbial cells being exposed to the antimicrobial agent is about 5.0 × 10³ cells / mL, or about 5.0 × 10⁴ cells / mL, or about 5.0 × 10⁵ cells / mL, or about 5.0 × 10⁶ cells / mL, or about 5.0 × 10⁷ cells / mL. In some exemplary embodiments the concentration of the actively dividing microbial cells being exposed to the antimicrobial agent is about 5.0 × 10⁵ cells / mL. It is desirable that the actively dividing microbial cells which are exposed to the test antimicrobial agent are present at the above mentioned concentrations for determining susceptibility of the a microorganism to an antimicrobial agent by acoustic flow cytometry using the method of any broad aspect described herein. Alternatively, or in addition, it is desirable that the actively dividing microbial cells which are exposed to the test antimicrobial agent are present at the above mentioned concentrations when determining the MIC of the microbial agent to which the microorganism has been determined to be susceptible. In some such examples, the microbial cells may be bacteria. In other examples, the microbial cells may be fungal cells. In yet other examples, the microbial cells may be yeast cells.

Accordingly, the method according to the present invention, comprises a step of incubating in a culture medium cells of the microorganism in or more from a sample obtained from a subject having or suspected of having an infection with said microorganism for a time and under conditions sufficient for the cells to actively divide, so as to obtain an actively dividing culture of the cells of said microorganism which comprises the above-mentioned concentrations of the of the actively dividing microbial cells. According to this example, it is desirable that the above-mentioned concentrations are attained before exposure of the cells in the actively dividing cell culture to the antimicrobial agent. Alternatively, or in addition, the above-mentioned concentrations of actively dividing cells in the cell culture may be achieved by combining and/or concentrating actively dividing microbial cells from two or more cultures to obtain a single culture of actively dividing microbial cells prior to exposure of the cells to the antimicrobial agent.

Methods for determining concentration of microbial cells in a cell culture or suspension are known to the skilled person and include for example, well documented measurements of optical density (OD) in a suitable Spectrophotometer (e.g., Thermo Fisher Scientific), and/or *in situ* using Lambda pump flow integrator according to manufacture's instructions (e.g., Lambda Laboratory Instruments, Ruessenstrasse 6, CH-6340Baar, SWITZERLAND - EUROPE). For example cell density of actively dividing microbial cells such as bacterial or yeast cells can be determined by measuring OD 600-650nm in a Spectrophotometer, or measuring total cell density by counting in a Neubauer cell counting chamber under a light microscope, or using a nephelometer. Alternatively, or in addition, concentration of cells can be determined by an automated cell counter such as a "coulter counter" cell or particle counting system or a Countess II FL Automated Cell Counter (e.g., thermos fisher Scientific). Alternatively, or in addition, the concentration of microbial cells may be determined by optionally combining the suspension with any appropriate fluorescent marker (in the presence or absence of fluorescently labelled microsphere standards), and analyzing the suspension to accurately enumerate the numbers of microbial cells using any flow cytometer.

Methods for concentrating cells in a cell culture are also well documented and are known for the skilled artisan. For example, actively dividing cells from two or more separate cell cultures can be combined in a single culture by transferring cell suspensions from two or more cultures to a sterile centrifuge tube of appropriate size followed by centrifugation for a period of time e.g., for 5 to 10 minutes at e.g., 800 × g. Supernatant can then be removed preferably without disturbing the cell pellet. The pellet containing the remaining cell pellet can be resuspended at the desired volume of a suitable culture medium such as those described herein and/or transferred to an existing culture of actively dividing microbial cells so as to increase the number of of actively dividing microbial cells in that culture.

### Suitable nucleic acid binding fluorescent compounds

In one example of a method according to any broad aspect of the invention described herein, the nucleic acid binding fluorescent compound may comprise any one or more of the following characteristics: cell wall and membrane permeable, bind nucleic acids (such as DNA and/or RNA), have some degree of natively fluorescent, be photo-stable.

Preferably, the nucleic acid binding fluorescent compound binds to cytoplasmic, mitochondrial and nuclear nucleic acids in the cells of the microorganism. For example, the nucleic acid binding fluorescent compound binds to deoxyribonucleic acid (DNA) and/or ribonucleic acid (RNA) in the cells of the microorganism.

In one example, the nucleic acid binding fluorescent compound comprises a nucleic acid binding moiety and a fluorescent tag moiety. However, in another example, nucleic acid binding fluorescent compounds suitable for use in the present invention may be a fluorophore compound not having separate nucleic acid binding moiety and fluorescent tag moiety. For example, the nucleic acid binding fluorescent compound may be a nucleic acid intercalating flurophore dye.

In one example, the nucleic acid binding fluorescent compound is a flurophore dye selected from the commercially available Quant-iT^{™} range dyes (e.g., ThermoFisher Scientific), and/or from the commercially available membrane permeable SYBR^{™} range dyes (e.g., ThermoFisher Scientific) and/or from the commercially available Hoechst range dyes (e.g., ThermoFisher Scientific; or Invitrogen) including but not limited to Hoechst 33258 (pentahydrate bis-benzimide), Hoechst 33342 [also known as [2-(4-ethoxyphenyl)-6-[6-(4-methylpiperazin-1-yl)-1H-benzimidazol-2-yl]-1H-benzimidazole or trihydrochloride trihydrate], Hoechst 34580 [also known as N,N-dimethyl-4-[6-[6-(4-methylpiperazin-1-yl)-1H-benzimidazol-2-yl]-1H-benzimidazol-2-yl]aniline], or nuclear yellow Hoechst S769121 and/or diaminophenylindole [also known as 4',6-diamidino-2-phenylindole or 2-(4-carbamimidoylphenyl)-1H-indole-6-carboximidamide or DAPI] and membrane-permeable DAPI derivatives (e.g., Biotium or ThermoFisher Scientific) and/or propidium iodide (PI) (e.g., ThermoFisher Scientific or Stemcell Technologies), and/or ethidium bromide (EB or EtBr) (e.g., Sigma-Aldrich, Merck) and/or Hexidium iodide (e.g., ThermoFisher Scientific) and/or acridine orange [also known as (3-N,3-N,6-N,6-N-tetramethylacridine-3,6-diamine or AO] (e.g., ThermoFisher Scientific) and/or DRAQ5^{™} and DRAQ7^{™} range dyes (e.g., Biostatus, BioLegend, or Beckman Coulter, Inc) and/or SYTOX^{™} (also known as SYTO Green) range dyes (e.g., ThermoFisher Scientific) and/or SYTO ^{™} range dyes such as SYTO 9, SYTO 11, SYTO 12, SYTO 13, SYTO 14, SYTO 15, SYTO 16, SYTO 18, SYTO 20, SYTO 21, SYTO 22, SYTO 23, SYTO 24, SYTO 25, SYTO BC (e.g., ThermoFisher Scientific).

In one example, the nucleic acid binding fluorescent compound is a flurophore dye selected from the group consisting of SYTO 9, SYTO 11, SYTO 12, SYTO 13, SYTO 14, SYTO 15, SYTO 16, SYTO 18, SYTO 20, SYTO 21, SYTO 22, SYTO 23, SYTO 24, SYTO 25, SYTO BC, Hoechst 33342, propidium iodine (PI), DAPI and any combination thereof. For example, the nucleic acid binding fluorescent compound is a flurophore dye selected from the group consisting of SYTO 9, Hoechst 33342, and a combination of SYTO 9 and propidium iodine (PI).

Preferably, the nucleic acid binding fluorescent compound is SYTO 9 dye.

In one example of a method according to this embodiment, the step of labelling cells of the microorganism exposed to the antimicrobial agent with the nucleic acid binding fluorescent compound, comprises staining the cells of the microorganism with the nucleic acid binding fluorescent compound to discriminate between microbila evenst (such as bacterial events) and non-microbila debris (such as non-bacterial debris) when measuring effect of the antimicrobial agent on cellular morphology of the cells of the microorganism by acoustic flow cytometry.

A skilled person would readily be able to determine the ability of a nucleic acid binding fluorescent compound to be membrane permeable for example by the relative polarity of the compounds, and specifically the lipophilic nature of exposed moieties on the compound using routine methods known in the art. In another example, the following method may be employed to determine that a nucleic acid binding fluorescent compound is membrane permeable. First, a suspension of microorganism such as a bacteria may be standardized to an appropriate working concentration such as about 5.0 × 10³ to 5.0 × 10⁷ microbial cells / mL. The standardized suspension may then be divided into a range of tubes (typically 10 may be used, but any number greater than or including 1 tube may be used). The nucleic acid binding fluorescent compound is then added to each of the tubes being tested, across a range of controlled concentrations. Typically, compounds may be assayed in doubling dilutions downwards from their maximum solubility. By way of example only, if a compound is soluble at 10mM/mL, it is desirable to test 10mM, 5mM, 2.5mM etc. The nucleic acid binding fluorescent compound is allowed to incubate for a period of time - typically staining times are about 5 minutes to about 30 minutes, but any time period can be tested. The cells may then be washed for example by centrifugation (preferably 9,800 × g for 5 minutes, but less time (1 minute - 30 minutes may also be employed) and variable speeds may also be applied e.g., from 4,000 × g to 30, 000 × g. Alternatively, or in addition, the cells may be subjected to a filtration, where the cell/ nucleic acid binding fluorescent compound suspension may be passed through a filter with a mesh small enough to capture bacteria (1 µM pore size or lower) but remove the nucleic acid binding fluorescent compound. The cells may then be analysed following washing/filtering using any fluorescence device capable of exciting the fluorescent compound and measuring the emission. A fluorescence microscope, a flow cytometer, or spectrophotomer are all examples of commonly used equipment. At this time, a negative control (an aliquot of the bacterial suspension that has not been exposed to the dye) may be used to determine the presence of the dye remaining in the cell.

Similarly, it is well within the ken of the skilled person to determine that a nucleic acid binding fluorescent compound is natively fluorescent within cells. For example, determination may be carried out by the same method described above in respect of determining that the compound is cell membrane permeable. For example, for the purpose of determining that that a nucleic acid binding fluorescent compound is natively fluorescent within cells a control condition, using a membrane-derived vesicle containing no nucleic acids may be used to assay that fluorescence exists in the cells in the absence of nucleic acids.

Similarly, it is well within the ken of the skilled person to determine that a nucleic acid binding fluorescent compound is photostable within the cells. For example, measurements may performed by the same method described above in respect of determining that the compound is cell membrane permeable and/or natively fluorescent within cells, with direct comparison between a membrane derived vesicle, and a structure containing a nucleic acid such as a DNA (may be a membrane derived vesicle, or a cell/organism). For example, measuring a ratio of arbitrary fluorescence units between the sample without a nucleic acid present, and with a nucleic acid present, yields a unit of measurement known as fluorescence enhancement. Manufacturers' instructions and information documentations that contains this information, can also be consulted to determine that a nucleic acid binding fluorescent compound is photostable within the cells.

Preferably the nucleic acid binding fluorescent compound binds to cytoplasmic, mitochondrial and nuclear nucleic acids in the cells of the microorganism. For example, the nucleic acid binding fluorescent compound binds to deoxyribonucleic acid (DNA) and/or ribonucleic acid (RNA) in the cells of the microorganism. Measurements may be performed as detailed above, with direct comparison between a membrane derived vesicle, and a structure containing DNA (can be a membrane derived vesicle, or a cell/organism). Measuring a ratio of arbitrary fluorescence units between the sample without DNA or RNA present, and with DNA or RNA present, yields fluorescence enhancement measurements. Again, manufacturers' instructions may be consulted to determine if a commercially available compound binds to cytoplasmic, mitochondrial and nuclear nucleic acids in the cells of the microorganism and/or binds DNA and/or RNA.

### Conditions for culturing microorganisms

### i) Culture media

In another example of a method according to any broad aspect of the invention described herein, the culture medium for incubating cells of the form a sample obtained from the subject, may be nutritionally complete and/or free of microbial components and/or contain minimal macro-protein. Preferably the culture medium used will nourish the cells during the incubation with the antibiotic. In one example, the culture medium is any one or more of the following: Brain Heart Infusion Broth, Lysogeny Broth, Nutrient Broth, Oxoid Iso-Sensitest^{™} Medium, Mueller-Hinton Broth.

Culturing cells of a microorganism that is present in a biological sample obtained from a subject in the context of the present invention, can be achieved by adding the clinical sample obtained from the subject or an aliqout thereof, and/or microbial cells purified and/or isolated from the clinical sample obtained from the subject to a culture or growth medium containing suitable nutrients such as a carbon source (preferably no less than a simple sugar), a nitrogen source (preferably no less than amino acids), and be of a pH compatible with the pH found in the body (preferably a pH in the range of pH 6.2 - pH 8.2).

Media used for culturing cells of a microorganism for microbial growth and/or maintenance are prepared by procedures known to those skilled in the art and described, for example in BD Diagnostics (Manual of Microbiological Culture Media, Sparks, Maryland, Second Edition, 2009); Versalovic et al. (in Manual of Clinical Microbiology, American Society for Micobiology, Washington D.C., 10th Edition, 2011); Garrity et al. (In Bergey's Manual of Systemic Bacteriology, Springer, New York, Second Edition 2001). As will be apparent to the skilled artisan, microbial morphology such as a morphology of a bacteria may be performed by performing staining such as Gram staining (see *e.g.,* Cioco 2005 Curr. Protoc. Microbiol. Appendix 3) and viability may be assessed as described, for example, by Murray et al. (In: Manual of Clinical Microbiology, American Society of Microbiology, Washington D.C, Ninth Edition 2007). Exemplary suitable culture media for culturing cells of a microorganism for microbial growth and/or maintenance and/or for the purpose of incubating cells of microorganisms for a time and under conditions for said cells to actively divide to thereby obtain an actively dividing culture of said cells, are outlined below.

A suitable MHB culture medium may comprise about 300.0g beef extract (dehydrated infusion from), about 17.5g casein hydrolysate, and about 1.5g starch, e.g., reconstituted in about 1L of water, e.g., and pH may be adjusted to about pH 7.3 ± 0.1 as measured at about 25°C. Preferably, the MHB culture medium comprises about 300.0g beef extract (dehydrated infusion from), about 17.5g casein hydrolysate, and about 1.5g starch, reconstituted in about 1L of water, and pH may be adjusted to about pH 7.3 ± 0.1 as measured at about 25°C.

A suitable BHIB culture medium may comprise about 12.5g brain infusion solids, about 5.0g beef heart infusion solids, about 10.0g proteose peptone, about 2.0g glucose, about 5.0g sodium chloride, about 2.5g disodium phosphate, e.g., reconstituted in about 1L of water, and pH may be adjusted to about pH 7.4 ± 0.2 as measured at about 25°C. Preferably, the BHIB culture medium comprises about 12.5g brain infusion solids, about 5.0g beef heart infusion solids, about 10.0g proteose peptone, about 2.0g glucose, about 5.0g sodium chloride, about 2.5g disodium phosphate, reconstituted in about 1L of water, and pH adjusted to about pH 7.4 ± 0.2 as measured at about 25°C.

A suitable Lysogeny Broth culture medium may comprise about 10g peptone such as peptone 140, about 5g yeast extract, about 5g sodium chloride, and may be reconstituted in e.g., about 1L of water. Preferably, the Lysogeny Broth culture medium comprises about 10g peptone such as peptone 140, about 5g yeast extract, about 5g sodium chloride, reconstituted in about 1L of water.

A suitable Nutrient Broth culture medium may comprise about 1.0g 'Lab-Lemco' meat extract powder (e.g., Thermo Scientific), about 2.0g yeast extract, about 5.0g peptone, about 5.0g sodium chloride, and may be reconstituted in about 1L of water, and pH may be adjusted to abut pH 7.4 ± 0.2 as measured e.g., at about 25°C. Preferably, the Nutrient Broth culture medium comprises about 1.0g 'Lab-Lemco' meat extract powder (e.g., Thermo Scientific), about 2.0g yeast extract, about 5.0g peptone, about 5.0g sodium chloride, and reconstituted in about 1L of water, and pH adjusted to abut pH 7.4 ± 0.2 as measured e.g., at about 25°C.

A suitable Oxoid Iso-Sensitest Medium culture medium may comprise about 11g hydrolysed casein, about 3.0g peptones, about 2.0g glucose, about 3.0g sodium chloride, about 1.0g soluble starch, about 2.0g disodium hydrogen phosphate, about 1.0g sodium acetate, about 0.2g magnesium glycerophosphate, about 0.1g calcium gluconate, about 0.001 cobaltous sulphate, about 0.001 cupric sulphate, about 0.001 zinc sulphate, about 0.001g ferrous sulphate, about 0.002 mangous chloride, about 0.001g menadione, about 0.001g cyanocobalamin, about 0.02g L-cysteine hydrochloride, about 0.02g L-tryptophan, about 0.003g pyridoxine, about 0.003g pantothenate, about 0.003g nicotinamide, about 0.0003g biotin, about 0.00004g thiamine, about 0.01g adenine, about 0.01g guanine, about 0.01g xanthin, about 0.01g uracil, and may be reconstituted e.g., in about 1L of water, and pH may be adjusted to e.g., about pH 7.4 ± 0.2 as measured at about 25°C.

Preferably, the Oxoid Iso-Sensitest Medium culture medium comprises about 11g hydrolysed casein, about 3.0g peptones, about 2.0g glucose, about 3.0g sodium chloride, about 1.0g soluble starch, about 2.0g disodium hydrogen phosphate, about 1.0g sodium acetate, about 0.2g magnesium glycerophosphate, about 0.1g calcium gluconate, about 0.001 cobaltous sulphate, about 0.001 cupric sulphate, about 0.001 zinc sulphate, about 0.001g ferrous sulphate, about 0.002 mangous chloride, about 0.001g menadione, about 0.001g cyanocobalamin, about 0.02g L-cysteine hydrochloride, about 0.02g L-tryptophan, about 0.003g pyridoxine, about 0.003g pantothenate, about 0.003g nicotinamide, about 0.0003g biotin, about 0.00004g thiamine, about 0.01g adenine, about 0.01g guanine, about 0.01g xanthin, about 0.01g uracil, reconstituted in about 1L of water, and pH may be adjusted to e.g., about pH 7.4 ± 0.2 as measured at about 25°C.

A suitable Trypticase Soy Broth culture medium may comprise about 17g tryptone (e.g., from pancreatic digest of casein), about 3.0g soytone (e.g., from peptic digest of soybean), about 2.5g glucose (e.g., dextrose), about 5.0g sodium chloride, about 2.5g dipotassium phosphate, and may be reconstituted e.g., in about 1L of water. Preferably, the Trypticase Soy Broth culture medium comprises about 17g tryptone (e.g., from pancreatic digest of casein), about 3.0g soytone (e.g., from peptic digest of soybean), about 2.5g glucose (e.g., dextrose), about 5.0g sodium chloride, about 2.5g dipotassium phosphate, and reconstituted in about 1L of water.

Alternatively, or in addition, e.g., for rare and unusual microorganisms specific media requirements can be selected from repositories of information such as: https://www.dsmz.de/catalogues/catalogue-microorganisms/culture-technology/list-of-media-for-microorganisms.html . Preferably, the vulture medium used is Mueller-Hinton Broth is e.g., because it encompasses all the above mentioned desirable characteristic of a culture medium, and is the standard medium to which all antibiotic sensitivity practices currently in place are aligned.

### ii) Times and conditions sufficient for microbial cells to actively divide to thereby obtain an actively dividing culture

To obtain actively dividing cells it is desirable to achieve suitable environmental conditions for cells of a microorganism to grow and divide such as temperature, humidity, and atmospheric condition atmospheric.

An actively dividing culture of microbial cells can be prepared by incubating a biological sample comprising viable cells of a microorganism which has been obtained from a subject having or suspect of having an infection with the microorganism or incubating viable microbial cells isolated from the biological sample in a suitable culture medium such as that described above.

In one example, the method comprises incubating an aliquot of the biological sample or an aliquot of the cells from the biological sample in a suitable culture for a time period of about 1 to about 5 cell divisions to occur, and more preferably for a time and under conditions sufficient for the cells to undergo about 1 to about 3 cell divisions. It will be understood that the time period required for cells of a microorganism to undergo a cell division would vary depending on the type or species of the microorganism found in the biological sample. Some microorganisms may undergo cell division in about 20-30 minutes, others may be slower to undergo a cell division and may take as much as up to 3 hours to undergo a single cell division.

For example, the microorganism may be a bacteria and the method may comprise incubating cells of the microorganism in and/or from the subject sample in a culture medium for a period of at least about 20 minutes to at least about 3 hours. In one such example, method may comprise incubating cells of the microorganism in and/or from the subject sample in a culture medium for a period of about 20 minutes to about 180 minutes, or about 30 minutes to about 180 minutes, or about 20 minutes to about 170 minutes, or about 20 minutes to about 160 minutes, or about 20 minutes to about 150 minutes, or about 20 minutes to about 140 minutes, or about 20 minutes to about 130 minutes, or about 20 minutes to about 120 minutes, or about 20 minutes to about 110 minutes, or about 20 minutes to about 100 minutes, or about 20 minutes to about 90 minutes, or about 20 minutes to about 80 minutes, or about 20 minutes to about 70 minutes, or about 20 minutes to about 60 minutes or about 20 minutes to about 50 minutes, or about 20 minutes to about 40 minutes, or about 20 minutes to about 30 minutes. In one example, the method may comprise incubating cells of the microorganism in and/or from the subject sample in a culture medium for a period of about 20 minutes or about 30 minutes or about 40 minutes or about 50 minutes or about 60 minutes or about 70 minutes or about 80 minutes or about 90 minutes or about 100 minutes or about 110 minutes or about 120 minutes or about 130 minutes, or about 140 minutes, or about 150 minutes, or about 160 minutes, or about 170 minutes, or about 180 minutes, or about 190 minutes, or about 200 minutes, or about 210 minutes, or about 220 minutes, or about 230 minutes, or about 240 minutes. More preferably, incubation time is about 30 minutes, or about 1 hour or about 1.5 hours. The inventors have speculated that incubation time of about 30 minutes to about 180 minutes may be used to capture majority of possible clinically relevant microorganisms. Utility in the present method of a minimum time that is required to achieve 1-3 cell divisions contributes to the ability of the method to determine susceptibility in a very short time.

The method may comprise incubating the cells of the microorganism in and/or from the subject sample in the culture medium under temperature conditions from about 25°C to about 42°C, preferably about 33°C to 42°C, and more preferably about 37°C and with aeration or without aeration.

For example, the method of the present invention according to any aspect or embodiment or example described herein may archive actively dividing cell cultures by first obtaining a clinical sample containing the microorganism, adding viable cells of the microorganism organisms to a suitable culture/growth medium such as those described above, and incubating the cells in the culture/growth medium for enough time sufficient for about 1 to about 3 cells divisions to occur.

Optionally, presence of actively dividing cells in a cell culture may be confirmed by any method known in the art for example including, but not limited to, measurements of CO₂ production and/or using fluorescent reagents. For example, density of the microorganism in a cell culture may be assayed by flow cytometry to count a suspension stained for 5 minutes with the nucleic acid binding fluorescent compound as described according to any example or embodiment hereof such as using with 5µM SYTO 9 to count the number of organisms. However, any technique and/or instrument capable of enumerating organism numbers may be used, including a flow cytometer (of any type), a microscope (optical or fluorescence), a spectrophotometer measuring optical density e.g., at 600nm, or a nephelometer. Measuring density of actively dividing cells after about 1-3 cell division helps to establish baseline density, where the total number of actively dividing cells in the cell culture may preferably be no less than 1000 cells.

The resulting suspension may then be placed in an incubator to provide appropriate heat, humidity and/or atmospheric gases. For example, for clinical specimens, this range is 33°C to 42°C, with either atmospheric gas conditions, atmospheric gas conditions with 5% enriched CO2, or anaerobic growth conditions (such as those less than or equal to 5% H₂ with the remainder N₂).

Optionally, a further measurement of cell density may be performed as described above to confirm that the "actively dividing" cell culture may have no less than a 60% increase in total cell numbers (or a proxy measurement - such as with a nephelometer - equal to an increase in cell numbers of 60%) may be observed. Such 60% increase in cell numbers may assist in determining that sufficient number of cells in the culture are actively dividing. The time at which this measurement may be made may vary. In one example, sampling may take place at about 180 minutes since inoculation of the culture medium with the microbial cells. Alternatively, sampling may take place at any number of intervals to ascertain the point at which the 60% increase in cell numbers is reached. To capture the majority of possible clinically relevant microorganisms, it is desirable that cells are incubated for about 30 minutes to about 180 minutes.

### iii) Exposing microbial cells in and/or from an actively dividing cell culture to an antimicrobial agent for a time and under conditions sufficient for the cells to actively divide

In preferred embodiments of the methods according to any broad aspect or example or embodiment described herein, the incubation conditions for active division during the antimicrobial exposure are similar to or mirror those used to obtain an actively dividing culture as described above i.e., before exposure to the antimicrobial agent. For example, it is preferred that exposure of actively dividing microbial cells to an antimicrobial agent is performed in the same medium and/or for the same or similar time period and/or the same or similar temperature and/or the same or similar atmospheric conditions used to obtain an actively dividing culture of said cells of the microorganism before exposure to the antimicrobial agent.

In one example, exposure of actively dividing microbial cells to an antimicrobial agent is performed in the same medium as that used to obtain an actively dividing culture of said cells of the microorganism before exposure to the antimicrobial agent.

In another example, the exposure of actively dividing microbial cells to an antimicrobial agent is performed for a period of at least about 20 minutes to at least about 3 hours. In one such example, method may comprise exposing actively dividing microbial cells to an antimicrobial agent for a period of about 20 minutes to about 180 minutes, or about 30 minutes to about 180 minutes, or about 20 minutes to about 170 minutes, or about 20 minutes to about 160 minutes, or about 20 minutes to about 150 minutes, or about 20 minutes to about 140 minutes, or about 20 minutes to about 130 minutes, or about 20 minutes to about 120 minutes, or about 20 minutes to about 110 minutes, or about 20 minutes to about 100 minutes, or about 20 minutes to about 90 minutes, or about 20 minutes to about 80 minutes, or about 20 minutes to about 70 minutes, or about 20 minutes to about 60 minutes or about 20 minutes to about 50 minutes, or about 20 minutes to about 40 minutes, or about 20 minutes to about 30 minutes. In one example, the method may comprise exposing actively dividing microbial cells to an antimicrobial agent for a period of about 20 minutes or about 30 minutes or about 40 minutes or about 50 minutes or about 60 minutes or about 70 minutes or about 80 minutes or about 90 minutes or about 100 minutes or about 110 minutes or about 120 minutes or about 130 minutes, or about 140 minutes, or about 150 minutes, or about 160 minutes, or about 170 minutes, or about 180 minutes, or about 190 minutes, or about 200 minutes, or about 210 minutes, or about 220 minutes, or about 230 minutes, or about 240 minutes. More preferably, incubation time is about 30 minutes, or about 1 hour or about 1.5 hours. The inventors have speculated that incubation time in the presence of an antimicrobial agent of about 30 minutes to about 180 minutes may be used to capture the majority of possible clinically relevant microorganisms.

Alternatively or in addition, exposing actively dividing microbial cells to an antimicrobial agent at a temperature from about 25°C to about 42°C, preferably about 33°C to 42°C, and more preferably about 37°C and with aeration or without aeration. For example, during exposure to the antimicrobial agent the cell suspension may be placed in an incubator to provide appropriate heat, humidity and/or atmospheric gases. For example, for clinical specimens, this range is 33°C to 42°C, with either atmospheric gas conditions, atmospheric gas conditions with 5% enriched CO₂, or anaerobic growth conditions (such as those less than or equal to 5% H₂ with the remainder N₂).

In one preferred embodiment, the exposure time to the antimicrobial agent may be tailored to a specific clinical isolate/microorganism by measuring, as described directly herein above, the minimum time to obtain "actively dividing cells" for that specific isolate/microorganism, and setting the antimicrobial exposure challenge as that measured period of time.

In one example, the method comprises exposing the actively dividing cells of the microorganism to a range of different concentrations of the antimicrobial agent such as one or more concentrations selected from 0.25 mg/L, 0.5 mg/L, 1.0 mg/L, 2.0 mg/L, 4.0 mg/L, 8.0 mg/L, 16.0 mg/L, 32.0 mg/L, 64.0 mg/L, 128.0 mg/L, and 256.0 mg/L. Alternatively, or in addition, the range of different concentrations of the antimicrobial agent may comprise 1:2 dilution series ranging from about 2560 mg/ml to about 2.5 mg/ml of said antimicrobial agent.

### iv) Labelling cells of the microorganism exposed to the antimicrobial agent with a nucleic acid binding fluorescent compound

It will be understood that labelling cells of the microorganism exposed to the antimicrobial agent with a nucleic acid binding fluorescent compound may be performed in any fluid or suspension in which the label being used remains chemically stable. It is desirable that manufacturers' instructions for any specific nucleic acid binding fluorescent compound label which is employed are consulted.

In one preferred example, microbial cells such as bacterial cells may be labelled with a nucleic acid binding fluorescent compound which is present in either Hank's Balanced Salt Solution or 0.85% saline solution.

### Subjects and therapeutic uses

It will be understood that the term "subject" as referred to in the context of the present invention to any subject having or suspect of having an infection with a microorganism is intended to encompass as subject including a human or animal subject. In one example, the subject is a human subject.

In such example, the subject having or suspected of having an infection with a microorganism may present one or more symptoms indicative or suggestive to a clinician such as a medical doctor or a nurse of an infection with one or more microorganisms. By way of a non-limiting example, a subject having or suspected of having an infection with a microorganism may suffer from or present one or more symptoms selected from elevated body temperature such as greater than 38°C (or 100.4°F), muscle weakness, organ or site specific pain, tissue necrosis or putrefaction, excessive drowsiness or sleepiness, muscle weakness, excessive pre-nasal or post-nasal mucous production, diarrhea, abdominal pain, coughing and/or sneezing, non-specific joint and muscle pain, and/or an organ specific inflammation such as cellulitis, folliculitis, impetigo, otitis or otitis media, inflammation of the urinary track, gastroenteritis and/or meningitis. Alternatively, or in addition, the subject having or suspected of having an infection with a microorganism may present one or more symptoms indicative or suggestive to a clinician such as a medical doctor or a nurse and/or one or more symptoms indicative or suggestive of sepsis such as one or more symptoms selected from elevated body temperature greater than 38°C (100.4°F) or a lower body temperature of less than 36°C (96.8°F), increased heart rate (e.g., of about more than 90 beats per minute), irregular or changing heart rhythm, shortness of breath or increased breathing rate (e.g., breathing rate greater than 20 breaths per minute), difficulty or pain associated with breathing, altered mental state such as disorientation, confusion, unprovoked aggression, decreased urine production, abnormal blood clotting, evidence of microbial infection presenting at multiple body sites, a drop in blood pressure (e.g., relative to individual patient norm), weakness or extreme weakness, unconsciousness, and/or death.

In a third broad aspect, described but not claimed, the present invention provides a method of treating a subject having or suspect of having an infection with a unicellular or multicellular microorganism. The method comprises determining susceptibility of the microorganism in said subject to any one or more antimicrobial agent(s) by performing the method according to the first or second broad aspects of the invention. The method further comprises identifying one or more antimicrobial agent(s) to which the microorganism has been determined to be susceptible, and administering to the subject a therapeutically effective amount of the one or more antimicrobial agent(s).

Compositions comprising one or antimicrobial agents which are administered to the subject, may be formulated for daily or periodic administration. For example, the composition may be administered to the subject daily for a period of at least about one week, or at least about two weeks or at least about three weeks or at least about four weeks or more than four weeks. In another example, the composition may be administered periodically such as for example, every day, or every second day or every third day or every fourth day or every sixth day, or once weekly or more than once weekly. In another example the composition comprising the antimicrobial agent may be formulated or administrated in a dosage form. Means and modes of administration of antimicrobial agents are known in the art and are readily available to the skilled artisan.

In a fourth broad aspect, described but not claimed, the present invention provides use of a therapeutically effective amount of any one or more antimicrobial agent(s) in the preparation of a medicament for the treatment of a subject having or suspect of having an infection with a unicellular or multicellular microorganism, wherein the microorganism has been determined to be susceptible to the one or more antimicrobial agent(s) by performing the method according to the first or second broad aspects.

In a fifth broad aspect, described but not claimed, the present invention provides use of a therapeutically effective amount of any one or more antimicrobial agent(s) in the treatment of a subject having or suspect of having an infection with a unicellular or multicellular microorganism, wherein the microorganism has been determined to be susceptible to the one or more antimicrobial agent(s) by performing the method according to the first or second broad aspects.

### Suitable types of microorganisms

In one preferred example according to any aspect or embodiment or example of the present invention described hereof, the microorganism is a unicellular or multicellular microorganism selected from a bacteria, a fungus or a yeast.

In one such preferred example, the microorganism is a bacteria. The bacteria may be a Gram negative bacteria or a Gram positive bacteria. The bacteria may be aerobic or anaerobic or facultative anaerobe or facultative aerobe or obligate aerobe or obligate anaerobe and/or may be any pathogenic or non-pathogenic bacteria.

In one example, the bacteria is selected from bacteria belonging to one or more of the following bacterial genus or species *Enterobacter,* Enterobacteriaceae, *Campylobacter, Klebsiella, Pseudomonas, Acinetobacter, Burkholderia, Clostridium, Stenotrophomonas, Serratia, Haemophilus, Neisseria, Burkholderia, Staphylococcus, Streptococcus, Clostridium, Bacillus, Bacteroides, Listeria, Mycobacterium, Salmonella, Brucella, Bordetella, Borrelia, Nocardia, Francisella, Legionella, Yersinia, Leptospira, Borrelia, Shigella, Shewanella, Campylobacter, Chlamydia, Clostridium, Corynebacterium, Escherichia, Haemophilus, Helicobacter, Leptospira, Mycoplasma, Rickettsia, Treponema,* and *Vibrio.*

For example, the bacteria is selected from any one or more of the following: Acinetobacter baumanii, Bacillus anthracis, Bacillus cereus, Bordetella pertussis, Borrelia burgdorferi, Brucella abortus, Brucella canis, Brucella melitensis, Brucella suis, Burkholderia pseudomallei, Burkholderia cepacia, Burkholderia cenocepacia, Campylobacter coli, Campylobacter jejuni, Chlamydia pneumoniae, Chlamydia psittaci, Chlamydia trachomatis, Clostridium botulinum, Clostridium difficile, Clostridium perfringens, Clostridium tetani, Corynebacterium diphtheriae, Enterobacter cloacae, Enterobacter aerogenes, Enterococcus faecalis, Enterococcus faecium, Escherichia coli, Enterotoxigenic Escherichia coli (ETEC), Enteropathogenic E. coli, E. coli (O157:H7), Francisella tularensis, Haemophilus influenza, Haemophilus parainfluenzae, Klebsiella pneumoniae, Klebsiella oxytoca, Helicobacter pylori, Legionella pneumophila, Legionella longbeachae, Leptospira interrogans, Leptospira Santarosai, Listeria monocytogenes, Mycobacterium avium, Mycobacterium intracellulare, Mycobacterium kansasii, Mycobacterium tuberculosis, Mycoplasma pneumoniae, Neisseria gonorrhoeae, Neisseria meningitidis, Pseudomonas aeruginosa, Rickettsia rickettsiae, Salmonella enterica var. Typhi, Salmonella enterica var. Typhimurium, Serratia marcescens, Shewanella oneidensis, Shigella sonnei, Shigella dysenteriae, Shigella boydii, Shigella flexneri, Staphylococcus aureus, Staphylococcus epidermidis, Staphylococcus lugdunensis, Staphylococcus saprophyticus, Stenotrophomonas maltophila, Streptococcus agalactiae, Streptococcus pneumoniae, Streptococcus pyogenes, Streptococcus anginosus-constellatus-intermedius, Streptococcus mitis, Vibrio cholera, Yersinia pseudotuberculosis and Yersinia pestis.

In one example, the bacteria belong to the Enterobacteriaceae, or the *Klebsiella* species or the *Pseudomonas* species or the *Acinetobacter* species.

For example, the bacteria are a strain of *Klebsiella pneumoniae* or *Pseudomonas aeruginosa.*

In another preferred example, the microorganism is a yeast. For example, the yeast is selected from a yeast belonging to one or more of the following species of yeast: *Candida, Saccharomyces,* and *Cryptococcus.*

For example, the yeast is selected from any one or more of the following: *Candida albicans, Candida tropica, Cryptococcus neoformans.*

In another preferred example, the microorganism is a fungus. For example, the fungus is selected from one or more of the following genus or species: *Aspergillus, Candida, Histoplasma, Pneumocystis,* and *Stachybotrys.*

For example, the fungus is selected from any one or more of the following: Aspergillus fumigatus, Aspergillus flavus, Candida albicans, Cryptococcus neoformans, Cryptococcus gattii, Histoplasma capsulatum, Pneumocystis jirovecii (also known as Pneumocystis carinii), and Stachybotrys chartarum.

### Antimicrobial agents

Preferably, in the method according to any aspect, embodiment and/or example of the present invention described hereof, the antimicrobial agent is or comprises an anti-bacterial agent and/or anti-fungal agent and/or anti-yeast agent. The antimicrobial agent may have biostatic and/or microbicidal effect on the unicellular and/or multicellular microorganisms of the present invention. Accordingly, the antimicrobial agent according to any aspect, embodiment and/or example described hereof may inhibit growth and/or kill actively dividing cells of the microorganism assayed in the method of the present invention. In one example the antimicrobial agent may be a compound of a chemical. Similarly, the antimicrobial agent may be synthetic and/or chemically-produced and/or naturally occurring and/or derived whether directly or indirectly from an organism or a plant or an animal. Preferably, the antimicrobial agent is or comprises an anti-bacterial agent such as a bactericidal agent and/or bacteriostatic agent.

In one example, the antimicrobial agent may be any compound such as a chemical compound or a pharmaceutical composition such as a drug. In another example the antimicrobial agent is a composition comprising a compound such as a chemical compound or a pharmaceutical or a drug. In one such example the antimicrobial agent may be a composition comprising two or more compounds such as chemical compounds, pharmaceuticals and/or drugs.

For example, the antimicrobial agent may be or comprise any one of more of the following: amino-acids or amino acid derivatives, fatty acids or fatty acid derivatives, antimicrobial peptides or peptide derivatives, aminoglycosides, aureolic acids, aziridines, benzenoids, Benzimidazoles, coumarin-glycosides, diphenyl ether derivatives, epipolythiodioxopiperazines, glucosamines, glycopeptides, Imidazoles, indol derivatives, macrolactam, macrolides, nucleosides, beta-Lactams such as penicillins and cephalosporins, peptidyl nucleosides, phenicoles, polyenes, pyridines and pyrimidines, quinolones and fluoroquinolones, statins, steroids, sulfonamides, taxoides, tetracyclines, metal alloys such as copper alloys.

In one particularly preferred example, the antimicrobial agent is or comprises an antibiotic. For example, the antibiotic is an anti-bacterial and/or anti-fungal and/or anti-yeast antibiotic.

In one example, the antimicrobial agent is or comprises a beta lactam antibiotic such as penicillins, cephalosporins, monobactams and/or carbapenems.

For example the antimicrobial agent is or comprises a penicillin antibiotic selected from one or more of penicillin G such as penicillin G potassium salt or penicillin G sodium salt, ampicillin such as ampicillin anhydrous, ampicillin trihydrate and/or ampicillin sodium salt, amoxicillin, ticarcillin piperacillin, (+)-6-Aminopenicillanic acid (6-APA), azlocillin such as azlocillin sodium salt, carbenicillin such as carbenicillin disodium salt, cloxacillin such as cloxacillin sodium salt (e.g., cloxacillin sodium salt monohydrate), dicloxacillin such as dicloxacillin sodium salt monohydrate, flucloxacillin, mezlocillin, methicillin, nafcillin such as nafcillin sodium salt monohydrate, oxacillin such as oxacillin sodium salt, D-penicillamine, penicillin V such as penicillin V potassium salt, phenoxymethylpenicillinic acid such as phenoxymethylpenicillinic acid potassium salt, piperacillin such as piperacillin sodium salt, temocillin, and ticarcillin such as ticarcillin disodium salt. Each of these penicillins is commercially available e.g., from Sigma-Aldrich.

In one such example, the antimicrobial agent which is or comprises a penicillin antibiotic provides antimicrobial effect such as antibacterial effect on actively dividing cells of the microorganism (e.g., bacteria) by disrupting synthesis of the peptidoglycan layer of the cell walls of the microorganism.

In another example, the antimicrobial agent is or comprises a cephalosporin antibiotic selected from one or more of cephalothin, cefalexin, cefaclor, cefixime such as aefixime trihydrate, cefmetazole such as cefmetazole sodium salt, cefoperazone such as cefoperazone sodium salt, sodium salt, cefotaxime such as cefotaxime sodium salt, cefuroxime, cefprozil, cefoxitin, ceftriaxone, ceftazidime, cefpodoxime, cefepime, 7-Aminocephalosporanic acid (7-ACA), 7-Aminodesacetoxycephalosporanic acid (7-ADCA), cefotiam such as cefotiam dihydrochloride hydrate, ceftazidime such as ceftazidime hydrate, cefsulodin such as cefsulodin sodium salt hydrate, ceftibuten such as ceftibuten hydrate, ceftriaxone such as ceftriaxone disodium salt hemi(heptahydrate), cephalexin such as cephalexin hydrate, cephalomannine, cephalothin such cephalothin as sodium salt, cephradine, imipenem e.g., imipenem monohydrate, moxalactam such as moxalactam sodium salt, and tazobactam sodium salt. Each of these cephalosporins is commercially available *e.g.,* from Sigma-Aldrich.

In another example, the antimicrobial agent is or comprises a first generation cephalosporin antibiotic selected from: cefadroxil, cefazolin, cefalotin or cefalothin, and cephalexin.

In another example, the antimicrobial agent is or comprises a second generation cephalosporin antibiotic selected from: cefaclor, cefamandole, cefoxitin, cefprozil, and cefuroxime.

In another example, the antimicrobial agent is or comprises a third generation cephalosporin antibiotic selected from: cefixime, cefdinir, cefditoren, cefotaxime, cefoperazone, cefotaxime, cefpodoxime, ceftibuten, ceftizoxime, and ceftriaxone.

In another example, the antimicrobial agent is or comprises a fourth generation cephalosporin antibiotic such as cefepime.

In another example, the antimicrobial agent is or comprises a fifth generation cephalosporin antibiotic such as ceftaroline fosamil and/or ceftobiprolel.

In one example, according to any example or embodiment or aspect described hereof involving an antimicrobial agent which is or comprises one of more cephalosporin (whether first, second, third, fourth and/or fifth generation cephalosporin antibiotic), the antimicrobial agent provides antimicrobial effect such as antibacterial effect on actively dividing cells of the microorganism (e.g., bacteria) by disrupting synthesis of the peptidoglycan layer of the cell walls of the microorganism.

In another example, the antimicrobial agent is or comprises a monobactam antibiotic such as aztreonam, which is commercially available *e.g.,* from Sigma-Aldrich.

In one such example, the antimicrobial agent comprising a monobactam antibiotic such as aztreonam provides antimicrobial effect such as antibacterial effect on actively dividing cells of the microorganism (e.g., bacteria) by disrupting synthesis of the peptidoglycan layer of the cell walls of the microorganism.

In another example, the antimicrobial agent is or comprises a carbapenem antibiotic selected from one or more of imipenem or cilastatin, meropenem, ertapenem and doripenem. Each of these exemplary carbapenem antibiotic is commercially available *e.g.,* from Sigma-Aldrich.

The antimicrobial agent which is or comprises a carbapenem antibiotic may provide antimicrobial effect such as antibacterial effect by inhibiting cell synthesis in actively dividing cells of the microorganism.

In another example, the antimicrobial agent is or comprises an amino acid or amino acid derivative. For example, the antimicrobial agent is or comprises an amino acid derivative selected from one or more of: L-alanyl-L-1-aminoethylphosphonic acid, azaserine (O-diazoacetyl-L-serine), bestatin hydrochloride (N-[(2S,3R)-3-Amino-2-hydroxy-4-phenylbutyryl]-L-leucine hydrochloride), D-cycloserine [(*R*)-4-Amino-3-isoxazolidone, 4-Amino-3-isoxazolidinone], and 6-Diazo-5-oxo-L-norleucine [(S)-2-Amino-6-diazo-5-oxocaproic acid, or DON]. Each of these amino acids or derivatives is commercially available e.g., from Sigma-Aldrich.

In another example, the antimicrobial agent is or comprises an aminoglycoside. For example, the antimicrobial agent is or comprises an aminoglycoside selected from one or more of: gentamicin, tobramycin, amikacin, daunorubicin such as hydrochloride, dihydrostreptomycin sesquisulfate, antibiotic G418 such as antibiotic G418 disulfate salt (C₂₀H₄₀N₄O₁₀ · 2H₂SO₄) (e.g., Sigma-Aldrich), gentamicin, hygromycin B (e.g., from *Streptomyces hygroscopicus* lyophilized powder) (e.g., Sigma-Aldrich), Kanamycin, neomycin such as neomycin trisulfate salt hydrate, netilmicin such as netilmicin sulfate salt, paromomycin such as paromomycin sulfate salt, ribostamycin such as ribostamycin sulfate salt, sisomicin such as sisomicin sulfate salt, spectinomycin such as spectinomycin dihydrochloride pentahydrate, streptomycin such as streptomycin sulfate salt, and tobramycin aminoglycoside antibiotic such as tobramycin sulfate salt (aminoglycoside antibiotic), paromomycin, each of which is commercially available *e.g.,* from Sigma-Aldrich.

In one such example, the antimicrobial agent is or comprises an aminoglycoside antibiotic selected from: amikacin, gentamicin, kanamycin, neomycin, netilmicin, tobramycin, streptomycin, and spectinomycin.

The antimicrobial agent which is or comprises an aminoglycoside may provide antimicrobial effect such as antibacterial effect by binding to the microbial (e.g., bacterial) 30S ribosomal subunit and/or the 50S subunit, thereby inhibiting the translocation of the peptidyl-tRNA from the A-site to the P-site and/or causing misreading of mRNA, leaving the microorganism cells (e.g., bacterium cells) unable to synthesize proteins vital to its growth.

In another example, the antimicrobial agent is or comprises an ansamycin antibiotic selected from: geldanamycin, herbimycin, and rifaximin.

In another example, the antimicrobial agent is or comprises a macrolide. For example, the macrolide is or comprises a compound selected from: anhydroerythromycin A (also known as erythromycin anhydride), apoptolidin A, azithromycin, dafilomycin A1 *e.g.,* from *Streptomyces griseus,* bafilomycin B1 *e.g.,* from *Streptomyces sp.,* borrelidin *e.g.,* from *Streptomyces sp.* (such as *Streptomyces parvulus*), clarithromycin, clindamycin such as clindamycin hydrochloride lincosamide antibiotic or clindamycin 2-phosphate aminoglycoside antibiotic, concanamycin A *e.g.,* from *Streptomyces sp.,* dirithromycin, dirithromycin, erythromycin such as erythromycin estolate or erythromycin ethyl succinate or erythromycin stearate, filipin complex *e.g.,* from *Streptomyces filipinensis,* Josamycin, lincomycin such as lincomycin hydrochloride, mepartricin, nonactin *e.g.,* from *Streptomyces sp.,* (such as *Streptomyces griseus*), oligomycin *e.g.,* from *Streptomyces diastatochromogenes,* oligomycin A, pimaricin *e.g., Streptomyces chattanoogensis,* rapamycin *e.g.,* from *Streptomyces hygroscopicus* such as rapamycin sodium salt, rifapentine, rifaximin, roxithromycin, spiramycin *e.g.,* from *Streptomyces sp.* such as spiramycin adipate, tiamulin, telithromycin, troleandomycin, tylosin such as tylosin tartrate, virginiamycin S1 (also known as Antibiotic 899 or Staphylomycin S), and virginiamycin M1 (also known as mikamycin A or staphylomycin). Each of these macrolides is commercially available *e.g.,* from Sigma-Aldrich.

For example, the macrolide is or comprises a compound selected from: azithromycin, clarithromycin, dirithromycin, erythromycin, roxithromycin, troleandomycin, telithromycin, spiramycin.

The antimicrobial agent which is or comprises one or more macrolide(s) may provide antimicrobial effect such as antibacterial effect by inhibiting microbial (e.g., bacterial) protein biosynthesis by binding reversibly to the subunit 50S of the microbial ribosome, thereby inhibiting translocation of peptidyl tRNA *e.g.,* in actively dividing cells of the microorganism.

In another example, the antimicrobial agent is or comprises a tetracycline antibiotic such as a tetracycline selected from any one or more of: chlortetracycline such as chlortetracycline hydrochloride, demeclocycline such as demeclocycline hydrochloride, doxorubicin such as doxorubicin hydrochloride, doxycycline such as doxycycline hyclate, duramycin *e.g.,* from *Streptoverticillium cinnamoneus,* meclocycline such as meclocycline sulfosalicylate salt, minocycline such as minocycline hydrochloride, oxytetracycline such as oxytetracycline dehydrate or oxytetracycline hydrochloride, and tetracycline such as tetracycline hydrochloride. Each of these tetracyclines is commercially available *e.g.,* from Sigma-Aldrich.

The antimicrobial agent which is or comprises a tetracycline antibiotic may provide antimicrobial effect such as antibacterial effect by inhibiting binding of microbial aminoacyl-tRNA to the mRNA-ribosome complex *e.g.,* in actively dividing cells of the microorganism, for example by binding to the 30S ribosomal subunit in the mRNA translation complex of the microbial cells such as of actively dividing cells of the microorganism.

In another example, the antimicrobial agent is or comprises a polymixin antibiotic such as polymixin B and/or colistin, each of which is commercially available *e.g.,* from Sigma-Aldrich.

In another example, the antimicrobial agent is or comprises a lincosamide such as clindamycin and/or lincomycin, each of which is commercially available *e.g.,* from Sigma-Aldrich. For example, the antimicrobial agent which is or comprises the lincosamide(s) provides antimicrobial effect such as antibacterial effect by binding to the microbial (e.g., bacterial) 50S subunit of (e.g., bacterial) ribosomal RNA, thereby inhibiting protein synthesis in the actively dividing cells of the microorganism.

In another example, the antimicrobial agent is or comprises a nitrofuran such as furazolidone and/or nitrofurantoin, each of which is commercially available *e.g.,* from Sigma-Aldrich.

In another example, the antimicrobial agent is or comprises an oxazolidinone such as linezolid (e.g., Sigma-Aldrich) and/or posizolid (e.g., Sigma-Aldrich) and/or radezolid and/or torezolid.

In one such example, the antimicrobial agent which is or comprises an oxazolidinone provides antimicrobial effect such as antibacterial effect by inhibiting protein initiation of protein synthesis *e.g.,* in actively dividing cells of the microorganism.

In another example, the antimicrobial agent is or comprises a peptide or polypeptide or peptide derivative or polypeptide derivative. For example, the peptide or peptide derivative is selected from one or more of: actinomycin (actinomycin C1), actinonin, amastatin hydrochloride hydrate, 7-aminoactinomycin D (7-AAD), antimycin A (e.g., from *Streptomyces* sp.), antimycin A₂, antipain such as antipain dihydrochloride, bacitracin such as bacitracin zinc salt (e.g., from *Bacillus licheniformis*), bactenecin, BD-3 human recombinant (e.g., Sigma-Aldrich), Beta D-4 human recombinant (e.g., Sigma-Aldrich), carbenicillin such as carbenicillin disodium salt, cecropin A, cecropin B, cecropin P1 procine, cinnamycin *e.g.,* from *Streptomyces cinnamoneus,* colistin such colistin as sulfate salt or colistin sodium methanesulfonate, cyclosporin A e.g., from *Tolypocladium inflatum,* β-Defensin 1 human (e.g., Sigma-Aldrich), β-Defensin 2 human recombinant (e.g., Sigma-Aldrich), defensin HNP-1 human (e.g., Sigma-Aldrich), defensin HNP-2 human (e.g., Sigma-Aldrich), Elafin human (e.g., recombinant, expressed in *Saccharomyces cerevisiae*) (e.g., Sigma-Aldrich), fengycin (e.g., Sigma-Aldrich), gramicidin polypeptide e.g., from *Bacillus aneurinolyticus* such as gramicidin A polypeptide or gramicidin C polypeptide, Kendomycin *e.g.,* from *Streptomyces violaceoruber,* lactoferricin B (fragment 4-14 trifluoroacetate salt) (e.g., Sigma-Aldrich), LL-37 (human) trifluoroacetate salt (e.g., Sigma-Aldrich), lysostaphin *e.g.,* from *Staphylococcus staphylolyticus,* magainin I, magainin II, melittin *e.g.,* from honey bee venom, mycosubtilin, nisin e.g., *from Lactococcus lactis,* NP-1 human recombinant (e.g., Sigma-Aldrich), pediocin *e.g.,* from *Pediococcus acidilactici,* PGLa, Polymyxin B, Surfactin *e.g.,* from Bacillus subtilis, thiostrepton *e.g.,* from *Streptomyces azureus,* and valinomycin. All the above mentioned peptide or peptide derivatives are commercially available e.g., from Sigma-Aldrich.

In one such example, the antimicrobial agent is or comprises the bacitracin polypeptide. An antimicrobial agent which may be or comprises the bacitracin polypeptide may provide antimicrobial activity such as anti-bacterial activity by inhibiting the microbial isoprenyl pyrophosphate *e.g.,* in actively dividing cells of the microorganism. Without being bound by any specific theory or mode of action, the microbial isoprenyl pyrophosphate is a molecule that carries the building blocks of the peptidoglycan microbial *e.g.,* bacterial cell wall outside of the inner membrane of the cells such as in actively dividing cells of the microorganism. Accordingly, inhibiting activity of the isoprenyl pyrophosphate in actively dividing microbial cells by the bacitracin polypeptide prevents further cellular replication of the microorganism.

In another such example, the antimicrobial agent is or comprises the colistin polypeptide or the polymyxin B polypeptide. For example, such antimicrobial agent may provide antimicrobial activity such as anti-bacterial activity by interacting with a Gram-negative bacterial outer membrane and cytoplasmic membrane, and displacing bacterial counterions, thereby destabilizing the bacterial outer membrane. Alternatively, or in addition, such antimicrobial agent may function as a detergent against the cytoplasmic membrane *e.g.,* of actively dividing cell of the microorganism thereby altering their cellular permeability.

In another example, the antimicrobial agent is or comprises a glycopeptide. For example, the glycopeptide is selected from one or more of: vancomycin, teicoplanin, bleomycin such as bleomycin sulfate *e.g.,* from *Streptomyces verticillus,* phleomycin *e.g.,* from *Streptomyces verticillus,* ramoplanin, ristomycin *e.g.,* ristomycin monosulfate, vancomycin such as vancomycin hydrochloride *e.g.,* from *Streptomyces orientalis,* telavancin, dalbavancin, and oritavancin. All the above mentioned glycopeptides are commercially available *e.g.,* from Sigma-Aldrich. In one such example, the antimicrobial agent is or comprises a glycopeptide selected from teicoplanin, vancomycin, telavancin, dalbavancin and/or oritavancin. For example, the antimicrobial agent which is or comprises a glycopeptide provides antimicrobial effect such as antibacterial effect by binding to the microbial (e.g., bacterial) Inhibits peptidoglycan synthesis *e.g.,* of actively dividing cells of the microroganism.

In another example, the antimicrobial agent is or comprises a nucleoside. For example, the nucleoside is selected from: Adenine 9-β-D-arabinofuranoside, 5-Azacytidine, blasticidine S hydrochloride, cordycepin *e.g.,* from *Cordyceps militaris,* 5-Fluorocytosine nucleoside analog, formycin A *e.g.,* from *Streptomyces kaniharaensis,* ganciclovir, ribavirin [1-β-D-Ribofuranosyl-1,2,4-triazole-3-carboxamide], sangivamycin such as sangivamycin hydrate *e.g.,* from *Streptomyces rimosus,* sinefungin [5'-Deoxy-5'-(1,4-diamino-4-carboxybutyl)adenosine], tubercidin e.g., from *Streptomyces tubercidicus,* tunicamycin e.g., from *Streptomyces sp.,* and tunicamycin C₂ homolog. Each one of the above mentioned nucleosides is commercially available *e.g.,* from Sigma-Aldrich.

In another example, the antimicrobial agent is or comprises a phenicole compound. For example, the phenicole compound is selected from: chloramphenicol such as chloramphenicol succinate sodium salt and/or thiamphenicol [D-threo-2,2-Dichloro-N-(β-hydroxy-α-[hydroxymethyl]-4-[methylsulfonyl]phenethyl)acetamide], each of which is commercially available *e.g.,* from Sigma-Aldrich.

In another example, the antimicrobial agent is or comprises a polyene compound. For example, the polyene compound is selected from: Amphotericin B *e.g.,* form *Streptomyces* sp., kirromycin (also known as antibiotic Myc-8003 or mocimycin) *e.g.,* from *Streptomyces collinus,* and nystatin (also known as fungicidin or mycostatin), each of which is commercially available *e.g.,* from Sigma-Aldrich.

In another example, the antimicrobial agent is or comprises a polyether compound. For example, the polyether compound is selected from: monensin (also known as monensin A) such as monensin sodium salt or monensin sodium salt hydrate, nigericin (also known as antibiotic K178, antibiotic X464, azalomycin M, helexin C, polyetherin A) such as nigericin sodium salt, and Salinomycin *e.g.,* from *Streptomyces albus,* each of which is commercially available e.g., from Sigma-Aldrich.

In another example, the antimicrobial agent is or comprises a pyridines and/or pyrimidine compound. For example, the pyridines and/or pyrimidine compound is selected from: fusaric acid [5-Butylpyridine-2-carboxylic acid] e.g., from *Gibberella fujikuroi,* isoniazid [4-Pyridinecarboxylic acid hydrazide], prothionamide [2-propyl-4-pyridinecarbothioamide], tioconazole [1-[2-[(2-chloro-3-thienyl)methoxy]-2-(2,4-dichlorophenyl)ethyl]-1H-imidazole], trimethoprim [2,4-Diamino-5-(3,4,5-trimethoxybenzyl)pyrimidine] such as trimethoprim lactate salt. Each of these exemplified pyridines and/or pyrimidine compound is commercially available *e.g.,* from Sigma-Aldrich.

In another example, the antimicrobial agent is or comprises a quinolone compound and/or a fluoroquinolone compound. For example, the quinolone compound and/or a fluoroquinolone compound is/are selected from: cinoxacin [1-ethyl-1,4-dihydro-4-oxo-[1,3]dioxolo[4,5-g]cinnoline-3-carboxylic acid], ciprofloxacin [1-Cyclopropyl-6-fluoro-4-oxo-7-(piperazin-1-yl)-1,4-dihydroquinoline-3-carboxylic acid], difloxacin hydrochloride [6-Fluoro-1-(4-fluorophenyl)-1,4-dihydro-7-(4-methylpiperazino)-4-oxo-3-quinolinecarboxylic acid hydrochloride], enoxacin, enrofloxacin (also known as baytril), flumequine, 8-Hydroxyquinoline, nadifloxacin [9-fluoro-6,7-dihydro-8-(4-hydroxy-1-piperidinyl)-5-methyl-1-oxo-1H,5H-benzo[ij]quinolizine-2-carboxylic acid], nalidixic acid [4-Dihydro-1-ethyl-7-methyl-1,8-naphthyridin-4-one-3-carboxylic acid] such as nalidixic acid sodium salt, ofloxacin fluoroquinolone antibiotic (also known as fluoroquinolone antibiotic), oxolinic acid quinolone antibiotic (also known as quinolone antibiotic) [5,8-Dihydro-5-ethyl-8-oxo-1,3-dioxolo[4,5-g]quinoline-7-carboxylic acid], pefloxacin mesylate dehydrate [3-Quinolinecarboxylic acid, 1-ethyl-6-fluoro-1,4-dihydro-7-(4-methyl-1-piperazinyl)-4-oxo-, monomethanesulfonate], pipemidic acid [8-ethyl-5,8-dihydro-5-oxo-2-(1-piperazinyl)pyrido(2,3-d)pyrimidine-6-carboxylic acid], 8-quinolinol [8-Hydroxyquinoline] such as 8-quinolinol hemisulfate salt, levofloxacin, gatifloxacin, gemifloxacin, lomefloxacin, moxifloxacin, nalidixic acid, and norfloxacin. Each of these exemplified quinolones and fluoroquinolones is commercially available *e.g.,* from Sigma-Aldrich.

An antimicrobial agent which is or comprises quinolone compound and/or a fluoroquinolone compound may provide antimicrobial effect such as antibacterial effect by inhibiting the microbial (e.g., bacterial) DNA gyrase and/or the topoisomerase IV enzyme, thereby inhibiting DNA replication and transcription *e.g.,* in actively dividing cells of the microorganism.

In another example, the antimicrobial agent is or comprises a sulfonamide compound. For example, the sulfonamide compound is selected from: succinylsulfathiazole, sulfabenzamide [N-(4-aminobenzenesulfonyl)benzamide], sulfacetamide [N-(4-Aminobenzenesulfonyl)acetamide] such as sulfacetamide sodium salt monohydrate, sulfadiazine [4-Amino-N-(2-pyrimidinyl)benzenesulfonamide], sulfadimethoxine [4-Amino-N-(2,6-dimethoxy-4-pyrimidinyl)benzenesulfonamide], sulfadoxin [4-Amino-N-(5,6-dimethoxy-4-pyrimidinyl)benzenesulfonamide], silver sulfadiazine, sulfaguanidine [-amino-N-(aminoiminomethyl)benzenesulfonamide], sulfameter [5-Methoxysulfadiazine], sulfamethazine [4,6-Dimethylsulfadiazine], sulfamonomethoxine [4-methoxy-6-sulfanilamidopyrimidine], sulfanilamide [*p*-aminobenzenesulfonamide], sulfaquinoxaline *e.g.,* as sodium salt, sulfasalazine [2-hydroxy-5-{{4-[(2-pyridinylamino)sulfonyl]phenyl}azo}benzoic acid], sulfathiazole e.g., as sodium salt [4-amino-N-(2-thiazolyl)benzenesulfonamide sodium salt], sulfamethoxazole, mafenide, sulfamethizole, sulfanilamide, trimethoprim, trimethoprim-sulfamethoxazole (co-trimoxazole; also known as TMP-SMX) and sulfonamidochrysoidine. Each of these exemplified sulfonamides is commercially available e.g., from Sigma-Aldrich.

The antimicrobial agent which is or comprises a sulfonamide compound may provide antimicrobial effect such as antibacterial effect by inhibiting folate synthesis e.g., in actively dividing cells of the microorganism. For example, the sulfonamide compound may be a competitive inhibitor of the microbial enzyme dihydropteroate synthetase (DHPS). Accordingly, in the absence of the antimicrobial agent, the microbial DHPS may generally catalyze the conversion of PABA (para-aminobenzoate) to dihydropteroate, a step in folate synthesis. Folate may be necessary for microbial cells to synthesize nucleic acids (such as microbial DNA and/or RNA). Accordingly, the inventors speculate that antimicrobial agents comprising a sulfonamide compound may act to prevent synthesis microbial nuclei acids *e.g.,* in actively dividing cells of the microorganism.

In another example, the antimicrobial agent is or comprises a statin such as mevastatin (also known as compactin or ML-236B), which is commercially available *e.g.,* from Sigma-Aldrich.

In another example, the antimicrobial agent is or comprises a steroids such as fusidic acid sodium salt (also known as fucidin) which is commercially available *e.g.,* from Sigma-Aldrich.

In another example, the antimicrobial agent is or comprises a taxoide compound such as paclitaxel *e.g.,* from *Taxus brevifolia* which is commercially available *e.g.,* from Sigma-Aldrich.

Other exemplary antimicrobial agents encompassed within the scope of the methods of any aspect, embodiment or example of the invention described hereof, include antimicrobial agents comprising any one or more of the following: anisomycin [(2R,3S,4S)-2-(4-Methoxybenzyl)-3,4-pyrrolidinediol-3-acetate] *e.g.,* from *Streptomyces griseolus,* cycloheximide [3-[2-(3,5-Dimethyl-2-oxocyclohexyl)-2-hydroxyethyl]glutarimide] (also known as actidione or naramycin A), cytochalasin D (also known as Zygosporin A) *e.g.,* from *Zygosporium mansonii,* cytochalasin B (also known as Phomin) *e.g.,* from *Drechslera dematioidea,* emetine dihydrochloride hydrate, ionomycin such ionomycin as calcium salt *e.g.,* from *Streptomyces conglobatus,* and Piericidin A (also known as Shaoguanmycin B) *e.g.,* from *Streptomyces mobaraensis,* each of which is commercially available *e.g.,* from Sigma-Aldrich.

In another example, the antimicrobial agent is or comprises a peptidyl nucleoside. For example, the peptidyl nucleoside is any one or more of: nikkomycin Z *e.g.,* from *Streptomyces tendae,* nourseothricin sulfate and puromycin dihydrochloride e.g., from *Streptomyces alboniger.* All the above mentioned peptidyl nucleoside are commercially available *e.g.,* from Sigma-Aldrich.

In another example, the antimicrobial agent is an aureolic acid such as chromomycin A3 *e.g.,* from *Streptomyces griseus* and/or mithramycin A *e.g.,* from *Streptomyces plicatus,* each of which readily commercially available *e.g.,* form Sigma-Aldrich.

In another example, the antimicrobial agent is or comprises an aziridine compound such as mitomycin C *e.g.,* from *Streptomyces caespitosus* and/or Thio-TEPA [N,N',N"-Triethylenethiophosphoramide, also known as Tris(aziridinyl)phosphine sulfide], each of which is commercially available *e.g.,* from Sigma-Aldrich.

In another example, the antimicrobial agent is or comprises a benzenoid compound such as 17-(Allylamino)-17-demethoxygeldanamycin, 17-Dimethylaminoethylamino-17-demethoxygeldanamycin, geldanamycin *e.g.,* from *Streptomyces hygroscopicus,* and/or herbimycin A *e.g.,* from *Streptomyces hygroscopicus,* each of which is commercially available *e.g.,* from Sigma-Aldrich.

In another example, the antimicrobial agent is or comprises a Benzimidazole compound such as albendazole [also known as Methyl 5-(propylthio)-2-benzimidazolecarbamate] and/or ricobendazole [also known as Albendazole oxide, or Methyl [5-(propane-1-sulfinyl)-1H-benzoimidazol-2-yl]-carbamate], each of which is commercially available *e.g.,* from Sigma-Aldrich.

In another example, the antimicrobial agent is or comprises a coumarin-glycoside compound such as coumermycin A1 and/or novobiocin e.g., novobiocin sodium salt, each of which is commercially available *e.g.,* from Sigma-Aldrich.

In another example, the antimicrobial agent is or comprises a diphenyl ether derivative such as irgasan [also known as 5-Chloro-2-(2,4-dichlorophenoxy)phenol] which is commercially available *e.g.,* from Sigma-Aldrich.

In another example, the antimicrobial agent is or comprises an epipolythiodioxopiperazine such as gliotoxin from *Gliocladium fimbriatum* which is commercially available *e.g.,* from Sigma-Aldrich.

In another example, the antimicrobial agent is or comprises a fatty acid or a fatty acid derivative such as cerulenin [(2R,3S,E,E)-2,3-Epoxy-4-oxo-7,10-dodecadienamide] *e.g.,* from *Cephalosporium caerulens* which is commercially available *e.g.,* from Sigma-Aldrich.

In another example, the antimicrobial agent is or comprises a glucosamine such as 1-deoxymannojirimycin hydrochloride [1,5-Dideoxy-1,5-imino-D-mannitol hydrochloride] and/or N-methyl-1-deoxynojirimycin [1,5-ddeoxy-1,5-imino-1-methyl-D-sorbitol], each of which is commercially available *e.g.,* from Sigma-Aldrich.

In another example, the antimicrobial agent is or comprises an imidazole compound. For example, the imidazole compound is selected from: clotrimazole, econazole such as econazole nitrate salt, ketoconazole, metronidazole, miconazole, such as (±)-miconazole nitrate salt, sulconazole such as sulconazole nitrate salt, and thiabendazole, each of which is commercially available *e.g.,* from Sigma-Aldrich.

In another example, the antimicrobial agent is or comprises an indol derivative such as fumitremorgin C *e.g.,* from *Neosartorya fischeri* and/or staurosporine *e.g.,* from *Streptomyces sp*., each of which is commercially available e.g., from Sigma-Aldrich.

In another example, the antimicrobial agent is or comprises a macrolactam such as ascomycin *e.g.,* from *Streptomyces hygroscopicus* var. *ascomyceticus* which is commercially available *e.g.,* from Sigma-Aldrich.

In one particularly preferred example, the antimicrobial agent is or comprises an antibiotic. For example, the antibiotic may be a beta-lactam antibiotic such as penicillin antibiotic, a cephalosporin antibiotic, a monobactam antibiotic and/or a carbapenem antibiotic. For example, the antimicrobial agent is or comprises a cephalosporin antibiotic and/or a carbapenem antibiotic. The antimicrobial agent may be or comprises a cephalosporin antibiotic. Alternatively, the antimicrobial agent may be or comprises a carbapenem antibiotic

In one example, the beta-lactam antibiotic is a carbapenem antibiotic selected from meropenem, imipenem and ertapenem.

In another example, the beta-lactam antibiotic is a cephalosporin antibiotic selected from ceftazidime, cefepime and ceftriaxone. Preferably, the cephalosporin antibiotic is a ceftriaxone antibiotic. Ceftriaxone is a third generation cephalosporin antibiotics and may be used in the method of the present invention according to any aspect described hereof as an indicator of extended-spectrum beta-lactamase activity.

In another example, the antimicrobial agent may comprise or consist of a physical and/or chemical phenomenon or stress applied to the cells of the microorganisms described according to broad aspect, embodiment of example hereof. According to this example, the antimicrobial agent may comprise or consist of one or more physical and/or chemical phenomenon or stress applied to actively dividing cells of a microorganism selected from heat stress, ultraviolet light, blue light, radiation such as electromagnetic radiation, microwave radiation, radiation with X-rays or gamma rays, particle radiation such as alpha, beta or neurons particle radiation, and/or oxidative stress. According to this example, the antimicrobial exposure-dependent susceptibility determined by the acoustic flow cytometry method as described according to any broad aspect, embodiment of example hereof is that determined following exposure of actively dividing cells of a microorganism to one or more of the physical and/or chemical phenomenon or stress described above. The preparation of bacterial suspensions of the susceptibility analysis when applying according to the method of the invention and exposure parameters will be well understood to the skilled artisan and may be used as described in the art for conventional antimicrobial susceptibility testing.

### Exemplary combinations of microorganism / antimicrobial agents

In one preferred example of any broad aspect of the invention as described hereof, the microorganism present in the sample obtained from the subject is a bacteria belonging to the *Klebsiella* species and/or the cells of the microorganism present in the sample obtained from the subject are cells of bacteria belonging to the *Klebsiella* species, and the antimicrobial agent assayed in the method according to any broad aspect of the invention described hereof comprises a beta-lactam antibiotic. In one such preferred example, the microorganism is a strain of *K. pneumoniae* or *K. oxytoca* and/or the cells of the microorganism are cells of a strain of *K*. *pneumoniae* or *K. oxytoca,* and the antimicrobial agent comprises a carbapenem antibiotic such as meropenem and/or imipenem and/or ertapenem. In an alternative preferred example, the microorganism is a strain of *K. pneumoniae* and/or the cells of the microorganism are cells of a strain of *K. pneumoniae,* and the antimicrobial agent comprises a cephalosporin antibiotic such as ceftriaxone.

In an alternative preferred example of any broad aspect of the invention as described hereof, the microorganism present in the sample obtained from the subject is a bacteria belonging to the *Staphylococcus* species and/or the cells of the microorganism present in the sample obtained from the subject are cells of bacteria belonging to the *Staphylococcus* species, and the antimicrobial agent assayed in the method according to any broad aspect of the invention described hereof comprises a beta-lactam antibiotic. In one such preferred example, the microorganism is a strain of *Staphylococcus aureus* and/or the cells of the microorganism are cells of a strain of *Staphylococcus aureus,* and the antimicrobial agent comprises a cephalosporin antibiotic such as cefoxitin. In an alternative preferred example, the microorganism is a strain of *Staphylococcus aureus* and/or the cells of the microorganism are cells of a strain of *Staphylococcus aureus,* and the antimicrobial agent comprises a penicillin antibiotic such as oxacillin.

### High-throughput qualitative and/or quantitative screening for susceptibility to antimicrobial agents

The method according to any broad aspect, example or embodiment as described herein may be adapted for high-throughput qualitative and/or quantitative screening for susceptibility of microorganisms in or form a clinical sample to one or more antimicrobial agents simultaneously. The method can be adapted for simultaneous high-throughput screening of susceptibility of multiple sample of microorganisms obtained from different clinical samples and/or for multiple antimicrobial agents. This rapid high throughput method may be employed to rapidly predict qualitatively susceptibility of one or plurality of microorganisms to one or plurality of antimicrobial agents within about 1 to 3 hours from the time of obtaining the sample comprising cells of the microorganism from the subject; and/or within 1 to 3 hours from the time of first incubating cells of the microorganism in a culture medium to obtain actively dividing culture of the microorganism. Alternatively, or in addition, the rapid high throughput method can be employed to rapidly predict quantitatively susceptibility of one or plurality of microorganisms to one or plurality of antimicrobial agents by determining MIC FAST values within about 2 to 6 hours, preferably within about 2 to 4 hours, and most preferably within about 3 to about 4 hours, or about 3 hours from the time of obtaining the sample comprising cells of the microorganism from the subject; and/or within 1 to 3 from the time of obtaining the sample comprising cells of the microorganism from the subject; and/or from the time of first incubating cells of the microorganism in a culture medium to obtain actively dividing culture of the microorganism.

In one example, using a single 96 well plate, the high-throughput method can be used to determine qualitatively and/or quantitatively (by determining MIC FAST values) susceptibility of as many as 13 different microorganisms obtained from different clinical samples such as blood samples to one or more antimicrobial agents.

In another example, using a single 96 well plate, the high-throughput method can be used to simultaneously determine qualitatively and/or quantitatively (by determining MIC FAST values) susceptibility of a single microorganism (obtained from one clinical sample such as a blood sample) to as many as 13 different antimicrobial agents. In one such example, using a single 96 well plate, the high-throughput method can be used to simultaneously determine qualitatively and/or quantitatively (by determining MIC FAST values) susceptibility of two or more microorganisms (obtained from two or more clinical samples to at least four different antimicrobial agents for example tested at different concentrations of each antimicrobial agent of 0.25 mg/L, 0.5 mg/L, 1.0 mg/L, 2.0 mg/L, 4.0 mg/L, 8.0 mg/L, 16.0 mg/L, 32.0 mg/L, 64.0 mg/L, 128.0 mg/L, and 256.0 mg/L. In another such example, using a single 96 well plate, the high-throughput method can be used to simultaneously determine qualitatively and/or quantitatively (by determining MIC FAST values) susceptibility of two or more microorganisms (obtained from two or more clinical samples) to at least eight different antimicrobial agents for example tested at different concentrations of each antimicrobial agent e.g., selected from 0.25 mg/L, 0.5 mg/L, 1.0 mg/L, 2.0 mg/L, 4.0 mg/L, 8.0 mg/L, 16.0 mg/L, 32.0 mg/L, 64.0 mg/L, 128.0 mg/L, and/or 256.0 mg/L. In yet a further such example, using a single 96 well plate, the high-throughput method can be used to simultaneously determine qualitatively and/or quantitatively (by determining MIC FAST values) susceptibility at least one microorganism (obtained from a single clinical sample) to at least 13 different antimicrobial agents for example tested at different concentrations e.g., selected from 0.25 mg/L, 0.5 mg/L, 1.0 mg/L, 2.0 mg/L, 4.0 mg/L, 8.0 mg/L, 16.0 mg/L, and 32.0 mg/L. The person skill in the art would recognize that other permutations of number of microorganisms and antimicrobial agents other than those listed herein can be tested in the high-throughput method of the invention without affecting efficacy of the method to achieve rapid determination of susceptibility to antimicrobial agents tested.

### Examplary method 1

In one example, the high-throughput method comprises obtaining from a subject having or suspected of having infection with a microorganism (e.g., bacteria) a sample comprising cells of the microorganism. The sample obtained from the subject may be examined to determine that the sample comprises at least about 1.0 × 10⁵ actively dividing microbial cells. Alternatively, the cells of the microorganisms in or from the sample are incubated for a time and under conditions sufficient for aid cells to actively divide to thereby obtain actively dividing culture of said cells of said microorganism. It is preferred that the actively dividing culture of said cells of said microorganism comprises a concentration of actively dividing cells of the microorganism in the range of about 5.0 × 10³ to about 5.0 × 10⁷ microbial cells/ mL, preferably 5.0 × 10⁴ to about 5.0 × 10⁷ microbial cells / mL, and most preferably to a concentration of about 5.0 × 10⁵ microbial cells / mL. A person skilled in the art would be aware of techniques for measuring actively dividing cells in a sample or suspension. For example, a concentration of actively dividing cells may be determined using measurements of CO₂ production and/or using fluorescent reagents to indicate presence of a sufficient number or concentration of actively dividing microbial cells.

For example, 500 microliters of a 10% suspension (in 0.85% saline) of a non-ionic detergent (such as Triton-X100) may be added 500 microliters of a clinical sample or a culture sample comprising actively dividing microbial cells and incubated at room temperature to separate the microbial cells from non-microbia cells (e.g., blood cells) that may be present in the culture. Alternatively, filtration may be used to isolate and separate the microbial cells from the culture. The isolated actively dividing microbial cells may then be resuspended in a suitable culture medium described herein above such as in 1 mL of Hank's Balanced Salt Solution. A dilution such as 1:10 dilution in a suitable culture medium as described herein above such as Hank's Balanced Salt Solution may be performed for example in a total of 1mL of Hank's Balanced Salt Solution containing a working concentration of the nucleic acid binding fluorescent compound described herein (for example 5 µM of SYTO 9). Alternatively, a 1:10 dilution of the bacterial suspension in Hank's Balanced Salt Solution (optionally pre-warmed to 37°C) may be prepared.

This suspension containing the actively dividing microbial suspension cells is then analyzed using an acoustic flow cytometer. For example, an Attune NxT flow cytometer may be used, with the following settings: Flow Rate 25 microliters/minute, Recording Time 1 minute. On the basis of selecting events from the flow cytometry data that have FSC signal intensity greater than 10³ arbitrary fluorescence units, SSC intensity greater than 10³ arbitrary fluorescence units and 488nm-excited 515-545nm emitted fluorescence greater than 10² arbitrary fluorescence units (which are positive to staining with the nucleic acid binding fluorescent compound), a count for the number of bacteria/mL of positive blood culture may be obtained. The microbial cells suspension previously subjected to detergent treatment, can be centrifuged to separate the microbial cells from the rest of the suspension, and the cells may then be resuspended in a suitable culture medium described herein such as Mueller-Hinton Broth.

The microbial cell suspension may then be standardized by any technique or instrument capable of standardising microbial suspensions (e.g., using a nephelometer or a spectrophotometer measuring at OD at 600nm) such that the density of the microbial cells at the time of exposure to an antimicrobial agent is in the range of about 5.0 × 10³ to about 5.0 × 10⁷ 0⁷microbial cells per milliliter, preferably 5.0 × 10⁴ to about 5.0 × 10⁷ microbial cells per milliliter, and most preferably to a concentration of 5.0 × 10⁵ microbial cells per milliliter.

This standridised suspension may then be incubated e.g., at 37°C for a period of time that is one of: 30 minutes, 60 minutes, 90 minutes, 120 minutes, 150 minutes or 180 minutes.

The exact time of incubation may be determined by, at each increment of 30 minutes, performing the following steps: **(a)** removing an aliquot (*e.g*., 100 microliters) of the microbial cell suspension; **(b)** preparing a 1:10 dilution of the aliquot in a suitable culture medium described herein (e.g., by adding an amount of about 900 microliters of Hank's Balanced Salt Solution to the aliquot); **(c)** centrifuging the diluted aliquot suspension; **(d)** removing the supernatant from the centrifuged suspension; **(e)** stating the suspension with a nucleic acid dining fluorescent compound e.g., by adding 1mL of the culture medium e.g., Hank's Balanced Salt Solution containing a working concentration of the nucleic acid binding fluoresenct compound (e.g., of 5 µM SYTO 9); **(f)** analysing the stained suspension using the acoustic flow cytometer (e.g., Attune NxT flow cytometer) with the following settings: Flow Rate 25 microliters/minute, Recording Time 1 minute. On the basis of selecting events from the flow cytometry data that had FSC signal intensity greater than 10³ arbitrary fluorescence units, SSC intensity greater than 10³ arbitrary fluorescence units and 488nm-excited 515-545nm emitted fluorescence greater than 10² arbitrary fluorescence units (positive SYTO 9 staining), a count for the number of bacteria/mL of bacterial growth suspension; and **(g)** if this count reached 2.0 × 10⁶ bacteria per milliliter, the incubation is terminated and the assay progressed.

Alternatively, in situations where the biological sample collected from the subject is flagged positive for a microorganism and retained at 37°C, pre-incubation steps (a) to (f) can be omitted. In which case the suspension is standardized (for example using flow cytometry counts, a nephelometer or a spectrophotometer measuring OD 600) to a cell concentration approximately in the range of about 5.0 × 10³ to about 5.0 × 10⁷ microbial cells per milliliter, preferably about 5.0 × 10⁴ to about 5.0 × 10⁷ microbial cells per milliliter, and most preferably to a concentration of about 5.0 × 10⁵ microbial cells per milliliter.

In one example where pre-incubation steps (a) to (f) were not omitted, a working suspension of microorganisms may be prepared from this bacterial incubation suspension. The empirical determination of the number of microorganisms per microliter may be used to determine a volume of suspension to be added to a volume of Mueller-Hinton Broth (pre-warmed to 37°C) such that the final concentration of cells microorganisms is about 5.0 × 10³ to 5.0 × 10⁷ microbial cells / mL, preferably about 5.0 × 10⁵ microorganism / mL.

A volume of this microbial growth suspension can be added to each well of a standard format 96 well plate (e.g., Merck) containing an antimicrobial agent such as antibiotic standard to achieve a final concentration equal to the appropriate working suspensions of the antimicrobial agents such as antibiotics tested. It will be understood that the plate format and number of wells or samples that can be tested is not limited to standard 96 well plate format. The range of concentrations for each antimicrobial agent tested may be assayed at 0.25 mg/L, 0.5 mg/L, 1.0 mg/L, 2.0 mg/L, 4.0 mg/L, 8.0 mg/L, 16.0 mg/L, 32.0 mg/L, 64.0 mg/L, 128.0 mg/L, and/or 256.0 mg/L. However, any concertation ranges of the antimicrobial agent may be tested and is not limited to this concentration range.

Following inoculation with the antimicrobial agent(s), the 96-well plate may be incubated for about 30 minutes or about 60 minutes or about 90 minutes or about 120 minutes. For Gram negative bacteria it is preferred that incubation is for about 30 minutes. For Gram positive bacteria, the period of time may be matched to the pre-incubation time. As an example, if the microorganism took about 90 minutes to grow from 1.0 × 10⁶ to 2.0 ×10⁶ microbial cells per milliliter, the incubation with the antimicrobial agent may also be about 90 minutes. In the absence of a prior knowledge of the identity of the organism, the duration of the pre-incubation step may be matched with the antibiotic exposure step, described herein. After exposure to of the microbial cells to the antimicrobial agent, the 96-well plate is preferably subject to centrifugation for example at 2300 × g for 5 minutes, and supernatant is aspirated, and cells are resuspended in a saline solution (e.g., 0.85%).

The suspension of microbial cell which have been exposed to the antimicrobial agent that are now present in each well of the 96 well plate are then subjected to labelling with the nucleic acid binding fluorescent compound as described herein. For example, an aliquot of 40 microliters of the cells which have been resuspended in the saline solution is added to 160 microliters of Hank's Balanced Salt Solution stained with a suitable nucleic acid binding fluorescent compound such as SYTO 9 (for example to prepare a final working concentration of 5 µM SYTO 9.). The plate may then be incubated in the absence of light, at ambient temperature, for about 5 minutes to allow sufficient labelling.

The plate is then loaded into a suitable acoustic flow cytometer as described herein such as the Attune NxT Flow Cytometer by way of an Attune NxT Flow Cytometer AutoSampler. From each well of the plate, a sample is analysed by the acoustic flow cytometer. For example, where the Attune NxT Flow Cytometer, via the Attune NxT Flow Cytometer AutoSampler, is used the following settings may be applied: Flow Rate - 25 microliters per minute, Acquisition Volume - 18 microliters per well, Wait Before Recording - 8 seconds, Rinse Between Wells - active. However, where the dilution method replaces centrifugation of plates, the above Attune NxT Flow Cytometer AutoSampler setting may be replaced with the following settings: Flow Rate - 100 microliters per minute, Acquisition Volume - 60 microliters per well, Wait Before Recording - 8 seconds, Rinse Between Wells - active.

Data for each well of the 96 well plate may then be exported for analysis.

By way of example only, Data for each well of the 96 well plate is exported in FCS 3.1 file format from the Attune NxT acquisition software for analysis, and data each well of the 96 well plate are then imported into FlowJo v10.4.2. However it will be understood that any other software (such FCS Express, R (FlowCore and FlowViz)) capable of performing the below analysis steps would be acceptable. Alternatively, any statistical package capable of accepting the FCS data (converted to CSV files), capable of performing the variable subset analysis listed here (where a gate is considered a subset of data with the appropriate parameters) can be used. Examples of this are: R (BioConductor, FlowStats), Orange, bespoke statistical code written in any appropriate programming language (e.g., Python, Ruby, Visual Basic, C, Ansi C, C++).

To each data file, the following *in silico* analysis steps A to H can be applied: **(A)** A histogram gate is added, selecting all events of arbitrary fluorescence units in the 488nm excited, 515nm-545nm emitted fluorescence greater than 10². **(B)** On a plot of Forward Scatter - Area and Forward Scatter - Height, all events with a ratio of height to area below 1.5 are selected to ensure that only single cells are analyzed. **(C)** Data files corresponding with the unexposed control samples are identified. For assays where multiple control samples are run, the mode or median number of events per microliter (whichever is higher) is identified. **(D)** On a plot of Forward Scatter Height, and 488nm excited, 515nm-545nm emitted fluorescence, nearest neighbour contouring is applied at the 10% threshold, and a region bounded by the outermost contour is selected encompassing all of the events within this region. This designates the UCM gate. **(E)** The number of recorded events is determined, standardized as events per microliter, and identified as falling within the bound of the UCM gate for each data file sampled from the 96 well plate. This represented a count of microbial cells per microliter, with the measured optical and fluorescence parameters consistent with microorganisms not effected by exposure to the antimicrobial agent. **(F)** Using the software, he UCM gate is applied to a plot of Forward Scatter Height, and 488nm excited, 515nm-545nm emitted fluorescence to all data files from the assay. This step identifies the number of microbes per microliter, in each data file, consistent with microorganisms not effected by exposure to the antimicrobial agent. **(G)** For each data file across the assay, the number of events in the UCM gate per microlitre is divided by the number of events in the UCM gate per microlitre in the unexposed control sample from which the UCM gate was originally defined. This value may be expressed as a percentage to identify the proportion of cells of microorganisms in each data file with optical and fluorescence parameters consistent with microorganisms not effected by exposure to the antimicrobial agent (see results shown in Figure 23). **(H)** All files across the assay having received the same antimicrobial challenge were compared. The minimum inhibitory concentration (MIC) is defined as the lowest antimicrobial concentration at which the percentage of remaining microbial cells has fallen below a pre-determined antimicrobial agent-specific threshold.

With respect to step **(H)** above, for the purpose of defining the minimum inhibitory concentration (MIC) of the antimicrobial agent tested, the predetermined thresholds can be obtained for example by exposing 122 isolates (with a minimum of 34 resistant isolates) of the microorganism of interest, to the antimicrobial agent of interest in the manner described above to generate a hypothetical value - for example, a new antimicrobial agent may appear to have a threshold of susceptibility of 20% remaining when compared with the unexposed control. This threshold may then be tested by comparison to results generated, for the isolates of microorganism exposed to the same drug, by a traditional reference method such as broth microdilution. If the MIC correlation is greater than 0.90, for example with fewer than 3% "very major errors" ("very major errors" being a microorganism with a drug resistant phenotype being assayed as having an MIC consistent with a drug susceptible phenotype) the threshold is then confirmed and a new threshold is set.

In the absence of empirically obtained, statistically validated thresholds, arbitrary thresholds could be used. For example, for an antimicrobial agent with a known bacteriostatic effect (i.e., where the presence of the antimicrobial agent inhibits the growth of, but does not directly kill cells of the microorganism), 60% cells remaining, compared with the unexposed control, would be considered sufficient to make a determination of MIC. For an antimicrobial agent with a known bactericidal (a compound that directly kills microbial cells) activity, 30% cells remaining, compared with the unexposed control, may be sufficient to make a determination of MIC. For antimicrobial agents with an unknown mechanism of action, statistical certainty of inhibition can be achieved by applying a threshold at 10% of cells remaining, compared with the unexposed control.

### Examplary method 2

In another example, the high-throughput method may be a modified and simplified version of the exemplary method 1 described above. For example, the high-throughput method may comprise obtaining from a subject having or suspected of having infection with a microorganism (e.g., bacteria) a sample comprising cells of the microorganism. The sample obtained from the subject may be examined to determine that comprises at least about 1.0 × 10⁵ actively dividing microbial cells.

A person skilled in the art would be aware of techniques for measuring actively dividing cells in a sample or suspension. For example, a concentration of actively dividing cells may be determined using measurements of CO2 production and/or using fluorescent reagents to indicate presence of a sufficient number or concentration of actively dividing microbial cells.

For example, 500 microliters of a 0.1% suspension (in 0.85% saline) of a non-ionic detergent (such as Triton-X100) may be added 500 microliters of a clinical sample or a culture sample comprising actively dividing microbial cells and incubated at room temperature to separate and/or isolate the microbial cells from other components of present in the culture e.g., to separate the microbial cells from non-microbial cells such as blood cells. Alternatively, filtration may be used to isolate and separate the microbial cells from the culture.

A 1:10 dilution of the isolated actively dividing microbial cells can then be prepared by resuspending the cells in a suitable culture medium described herein such as Hank's Balanced Salt Solution. This suspension is then preferably analyzed with a nephelometer and, by the dropwise addition of the suitable culture media such as Hank's Balanced Salt Solution (e.g., pre-warmed to 37°C), diluted until it reaches parity, for example by the resolution of said nephelometer, with a 0.5 McFarland standard (0.05 ml of 1.0% barium chloride in 9.95mL of 1.0% sulfuric acid). This equates to approximately 1.5 × 10⁸ microbial cells /mL.

An aliquot of this suspension is added to a further volume of Mueller-Hinton Broth (pre-warmed to 37°C) such that the final concentration of microorganisms is in the range of about 5.0 × 10³ to about 5.0 × 10⁷ microbial cells per milliliter, preferably about 5.0 × 10⁴ to about 5.0 × 10⁷ microbial cells per milliliter, and most preferably at concertation of 5.0 × 10⁵ microbial cells per milliliter. A volume of this standardised microbial growth suspension is added to each well of a standard format 96 well plate containing antimicrobial agent (e.g., antibiotic) standard to achieve a final concentration equal to the appropriate working suspensions of the antimicrobial agents (e.g., antibiotics) being tested. The range of concentrations for each antimicrobial agent that may be tested is selected from 0.25 mg/L, 0.5 mg/L, 1.0 mg/L, 2.0 mg/L, 4.0 mg/L, 8.0 mg/L, 16.0 mg/L, 32.0 mg/L, 64.0 mg/L, 128.0 mg/L, and/or 256.0 mg/L.

Following inoculation of the microbial growth suspension with an antimicrobial agent, the 96-well plate may be incubated for either about 30 minutes or about 120 minutes. For Gram negative bacteria, incubation may be for 30 minutes. For microorganisms classified as Gram positive bacteria, or in the absence of a prior knowledge of the identity of the microorganism, the duration of the pre-incubation step may be set at about 120 minutes.

After exposure time with the antimicrobial agent has passed, the volume within each well is preferably supplemented with 4 volumes of a working solution of a nucleic acid binding fluorescent compound described herein, for example SYTO 9 stained Hank's Balanced Salt Solution such that the final SYTO 9 concentration is 5 µM. The plate is then incubated in the absence of light, at ambient temperature, for about 5 minutes to allow staining of the microbial cells. The plate may then be loaded into any suitable acoustic flow cytometer such as the Attune NxT Flow Cytometer by way of an Attune NxT Flow Cytometer AutoSampler.

From each well of the plate, a sample is analysed by the acoustic flow cytometer such as Attune NxT Flow Cytometer, via the Attune NxT Flow Cytometer AutoSampler for example using the following settings: Flow Rate - 100 microliters per minute, Acquisition Volume - 60 microliters per well, Wait Before Recording - 8 seconds, Rinse Between Wells - active.

SYTO 9 stained Hank's Balanced Salt Solution such that the final SYTO 9 concentration is 5 µM. The plate is then incubated in the absence of light, at ambient temperature, for about 5 minutes. The plate is loaded into the Attune NxT Flow Cytometer by way of an Attune NxT Flow Cytometer AutoSampler.

As in the exemplary method 1 described above, data for each well of the 96 well plate may then be exported for analysis. By way of example only, data for each well of the 96 well plate may be exported in FCS 3.1 file format from the Attune NxT acquisition software for analysis, and data each well of the 96 well plate are then imported into FlowJo v10.4.2. However it will be understood that any other software (such FCS Express, R (FlowCore and FlowViz)) capable of performing the below analysis steps would be acceptable or any statistical package capable of accepting the FCS data (converted to CSV files) and which is capable of performing a variable subset analysis where a gate is considered a subset of data with the appropriate parameters may be used.

For each data file, the *in silico* analysis steps A to H as already described in detail above in relation to exemplary method 1 may also be performed to determine the MIC values.

### Discussion

The results provided in the working examples that follow demonstrate that AFC-assisted susceptibility testing method of the invention was able to accurately and very rapidly qualitative profile susceptibility to antimicrobial agents of microorganisms e.g., bacteria into susceptible/non-susceptible classification. The results provided in the working examples herein, also demonstrate that AFC-assisted susceptibility testing method of the invention was further very effective in measuring of and/or predicting quantitative (MIC) susceptibility of the microorganisms to the antimicrobial agents.

For example, antimicrobial susceptibility profiling of carbapenem-resistant *K. pneumoniae* by AFC by the method of the present invention has predicted both quantitative (MIC) and qualitative (susceptible/non-susceptible) carbapenem susceptibility. While hydrodynamic flow cytometry may have been used for antimicrobial susceptibility testing before the present invention, the use of acoustic flow cytometry for antimicrobial susceptibility testing as exemplified in the working examples and detailed herein throughout is the first report of any validated method capable of generating clinically relevant quantitative end-points e.g., using any flow cytometry. Furthermore, prediction of qualitative and/or quantitative susceptibility achieved by the AFC-assisted susceptibility testing method of this invention is the fastest reported phenotypic determination of antimicrobial susceptibility achieved to date, has a high degree of accuracy, and represents a significant step forward to alignment of laboratory susceptibility of microorganisms with clinical decision timelines. The AFC-assisted susceptibility testing method of the present invention provides rapid phenotypic determination of antimicrobial susceptibility and accurately predicts qualitative results.

The internationally recognized susceptibility testing method by broth microdilution method is too labor intensive for use in clinical (e.g., pathology) laboratories, which favour other methods of susceptibility determination.

The description of the invention provided herein *inter alia* presents performance statistics for AFC-assisted susceptibility qualitative susceptibility test. The working examples herein are able to demonstrate the power of single-cell level analysis, with a view for possible adaptation of this method in the clinical laboratory. To the prescribing physician, a rapid qualitative susceptibility testing method such as that broadly described herein represents an ability to align the decision-making process of antimicrobial agents (*e.g.,* antibiotic) prescribing to the best-practice ideals of effective anti-microbial stewardship.

The AFC-assisted susceptibility test method as broadly described herein is suitable for application as a rapid method to determining resistance phenotype to antimicrobial agents on the ground of strong correlation between MIC_{BMD} and MIC_{FAST}. By way of example, the AFC-assisted susceptibility test method as broadly described herein is suitable for application as a rapid method to determined carbapenem resistance phenotype on the grounds of a strong correlation between MIC_{BMD} and MIC_{FAST} for carbapenem.

The MIC_{FAST} measure generated by AFC-assisted susceptibility test method of the present invention follows a pre-determined heuristic to generate quantitative results, rather than relying on potentially user-biased subjective end points. Any optional use of workspace templates can allow replication of results by non-specialists users after minimal instruction by a skilled operator using a software package such as the proprietary software package FlowJo. However, the use of proprietary software is not strictly necessary - any software platform capable of generating a contouring output may be suitable for use with the AFC-assisted susceptibility test method of this invention.

The robust nature of the AFC-assisted susceptibility test assay of this invention is *inter alia* underpinned by the reproducible flow cytometry modeling of a complex series of physiological interactions. For example, Forward Scatter (FSC) is often used a surrogate for particle size, but this oversimplifies the dynamic behaviour of non-spherical particles [Caron, G.N., Stephens, P., Badley, R. A. (1998). Assessment of bacterial viability status by flow cytometry and single cell sorting. J. Appl. Microbiol. 84, 988-98.] There is much more information in this single measurement than the size and orientation of a particle passing through the flow cytometer. For example, changes in granularity and autofluorescence profiles are also capable of altering the absolute numbers of photons reaching the FSC detector. In a similar manner, photons absorbed and emitted by fluorescence signals can also alter FSC measurements [Shapiro, H. M., Nebevon-Caron G. (2004). Multi-parameter flow cytometry of bacteria. Methods Mol. Biol. 263, 33-44].

The choice of use of a nucleic acid binding fluorescent compound *e.g.,* a natively fluorescent nucleic acid binding dye (such as SYTO 9) ensures that the measurement in the BL1 channel (*e.g.,* 530/30 nm) also contains information regarding cellular nucleic acid (e.g., DNA) content, cytoplasmic volume, and autofluorescence profiles.

Observed staining intensity profiles from a assuming rigorously controlled experimental method offer additional offer additional insight into physiological properties such as membrane permeability and efflux of fluorescent compound (e.g., dye) molecules. Isolates with the osmoporin *ompK36* third eyelet insertion mutation (ins AA 134-135 GD) can display reduced BL1 intensity *e.g.,* as shown in Hall, J.M. *et al.* (2014) and Hall, J. M., Ingram, P. R., O'Reilly, L. C., & Inglis, T. J. J. (2016) mentioned previously above. This porin mutation excludes positively charged compounds (such as SYTO 9) [Dutzler, R. et al. (1999). Crystal structure and functional characterization of the OmpK36, the osmoporin of K. pneumoniae. Structure. 7, 425-434]. The consistency of results obtained across a collection isolates such diverse geographic origins and resistance mechanisms supports a conserved microbial (*e.g.,* bacterial) physiology, and therefore supports the conserved nature of the underlying biology being quantified.

The physiological response we detected by the FAST method after antimicrobial exposure resembles the range of carbapenem-induced morphotypes described previously [Choi J. et al. (2014). A rapid antimicrobial susceptibility test based on single-cell morphological analysis. Sci Transl Med. 6, 267ra174]. Arrested cell division after inhibition of penicillin-binding proteins [Kitano, K., & Tomasz, A. (1979). Triggering of autolytic cell wall degradation in Escherichia coli by beta-lactam antibiotics. Amicrob. Agents Cemother. 16, 838-848; and Lakaye, B. et al. (1999). When drug inactivation renders the target irrelevant to antibiotic resistance: a case story with beta-lactams. Mol. Microbiol. 31, 89-101] leads to an overall increase in cellular DNA and increases the DNA-bound SYTO dye detectable in BL1. The overall decrease in cell numbers by fluorescence microscopy and flow cytometry, and the corresponding increase in flow cytometer event populations with low forward scatter and varied BL1, is likely to reflect mixed cell debris from bacterial cell lysis during antimicrobial exposure.

More specifically, orthogonal validation of the susceptibility-associated signature (SAS) by fluorescence microscopy (examples of which provided in Figure 4) provides further evidence of the underlying physiological response to antimicrobial insult. While the SAS describes the changes in prevalence of populations across biaxial AFC plots, aligning these changes to microscopy demonstrates a consistency with the known activity of meropenem in susceptible *Klebsiella* isolates. The elongation and ballooning of cells increases overall event size (increased FSC) and cytoplasmic volume (increased native-fluorescence SYTO 9 contribution to BL1). Failure of cell division following inhibition of penicillin-binding proteins [Kitano, K., & Tomasz, A. (1979); and Lakaye, B. et al. (1999)] occurs immediately prior to binary fission and results in an overall increase in genomic DNA content per cell (increase in DNA-bound SYTO 9 specific component of BL1). The overall decrease in cell numbers observed both by microscopy, and by AFC, showed an inverse association with low FSC/ variable BL1 event populations. The inventors speculate that populations are a mixture of proteinaceous debris, cell fragments, cell carcasses, and DNA-encapsulating lipid micelles that may be present following cell lysis dye to antimicrobial exposure.

Without being bound by any theory or any specific mechanism or mode of action, the inventors have speculated that in the context of the orthogonal validation of the susceptibility-associated signature (SAS), those minority sub-populations showing a resistance phenotype differing from the majority of the population in a single sample from a dilution series (such as those examples highlighted in Figure 6) posit biological relevance that only emerges from single-cell level analysis. The ability to discriminate between low prevalence contaminating isolates whether they were (in the case of Isolate 374's contamination with *S*. *aureus*) of differing species or morphologically distinct colonies of the same species, demonstrates the power of single-cell level analysis. BMD requires extended incubation to produce a suspension turbid to the naked eye. This period allows, under a strong selective pressure (such as following exposure to any antimicrobial agent), for a highly resistant sub-population to persist when the susceptible phenotype majority is inhibited. The significance of detection by acoustic flow-cytometry-assisted susceptibility test method of the present invention of sub-populations is two-fold. Firstly, it gives a resolution to measure the precise resistance phenotype of all events within the sample provided to the assay (including valuable information about multiple phenotypes), and secondly adds to the mounting burden evidence of naturally existing heterogeneity in resistance phenotype among otherwise homogenous seeming isolates.

In other words, the broth microdilution MIC method relies on a subjective end-point and requires extended incubation [Wiegand, I., Hilpert, K., & Hancock, R. E. W. (2008). Agar and broth dilution methods to determine the minimal inhibitory concentration (MIC) of antimicrobial substances. Nat. Protoc. 3, 163-175], allowing persistence of resistant sub-populations after inhibition of the susceptible majority of bacteria. On the other hand, the acoustic FAST method of the preset invention *inter alia* measures the resistance phenotype of all microorganism (e.g., bacterial) cells in each aliquot, and adds to the evidence that microorganisms that are resistant to the antimicrobial agent (e.g., carbapenem-resistant Enterobacteriaceae) are phenotypically heterogeneous.

Without being bound by any theory or specific mechanism or mode of action, the inventors have speculated that broth microdilution over-simplifies the susceptibility test method and overestimates the dose required to demonstrate antimicrobial efficacy.

Highly resistant bacterial sub-populations have been implicated in failed meropenem monotherapy before. These bacteria may respond to meropenem combination therapy provided sufficient breakthrough growth has not occurred [Qureshi, Z. A. et al. (2012). Treatment outcome of bacteremia due to KPC-producing Klebsiella pneumoniae: superiority of combination antimicrobial regimens. Antimicrob. Agents Chemother. 56, 2108-2113.31,34; and Nabarro, L. E. B., & Veeraraghavan, B. (2015). Combination therapy for carbapenem-resistant Enterobacteriaceae: increasing evidence, unanswered questions, potential solutions. Eur. J. Clin. Microbiol. Infect. Dis. 34, 2307-2311]. Therefore, identification of these features of bacterial susceptibility in a shorter time could become the basis of more timely antimicrobial treatment guidance.

While our method eliminates the necessity of the secondary culture step required for either broth microdilution or other growth-dependent qualitative susceptibility determination, optional additional development may be needed to purify bacteria directly from patient samples so that laboratory results are available to the physician within a shorter time frame, particularly for patients with sepsis and other severe infections.

Discrepancies ≥ 2 dilutions were observed between MIC_{BMD} and MIC_{FAST} for pAmpC- and IMP-4-producing isolates. These types of resistance cause inducible meropenem resistance phenotypes [Espedido, B., Iredell, J., Thomas, L., Zelynski, A. (2005). Wide dissemination of a carbapenemase plasmid among Gram-negative bacteria: implications of the variable phenotype. J. Clin. Microbiol. 43, 4918-4919.33; and Bartowsky, E., & Normark, S. (1993). Interactions of wild-type and mutant AmpR of Citrobacter freundii with target DNA. Mol. Microbiol. 10, 555-565]. Induction of meropenem resistant pAmpC-producing *K. pneumoniae* has been demonstrated following cytosolic accumulation of by-products of inhibited transpeptidation over time. Selection of low-prevalence sub-populations with constitutively expressed AmpC can also lead to rapid, time-dependent shifts in overall resistance phenotype [Jacobs, C., et al. (1994). Bacterial cell wall recycling provides cytosolic muropeptides as effectors for beta-lactamase induction. EMBO J. 13, 4684-4694; and Jacobs, C., Frere, J. M., & Normark, S. (1997). Cytosolic intermediates for cell wall biosynthesis and degradation control inducible beta-lactam resistance in Gram-negative bacteria. Cell. 88, 823-832.] Induction of expression does not occur within 30 minutes of antimicrobial exposure and may therefore contribute to discrepancies between MIC_{BMD} and MIC_{FAST}. In the case of IMP-4-producing isolates, high-level induced meropenem-resistance is thought to be caused by the intrinsically-resistant sub-populations [Espedido, B., Iredell, J., Thomas, L., Zelynski, A. (2005). Wide dissemination of a carbapenemase plasmid among Gram-negative bacteria: implications of the variable phenotype. J. Clin. Microbiol. 43, 4918-4919]. The presence of persistent populations at higher meropenem concentrations in the UCM gate indicates a sub-population of inducible IMP-4-mediated meropenem resistant cells. Identification of inducible resistance is a significant challenge with any rapid antimicrobial susceptibility test, but determination of the results shortly after the start of antimicrobial exposure should reduce the complex effect of prolonged exposure and improve the accuracy of test endpoints.

The AFC-assisted susceptibility test method of the present invention for determination of MIC_{FAST}, has potential many potential clinical and research applications such as resistant phenotype surveillance, determining the impact of altered environmental/chemical conditions on resistance phenotype, and modelling the interactions between mixed bacterial populations following antimicrobial insult. Another potential use of this method is its incorporation in a suite of orthogonal analyses that combine genomic and phenotypic investigations to assess the physiological features of a particular resistance phenotype (24-27, 33-38).

The precision of the AFC-assisted susceptibility test method of the present invention in determining and/or measuring quantitative and qualitative susceptibility to antimicrobial agents (e.g., to meropenem in *Klebsiella* species) compares favourably with the current international standard, while preferably returning results in about 1 (qualitative) to about 3 (quantitative) hours after receipt of primary culture - in most cases a full 24 hours earlier than current standard practice.

Also, the AFC-assisted susceptibility test method of the present invention enables transition from subjectively interpreted end-points to objectively-generated, single bacterial cell analysis that can improve the resolution of an antimicrobial susceptibility test, without sacrificing either precision or specificity.

Also, the strong positive correlation observed between MIC_{BMD} and MIC_{FAST} supports the application of the FAST method as a tool for a rapid assay of antimicrobial (*e.g*., carbapenem) resistance phenotype. Rather than relying on potentially user-biased subjective end-points, MIC_{FAST} measurements achieved by the AFC-assisted susceptibility test method of this invention follows a pre-determined heuristic to generate qualitative determinations..

To the prescribing physician, the rapid qualitative and quantitative susceptibility measurements/determination achieved by the method of the present invention represents an ability to improve the decision-making process of prescription to the best-practice ideals of effective anti-microbial stewardship. The method of the present invention eliminates the necessity of the secondary culture step required to perform either BMD or other growth-dependent qualitative susceptibility determination.

Furthermore, as demonstrated in the working examples that follow, the definition of unexposed cell morphotype (UCM) and its use in subsequent suscpetibilty determination/analysis is conducted in the present invention with the acoustic flow cytometer parameters that best differentiate the unexposed bacterial cell population from antimicrobial-dependent effects that comprise the exposure signature. Demonstration of an exposure signature is performed in the smallest number of acoustic flow cytometer channels that accurately reflect variance in the UCM, irrespective of which channels they may be. Therefore, in the methods of the present invention, the optimal combination of channels and interpretive threshold may differ with antimicrobial/species combinations. However, determination of variance from the UCM and its attribution to an antimicrobial effect may be performed with any clustering, contouring, sorting or classification tool that is capable of interpretation of the same acoustic flow cytometer data output that can be correlated with a numeric reference standard for antimicrobial susceptibility.

Futhermore, the current reference standard used in the art for measuring MIC (BMD) is a discontinuous measurement with naked eye determination of endpoint. BMD standard MIC is defined differently by different regulatory jurisdictions, and does not apply to antimicrobial/species combinations for which there is no accepted regulatory standard. A more general threshold for biomedical significance may be >90%, which has been widley used for lab processes that output a numeric result. However, when using BMD MIC for some antimicrobial/species combinations, the endpoint is gradual and NOT sharp, introducing error before a comparator test has commenced. A consequence is that some mechanisms of antimicrobial action will be concentration dependent and some will be time dependent, or a combination of both.

The superior accuracy of the acoustic flow cytometry method of the present invention enables us to demonstrate these effects, differentiate between them and determine the MIC levels of an antimicrobial agent for an infection microorganism present in the subject in real time, which cannot be acheived using BMD MIC standard techniques.

The present invention is described further in the following non-limiting examples:

### Example 1: Bacterial isolates

This example demonstrates assembly of a representative panel of known isolates of a microorganism to which MICs of antimicrobial agents are known. The representative panel can be used as a reference or standard for rapid determination of MICs from output data obtained by acoustic flow cytometry methods of the invention.

This example also demonstrates assembly of a representative panel of known isolates of a microorganism for validating susceptibility classification and susceptibility testing results obtained with the acoustic flow cytometry methods of the invention, against broth microdilution analysis to demonstrate effectiveness of the method of the present invention.

An internationally representative panel of 48 carbapenemase-producing *Klebsiella* isolates covering a susceptibility range to carbapenemase from sensitive to highly resistant was assembled. The isolates were collected from geographical different locations across the globe, as illustrated in Figure 1. In particular, the panel comprises of *Klebsiella pneumoniae* (n=45) and *Klebsiella oxytoca* (n=3) isolates which were collected from collaborators in England (n=9), Sweden (n=10), Norway (n=9), Sri Lanka (n=4), United States of American (culture collection isolates, n=3), and Australia (n=13) to ensure representation of a diverse panel of mechanisms and lineages. All 48 isolates are shown in Table 2.

The carbapenemase-producing *K. pneumoniae* isolates were obtained from the Western Australian Culture Collection (WACC, PathWest Laboratory Medicine, Western Australia which included American Type Culture Collection ATCC *K. pneumoniae:* ATCC BAA1705, ATCC BAA1706 and ATCC 700603), Public Health England's Antimicrobial Resistance and Healthcare Associated Infections (AMRHAI) Reference Unit, (London, UK); the Norwegian Public Health Institute (Oslo, Norway), the EUCAST development Laboratory (Växjö, Sweden) (Table 2).

All bacterial isolates listed in Table 2 are known in the art. For example, the isolate ATCC 700603 is a previously deposited (with ATCC) strain of *K. pneumoniae* ESBL and was employed in the method of the present as positive control (carbapenemase-producing positive control strain). The bacterial isolate ATCC BAA1706 was also previously deposited (with ATCC) strain of *K. pneumoniae* and was employed as a wild type control strain of *K. pneumoniae.* The isolate ATCC BAA1705 was also a previously deposited (with ATCC) strain of *K. pneumoniae* KPC and was as employed as a carbapenemase- producing control isolate. The remaining *Klebsiella* strains listed in Table 2 were all known and were previously described e.g., in Hall, J.M. et al. Molecular mechanisms of β-lactam resistance in carbapenemase-producing Klebsiella pneumoniae from Sri Lanka. J. Med. Microbiol. 63, 1087-92 (2014); and Hall, J. M., Ingram, P. R., O'Reilly, L. C., & Inglis, T. J. J. Temporal flux in beta-lactam resistance among Klebsiella pneumoniae in Western Australia. J. Med. Microbiol. 65, 429-437 (2016).

All referred isolates were from curated culture collections, with confirmed identity to species level, had known carbapenem MICs and a defined mechanism of carbapenem resistance for the majority of resistant isolates (Table 2). Accordingly, the internationally representative panel provided a broad coverage of geographic distribution, and antimicrobial resistance mechanisms. The collection contained 23 isolates with carbapenemases (KPC, NDM, VIM, IMP, and OXA-48 families), eight with extended-spectrum β-lactamases (ESBL) or plasmid encoded AmpC β-lactamases (pAMPC) and 17 with no demonstrable acquired beta-lactamases. Meropenem susceptibility phenotypes ranged from highly-susceptible (MIC ≤0.006 mg/L) to high-level resistance (MIC >256 mg/L).

Isolates were shipped to Perth, Western Australia under International Air Transport Association (IATA) regulations for biological goods and imported under Australian biosecurity regulations into an approved quarantine premises.

All imported isolates were subcultured onto 5% v/v horse blood agar, and had their species identity re-confirmed by MALDI-TOF (Bruker Daltonics, Germany). Isolates were stored in 15% glycerol brain heart infusion broth at -80°C for subsequent analysis.

Prior to susceptibility testing by the FAST method of the present invention and parallel broth microdilution MIC analysis, bacteria were recovered from frozen storage in accordance with ATCC guidelines to provide a standard cell density in suspension as previously described [in American Type Culture Collection. (2015). ATCC® Bacterial culture guide tips and techniques for culturing bacteria and bacteriophages (pp. 1-36). Manassas, Virginia: American Type Culture Collection]. This is illustrated in Figure 2. In summary, bacterial isolates were reconstituted from -80°C glycerol stocks, streaked on to 5% v/v horse blood agar plates for incubation at 37°C overnight to ensure purity of growth, then inoculated into 20 mL of trypticase soy broth (TSB) [comprising about 17g tryptone (pancreatic digest of casein), about 3.0g soytone (peptic digest of soybean), about 2.5g glucose (dextrose), about 5.0g sodium chloride, about 2.5g dipotassium phosphate, reconstituted in about 1L of water] for an incubation of 10-18 hours (overnight) to ensure recovery from cryopreservation-induced insult.

The initial experimental iteration used 10 isolates from the Western Australian Culture Collection (WACC, PathWest Laboratory Medicine, Western Australia) which included American Type Culture Collection (ATCC) *K. pneumoniae.* Further validation work was conducted after accepting a further 38 *Klebsiella* isolates from a series of international collaborating centres: the PHE Antimicrobial Resistance and Healthcare Associated Infections (AMRHAI) Reference Unit, (London, UK), the Norwegian Public Health Institute (Oslo, Norway), the EUCAST development Laboratory (Växjö, Sweden) and the University of Colombo, Sri Lanka [Hall, J.M. et al. (2014) Molecular mechanisms of β-lactam resistance in carbapenemase-producing Klebsiella pneumoniae from Sri Lanka. J. Med. Microbiol. 63, 1087-92]. As already mentioned above, this provided a broad coverage of geographic distribution, and resistance mechanisms.

### Example 2: Material and methods

### 1. Antimicrobial agents

The following antimicrobial agents were used: pharmaceutical grade meropenem (Ranbaxy, Haryana, India), and analytical grade imipenem, ertapenem, meropenem, ceftriaxone, cefoxitin and oxacillin (all from Sigma-Aldrich, Missouri, USA).

Lyophilised antibiotics were dissolved in sterile 0.85% saline to produce a 5120 mg/L stock, syringe-filtered at 0.1 µm using hydrophilic polyvinylidene fluoride (PVDF) syringe filter units (Millipore, Massachusetts), and stored below -20°C, Stored antibiotics were thawed immediately prior to use.

To prepare FAST working stocks of the antibiotics (e.g., FAST meropenem working stocks), stock suspensions were dispensed in serial 1:2 dilutions in filtered Mueller-Hinton broth (MHB) to give a dilution series of 1mL aliquots at concentrations ranging from 2560 mg/L to 2.5 mg/L.

### 2. Bulk Fluid Handling

All bulk fluids required for preparation of bacterial samples, or for operation of the acoustic flow cytometry, were filtered prior to use with 0.1 µm disposable polyethersulfone filter units (Sartorius, Stedim, Germany) to minimise particulate contamination. Small volume solutions (resuspension buffers, wash solutions etc.) were filtered using 0.1 µm disposable syringe filters (Merck, Darmstadt, Germany) immediately prior to use.

### 3. Preparation of bacteria and staining of actively dividing bacterial cells with a nucleic acid binding fluorescent compound

A 1 mL aliquot of bacterial suspension was centrifuged, washed and resuspended in 1 mL filtered Hank's Buffered Salt Solution (HBSS; comprising about 0.137 M NaCl, about 5.4 mM KCI, about 0.25 mM Na₂HPO₄, about 0.1g Glucose, about 0.44 mM KH₂PO₄, 1.3 mM CaCl₂, 1.0 mM MgSO₄, about 1.3 mM CaCl₂, 1.0 mM MgSO₄, and about 4.2 mM NaHCO₃) and diluted in series (e.g., in a 1:10 serial dilution) to a final 1:1000 dilution.

One µL (microliter) of SYTO 9 stain (e.g., ThermoFisher Scientific) working stock solution was added to the final dilution at a final concentration of 5 µM and incubated for 5 minutes before counting bacterial events by acoustic flow cytometer (AFC) (Attune, ThermoFisher, Eugene, OR, USA), which was used to prepare a standardised inoculum density for susceptibility testing.

An aliquot of bacterial suspension was added to 50 mL centrifuge tubes (Corning, New York) containing 9 mL of pre-warmed (37°C) filtered MHB to produce a final density of 5 × 10⁵ - 1 × 10⁶ bacteria per mL. This suspension was incubated at 37°C with shaking at 100 RPM for 30 minutes to obtain an actively dividing culture. An aliquot of antibiotic working solution from the previously described dilution series (appropriate to the concentration tested) was then added to each tube before a further 30-minute incubation with shaking, during which time a microbroth dilution (MBD) plate was prepared for overnight incubation at 37°C as previously described in Clinical and Laboratory Standards Institute (2015) Performance Standards for Antimicrobial Susceptibility Testing: Informational Supplement. CLSI document M100-S25. Clinical and Laboratory Standards Institute, 950, West Valley Road, Suite 2500, Wayne, Pennsylvania, 19087, USA.

One millilitre of bacterial suspension from each antibiotic concentration was harvested by centrifugation at 7800 × g for 5 minutes, washed and diluted 1:10 in filtered HBSS in a light-impermeable microcentrifuge tube, then stained with SYTO 9 stain at a final concentration of 5 µM, and incubated for 5 min before AFC sampling. Hoechst 33342 dye (NucBlue, Thermo Fisher Scientific, Eugene, OR, USA) and a SYTO 9/propidium iodide (PI) combination (Thermo Fisher Scientific) were used in a series of replicate experiments.

### 4. Acoustic Flow Cytometry (AFC) operation

The acoustic flow cytometer employed was Attune acoustic flow cytometer (Thermo Fisher Scientific), having installed therein a 488nm blue laser with a 530/30 nm filter on the photomultiplier tube for measuring fluorescence of cells assayed. Alternatively, the Attune NxT acoustic flow cytometer (Thermo Fisher Scientific) can be employed. AFC was calibrated at the beginning of each acquisition session using fluorescently labelled size calibration beads, in accordance with the manufacturer's instructions (ThermoFisher Scientific). The AFC settings were as follows: Forward Scatter (FSC) voltage 3100, FSC threshold 4 × 1000 AND, blue laser 1 (BL1 - 530/30 nm) voltage 1900, BL1 threshold 1 × 1000 AND, high sensitivity, flow rate 25 µL / minute, and an acquisition volume of 125 µL. Acquisition was halted after either collection of 20,000 events across all gates, or after 3 minutes, with each sample acquired in technical triplicate.

### 5. AFC data analysis

Collected data was exported in the FCS 3.0 file format and analysed in Flow v10.0 (FlowJo LLC, Ashland, OR, USA) by a single user, blinded to the MIC results by BMD (MIC_{BMD}).

### 6. Digital fluorescence microscopy

A 1 mL aliquot of growth from each antimicrobial concentration was harvested while conducting AFC measurements of antimicrobial-exposed bacteria, centrifuged at 7800 × g for 5 minutes, and resuspended in 10 µl of HBSS. A 0.1 µL aliquot of SYTO 9 stain was added to each tube (at a final concentration 50 µM) and incubated for 5 minutes. A 2 µl aliquot was placed on a poly-L-lysine slide, sealed beneath a coverslip, and observed at 60 x magnification by digital fluorescence microscopy. Samples were observed on the EVOS-FL digital fluorescence microscopy platform (Thermo Fisher, Eugene OR), with a representative field of view captured for each sample across the antimicrobial dilution series. Demonstrative high resolution images were acquired using a Nikon Ts2R Eclipse inverted digital fluorescence microscope (Nikon, Tokyo, Japan).

### 7. Qualitative susceptibility testing subset assembly

The inventors sought to determine whether a qualitative susceptibility test could be developed using the FAST platform illustrated in these examples. To this effect, a subsequent quantitative MIC data determination was performed as described below.

Subsequent to quantitative MIC_{FAST} determination, the acoustic flow cytometer data were re-analysed *in silico* to produce a limited sub-set for qualitative susceptibility determination. The six antibiotic dilutions (0 mg/L, 0.25 mg/L, 1 mg/L, 2 mg/L, 4 mg/L and 16 mg/L) most relevant to qualitative susceptibility assessment were used. *In silico* analysis was restricted to the first technical triplicate of each recorded sample. Gating strategies remained consistent, with the addition of a gate restricting analysis to only those events recorded in the first 60 seconds of acquisition. Isolates were defined as meropenem susceptible (S) or non-susceptible (NS) using the EUCAST clinical breakpoints for Enterobacteriaceae (S ≤2 mg/L, NS > 8 mg/L).

### 8. Statistical analysis

Statistical software (Prism v 6.1, GraphPad, San Diego, CA, USA) was used to analyse both S/NS categorization and quantitative MIC results. SINS results were analysed using a χ² format. Clinical laboratory test performance measurements (sensitivity, specificity, positive predictive value, negative predictive value, Matthews Correlation Coefficient) were used to assess the ability of the FAST method to correctly determine antimicrobial e.g., carbapenem susceptibility by qualitative breakpoint. The correlation between MIC_{BMD} and MIC_{FAST} was analysed by calculating Spearman's coefficient for non-parametric data. The MIC data were plotted on a log-log biaxial plot, using the microdilution results as the determinant (Prism, 6.1).

### 9. Discrepancy investigation

All isolates demonstrating anomalous SINS categories (MIC_{BMD} vs. MIC_{FAST}) or MIC discrepancies outside the tolerance of the microbroth assay (+/- one two-fold dilution step) were examined in more detail.

These isolates were all subcultured once per day for three days, to exclude the possibility of low prevalence contamination of cryo-preserved stocks by bacteria other than *Klebsiella* species. Any isolates displaying variable colony appearance on solid media had each observed colony morphotype sub-cultured separately, and their identity verified. The molecular basis of carbapenem resistance was reconfirmed using mechanism-specific PCR assays as previously described [Hall, J.M. et al. Molecular mechanisms of β-lactam resistance in carbapenemase-producing Klebsiella pneumoniae from Sri Lanka. J. Med. Microbiol. 63, 1087-92 (2014); and Goire N. et al., The implications of endemic IMP-4 carbapenemase for clinical laboratory susceptibility testing. J. Microbiol. Methods. 124, 10-2 (2016)].

In cases where contaminants or complex resistance mechanisms were identified, isolates were subjected to a further round of FAST following determination of identity and molecular basis of resistance.

### 10. Subpopulation investigation

Populations observed on bi-variate flow cytometry plots that seemed to segregate into two populations across a dilution series were observed in many isolates during validation.

To investigate this effect further, a demonstrative example (K16, an IMP-4 producing isolate) was selected and subjected to the FAST MIC on Day 1 assay detailed above. This was referred to as "Day one". Once sample preparation was completed, one mL of the bacterial suspension exposed to 4 mg/L meropenem was harvested (e.g., by centrifugation), washed in fresh HBSS to remove the presence of meropenem, and inoculated into fresh 19 mL TSB for overnight incubation to provide input for a second round of FAST on "Day two". On Day 2, FAST was repeated and final results compared. MIC_{FAST}, population shapes, and progression to susceptibility associated signature were compared between both experiments (i.e., Day one and Day two experiments).

### Example 3:

### Development of a rapid acoustic flow cytometry-assisted susceptibility test

This example outlines the development of a new AFC-assisted susceptibility testing method for assaying qualitatively and/or quantitatively susceptibility of a microorganism (*e*.*g*., a bacteria) in a biological sample to antimicrobial agent(s).

The methodology outlined in working examples 1 and 2 above together with the results provided in the working examples that follow illustrate development by the present inventors of a new and improved method by which susceptibility to an antimicrobial agent such as an antibiotic (in this case, exemplified using meropenem) can be rapidly assayed in any unicellular or multicellular microorganism such as a bacteria (in this case, exemplified in *K. pneumoniae*)*.* This new and improved method is briefly exemplified in the schematic diagram outlined in Figure 2.

The method was achieved using an acoustic flow cytometer to obtain optimal resolution of small particles, and a nucleic acid binding fluorescent compound such as a nucleic acid binding intercalating fluorophore dye (*e*.*g*., SYTO 9) to discriminate between bacterial events and background debris. Of the dyes or dye combinations tested by the inventors in Example 2, optimal results were achieved with SYTO 9. In the present working examples, changes in size, shape cytoplasmic volume and overall event numbers were used to predict susceptibility of *K*. *pneumoniae*) to meropenem in 1 hour, and MIC in 3 hours.

Figure 2 outlines the work flow as exemplified herein. In brief, an isolate was retrieved from cryopreservation, plated onto blood agar to ensure purity, and inoculated into trypticase soy broth (TSB, described above) overnight to simulate biological fluids. A 1 mL aliquot of this overnight suspension was inoculated into Mueller-Hinton broth (MHB) [comprising about 300.0g beef extract (dehydrated infusion from), about 17.5 g casein hydrolysate, about 1.5g starch, reconstituted in about 1L of water, pH 7.3 ± 0.1 as measured and kept at 25°C] and incubated at 37°C to ensure bacteria were actively dividing. One aliquot was subjected to traditional microbroth dilution (MBD) susceptibility testing. Another aliquot was exposed to meropenem for 30 minutes, harvested, stained with SYTO 9 dye, and then assayed with an acoustic flow cytometer.

### Example 4:

### Defining susceptibility to antimicrobial agent(s) by AFC

This example demonstrates the use of acoustic flow cytometry- assisted method of the invention to define susceptibility of a microorganism *e*.*g*., a bacteria to one or more antimicrobial agent(s). For instance, this example demonstrates use of acoustic flow cytometry-assisted method of the invention to define susceptibility of *Klebsiella* to meropenem.

Susceptibility to meropenem was defined by careful pairing of observed shifts in FSC and BL1 fluorescence (530/30nm - ideal collection for SYTO 9) in bi-axial AFC plots, and observation of bacterial structures consistent with meropenem compromise by fluorescence microscopy. Exposure of actively dividing meropenem-susceptible isolates to inhibitory concentrations of the drug has been demonstrated to produce a range of cellular morphotypes; cells elongate, swell, balloon, and eventually proceed to complete cell lysis as they become compromised [Choi J. et al., (2014). A rapid antimicrobial susceptibility test based on single-cell morphological analysis. Sci Transl Med. 6, 267ra174].

When comparison between microscopy and biaxial AFC plots were made, it was found that an increased prevalence of aberrant cell morphotypes (consistent with meropenem compromise) correlated with an increase in FSC, increased BL1 fluorescence, and formation of populations that contour independently on biaxial plots. In a meropenem- susceptible isolate, these changes were observed at the lowest concentration tested (Figure 3A), whereas in an isolate with a raised meropenem MIC, these changes were not observed at concentrations below the MIC_{BMD} (Figure 3B). When a not susceptible (i.e., resistant) isolate was exposed to concentration approaching, and exceeding, the MIC_{B}, the forward scatter (FSC) and BL1 changes associated with susceptibility were observed. For convenience, we refer here to this progressive change in morphotype approaching, and exceeding, the MIC_{BMD} as the susceptibility-associated signature (SAS).

In more detail, Figure 3, in panel (A), demonstrates that exposure of the *K*. *pneumoniae* susceptible type strain (ATCC 700603) produced an increase in forward scatter, SYTO 9 fluorescence, reduced overall event numbers, and formed a new contouring focus at the isolate's MIC (0.25 mg/L). At 32x MIC, a total of four contouring foci were observed, with an overall shift towards low forward scatter, low fluorescent debris. The progression of these features, when observed in combination, constitutes the susceptibility-associated signature. Colouring on the biaxial plots indicates separate clustering foci. Fluorescence micrographs (acquired at 60x magnification) show reduced overall cell numbers and increased aberrant cell morphotypes as meropenem concentration increases.

Figure 3, in panel (B), demonstrates that exposure of highly resistant clinical *K*. *pneumoniae* strain K8 to meropenem shows an absence of susceptibility-associated signature across clinically relevant meropenem concentrations by acoustic flow cytometer bi-axial plot, and an absence of aberrant cell morphotypes by fluorescence microscopy.

### Example 5:

### Standardised gating strategies results in reproducible quantitative end-point report

This example demonstrates a standardised gating strategy to produce FAST MIC measurements on AFC biaxial plots. The strategy as exemplified herein involves *inter alia* defining a rational gate of unexposed cellular morphology of the cells of the microorganism on AFC forward scatter (FSC) and fluorescence biaxial plots that capture acoustic flow cytometry events corresponding to all cells in the unexposed control sample having a cellular morphology corresponding a cellular morphology of the microorganism which is not compromised by the effects of the anti-microbial agent.

Bacterial events of interest were defined by SYTO 9 fluorescence greater than 10⁴ arbitrary fluorescence units. Aggregate and co-incident events were excluded by rational gating on FSC-A vs FSC-H bivariate plots, and background fluorescence (auto-fluorescence and electronic noise) was minimised by rational gating on populations of interest by BL1-H vs BL3-H plot (Figure 4A). Using the technical triplicate of the unexposed bacteria with the median BL1-H geometric mean fluorescence intensity (GMFI), the auto gate tool (FlowJo) was used to define a gate that bounded all contoured events on a bivariate contour plot of FSC-H vs BL1-H at the 10% threshold (Figure 4B). This gate and the events it bounded were referred to as the Unexposed Cell Morphotype (UCM), and the gate was then applied consistently to all samples across the antimicrobial agent dilution series. The absolute count of event numbers in this gate was calculated to give a comparable measure of UCM for each sample, standardised by volume (events / µL). Changes in the prevalence of morphotypes when bacteria were exposed to meropenem at concentrations approaching or exceeding the MIC_{BMD} were evident as a distinct susceptibility-associated signature. Iterative comparisons between UCM event rates / µL in antibiotic-exposed samples and the unexposed control samples were used to determine the flow-associated susceptibility test MIC (MIC_{FAST}).

### Example 6:

### Prediction of MIC_{BMD} by AFC

This example demonstrates the efficacy of the AFC-assisted susceptibility testing method of the invention to accurately measure the MIC of an antimicrobial agent to which a microorganism is susceptible, and demonstrates that MIC_{FAST} measurements obtained by the method of the invention are consistent with the internationally recognized standard for AST using MIC determination by broth microdilution (MIC_{BMD}). As such, this example demonstrates that quantitative susceptibly (MIC_{FAST}) measurements obtained by the AFC-assisted susceptibility testing method of the invention accurately (and rapidly) predicts MIC_{BMD} values of an antimicrobial agent to which a microorganism is susceptible.

Our initial range-finding series of 10 isolates demonstrated close correspondence between the meropenem concentration that caused appearance of the susceptibility-associated signature and the MIC_{BMD} values. Numbers of events bounded by the UCM gate per µL (UCMµ) in antibiotic-exposed samples were compared with unexposed control samples, with a particular focus on cell numbers falling into and out of gated regions in those samples displaying the susceptibility-associated signature. It was observed that the flow cytometer results accurately predicted meropenem MIC_{BMD} when a cut-off point was established as the first concentration in an antimicrobial dilution series in which two or more of the technical replicates had a UCMµ equal to or less than 30% of events falling into the UCM gate of the unexposed control (see Table 1 and Figure 5A). This concentration as referred to as the MIC_{FAST}.

Paired MIC_{BMD} and MIC_{FAST} results for the entire isolate collection are shown in Table 2. There was a strong positive correlation between MIC_{BMD} and MIC_{FAST} across the entire isolate collection (r = 0.913, p < 0.0001, sensitivity 1.00, specificity 0.90, positive predictive value 0.86, negative predictive value 1.00 and Matthew's correlation coefficient of 0.878) (Figure 5B).

MIC_{FAST} determination required 158 minutes from actively growing primary culture (35 minutes of incubation, 12 minutes for manual handling, 108 minutes for data acquisition, and 3 minutes of data interpretation from prepared workspace template).

### Example 7:

### Prediction of qualitative susceptibility by AFC

This example demonstrates that the AFC-assisted susceptibility testing method of the invention is also effective in rapidly provide qualitative susceptibility determination and effectively assign susceptibility categories (S/NS) to tested antimicrobial agent for the microorganism.

Qualitative meropenem susceptibility was assessed for the entire isolate collection from the previously described data subset.

Only three isolates were incorrectly determined; two isolates (KS1, OXA-181, and 500638, pAMPC) were incorrectly categorised as susceptible (S) despite being non-susceptible (NS) (MIC_{FAST} 2, MIC_{BMD} 4), however this two-fold inter-test variation is within the accepted tolerance of the broth microdilution (BMD) assay. K16 (IMP-4, MIC_{BMD} 16; MIC_{FAST} 2) was the subject of extensive further investigation.

Despite these isolates, the FAST susceptible / non-susceptible threshold / interpretive criterion was highly concordant with BMD-derived susceptibility (χ² = 37.03, df = 1, p < 0.0001, sensitivity 1.00, specificity 0.90, positive predictive value 0.875, negative predictive value, 1.00 Matthew's correlation coefficient 0.887).

Based on the conditions selected for the data set assembly, the theoretical time-to-result for this qualitative test is 54 minutes from actively dividing primary culture (35 minutes of incubation, 11 minutes for manual handling, 6 minutes for data acquisition, and 2 minutes of data interpretation from prepared workspace template).

**Table 1 - Assay validation with ten Klebsiella pneumoniae isolates exposed to therapeutic meropenem. Each isolate was exposed in biological triplicate, with each sample being acquired in technical triplicate by AFC, with the MIC reported as the mode of replicates. S/NS calls according to the following EUCAST criteria: (S: MIC ≤ 2 mg/L, NS: MIC > 2 mg/L).**

| | MIC_{BMD} | MIC_{FAST} | BMD S/NS | FAST S/NS |
|---|---|---|---|---|
| | ≤0.25 | ≤0.25 | S | S |
| ATCC 700603 | ≤0.25 | ≤0.25 | S | S |
| | ≤0.25 | ≤0.25 | S | S |
| | ≤0.25 | ≤0.25 | S | S |
| 43292 | ≤0.25 | ≤0.25 | S | S |
| | ≤0.25 | ≤0.25 | S | S |
| | ≤0.25 | ≤0.25 | S | S |
| 43358 | ≤0.25 | ≤0.25 | S | S |
| | ≤0.25 | ≤0.25 | S | S |
| | ≤0.25 | ≤0.25 | S | S |
| 18397 | ≤0.25 | ≤0.25 | S | S |
| | ≤0.25 | ≤0.25 | S | S |
| | 0.5 | 0.5 | S | S |
| 44271 | 0.5 | 0.5 | S | S |
| | 0.5 | 0.5 | S | S |
| | 4 | 4 | NS | NS |
| KS1 | 2 | 1 | S | S |
| | 2 | 2 | S | S |
| | 2 | 1 | S | S |
| KS11 | 4 | 1 | NS | S |
| | 4 | 2 | NS | S |
| | 16 | 8 | NS | NS |
| ATCC BAA1705 | 16 | 8 | NS | NS |
| | 16 | 8 | NS | NS |
| | 32 | 64 | NS | NS |
| K1 | 64 | 64 | NS | NS |
| | 32 | 32 | NS | NS |
| | 128 | 256 | NS | NS |
| K14 | 256 | 256 | NS | NS |
| | 256 | 128 | NS | NS |

In Table 1 above: MIC_{FAST} is FAST method MIC; MIC_{BMD} is broth microdilution MIC; S is susceptible to meropenem (MIC ≤ 2 mg/L); NS is non-susceptible to meropenem (MIC > 2 mg/L).

**Table 2 - Meropenem susceptibilities (mg/L) for 48 Klebsiella isolates from diverse locations and with diverse resistance mechanisms. 48 isolates were exposed to therapeutic grade meropenem and subjected to FAST in technical triplicate, with the MIC_{FAST} and MIC_{BMD} values reported as the mode of replicates. Interpretation was according to EUCAST criteria: (S = MIC ≤2 mg/L; NS = MIC >2 mg/L).**

| **Isolate** | **Country of Origin** | **Mechanism** | **Referring Lab's MIC** | **MIC_{BMD}** | **MIC_{FAST}** | **MBD S/NS** | **FAST S/NS** |
|---|---|---|---|---|---|---|---|
| 2440606 | England | | ≤ 0.06 | ≤0.25 | ≤0.25 | S | S |
| 2880622 | England | | ≤ 0.06 | ≤0.25 | ≤0.25 | S | S |
| 51575 | Sweden | ESBL | 0.064 | ≤0.25 | ≤0.25 | S | S |
| 515870 | Sweden | ESBL | 0.125 | ≤0.25 | ≤0.25 | S | S |
| ATCC 700603 | USA | | ≤ ≤0.25 | ≤0.25 | ≤0.25 | S | S |
| ATCC BAA 1706 | USA | | ≤ ≤0.25 | ≤0.25 | ≤0.25 | S | S |
| 3354 | Australia | | ≤ ≤0.25 | ≤0.25 | 0.5 | S | S |
| 18397 | Australia | | ≤ ≤0.25 | ≤0.25 | ≤0.25 | S | S |
| 43288 | Australia | | ≤ ≤0.25 | ≤0.25 | ≤0.25 | S | S |
| 43292 | Australia | | ≤ ≤0.25 | ≤0.25 | ≤0.25 | S | S |
| 43358 | Australia | | ≤ ≤0.25 | ≤0.25 | ≤0.25 | S | S |
| 43854 | Australia | | ≤ ≤0.25 | ≤0.25 | ≤0.25 | S | S |
| A1 | Norway | | S | ≤0.25 | ≤0.25 | S | S |
| A2 | Norway | | S | ≤0.25 | ≤0.25 | S | S |
| A3 | Norway | | S | ≤0.25 | ≤0.25 | S | S |
| A4 | Norway | | S | ≤0.25 | ≤0.25 | S | S |
| A7 | Norway | | S | ≤0.25 | ≤0.25 | S | S |
| A13 | Norway | | S | ≤0.25 | ≤0.25 | S | S |
| A15 | Norway | | S | ≤0.25 | ≤0.25 | S | S |
| A16 | Norway | | S | ≤0.25 | ≤0.25 | S | S |
| 2890369 | England | | 1 | ≤0.25 | 0.5 | S | S |
| 2980639 | England | | 1 | ≤0.25 | 0.5 | S | S |
| 10924 | Sweden | KPC | 4 | ≤0.25 | 0.5 | S | S |
| 3040820 | England | pAmpC | ≤ 0.06 | 0.5 | ≤0.25 | S | S |
| 270902 | England | | 0.125 | 0.5 | ≤0.25 | S | S |
| 44271 | Australia | | ≤ ≤0.25 | 0.5 | 0.5 | S | S |
| K23 | Australia | IMP - 4 | 0.5 | 2 | 0.5 | S | S |
| 500638 | Sweden | pAmpC | ≤0.25 | 4 | 2 | NS | NS |
| KS1 | Sri Lanka | OXA-181 | 1 | 4 | 2 | NS | S |
| 374 | Sweden | VIM | > 32 | 8 | 32 | NS | NS |
| K16 | Australia | IMP - 4 | 2 | 16 | 2 | NS | s |
| ATCC BAA 1705 | USA | KPC | 8 | 16 | 8 | NS | NS |
| 200822 | Sweden | KPC | 8 | 16 | 8 | NS | NS |
| A20 | Norway | NDM-1 | R | 16 | 16 | NS | NS |
| 70708 | Sweden | KPC | 16 | 32 | 32 | NS | NS |
| 71076 | Sweden | KPC | ≥ 32 | 32 | 32 | NS | NS |
| 3000770 | England | NDM-1 | > 32 | 32 | 8 | NS | NS |
| N17 | Sweden | NDM-1 | ≥ 32 | 32 | 32 | NS | NS |
| K2 | Australia | OXA-181 | 64 | 32 | 32 | NS | NS |
| 2880654 | England | KPC | 4 | 64 | 32 | NS | NS |
| 194 | Sweden | VIM | ≥ 32 | 64 | 16 | NS | NS |
| KS17 | Sri Lanka | OXA-181 | ≥ 32 | 64 | 64 | NS | NS |
| K1 | Australia | OXA-181 | 64 | 64 | 32 | NS | NS |
| KS2 | Sri Lanka | OXA-181 | ≥ 32 | 128 | 16 | NS | NS |
| KS23 | Sri Lanka | OXA-181 | ≥ 32 | 128 | 64 | NS | NS |
| 3080800 | England | KPC | > 32 | 256 | 32 | NS | NS |
| K8 | Australia | NDM-1 | > 256 | >256 | >256 | NS | NS |
| K14 | Australia | KPC | 256 | >256 | >256 | NS | NS |
| MIC_{FAST} : FAST method MIC; MIC_{BMD}: broth microdilution MIC | | | | | | | |
| S: Susceptible to meropenem, MIC ≤ 2 mg/L; NS: Non-susceptible to meropenem, MIC > 2 mg/L | | | | | | | |

### Example 8:

### AFC-assisted susceptibility test method can be applied to different antimicrobial agents

This example demonstrates that the AFC-assisted susceptibility testing method of the invention is also effective in rapidly determining susceptibility of microorganisms to antimicrobial agents. In particular, this example demonstrates that the method AFC-assisted susceptibility testing method of the invention can be applied to determining susceptibility to other carbapenem antibiotics.

To examine the applicability of the AFC-assisted susceptibility testing method of the invention to other carbapenems, carbapenem-susceptible (ATCC 700603) and resistant (i.e., non-susceptible; ATCC BAA 1705) stains were exposed to analytical grade meropenem, imipenem, ertapenem, and therapeutic grade meropenem. There was no difference between S/NS determination between MIC_{BMD} and qualitative FAST SINS across all tested conditions (see Table 3).

**Table 3 - Control K. pneumoniae strains exposed to carbapenems produced concordant results between broth microdilution and FAST. The ATCC control carbapenem susceptible (ATCC 700603) and control carbapenem resistant i.e., non-susceptible (ATCC BAA1705) K. pneumoniae strains were exposed to three carbapenems, across the limited clinical screen configuration of concentrations. There were no differences (outside the resolution of the broth microdilution test) in MIC_{FAST} and MIC_{BMD} observed.**

| Compound Used | ATCC 700603 | | ATCC BAA1705 | |
|---|---|---|---|---|
| | MIC_{FAST} | MIC_{BMD} | MIC_{FAST} | MIC_{BMD} |
| Therapeutic Meropenem | ≤0.25 | ≤0.25 | > 16 | > 16 |
| Analytical Meropenem | ≤0.25 | ≤0.25 | > 16 | > 16 |
| Analytical Ertapenem | ≤0.25 | ≤0.25 | > 16 | > 16 |
| Analytical Imipenem | 1 | 2 | > 16 | > 16 |

### Example 9:

### MIC_{BMD} vs MIC_{FAST} discrepancy analysis

Only five of the 48 (10.4%) isolates showed discrepancies between MIC_{BMD} and MIC_{FAST} that resulted in a different meropenem S/NS assessment. The first isolate 374, was found to contain a low prevalence *Staphylococcus aureus* contaminant. A single colony of the *K*. *pneumoniae* isolate present was sub-cultured, and re-analysed by FAST. Analysis of the pure *K*. *pneumoniae* growth produced perfect concordance between MIC_{BMD} and MIC_{FAST}.

Three of these isolates, two with an OXA-48-family enzyme and one with an IMP-4 (3000763, KS11 and K23 respectively), had MIC_{BMD} and MIC_{FAST} values within the two-fold dilution tolerance of the BMD the method, but straddled the EUCAST breakpoint. Accordingly, this was an error of classification, not an inaccuracy of the AFC-assisted susceptibility testing method of the invention.

The remaining isolate (K16, IMP-4) initially produced a MIC_{BMD} of 16 mg/L and a MIC_{FAST} of 2 mg/L. This isolate was sub-cultured once again to check for purity, whereupon smooth and rough colony variants were observed. In particular, while both these were 2-4 mm in size, mucoid, ivory-coloured, and circular, the dominant colony type was smooth in appearance, with a low prevalence form that appeared rough. Each colony was sub-cultured independently and subjected to FAST. Retesting of each colony type produced an MIC_{FAST} of 2 and 64 mg/L, respectively (Figure 6). The MIC_{BMD} (16 mg/L) and MIC_{FAST} of the rough colony variant (64 mg/L), were within the resolution of the BMD test, and resulted in a concordant SINS determination.

### Example 10:

### Identification of subpopulation and persisting populations in isolates with discrepant MIC results

Isolate K16, classified as susceptible by MIC_{FAST} was investigated across two days, as described previously herein. On Day 1, isolate K16 was found to contain a population of bacterial events consistent with unexposed cell morphotypes, that persisted until 16 mg/L on Day 1 (see Figure 7 - Day 1) (i.e., this is despite an MIC_{FAST} resulting in a susceptible classification of isolate K16).

Furthermore, on Day 2 the bacterial population characteristics exhibited a different progression towards the susceptibility-associated signature across the same meropenem dilution range. Bacterial cells exhibited a much higher forward scatter (FSC), without associated BL1 increase on Day 2, and starting at 4 mg/L a subpopulation of cells again became evident (Figure 7 - Day 2). Subpopulations such as these have been observed across approximately one third of all isolates assayed in the collection (n=17).

### Example 11:

### AFC-assisted susceptibility test method can be applied to additional antimicrobial agents

Further to the results shown in the working examples above, this particular example provides further demonstration that the AFC-assisted susceptibility testing method of the invention is effective in rapidly determining susceptibility of microorganisms to a range of antimicrobial agents. In particular, this example demonstrates that (in addition to the carbapenems antimicrobial agents exemplified previously) the AFC-assisted susceptibility testing method of the invention is also applicable to determining susceptibility to other antibiotics including cephalosporins *e*.*g*., as represented by ceftriaxone.

Ceftriaxone is a third generation cephalosporin and is commonly used in susceptibility testing of microorganisms as an indicator of extended-spectrum beta-lactamase activity.

14 *K. pneumoniae* isolates of the isolate listed in Table 2 (which include the 10 isolates listed in Table 1) were exposed to ceftriaxone as described above to determine susceptibility of these *K. pneumoniae* isolated to ceftriaxone and the MIC by the acoustic flow cytometry method of the present invention. Results were correlated to MIC_{BMD} values as before and are shown in Figure 8.

As shown in Figure 8, there was no difference between S/NS determination between MIC_{BMD} and qualitative FAST SINS and MIC_{BMD} across all tested dilutions ceftriaxone, thereby further demonstrating that the methods of the present invention are equally successful in determining rapid susceptibility and MIC values in respect of different types of antibiotics including carbapenems and cephalosporins beta-lactam antibiotics.

### Example 12:

### AFC-assisted susceptibility test method can be applied to additional microorganisms (part I)

Further to the results shown in the working above, this particular example provides further demonstration that the AFC-assisted susceptibility testing method of the invention is effective in rapidly determining susceptibility to antimicrobial agents of a range of different microorganism such as a range of different bacterial species, and also to a range of different antimicrobial agents. In particular, this example demonstrates that the acoustic flow cytometry assisted susceptibility method of the present invention can also be applied to rapidly determining susceptibility of *Staphylococcus aureus* bacteria to both cephalosporin antibiotics *e*.*g*., as represented by cefoxitin and penicillin antibiotics *e*.*g*., as represented by oxacillin.

The acoustic flow cytometry assisted method of the present invention was employed essentially as described in the previous working examples to determine susceptibility of *Staphylococcus aureus* to both cefoxitin and oxacillin. Susceptibility classification into SINS and quantitative MIC values obtained using the acoustic flow cytometry assisted method were compared to results obtained using the conventional broth microdilution (BMD) method. Results are shown in Table 4 (Panels A and B) below and in Figures 9-12. These results demonstrate utility of the method of the present invention in correctly and rapidly predicting susceptibility (S/SN) and MIC values obtained using BMD method.

**Table 4 - Acoustic flow cytometry (AFC) assisted susceptibility testing for Staphylococcus aureus in respect of cefoxitin antibiotic (Panel A) and oxacillin antibiotic (Panel B).**

| **(Panel A)** *S*. *aureus* - Cefoxitin | | | | | |
|---|---|---|---|---|---|
| ISOLATE | RESISTANCE MECHANISM | MIC (BMD) | MIC (FAST) | BMD (SINS) | FAST (SINS) |
| W0866111 | MRSA | 32 | 16 | NS | NS |
| W9346111 | MRSA | 32 | 32 | NS | NS |
| ATCC 29213 | MSSA | 4 | 2 | S | S |
| WACC 98 | MRSA | 128 | 64 | NS | NS |
| W....6111 | MRSA | 16 | 16 | NS | NS |
| W0236111 | MSSA | 2 | 4 | S | S |
| W0816111 | MSSA | 2 | 2 | S | S |
| W9196111 | MSSA | 2 | 4 | S | S |
| W0096111 | MSSA | 2 | 4 | S | S |
| W0296111 | MSSA | 4 | 4 | S | S |
| W0226111 | MSSA | 2 | 2 | S | S |
| W6496111 | MSSA | 4 | 4 | S | S |

| **(Panel B)** *S*. *aureus* - Oxacillin | | | | | |
|---|---|---|---|---|---|
| ISOLATE | RESISTANCE MECHANISM | MIC (BMD) | MIC (FAST) | BMD (SINS) | FAST (SINS) |
| W0866111 | MRSA | 16 | 16 | R | R |
| W9346111 | MRSA | 32 | 32 | R | R |
| W....6111 | MRSA | 8 | 16 | R | R |
| W0236111 | MSSA | 0.25 | ≤1 | S | S |
| W0816111 | MSSA | 0.25 | ≤1 | S | S |
| W9196111 | MSSA | 0.25 | ≤1 | S | S |
| W0096111 | MSSA | 0.25 | ≤1 | S | S |
| W0296111 | MSSA | 0.25 | ≤1 | S | S |
| W0226111 | MSSA | 0.25 | ≤1 | S | S |
| W6496111 | MSSA | 1 | 1 | S | S |
| ATCC 29213 | MSSA | 0.25 | ≤1 | S | S |
| WACC 98 | MRSA | 256 | 256 | R | R |

The material and methods utilized are briefly outlined herein below. The method used was similar to that outlined in the earlier examples *albeit* adjusted to allow for 3 hour incubation of *Staphylococcus aureus* to account for longer doubling times of *Staphylococcus aureus* relative to *K. pneumoniae* used in the previous examples.

### 1. Materials

1x Attune Focusing fluid; 1x Attune Wash fluid; 1x Attune Shutdown fluid; 100% reagent grade ethanol (0,1µm filtered); 5% sheep blood agar plates; Hanks Balanced Salt Solution (HBSS) (0,1µm filtered); Mueller Hinton Broth with 2% NaCl (MHB); Tryptic Soy Broth, 25 mL (TSB); MilliQ water (0.1 µm filtered); Bleach 10% (0,1 µm filtered); Analytical grade antibiotics (Sigma) - oxacillin / cefoxitin; tracking beads (Attune performance test) Life Technologies Ref 4449754; Falcon 50 mL Polystyrene Round-Bottom tube Ref 352054-sterile; Eppendorf tubes, safelock, 1,5 ml (Amber + blank); 0,1 µm (PES) Polyethersulfone Vacuum filter bottles (Sartorius Stedim) 1L; SYTO9 Nucleic acid stain Life Technologies Ref S34854 Lot 1746883; Surfynol; Sterivex GP 0.22 µm Filter Unit Cat No: SVGP01015.

### 2. Culture of microorganisms

Bacterial stocks were from storage and aseptically streaked out for single colonies on a pre-warmed (35°C for 10 minutes) blood agar plate, return to the incubator and incubated at atmospheric gas conditions at 35°C (overnight). If initial stocks were retrieved from cryopreserved glycerol stocks, cultivation was repeated to ensure no aberrant phenotypic expression resulting from cryopreservation remained.

One cultured colony was emulsified in 25mL TSB and incubate overnight at 35°C. Optionally, cultures were placed on a shaking platform to increase growth rates.

### 3. Preparation of acoustic flow cytometer

The acoustic flow cytometer (AFC) (Attune acoustic flow cytometer from Thermo Fisher Scientific) used was supplied with entirely filtered fluids. When the AFC device was previously used without pre-filtered fluids, all previous fluid stocks were decanted from all tanks. 100% reagent grade ethanol was filtered using a 0.1 µm filter bottle. All fluid reservoirs were rinsed with filtered ethanol, the ethanol discarded and device allowed to air dry air dry. Optionally this was repeated twice. High purity (de-ionised or double-distilled) water was filtered using a 0.1 µm filter bottle. All fluid reservoirs were thoroughly. All reservoirs were with appropriate Attune solutions. Using the Attune software suite, a 60 cycle clean was set, using the 0.1 µm filtered Attune Wash solution. Optionally this preparation was repeated once more.

### 4. Preparation of fluids

HBSS and MiliQ water were filtered using 0.1 µm filter bottles. A 1 mL aliquot was dispensed into a 1.5mL tube (one stained and one unstained). The Attune settings described below were used for the stained sample. Events were diffused with no obvious clustering focus, at event rates less than 50 events/ µL being acceptable event rates. If this was not the case, the filtration step was repeated until desired level was reached. For the unstained sample set threshold was ignored.

### 5. Daily Performance Testing

All fluid tanks were checked and made sure levels were greater than 50% (for Focusing Fluid, Wash Solution and Shutdown Solution) or less than 20% (for Waste). The fluid was decanted from the Waste Tank into a container for safe disposal. Approximately 10% of the Waste tank was filled with bleach solution.

The Attune was turned on. The software suite was launched and logged in. The fluidics Startup script was run. To ensure effective flushing of the fluidics system, the Startup script was optionally repeated up to a total of three times, or highly filtered MilliQ water was optionally flushed through the system (at the 1000 µL/minute rate) for no less than about 5 minutes.

A 1.5 mL tube was used, and a suspension of Attune Tracking Beads for the performance test was prepared. One mL of filtered Attune focusing fluid was added, 1 drops of tracking beads and then vortexed for greater than 3 seconds. The tube was loaded onto the SIP and then the automated performance test was run. For small particle analysis, CV values were determined as follows: FSC: <5%; SSC: <7.5%; Blue/Red Laser: <3%; Violet Laser: <3.5%. When the performance test did not reach these specifications, cleaning and/or decontamination procedures was proceeded with until quality control settings were achieved.

### 6. Attune acoustic flow cytometry settings

Events were acquired by forward scatter and BL1 (515nm - 545nm) using the Attune with voltages and thresholding set as follows: Forward Scatter Voltage: 3050; Forward Scatter Threshold: 3×1000 AND; Side Scatter Voltage: 2950; Side Scatter Threshold: IGNORE; BL1 Voltage: 1500; BL1 Threshold: 1×1000 AND.

Before recording data on Attune, no less than 8 µL of sample was allowed to be dispensed. This allowed fluctuations in the fluidics to stabilise and ensure efficient sample stream focusing before data was collected. In the stained aliquot, the density (events / µL) of the events were noted.

### 7. Preparation of test bacterial suspension

Nine mL of MHB was aliquoted into twelve, 50mL Falcon tubes. Each tube was labelled with the required final test concentration of antibiotic. For example: 0, 0.25, 0.5, 1, 2, 4, 8, 16, 32, 64, 128 and 256. Tubes were transferred to a 35°C incubator to pre-warm.

One 1mL of the overnight bacterial suspension was transferred into a 1.5mL microcentrifuge tube and centrifuge at 7,800×g for about 5 minutes. The supernatant was discarded, and the pellet re-suspended in 1mL of 0.1 µm filtered HBSS. This step was optionally repeat.

About 900 µL of HBSS was dispended into three 1.5 mL tubes - two clear tubes and one amber tube. About 100 µL of the bacterial suspension was inoculated into the first tube (clear) and serially dilute to reach 1:1000 by the third tube (amber). About 1µl of SYTO 9 (5µM final concentration) was added to the third tube. The dye was incubated, protected from light, for at least 5 minutes (individual sample types were optionally required with longer incubations) and then the Stained Control on the Attune acoustic flow cytometer instrument was read

The event density (events/ µL) from your stained control aliquots was used to calculate the inoculum volume required to produce a 5.5× 10⁵ bacteria mL-1 in 10mL of MHB. For example, (400 events/µL) / (10 mL × 550000) = 7.3µL, rounded up to the closest 10µL.

The required volume of bacterial suspension was added from the overnight culture into each 50 ml tube with 9 ml of preheated MHB. Optionally incubate with shaking and protected from light, at 100 RPM at 35°C for 60 minutes.

### 8. Antibiotic preparation

MHB (0.1 µm filtered) was used to dilute the antibiotics in this preparation. About one aliquot of antibiotic was removed from the -20°C freezer and equilibrated to room temperature by ambient heat exchange, and diluted to reach a range of working stock concentrations that were are ten times the desired test concentrations. As per the example above, working concentrations (prior to adding to 50mL Falcon tubes) were as follows: 0, 2.5, 5, 10, 20, 40, 80, 160, 320, 640, 1280 and 2560.

Alternatively, large stocks of working concentrations of antibiotic were prepared, aliquoted and stored away at -20°C prior to use. The antibiotics were ensured not to be exposed to more than one freeze-thaw event.

### 9. Exposure of bacterial suspensions to antibiotics

Once the 60-minute incubation of bacterial suspension was complete, about 1mL of 10x antibiotic stocks were added to tubes containing bacterial suspensions to reach final desired test concentrations (1ml antibiotic and total volume 10 ml). The 50 mL tubes were returned to the incubator and incubated, by shaking at 100 RPM at 35°C, for a further 3 hours.

### 10. Washing the exposed cells

After 3 hours, about 1mL suspension was harvested from each tube and transferred to a 1.5 mL clear microcentrifuge tube (marked with the corresponding antibiotic concentration), then centrifuge at 7,800×g for 5 minutes. The supernatant discarded and the pellet resuspended in 1mL of 0.1 µm filtered HBSS. This wash step was optionally repeated.

### 11. Preparation of dye

The vial of SYTO 9 was removed from refrigerated storage and immediately wrapped in aluminium foil to protect from light. Using the microcentrifuge, any dye that was adhered to the cap was descended with a 30 second spin at full power, then allowed to equilibrate to room temperature before use - this can was confirmed visually by dissolution of DMSO crystal from dye stock to liquid form. The tube threads were confirmed to be clear of dye before opening the vial - the previous centrifugation was optionally repeated multiple times to achieve this.

### 12. Setting up the Attune Mkl (acoustic flow cytometer)

Using the computer connected to the Attune, new samples were created ready for the collection of data. That the following parameters were set: Sensitivity: High Sensitivity; Flow Rate: 25 µL/minute; Acquisition Volume: 125 µL; Events Collected: No less than 20,000 from all gates, Time: 1 minute.

At this time, the instrument settings were double checked to ensure that all voltages and thresholds were correct across the new samples created in your workspace.

### 13. Staining cells

About 899 µL HBSS was dispensed into one 1.5mL amber tube per sample. About 100 µL of suspension per tube was added to achieve a 1 in 10 dilution. About 1µl of SYTO 9 was added to each tube and the tube vortexed for no more than 2 seconds (over vortexing can lead to bacterial clustering and/or attachment to tube walls).

At least 5 minutes were allowed to ensure all bacterial samples equilibrate fully with free dye. For new sample types, a range of staining conditions were investigated to ensure optimal staining.

samples were optionally stained sequentially such that no individual sample was left to incubate with dye longer than others due to time spent acquiring previous aliquots on the acoustic flow cytometer.

### 14. Control samples

About 1mL of filtered HBSS were aliquoted into an amber tube. Stained under the same conditions as your bacterial samples. This established background fluorescence of any components within the HBSS and served as a negative control.

About 899 µL of filtered HBSS were aliquoted into an amber tube. About 100 µL of the unexposed, washed bacterial sample were added and vortexed to mix. This unstained sample controls for specific activity of the dye. For daily quality assurance, an absence of a visible bacterial population in this sample was ideal. For validation work, the BL1 threshold was removed before the sample was recorded.

### 15. Acquiring Data

Samples were placed on the SIP and acquired, in technical triplicate, as per the parameters outlined above. The SIP did not need to be lowered between technical triplicates (Due to *S*. *aureus* being autofluorescent by nature, the SIP were lowered between the unstained triplicates as the autofluorescense profile was changed between the triplicates due to unknown reasons), as long as each sample was allowed to proceed until 8 µL had been passed through the instrument before recording. Between samples, an absorbent wipe was used to blot any liquid from the tip of the needle, without applying any pressure directly to the needle itself.

### 16. Cleaning the Attune acoustic flow cytometer

With each experiment run, the following cleaning procedures were be performed to ensure no live organisms remain in the instrument, and no particulate matter began to deposit. If this was not done, the quality of data produced might have been at risk of degrading over time.

A new sample entitled "Clean" was created, and the following parameters were set: Sensitivity: Standard; Flow Rate: 1000 µL / minute; Acquisition Volume: 4000 µL; Time: 4 minutes.

The following solutions were passed through the acoustic flow cytometer, in order: 5mL 10% filtered bleach; 5mL Attune Wash solution; 5mL MilliQ. Optionally, samples were recorded without the initial run step, as the data was not required.

### 17. Decontamination procedures for the the Attune acoustic flow cytometer samples

If debris particulates were observed in otherwise clean samples, optionally using Surfynol to clean the fluidics system was found to be helpful. For this effect, about 10 µL was to 1mL of Attune focusing fluid and ran through the system at the following settings: Sensitivity: Standard; Flow Rate: max; Acquisition Volume: 850 µL; Time: 1 minute.

Once this was complete, the wash procedures outlined above was run through. Optionally this step was repeated multiple times.

If this did not resolve any contamination issues, it was proceeded with running the System Decontamination function in the Attune software suite. Instructions appeared on screen, and were followed explicitly. During this process, the rear focusing fluid filter was changed. The Attune cytometer was moved to a position where the window on the left hand side was easily accessed. Particular care was taken not to drop or strike the instrument against another surface to avoid altering laser alignments. Once the wash steps have finished, a new filter was installed between the two ends of the fluidics lines.

### Example 13:

### AFC-assisted susceptibility test method can be applied to additional microorganisms (part II)

This example further demonstrates that the acoustic flow cytometry assisted susceptibility method of the present invention can also be applied to rapidly determining susceptibility of *Staphylococcus aureus* bacteria to both cephalosporin antibiotics e.g., as represented by cefoxitin and penicillin antibiotics e.g., as represented by oxacillin.

Methicillin resistance in *Staphylococcus aureus* is associated with poor patient outcomes, especially in developing nations and indigenous populations. Current detection methods either rely on molecular methods, and report potential resistance, or are limited by growth-based assays measuring the antimicrobial resistance phenotype at a gross population level.

Routinely employed phenotypic methods of antimicrobial susceptibility testing in *S*. *aureus* require at least 48 hours from sample receipt. This example demonstrates an AFC-assisted susceptibility testing method of this invention that reports single-cell level results, of clinical significance, within about 4 hours of primary culture.

Using a representative collection of methicillin resistant, and methicillin sensitive *S*. *aureus* isolates from the American Type Culture Collection (ATCC) and West Australian Sterile Sites Collection (WACC) e.g., as outlined in Example 12, this example employs the AFC-assisted susceptibility testing method of the invention for carbapenem resistance to screen oxacillin (OXA) and cefoxitin (FOX) against *S*. *aureus.* Using a flow cytometer and SYTO 9 (a green, fluorescent, nucleic acid dye), with confirmatory fluorescence microscopy, populations of single cells were analysed and compared with unexposed controls, to report a susceptible/non-susceptible result (SINS), as well as full minimum inhibitory concentration (MIC) as described herein above. Exemplary results for control methicillin sensitive S. *aureus* (MSSA) isolate ATCC 25912 and clinical methicillin resistant S. *aureus* (MRSA) isolate are shown in Figures 13 and 14.

Microscopy showed consistent results across exposure to both antimicrobials. As drug concentration increased, overall cell numbers decreased, cells switched from clusters to single planktonic cells, and an increase in fluorescence was observed. Flow cytometry data were used to develop a bespoke analysis pipeline that compares cell densities in antimicrobial exposed samples, with a paired unexposed control sample. Across a collection of representative isolates (n=10), perfect concordance was seen for SINS result, with all MIC_{FAST} results concordant within the resolution of the MIC_{BMD} (broth microdilution) test (data not shown).

The results were reported within 4 hours of primary culture. The results demonstrate strong concordance between the novel AFC-assisted susceptibility testing method of this invention and existing conventional testing methods (*e*.*g*., broth microdilution) across the panel of 10 isolates, significantly, with time to result saving of about 24-52 hours.

### Example 14:

### Rapid antimicrobial susceptibility testing of Burkholderia thailandensis and the role of efflux pump systems in antimicrobial resistance

This example demonstrates that application of the AFC-assisted susceptibility testing method of the invention to additional microorganism including to rapidly determining susceptibility of *Burkholderia* bacteria to antimicrobial agents *e.g.,* meropenem antibiotic. This example further demonstrates the applicability of the AFC-assisted susceptibility testing method of the invention as a tool to examine a role of the efflux pump system in *Burkholderia* in antimicrobial resistance.

*Burkholderia thailandensis* has been shown to be intrinsically resistant to many antimicrobials including aminoglycosides and macrolides and efflux pumps are thought to play a critical role in resistance. A closely related environmental bacterium, *B*. *pseudomallei,* is the etiologic agent of melioidosis and utilises identical efflux systems. In the past, *B*. *thailandensis* was used to study the effect of Resistance-Nodulation-Division (RND) efflux pumps in antimicrobial resistance in the *Burkholderia* species.

Prior to the instant invention, there has been a lack of rapid, quantitative and functional assays for efflux activity and a limited number of efflux pump mutants in *B*. *thailandensis.* This example sets to demonstrate utility of the AFC-assisted susceptibility testing method of the invention as a novel technique and tool for examining efflux systems in *Burkholderia.*

To this effect, the RND efflux pump was deleted by allelic exchange to generate a 8. *thailandensis*ΔRND mutant of *B*. *thailandensis.* Mutagenesis was performed using a suicide vector and sucrose counter-selection. The linear range of detection for flow cytometry was determined using a dilution series on overnight broth cultures of *B*. *thailandensis* stained with the DNA-intercalating fluorescent dye, STYO9, after which growth curves were performed as shown in Figure 16 panel B. The FAST protocol as described in the earlier examples was adapted to the growth kinetics of *B*. *thailandensis.* Results are shown in Figure 15.

As shown in Figure 15, the construction of the mutant met several waypoint goals. The linear range of detection for flow cytometry was 9×10³ to 4.4×10⁶ cells/ml (R² = 0.9924). The doubling time for *B*. *thailandensis* was estimated as 43.8 minutes by flow cytometry and 46.2 minutes using viable counts (p < 0.0004). The minimum inhibitory concentration determined by FAST (MIC_{FAST}) for meropenem was concordant with the standard broth microdilution method (MIC_{BMD}).

Accordingly, in this example an efflux system (RND) mutant was constructed in B. *thailandensis*, as a surrogate model for *B*. *pseudomallei.* Both bacterial species are intrinsically resistant to many antimicrobials such as aminoglycosides and macrolides. MIC_{FAST} for meropenem correspond to conventional MIC_{BMD}. Therefore the AFC-assisted susceptibility testing method of this invention is applicable in *B*. *thailandensis,* implicating its further utility for *B*. *pseudomallei* antimicrobial susceptibility testing. Adaptation of meropenem FAST assay to *Burkholderia* spp. shows proof-of-concept of efflux pump activity in the *B. pseudomallei.* Characterisation of mutant antimicrobial susceptibility phenotype using the AFC-assisted susceptibility testing method of this invention offers insights into efflux contribution to antimicrobial resistance, and demonstrates that deletion of RND efflux system can give rise to meropenem susceptible bacterial populations.

### Example 15:

### Piperacillin / tazobactam flow cytometry-assisted antimicrobial susceptibility test for Klebsiella pneumoniae

This example demonstrates that application of the AFC-assisted susceptibility testing method of the invention to rapidly determining susceptibility of *Klebsiella pneumoniae* bacteria to antimicrobial agents such a piperacillin / tazobactam.

Piperacillin / tazobactam often see routine use for intensive care settings. However, before the present invention, empirical antimicrobial chemotherapy has been administered to patients before the causative organism and the antimicrobials which can best treat it have been identified. Definitive treatment using know conventional methods relies on susceptibility results obtained after antimicrobial chemotherapy has commenced. Patient outcomes are therefore dependent on the turnaround time and accuracy of antimicrobial susceptibility testing (AST). Current conventional automated methods of AST may reduce time-to-result, however these methods generally require regrowth of the isolated bacteria and results can take up to 20 hours or even up to 72 hours. Moreover, the sensitivity of these conventional automated methods currently in use is variable. Consequently, there has been an increased reliance on the use of broad-spectrum antibiotics, including last-line carbapenems *e*.*g*., Piperacillin and tazobactam. The overuse of such drugs promoted the emergence of antimicrobial resistant organisms *i*.*e*., such overuse became significant contributor to antimicrobial resistance. Piperacillin / tazobactam (TZP) was identified as a potential carbapenem-sparing antimicrobial.

This example demonstrates the use of the AFC-assisted susceptibility testing method of this invention as a rapid phenotypic FAST test for TZP against *Klebsiella pneumoniae* and implicates the AFC-assisted susceptibility testing method of this invention in providing additional therapeutic options in order to reduce carbapenem use.

To this end, a cohort of *Klebsiella pneumoniae* isolates with varying resistance phenotypes was selected (n=10). Isolates were cultured overnight and enumerated using flow cytometry. Overnight cultures were counted using an Attune^{™} flow cytometer (Thermo Fisher Scientific) as described earlier above. Cell densities were adjusted to 10⁶ cells/ml in Mueller-Hinton broth, and cultured in the Mueller Hinton Broth for about 30 mins or until cell density doubled. Once cells densities doubled, cultures were split and exposed to varying concentrations of piperacillin (0 mg/l to 256mg/l) for 30 minutes. Tazobactam was present in each aliquot at a fixed concentration of 4mg/l, including the unexposed control. Inocula were washed, resuspended then stained with SYTO 9 and analysed by flow cytometry. MIC determinations were made based on the antibiotic concentration which reduced the cell concentration in the unexposed gate by 40%. MICs were determined by the FAST method of the invention and by conventional broth microdilution (BMD) method, conducted in parallel. The experimental work flow is shown in Figure 16 and results are shown in Figures 17 and 18.

Two isolates failed to double in the allocated time and excluded. One additional isolate was contaminated and invalidated. The results demonstrate that, in the remaining 7 isolates where QC criteria were met, there was excellent correlation between BMD and FAST (Spearman's R²= 0.98907 n=7), with all MIC_{FAST} results being within ±1 dilution resolution of BMD.

The results obtained demonstrate that TZP susceptibility can be determined using AFC-assisted susceptibility testing method of the invention, with quantitative results available in under 3 hours such as within 1 hour and/or within 2 hours from primary culture. Accordingly, based on these data, the FAST method of the invention has shown to have application in expediting clinical decision making, limit carbapenem use and, in turn, reduce selection for antimicrobial resistance.

The results provided herein also led the inventors to consider the results as early evidence of direct applicability of the AFC-assisted susceptibility testing method of the invention to directly to human samples such as direct applicability to blood culture. This is because, the inventors reason based on these results that the Gram negative bacteria *Klebsiella pneumoniae* can be separated from primary blood culture bottle within 1 hour of testing for susceptibility to antimicrobial agent using the FAST method of the invention once positive result is obtained.

### Example 16:

### Flow cytometry-assisted susceptibility testing directly from blood cultures

This example demonstrates successful application of the AFC-assisted susceptibility testing method of the invention to rapidly determining susceptibility of unidentified microorganisms e.g., bacteria to antimicrobial agents directly from positive blood cultures as rapidly as within 3 hours.

Antimicrobial resistance has been described as the largest Darwinian experiment. Whilst antimicrobials have added 20 years to the average human life, their use has created selective pressures favouring the evolution of resistant microbes. Serious infections, such as sepsis, require the rapid administration of the right antimicrobials. However, to date, selection of the right antimicrobial agent has been based on clinical presentation and supported by conventional antimicrobial susceptibility tests which do not produce results sufficiently rapidly to treat sepsis. Also, conventional susceptibility test results are not available in the acute clinical setting and decisions are made based on clinical suspicion and not based on actual susceptibility determination. Furthermore, as outlined above, use of inappropriate antimicrobials drives patient mortality and antimicrobial resistance.

This example sets out to investigate utility of the AFC-assisted susceptibility testing method of the invention as a clinical test and tool which can inform use antimicrobial choice in the early hours of sepsis presentation, e.g., by applying the AFC-assisted susceptibility testing method directly to blood cultures. Procedure and results are shown in Figures 19 to 21.

Blood culture FAST assays using the AFC-assisted susceptibility testing method of the invention described above, conducted on the first five isolates resulted in one susceptibility determination that agreed with the reference laboratory result. Three of the failures occurred in isolates that did not achieve a doubling in bacterial concentration during incubations. Quality control metrics were implemented to ensure active bacterial growth (as indicated by a doubling in cell density) after which, 10 clinical blood culture specimens had a 100% concordance with reference laboratory results.

These results demonstrate that determining susceptibility data earlier in sepsis can improve patient outcomes and also serve to mitigate the emergence of multi-resistant organisms. Use of the AFC-assisted susceptibility testing method of the invention developed by the present inventors as described herein, is a suitable tool for determining the most appropriate antimicrobial choice to use (against even unidentified bacteria), within a very short time of 3 hours, directly from a positive blood culture.

Routine blood cultures for serious infections represent the beginning of the testing pipeline. In this example, 10 isolates (30 AST tests), benchmarked in parallel to a local pathology service provided perfect concordance, with a time saving of 22-46 hours.

### Example 17:

### Development of a rapid high throughput system employing acoustic flow cytometry (AFC)-assisted susceptibility testing to one or more antimicrobial agents in one or more biological samples (Method I)

This example outlines the development of a rapid high-throughput method based on the new AFC-assisted susceptibility testing method for assaying qualitatively and/or quantitatively susceptibility of microorganism(s) (e.g., one or more different bacteria) in one or more biological sample(s) to antimicrobial(s). This example demonstrates application of the new AFC-assisted susceptibility testing method according to any broad aspect described herein simultaneously and in a high throughput manner to multiple biological samples / microbial cultures of actively dividing microbial cells and/or to multiples antimicrobial agents. This rapid high throughput method was used rapidly predict qualitatively susceptibility of one or plurality of microorganisms to one or plurality of antimicrobial agents in one hour, and measure susceptibility quantitatively by FAST MIC within 3 hours of sample handling. As exemplified herein the high-throughput system was carried out in a 96-well plates or trays. However it will be understood that the method can be modified to use any high-throughput format not limited to 96-well plates or trays. As exemplified, the high-throughput method allowed testing in a single 96 well plate /tray for assay susceptibility of one microbial sample against 13 different antimicrobial agents, or alternatively test at 13 microbial samples against one antimicrobial agent. It will be readily understood that different permutations of these combination are possible.

The methodology already outlined in the earlier examples above together with the examples that follow illustrate development of a new and improved method by which susceptibility to an antimicrobial agent such as an antibiotic can be rapidly assayed rapidly and at high-throughput for multiple biological samples of unicellular or multicellular microorganisms (such a bacteria). This new and improved high-throughput method is briefly exemplified in Figure 22.

The method was achieved as follows. Once a biological sample such as a blood culture sample obtained from a subject having or suspected of having infection with a microorganism (e.g., bacteria) has been determined, by the system in which it was incubated, to have returned a culture positive result, 500 microliters was removed and placed into a tube. A culture positive result was determined by measurements of CO₂ production and/or using fluorescent reagents to indicate there presence of a sufficient number *e*.*g*., about 1.0 × 10⁶ through to about 9.0 ×10⁹, of actively dividing microbial cells present in the microbial sample.

500 microliters of a 10% suspension (in 0.85% saline) of a non-ionic detergent (such as Triton-X100) was added to the 500 microliters of the positive blood culture sample and incubated at room temperature for 5 minutes to separate microbial cells from blood cells. Alternatively, filtration was used to separate blood cells from microbial cells.

After the 5 minutes had elapsed, the sample was centrifuged at 4000 × g for 5 minutes. The supernatant of the suspension was then removed, with the remaining material resuspended in 1 mL of Hank's Balanced Salt Solution. This sample was then centrifuged at 4000 × g for 5 minutes. The supernatant of the suspension was then removed, with the remaining material resuspended in 1 mL of Hank's Balanced Salt Solution. A 1:10 dilution in Hank's Balanced Salt Solution was performed in a total of 1mL of Hank's Balanced Salt Solution containing a working concentration of 5 µM SYTO 9 (1 microliter of 5mM SYTO 9 was added to 899 microliters of Hank's Balanced Salt Solution and 100 microliters of sample). Alternatively, these steps were replaced with a 1:10 dilution of the bacterial suspension in pre-warmed Hank's Balanced Salt Solution (37°C).

This suspension was then analyzed using the Attune NxT flow cytometer, with the following settings: Flow Rate 25 microliters/minute, Recording Time 1 minute. On the basis of selecting events from the flow cytometry data that had FSC signal intensity greater than 10³ arbitrary fluorescence units, SSC intensity greater than 10³ arbitrary fluorescence units and 488nm-excited 515-545nm emitted fluorescence greater than 10² arbitrary fluorescence units (positive SYTO 9 staining), a count for the number of bacteria/mL of positive blood culture was obtained. The blood culture suspension, previously subjected to detergent treatment, was centrifuged at 4000 × g for 5 minutes. The supernatant was discarded. The descended matter remaining in the tube was resuspended in Mueller-Hinton Broth pre-warmed to 37°C.

Based upon the empirically determined numbers of microorganism per milliliter of suspension, an aliquot of the suspension of micro-organisms from the blood culture bottle was added to a further volume of Mueller-Hinton Broth (pre-warmed to 37°C) such that the final concentration of micro-organisms was 1.0 × 10⁶ microorganisms per milliliter and the final volume was 10 milliliters. In one example, where the density of microorganisms in the suspension was determined by flow cytometry to be 4.5 × 10⁷ cells of microorganisms per milliliter, then 222.22 microliters of blood culture suspension was added to 9777.78 microliters of pre-warmed Mueller-Hinton Broth.

Alternatively, microbial suspensions standardized by any technique or instrument capable of standardising microbial suspensions (e.g., using a nephelometer or a spectrophotometer measuring at OD at 600nm) such that the initial density at time of exposure to an antimicrobial agent is in the range of about 5.0 × 10³ to about 5.0 × 10⁷microbial cells per milliliter, preferably 5.0 × 10⁴ to 5.0 × 10⁷ microbial cells per milliliter, and most preferably to a concentration of 5.0 × 10⁵ microbial cells per milliliter.

This suspension was then incubated at 37°C for a period of time that is one of: 30 minutes, 60 minutes, 90 minutes, 120 minutes, 150 minutes or 180 minutes. The exact time of incubation was determined by, at each increment of 30 minutes, performing the following steps: **(a)** removing a 100 microliters aliquot of the adjusted suspension of microorganism in Mueller-Hinton Broth; **(b)** adding 900 microliters of Hank's Balanced Salt Solution to the 100 microliters of suspension; **(c)** centrifuging the diluted suspension at 4000 × g for 5 minutes; **(d)** removing the supernatant from the centrifuged suspension; **(e)** adding a total of 1mL of Hank's Balanced Salt Solution containing a working concentration of 5 µM SYTO 9 (1 microliters of 5mM SYTO 9 was added to 899 microliters of Hank's Balanced Salt Solution and 100 microliters of sample); **(f)** analysing this SYTO 9 stained suspension using the Attune NxT flow cytometer, with the following settings: Flow Rate 25 microliters/minute, Recording Time 1 minute. On the basis of selecting events from the flow cytometry data that had FSC signal intensity greater than 10³ arbitrary fluorescence units, SSC intensity greater than 10³ arbitrary fluorescence units and 488nm-excited 515-545nm emitted fluorescence greater than 10² arbitrary fluorescence units (positive SYTO 9 staining), a count for the number of bacteria/mL of bacterial growth suspension; and **(g)** if this count reached 2.0 × 10⁶ bacteria per milliliter, the incubation was terminated and the assay progressed.

Alternatively, if the biological sample collected from the subject was flagged positive in using blood culture machine and been retained at 37°C, pre-incubation steps (a) to (f) could be omitted. Where the pre-incubation steps (a) to (f) were omitted, the suspension was standardised (using flow cytometry counts, a nephelometer or a spectrophotometer measuring OD600) to a cell concentration approximately in the range of about 5.0 × 10³ to about 5.0 × 10⁷ microbial cells per milliliter, preferably about 5.0 × 10⁴ to about 5.0 × 10⁷ microbial cells per milliliter, and most preferably to a concentration of about 5.0 × 10⁵ microbial cells per milliliter.

However, where pre-incubation steps (a) to (f) were not omitted, a working suspension of microorganisms was prepared from this bacterial incubation suspension. The empirical determination of the number of microorganisms per microliter was used to determine a volume of suspension to be added to a volume of Mueller-Hinton Broth (pre-warmed to 37°C) such that the final concentration of cells microorganisms was about 5.0 × 10⁵ microorganism per milliliter (preferred range can be about 5.0 × 10³ to 5.0 × 10⁷ microbial cells per milliliter). For example, if the count of the suspension determined that there were 2.0 ×10⁶ microorganisms per microliter, then 1333.33 microliters of bacterial growth suspension was added to 28666.67 microliters of Mueller Hinton Broth (pre-warmed to 37°C).

A volume of this diluted microbial growth suspension was added to each well of a standard format 96 well plate containing an antimicrobial agent such as antibiotic standard to achieve a final concentration equal to the appropriate working suspensions of the antimicrobial agents such as antibiotics tested. It will be understood that the plate format and number of wells or samples that can be tested is not limited to standard 96 well plate format. The range of concentrations for each antimicrobial agent tested was 0.25 mg/L, 0.5 mg/L, 1.0 mg/L, 2.0 mg/L, 4.0 mg/L, 8.0 mg/L, 16.0 mg/L, 32.0 mg/L, 64.0 mg/L, 128.0 mg/L, 256.0 mg/L.

In one example, four different antimicrobial agents such as four different antibiotics (ceftixalone (CRO), gentamicin (GEN), meropenem (MEM) and piperacillin-tazobactam (PZT)) were tested at the above concentrations on a single 96 well page plate, with 8 wells containing no antimicrobial agent as an unexposed growth control, as outlined in Figure 22. In another example, eight different antimicrobial agents such as eight different antibiotics were tested at the above concentrations on one single plate, with 8 wells containing no antimicrobial agent as an unexposed growth control. In yet a further example, 13 different antimicrobial agents (such as 13 different antibiotics) were tested on one single 96 well plate, omitting the 64.0 mg/L, 128.0 mg/L and the 256.0 mg/L antimicrobial dilutions, with either one or three wells containing no antimicrobial agent as unexposed growth controls.

Following inoculation with the adjusted microbial growth suspension, the 96-well plate was returned to an incubator for either 30 minutes or 60 minutes or 90 minutes or 120 minutes. For microorganisms classified as Gram negative bacteria, it was 30 minutes. For microorganisms classified as Gram positive bacteria, the period of time was matched to the period of time with the pre-incubation. As an example, if the microorganism took 90 minutes to grow from 1.0 × 10⁶ to 2.0 ×10⁶ microbial cells per milliliter, the incubation in this step was 90 minutes. In the absence of a prior knowledge of the identity of the organism, the duration of the pre-incubation step was matched with the antibiotic exposure step, described herein. After the antibiotic exposure time had elapsed, the 96-well plate was transferred to a centrifuge for centrifugation at 2300 × g for 5 minutes.

The supernatant from the wells was then aspirated, and 270 microliters of 0.85% saline solution was added. The 96-well plate was transferred to a centrifuge for centrifugation at 2300 × g for 5 minutes. The supernatant from the wells was aspirated again, and 270 microliters of 0.85% saline solution was added. For each well of the plate, 40 microliters of the antibiotic exposed microbial cell suspension was added to 160 microliters of Hank's Balanced Salt Solution stained with SYTO 9, for a final working concentration of 5 µM SYTO 9. Alternatively, these steps were omitted and replaced with an addition of 4 volumes of SYTO 9 stained Hank's Balanced Salt Solution so as to give a final working concentration of 5 µM SYTO 9.

The plate was incubated in the absence of light, at ambient temperature, for 5 minutes, prior to loading of the plate into the Attune NxT Flow Cytometer by way of an Attune NxT Flow Cytometer AutoSampler. From each well of the plate, a sample was analysed by the Attune NxT Flow Cytometer, via the Attune NxT Flow Cytometer AutoSampler with the following settings: Flow Rate - 25 microliters per minute, Acquisition Volume - 18 microliters per well, Wait Before Recording - 8 seconds, Rinse Between Wells - active. However, where the dilution method replaced centrifugation of plates, the above Attune NxT Flow Cytometer AutoSampler setting were replaced with the following settings: Flow Rate - 100 microliters per minute, Acquisition Volume - 60 microliters per well, Wait Before Recording - 8 seconds, Rinse Between Wells - active. Data for each well of the 96 well plate were exported in FCS 3.1 file format from the Attune NxT acquisition software for analysis, and data each well of the 96 well plate were imported into FlowJo v10.4.2.

However it will be understood that any other software (such FCS Express, R (FlowCore and FlowViz)) capable of performing the below analysis steps would be acceptable. Alternatively, any statistical package capable of accepting the FCS data (converted to CSV files), capable of performing the variable subset analysis listed here (where a gate is considered a subset of data with the appropriate parameters) could be used. Examples of this are: R (BioConductor, FlowStats), Orange, bespoke statistical code written in any appropriate programming language (e.g., Python, Ruby, Visual Basic, C, Ansi C, C++).

To each data file, the following *in silico* analysis steps A to H were applied: **(A)** A histogram gate was added, selecting all events of arbitrary fluorescence units in the 488nm excited, 515nm-545nm emitted fluorescence greater than 10². **(B)** On a plot of Forward Scatter - Area and Forward Scatter - Height, all events with a ratio of height to area below 1.5 were selected to ensure that only single cells are analyzed. **(C)** Data files corresponding with the unexposed control samples were identified. For assays where multiple control samples were run, the mode or median number of events per microliter (whichever is higher) was identified. **(D)** On a plot of Forward Scatter Height, and 488nm excited, 515nm-545nm emitted fluorescence, nearest neighbour contouring was applied at the 10% threshold, and a region bounded by the outermost contour was selected encompassing all of the events within this region. This was designated the UCM gate. **(E)** The number of recorded events was determined, standardized as events per microliter, and identified as falling within the bound of the UCM gate for each data file sampled from the 96 well plate. This represented a count of microbial cells per microliter, with the measured optical and fluorescence parameters consistent with microorganisms not effected by exposure to the antimicrobial agent. **(F)** Using the software, he UCM gate was applied to a plot of Forward Scatter Height, and 488nm excited, 515nm-545nm emitted fluorescence to all data files from the assay. This step identified the number of microbes per microliter, in each data file, consistent with microorganisms not effected by exposure to the antimicrobial agent. **(G)** For each data file across the assay, the number of events in the UCM gate per microlitre was divided by the number of events in the UCM gate per microlitre in the unexposed control sample from which the UCM gate was originally defined. This value was expressed as a percentage to identify the percentage/proportion of cells of microorganisms in each data file with optical and fluorescence parameters consistent with microorganisms not effected by exposure to the antimicrobial agent (see results shown in Figure 23). **(H)** All files across the assay having received the same antimicrobial challenge were compared. The minimum inhibitory concentration (MIC) was defined as the lowest antimicrobial concentration at which the percentage of remaining microbial cells had fallen below a pre-determined antimicrobial agent specific threshold.

Accordingly, as illustrated in Figure 23, following the exemplified high-throughput AFC method the number of microbial cells per microliter that fall within the UCM gated region for each antimicrobial dilution series can be plotted as a percentage of those found in the unexposed control. For example as shown in Figure 23 panel A, for a microbial isolate that previously been shown to be susceptible to all tested antimicrobials, the plot results in a rapid drop in the bacterial cells at the lowest concentrations (dilutions) of all four antibiotics tested (ceftixalone (CRO), gentamicin (GEN), meropenem (MEM) and piperacillin-tazobactam (PZT), with all antimicrobial agents falling there threshold of susceptibility before reaching a concentration associate with a resistant phenotype. In contrast as shown in Figure 23 panel B, for a multiple-drug resistant bacterial isolate, this drop does not occur until antimicrobial agent concentration are above the concentration associated with a resistant phenotype.

With respect to step **(H)** above, the following were taken into account for defining the minimum inhibitory concentration (MIC) of the antimicrobial agent tested. **(i)** For determining MIC of meropenem, the pre-determined antimicrobial agent specific threshold was 30%, i.e. being the lowest meropenem concentration at which there are 30% or less events within the UCM gate as compared with the unexposed control sample. **(ii)** For determining MIC of gentamicin, the pre-determined antimicrobial agent specific threshold was 60% i.e. being the lowest gentamicin concentration at which there are 60% or less events within UCM gate as compared with the unexposed control sample. **(iii)** For determining MIC of piperacillin-tazobactam, the pre-determined antimicrobial agent specific threshold was 50% i.e. being the lowest gentamicin concentration at which there are 50% or less events within UCM gate as compared with the unexposed control sample. **(iv)** The predetermined thresholds can be obtained by exposing 122 isolates (with a minimum of 34 resistant isolates) of the microorganism of interest, to the drug of interest in the manner described above to generate a hypothetical value - for example, a new antimicrobial may appear to have a threshold of susceptibility of 20% remaining when compared with the unexposed control. This threshold is then tested by comparison to results generated, for the isolates of microorganism exposed to the same drug, by a traditional reference method such as broth micro-dilution. If the MIC correlation is greater than 0.90, for example with fewer than 3% "very major errors" ("very major errors" being a microorganism with a drug resistant phenotype being assayed as having an MIC consistent with a drug susceptible phenotype) the threshold is confirmed and a new threshold is set. (v) In the absence of empirically obtained, statistically validated thresholds, arbitrary thresholds could be used. For an antimicrobial agent with a known bacteriostatic effect (i.e., where the presence of the antimicrobial agent inhibits the growth of, but does not directly kill cells of the microorganism), 60% cells remaining, compared with the unexposed control, would be considered sufficient to make a determination of MIC. For an antimicrobial agent with a known bactericidal (a compound that directly kills microbial cells) activity, 30% cells remaining, compared with the unexposed control, is sufficient to make a determination of MIC. For antimicrobial agents with an unknown mechanism of action, statistical certainty of inhibition can be achieved by applying a threshold at 10% of cells remaining, compared with the unexposed control.

Using the high-throughput system exemplified antimicrobial qualitative susceptibility results can be obtained within 1 hour of sample handing, ad FAST MIC results can be achieved within 3 hours of first sample handling, for a plurality of microbial samples and using a plurality of antimicrobial agents. For example as shown in Figure 22, MIC results using a full range of antimicrobial agent dilutions (of 0.25 mg/L, 0.5 mg/L, 1.0 mg/L, 2.0 mg/L, 4.0 mg/L, 8.0 mg/L, 16.0 mg/L, 32.0 mg/L, 64.0 mg/L, 128.0 mg/L, 256.0 mg/L), for as many as four different antibiotics (CRO, GEN, MEM and PZT) for 2 separate bacterial isolates from a positive blood sample culture can be returned in an average of approximately 3 hours from initial detection of positive blood culture. Specifically, across the 96 well samples tested, average time to AST MIC result had a mean of 3.4 hours, and a range of 3.0 hours to 4.2 hours. This is much more rapid compared with MIC BMD determination for the same 96 samples tested under reference laboratory time having a mean of 47.9 hours, and a range of 14.8-126.8 hours.

### Example 18:

### Development of a rapid high throughput system employing acoustic flow cytometry (AFC)-assisted susceptibility testing to one or more antimicrobial agents in one or more biological samples (Method II)

This example outlines a further method of a rapid high-throughput method based on the new AFC-assisted susceptibility testing method for assaying qualitatively and/or quantitatively susceptibility of microorganism(s) (e.g., one or more different bacteria) in one or more biological sample(s) to antimicrobial(s). The method exemplified herein comprises fewer steps than the high-throughput method outlined in Example 17, and is simplified.

This simpler method is performed as follows. Once a biological sample such as a blood culture sample obtained from a subject having or suspected of having infection with a microorganism (e.g., bacteria) has been determined, by the system in which it was incubated, to have returned a culture positive result for the microorganism, 500 microliters is removed and placed into a tube. A culture positive result can be determined by measurements of CO₂ production and/or using fluorescent reagents to indicate there presence of a sufficient number e.g., about 1.0 × 10⁶ to about 9.0 ×10⁹ of actively dividing microbial cells present in the microbial sample.

500 microliters of a 0.1% suspension (in 0.85% saline) of a non-ionic detergent (such as Triton-X100) is added to the 500 microliters of the positive blood culture sample and incubated at room temperature for 5 minutes to separate microbial cells from blood cells. Alternatively, filtration was used to separate blood cells from microbial cells.

After the 5 minutes have passed, the sample is diluted 1:10 in Hank's Balanced Salt Solution, pre-warmed to 37°C. This suspension is then analyzed with a nephelometer and, by the dropwise addition of Hank's Balanced Salt Solution (pre-warmed to 37°C), diluted until it reaches parity, by the resolution of said nephelometer, with a 0.5 McFarland standard (0.05 ml of 1.0% barium chloride in 9.95mL of 1.0% sulfuric acid). This equates to approximately 1.5 × 10⁸ microbial cells /mL.

An aliquot of the suspension of micro-organisms from the blood culture bottle is added to a further volume of Mueller-Hinton Broth (pre-warmed to 37°C) such that the final concentration of microorganisms is in the range of about 5.0 × 10³ to about 5.0 × 10⁷ microbial cells per milliliter, preferably about 5.0 × 10⁴ to about 5.0 × 10⁷ microbial cells per milliliter, and most preferably at concertation of 5.0 × 10⁵ microbial cells per milliliter. In this case, 400.00 microliters of blood culture suspension may be added to 29600 microliters of pre-warmed Mueller-Hinton Broth. A volume of this diluted microbial growth suspension is added to each well of a standard format 96 well plate containing antimicrobial agent (e.g., antibiotic) standard to achieve a final concentration equal to the appropriate working suspensions of the antimicrobial agents (e.g., antibiotics) being tested. The range of concentrations for each antimicrobial agent that mat be tested may be 0.25 mg/L, 0.5 mg/L, 1.0 mg/L, 2.0 mg/L, 4.0 mg/L, 8.0 mg/L, 16.0 mg/L, 32.0 mg/L, 64.0 mg/L, 128.0 mg/L, 256.0 mg/L.

In one embodiment, four different antimicrobial agents are tested at the above concentrations on one 96 well plate, with 8 wells containing no antimicrobial agent as an unexposed growth control. In another example, eight antimicrobial agents are tested at the above concentrations on one 96 well plate, with 8 wells containing no antimicrobial agent as an unexposed growth control. In yet a further example, 13 different antimicrobial agents are tested on one 96 well plate, with reduced number of dilutions tested for each antimicrobial agent (e.g., omitting the 64.0 mg/L, 128.0 mg/L and the 256.0 mg/L dilutions), with either one or three wells containing no antimicrobial agent as unexposed growth controls.

Following inoculation with the adjusted microbial growth suspension, the 96-well plate is placed in an incubator for either about 30 minutes or about 120 minutes. For microorganisms classified as Gram negative bacteria, incubation may be for30 minutes. For microorganisms classified as Gram positive bacteria, or in the absence of a prior knowledge of the identity of the microorganism, the duration of the pre-incubation step is about 120 minutes.

After exposure time with the antimicrobial agent has passed, the volume within each well is supplemented with 4 volumes of SYTO 9 stained Hank's Balanced Salt Solution such that the final SYTO 9 concentration is 5 µM. The plate is then incubated in the absence of light, at ambient temperature, for about 5 minutes. The plate is loaded into the Attune NxT Flow Cytometer by way of an Attune NxT Flow Cytometer AutoSampler.

From each well of the plate, a sample is analysed by the Attune NxT Flow Cytometer, via the Attune NxT Flow Cytometer AutoSampler with the following settings: Flow Rate - 100 microliters per minute, Acquisition Volume - 60 microliters per well, Wait Before Recording - 8 seconds, Rinse Between Wells - active.

As in Example 17 above, Data for each well of the 96 well plate is exported in FCS 3.1 file format from the Attune NxT acquisition software for analysis, and data each well of the 96 well plate were imported into FlowJo v10.4.2. It will be understood that any other software (such FCS Express, R (FlowCore and FlowViz)) capable of performing the below analysis steps would be acceptable. Alternatively, any statistical package capable of accepting the FCS data (converted to CSV files), capable of performing the variable subset analysis listed here (where a gate is considered a subset of data with the appropriate parameters) could be used. Examples of this are: R (BioConductor, FlowStats), Orange, Bespoke statistical code written in any appropriate programming language (e.g., Python, Ruby, Visual Basic, C, Ansi C, C++).

To each data file, the following *in silico* analysis steps A to H are applied: **(A)** A histogram gate is added, selecting all events of arbitrary fluorescence units in the 488nm excited, 515nm-545nm emitted fluorescence greater than 10². **(B)** On a plot of Forward Scatter - Area and Forward Scatter - Height, all events with a ratio of height to area below 1.5 are selected to ensure that only single cells are analyzed. **(C) D**ata files corresponding with the unexposed control samples are identified. For assays where multiple control samples are run, the mode or median number of events per microliter (whichever is higher) is identified. **(D)** On a plot of Forward Scatter Height, and 488nm excited, 515nm-545nm emitted fluorescence, nearest neighbour contouring is applied at the 10% threshold, and a region bounded by the outermost contour is selected encompassing all of the events within this region. This designates the UCM gate. **(E)** The number of recorded events is determined, standardized as events per microliter, and identified as falling within the bound of the UCM gate for each data file sampled from the 96 well plate. This represented a count of microbial cells per microliter, with the measured optical and fluorescence parameters consistent with microorganisms not effected by exposure to the antimicrobial agent. **(F)** Using the software, he UCM gate is applied to a plot of Forward Scatter Height, and 488nm excited, 515nm-545nm emitted fluorescence to all data files from the assay. This step identifies the number of microbes per microliter, in each data file, consistent with microorganisms not effected by exposure to the antimicrobial agent. **(G)** For each data file across the assay, the number of events in the UCM gate per microlitre is divided by the number of events in the UCM gate per microlitre in the unexposed control sample from which the UCM gate was originally defined. This value is expressed as a percentage to identify the proportion of cells of microorganisms in each data file with optical and fluorescence parameters consistent with microorganisms not effected by exposure to the antimicrobial agent (see results shown in Figure 23). **(H)** All files across the assay having received the same antimicrobial challenge were compared. The minimum inhibitory concentration (MIC) is defined as the lowest antimicrobial concentration at which the percentage of remaining microbial cells had fallen below a pre-determined antimicrobial agent specific threshold.
With respect to step **(H)** above, the following may be taken into account for defining the minimum inhibitory concentration (MIC) of the antimicrobial agent tested. The predetermined thresholds can be obtained for example by exposing 122 isolates (with a minimum of 34 resistant isolates) of the microorganism of interest, to the drug of interest in the manner described above to generate a hypothetical value - for example, a new antimicrobial may appear to have a threshold of susceptibility of 20% remaining when compared with the unexposed control. This threshold is then tested by comparison to results generated, for the isolates of microorganism exposed to the same drug, by a traditional reference method such as broth micro-dilution. If the MIC correlation is greater than 0.90, for example with fewer than 3% "very major errors" ("very major errors" being a microorganism with a drug resistant phenotype being assayed as having an MIC consistent with a drug susceptible phenotype) the threshold is confirmed and a new threshold is set.
In the absence of empirically obtained, statistically validated thresholds, arbitrary thresholds could be used. For an antimicrobial agent with a known bacteriostatic effect (i.e., where the presence of the antimicrobial agent inhibits the growth of, but does not directly kill cells of the microorganism), 60% cells remaining, compared with the unexposed control, would be considered sufficient to make a determination of MIC. For an antimicrobial agent with a known bactericidal (a compound that directly kills microbial cells) activity, 30% cells remaining, compared with the unexposed control, is sufficient to make a determination of MIC. For antimicrobial agents with an unknown mechanism of action, statistical certainty of inhibition can be achieved by applying a threshold at 10% of cells remaining, compared with the unexposed control.

## Claims

1. A method of determining the susceptibility of a unicellular or multicellular microorganism to an antimicrobial agent by acoustic flow cytometry, said method comprising the following steps:
(i) incubating in a culture medium
(a) cells of the microorganism from a sample that has been obtained from a subject having or
suspected of having an infection with said microorganism, and/or
(b) a sample that has been obtained from the subject having or suspected of having an infection with said microorganism, wherein said sample comprises cells of said microorganism, wherein said incubating is for a time and under conditions sufficient for said cells to actively divide to thereby obtain an actively dividing culture of said cells of said microorganism;
(ii) exposing cells of the microorganism in and/or from the actively dividing cell culture obtained in step (i) to an antimicrobial agent for a time and under conditions sufficient for said cells of the microorganism to actively divide;
(iii) labelling cells of the microorganism exposed to the antimicrobial agent in step (ii) with a nucleic acid binding fluorescent compound;
(iv) measuring effect of said antimicrobial agent on cellular morphology of said cells of the microorganism by acoustic flow cytometry; and
(v) determining susceptibility of the microorganism to the antimicrobial agent from the acoustic flow cytometry data output, wherein step (v) comprises
(A) determining susceptibility of the microorganism to the antimicrobial agent qualitatively by determining from the acoustic flow cytometry data output that said microorganism is either susceptible or not susceptible to said antimicrobial agent; and
(B) determining susceptibility of the microorganism to the antimicrobial agent quantitatively by determining from the acoustic flow cytometry data output the minimal inhibitory concentration (MIC) of said antimicrobial agent to which the microorganism is susceptible.

2. The method of claim 1, wherein labelling the cells of the microorganism exposed to the antimicrobial agent in step (ii) with the nucleic acid binding fluorescent compound results in arrest in cell division of intact and/or replicating cells of said microorganism.

3. The method of claim 1 or claim 2, wherein the microorganism is susceptible to the antimicrobial agent, and wherein measuring the effect of the antimicrobial agent on the cellular morphology of cells of the microorganism by acoustic flow cytometry comprises measuring effect of said antimicrobial agent on the cellular morphology of actively dividing cells of said microorganism as said cells become compromised by said antimicrobial agent and before cells lysis and/or complete cell breakdown following exposure of said actively dividing cells of said microorganism to said antimicrobial agent.

4. The method of any one of claims 1 to 3, wherein said method comprises incubating in the culture medium a sample that has been obtained from the subject having or suspected of having an infection with said microorganism, wherein the sample comprises cells of the microorganism, and wherein the sample that has been obtained from the subject is incubated directly in said culture medium without first isolating and/or removing said cells of said microorganism from said sample prior to said incubation of said sample in said culture medium.

5. The method of any one of claims 1 to 4, wherein measuring effect of the antimicrobial agent on the cellular morphology of cells of the microorganism by acoustic flow cytometry comprises
(a) measuring by acoustic flow cytometry the effect of the antimicrobial agent on
(i) the amount nucleic acid content in said cells of said microorganism; and/or
(ii) the cytoplasmic volume of said cells of said microorganism; and/or
(b) measuring a change in the cellular morphology of actively dividing cells of said microorganism as determined by acoustic flow cytometry following exposure of said cells of the microorganism to a concentration of said antimicrobial agent, relative to the cellular morphology of actively dividing cells of said microorganism in and/or from the sample that has been obtained from the subject as determined by acoustic flow cytometry absent exposure of said cells to said antimicrobial agent.

6. The method of any one of claims 1 to 5, wherein measuring effect of the antimicrobial agent on the cellular morphology of cells of the microorganism by acoustic flow cytometry comprises measuring an increase in the size of actively dividing cells of said microorganism following exposure of said cells to a concentration of said antimicrobial agent as determined by acoustic flow cytometry relative to the size of actively dividing cells of said microorganism in and/or from the sample obtained from the subject as determined by acoustic flow cytometry absent exposure of said cells to said antimicrobial agent,
and wherein measuring an increase in the size of actively dividing cells of said microorganism comprises measuring an increase in (i) the nucleic acid content in said cells of said microorganism and/or (ii) the cytoplasmic volume of said cells of said microorganism, and preferably wherein the concentration of the antimicrobial agent is an inhibitory concentration of said antimicrobial agent to which the cells of the microorganism are susceptible.

7. The method of claim 1, wherein determining from the acoustic flow cytometry data output that the microorganism is either susceptible or not susceptible to the antimicrobial agent comprises measuring from said acoustic flow cytometry data output the effect of said antimicrobial agent on cellular morphology of the actively dividing cells of said microorganism at different concentrations of said antimicrobial agent.

8. The method of claim 7, wherein said method further comprising determining that said microorganism is susceptible to said antimicrobial agent when:
the acoustic flow cytometry data output measures a change in the cellular morphology of actively dividing cells of said microorganism following exposure of said actively dividing cells of said microorganism to a concentration of said antimicrobial agent which is equals to or less than a pre-determined concentration of said antimicrobial agent, relative to the cellular morphology of actively dividing cells of said microorganism in and/or from the sample obtained from the subject absent exposure of said cells to said antimicrobial agent;
and preferably wherein a change in the cellular morphology of actively dividing cells of said microorganism comprises an increase in the size of the actively dividing cells of the microorganism.

9. The method of claim 7, wherein said method further comprising determining that said microorganism is not susceptible to said antimicrobial agent when:
the acoustic flow cytometry data output fails to measure a change in the cellular morphology of actively dividing cells of said microorganism following exposure of said actively dividing cells of said microorganism to a concentration of said antimicrobial agent which is equals to or less than said pre-determined concentration of said antimicrobial agent; and/or
the acoustic flow cytometry data output measures a change in the cellular morphology of actively dividing cells of said microorganism following exposure of said actively dividing cells of said microorganism to a concentration of said antimicrobial agent which is greater than said pre-determined concentration of said antimicrobial agent;
and preferably wherein a change in the cellular morphology of actively dividing cells of said microorganism comprises an increase in the size of the actively dividing cells of the microorganism.

10. The method of claim 8 or claim 9, wherein
the pre-determined concentration is an internationally recognised clinical susceptibility breakpoint concentration or an internationally recognised MIC of said antimicrobial agent for said microorganism in the sample obtained from the patient or a species to which said microorganism belongs; and/or
the pre-determined concentration is a clinical susceptibility breakpoint concentration or an internationally recognised MIC determined for the antimicrobial agent by the European Committee on Antimicrobial Susceptibility Testing (EUCAST) or by the Clinical & Laboratory Standards Institute (CLSI) in respect of said microorganism in the sample obtained from the patient or for a species to which said microorganism obtained from the patient belongs.

11. The method of any one of claims 1 to 10, wherein
(a) determining from the acoustic flow cytometry data output that said microorganism is either susceptible or not susceptible to said antimicrobial agent is achieved within
about 1 to 3 hours, preferably within about 1 hour, from the time of obtaining the sample comprising cells of the microorganism from the subject or
about 1 to 3 hours, preferably within about 1 hour from the time of first incubating the cells of the microorganism from or in said sample in the culture medium to obtain the actively dividing culture of said cells of said microorganism ; and/or
(b) determining susceptibility of the microorganism to the antimicrobial agent quantitatively by measuring from the acoustic flow cytometry data output the minimal inhibitory concentration of said antimicrobial agent to which the microorganism is susceptible, is achieved within
about 3 to 6 hours, preferably within about 3 hours, from the time of obtaining the sample comprising cells of the microorganism from the subject or
about 3 to 6 hours, preferably within about 3 hours from the time of first incubating the cells of the microorganism from or in said sample in the culture medium to obtain the actively dividing culture of said cells of said microorganism.

12. The method of any one of claims 1 to 11, wherein determining from the acoustic flow cytometry data output the MIC of the antimicrobial agent to which the microorganism is susceptible, comprises
(a) incubating in a culture medium
(i) cells of the microorganism from the sample that has been obtained from the subject
and/or
(ii) a sample that has been obtained from the subject wherein said sample comprises cells of the microorganism,
for a time and under conditions sufficient for said cells of said microorganism to actively divide to obtain an actively dividing culture of said cells of said microorganism, and
labelling said cells of said microorganism in or form said actively dividing culture with the nucleic acid binding fluorescent compound absent prior exposure to the antimicrobial agent, to thereby produce an unexposed control sample;
(b) measuring by acoustic flow cytometry the cellular morphology and amount of said cells of said microorganism in the unexposed control sample;
(c) performing steps (i) to (iv) as defined in claim 1; and
(d) determining from the acoustic flow cytometry data output the minimal concentration of said antimicrobial agent for which the amount or proportion of the cells of said microorganism exposed to said antimicrobial agent having a cellular morphology of non-antimicrobial agent-exposed cells in said unexposed control sample, equals to or less than a pre-determined amount or proportion of the cells in said unexposed control sample measured by the acoustic flow cytometry to exhibit said cellular morphology of non-antimicrobial agent-exposed cells.

13. The method of claim 12, wherein
the pre-determined amount or proportion of the cells in said unexposed control sample measured by the acoustic flow cytometry to exhibit the cellular morphology of non-antimicrobial agent-exposed cells, is determined by reference to known minimal inhibitory concentrations (MICs) of said anti-microbial agent to a panel of known isolates of the microorganism; and/or
the pre-determined amount or proportion of the cells in said unexposed control sample measured by the acoustic flow cytometry to exhibit said cellular morphology of non-antimicrobial agent-exposed cells, is about 1% to about 50% of total the cells in said unexposed control sample.

14. The method of any one of claims 1 to 13, further comprising
(a) incubating in a culture medium cells of the microorganism from the sample that has been obtained from the subject and/or incubating in the culture medium the sample that has been obtained from the subject wherein the sample comprises the cells of the microorganism,
for a time and under conditions sufficient for said cells to actively divide to obtain an actively dividing culture of said cells of said microorganism, and
labelling said cell of said microorganism in or form said actively dividing culture with the nucleic acid binding fluorescent compound absent prior exposure to the antimicrobial agent, to thereby produce an unexposed control sample; and
(b) measuring by acoustic flow cytometry the cellular morphology and amount of said cells of said microorganism in the unexposed control sample.

15. The method of any one of claims 1 to 14, wherein
(a) incubating cells of the microorganism in and/or from the sample in a culture medium for a time and under conditions sufficient for said cells to actively divide comprises incubating said cells of said microorganism for a time and under conditions sufficient for about 1 to about 5 cells divisions to occur, preferably for about 1 to about 3 cell divisions to occur; and/or
(b) exposing cells of the microorganism to the antimicrobial agent for a time and under conditions sufficient for said cells of the microorganism to actively divide, comprises incubating said cells of said microorganism in the presence of the antimicrobial agent for a time and under conditions sufficient for said cells to undergo about 1 to about 3 cell divisions.

16. A method of determining susceptibility of a unicellular or multicellular microorganism to a plurality of antimicrobial agents, said method comprising:
performing the method according to any one of claims 1 to 15 wherein said method comprises exposing the actively dividing cells of the microorganism in and/or from the sample that has been obtained from the subject to one or more representative antimicrobial agent(s) of a first family or panel of a plurality of antimicrobial agents, to thereby determine susceptibility of said cells of said microorganism to antimicrobial agents belonging to said first family or panel of the plurality of antimicrobial agents.

17. The method according to any one of claims 1 to 16, wherein said method is used for high-throughput screening of the susceptibility of one or more unicellular or multicellular micrroganisms to one or more antimicrobial agents.

## Patentansprüche

1. Verfahren zum Bestimmen der Empfindlichkeit eines einzelligen oder mehrzelligen Mikroorganismus gegenüber einem antimikrobiellen Mittel durch akustische Durchflusszytometrie, wobei das Verfahren die folgenden Schritte umfasst:
(i) Inkubieren in einem Kulturmedium
(a) von Zellen des Mikroorganismus aus einer Probe, die von einem Individuum erhalten worden ist, bei dem eine Infektion mit dem Mikroorganismus vorliegt oder vermutet wird, und/oder
(b) einer Probe, die von dem Individuum erhalten worden ist, bei dem eine Infektion mit dem Mikroorganismus vorliegt oder vermutet wird, wobei die Probe Zellen des Mikroorganismus umfasst, wobei das Inkubieren für einen Zeitraum und unter Bedingungen erfolgt, die ausreichen, damit sich die Zellen aktiv teilen, um dadurch eine sich aktiv teilende Kultur der Zellen des Mikroorganismus zu erhalten;
(ii) Aussetzen von Zellen des Mikroorganismus in und/oder aus der sich aktiv teilenden Zellkultur, die in Schritt (i) erhalten wurde, für einen Zeitraum und unter Bedingungen, die ausreichen, damit sich die Zellen des Mikroorganismus aktiv teilen, gegenüber einem antimikrobiellen Mittel;
(iii) Markieren von Zellen des Mikroorganismus, die in Schritt (ii) dem antimikrobiellen Mittel ausgesetzt wurden, mit einer nukleinsäurebindenden fluoreszierenden Verbindung;
(iv) Messen der Wirkung des antimikrobiellen Mittels auf die Zellmorphologie der Zellen des Mikroorganismus durch akustische Durchflusszytometrie; und
(v) Bestimmen einer Empfindlichkeit des Mikroorganismus gegenüber dem antimikrobiellen Mittel anhand der Datenausgabe der akustischen Durchflusszytometrie, wobei Schritt (v) Folgendes umfasst:
(A) qualitatives Bestimmen einer Empfindlichkeit des Mikroorganismus gegenüber dem antimikrobiellen Mittel durch Bestimmen anhand der Datenausgabe der akustischen Durchflusszytometrie, dass der Mikroorganismus gegenüber dem antimikrobiellen Mittel empfindlich oder nicht empfindlich ist; und
(B) quantitatives Bestimmen einer Empfindlichkeit des Mikroorganismus gegenüber dem antimikrobiellen Mittel durch Bestimmen der minimalen Hemmkonzentration (MHK) des antimikrobiellen Mittels, gegenüber dem der Mikroorganismus empfindlich ist, anhand der Datenausgabe der akustischen Durchflusszytometrie.

2. Verfahren nach Anspruch 1, wobei
das Markieren der Zellen des Mikroorganismus, die in Schritt (ii) dem antimikrobiellen Mittel ausgesetzt wurden, mit der nukleinsäurebindenden fluoreszierenden Verbindung zum Stillstand der Zellteilung intakter und/oder replizierender Zellen des Mikroorganismus führt.

3. Verfahren nach Anspruch 1 oder Anspruch 2, wobei der Mikroorganismus gegenüber dem antimikrobiellen Mittel empfindlich ist und wobei das Messen der Wirkung des antimikrobiellen Mittels auf die Zellmorphologie von Zellen des Mikroorganismus durch akustische Durchflusszytometrie das Messen einer Wirkung des antimikrobiellen Mittels auf die Zellmorphologie sich aktiv teilender Zellen des Mikroorganismus umfasst, wenn die Zellen durch das antimikrobielle Mittel geschädigt werden und vor der Zelllyse und/oder einem vollständigen Zellabbau nach Aussetzen der sich aktiv teilenden Zellen des Mikroorganismus gegenüber dem antimikrobiellen Mittel.

4. Verfahren nach einem der Ansprüche 1 bis 3, wobei das Verfahren ein Inkubieren einer Probe, die von dem Individuum erhalten worden ist, bei dem eine Infektion mit dem Mikroorganismus vorliegt oder vermutet wird, in dem Kulturmedium umfasst, wobei die Probe Zellen des Mikroorganismus umfasst, und wobei die von dem Individuum erhaltene Probe direkt in dem Kulturmedium inkubiert wird, ohne die Zellen des Mikroorganismus vor der Inkubation der Probe in dem Kulturmedium zuerst aus der Probe zu isolieren und/oder zu entfernen.

5. Verfahren nach einem der Ansprüche 1 bis 4, wobei das Messen der Wirkung des antimikrobiellen Mittels auf die Zellmorphologie von Zellen des Mikroorganismus durch akustische Durchflusszytometrie Folgendes umfasst:
(a) Messen der Wirkung des antimikrobiellen Mittels durch akustische Durchflusszytometrie auf
(i) die Menge an Nukleinsäuregehalt in den Zellen des Mikroorganismus; und/oder
(ii) das Zytoplasmavolumen der Zellen des Mikroorganismus; und/oder
(b) Messen einer Veränderung der Zellmorphologie von sich aktiv teilenden Zellen des Mikroorganismus, bestimmt durch akustische Durchflusszytometrie, nach Aussetzen der Zellen des Mikroorganismus gegenüber einer Konzentration des antimikrobiellen Mittels relativ zu der Zellmorphologie sich aktiv teilender Zellen des Mikroorganismus in und/oder aus der von dem Individuum erhaltenen Probe, bestimmt durch akustische Durchflusszytometrie, ohne Aussetzen der Zellen gegenüber dem antimikrobiellen Mittel.

6. Verfahren nach einem der Ansprüche 1 bis 5, wobei das Messen der Wirkung des antimikrobiellen Mittels auf die Zellmorphologie von Zellen des Mikroorganismus durch akustische Durchflusszytometrie das Messen einer Zunahme der Größe von sich aktiv teilenden Zellen des Mikroorganismus nach Aussetzen der Zellen des Mikroorganismus gegenüber einer Konzentration des antimikrobiellen Mittels, bestimmt durch akustische Durchflusszytometrie, relativ zu der Größe sich aktiv teilender Zellen des Mikroorganismus in und/oder aus der von dem Individuum erhaltenen Probe, bestimmt durch akustische Durchflusszytometrie, ohne Aussetzen der Zellen gegenüber dem antimikrobiellen Mittel, umfasst,
und wobei das Messen einer Zunahme der Größe von sich aktiv teilenden Zellen des Mikroorganismus das Messen einer Zunahme (i) des Nukleinsäuregehalts in den Zellen des Mikroorganismus und/oder (i) des Zytoplasmavolumens der Zellen des Mikroorganismus umfasst, und wobei es sich bei der Konzentration des antimikrobiellen Mittels vorzugsweise um eine Hemmkonzentration des antimikrobiellen Mittels handelt, gegenüber dem die Zellen des Mikroorganismus empfindlich sind.

7. Verfahren nach Anspruch 1, wobei das Bestimmen anhand der Datenausgabe der akustischen Durchflusszytometrie, dass der Mikroorganismus entweder empfindlich oder nicht empfindlich gegenüber dem antimikrobiellen Mittel ist, das Messen der Wirkung des antimikrobiellen Mittels auf die Zellmorphologie der sich aktiv teilenden Zellen des Mikroorganismus bei unterschiedlichen Konzentrationen des antimikrobiellen Mittels anhand der Datenausgabe der akustischen Durchflusszytometrie umfasst.

8. Verfahren nach Anspruch 7, wobei das Verfahren ferner umfasst zu bestimmen, dass der Mikroorganismus gegenüber dem antimikrobiellen Mittel empfindlich ist, wenn:
die Datenausgabe der akustischen Durchflusszytometrie eine Veränderung der Zellmorphologie von sich aktiv teilenden Zellen des Mikroorganismus misst, nachdem die sich aktiv teilenden Zellen des Mikroorganismus einer Konzentration des antimikrobiellen Mittels ausgesetzt worden sind, die gleich wie oder kleiner als eine vorgegebene Konzentration des antimikrobiellen Mittels ist, relativ zu der Zellmorphologie von sich aktiv teilenden Zellen des Mikroorganismus in und/oder aus der von dem Individuum erhaltenen Probe, ohne Aussetzen der Zellen gegenüber dem antimikrobiellen Mittel;
und wobei eine Veränderung der Zellmorphologie von sich aktiv teilenden Zellen des Mikroorganismus vorzugsweise eine Zunahme der Größe der sich aktiv teilenden Zellen des Mikroorganismus umfasst.

9. Verfahren nach Anspruch 7, wobei das Verfahren ferner umfasst zu bestimmen, dass der Mikroorganismus gegenüber dem antimikrobiellen Mittel nicht empfindlich ist, wenn:
die Datenausgabe der akustischen Durchflusszytometrie keine Veränderung der Zellmorphologie von sich aktiv teilenden Zellen des Mikroorganismus misst, nachdem die sich aktiv teilenden Zellen des Mikroorganismus einer Konzentration des antimikrobiellen Mittels ausgesetzt worden sind, die gleich wie oder kleiner als die vorgegebene Konzentration des antimikrobiellen Mittels ist, und/oder
die Datenausgabe der akustischen Durchflusszytometrie eine Veränderung der Zellmorphologie von sich aktiv teilenden Zellen des Mikroorganismus misst, nachdem die sich aktiv teilenden Zellen des Mikroorganismus einer Konzentration des antimikrobiellen Mittels ausgesetzt worden sind, die größer als eine vorgegebene Konzentration des antimikrobiellen Mittels ist,
und wobei eine Veränderung der Zellmorphologie von sich aktiv teilenden Zellen des Mikroorganismus vorzugsweise eine Zunahme der Größe der sich aktiv teilenden Zellen des Mikroorganismus umfasst.

10. Verfahren nach Anspruch 8 oder Anspruch 9, wobei
es sich bei der vorgegebenen Konzentration um eine international anerkannte klinische Empfindlichkeits-Breakpoint-Konzentration oder eine international anerkannte MHK des antimikrobiellen Mittels für den Mikroorganismus in der von dem Individuum erhaltenen Probe oder eine Spezies, zu welcher der Mikroorganismus gehört, handelt; und/oder
es sich bei der vorgegebenen Konzentration um eine klinische Empfindlichkeits-Breakpoint-Konzentration oder eine international anerkannte MHK handelt, die für das antimikrobielle Mittel vom European Committee on Antimicrobial Susceptibility Testing (EUCAST) oder vom Clinical & Laboratory Standards Institute (CLSI) in Bezug auf den Mikroorganismus in der von dem Patienten erhaltenen Probe oder für eine Spezies, zu welcher der von dem Patienten erhaltene Mikroorganismus gehört, bestimmt worden ist.

11. Verfahren nach einem der Ansprüche 1 bis 10, wobei
(a) das Bestimmen anhand der Datenausgabe der akustischen Durchflusszytometrie, dass der Mikroorganismus gegenüber dem antimikrobiellen Mittel entweder empfindlich oder nicht empfindlich ist, erreicht wird innerhalb
von etwa 1 bis 3 Stunden, vorzugsweise innerhalb von etwa 1 Stunde, nach dem Zeitpunkt des Erhaltens der Probe umfassend Zellen des Mikroorganismus von dem Individuum oder
von etwa 1 bis 3 Stunden, vorzugsweise innerhalb von etwa 1 Stunde, nach dem Zeitpunkt des ersten Inkubierens der Zellen des Mikroorganismus aus oder in der Probe in dem Kulturmedium, um die sich aktiv teilende Kultur der Zellen des Mikroorganismus zu erhalten; und/oder
(b) das quantitative Bestimmen einer Empfindlichkeit des Mikroorganismus gegenüber dem antimikrobiellen Mittel durch Messen der minimalen Hemmkonzentration des antimikrobiellen Mittels, gegenüber dem der Mikroorganismus empfindlich ist, anhand der Datenausgabe der akustischen Durchflusszytometrie erreicht wird innerhalb
von etwa 3 bis 6 Stunden, vorzugsweise innerhalb von etwa 3 Stunden, nach dem Zeitpunkt des Erhaltens der Probe umfassend Zellen des Mikroorganismus von dem Individuum oder
von etwa 3 bis 6 Stunden, vorzugsweise innerhalb von etwa 3 Stunden, nach dem Zeitpunkt des ersten Inkubierens der Zellen des Mikroorganismus aus oder in der Probe in dem Kulturmedium, um die sich aktiv teilende Kultur der Zellen des Mikroorganismus zu erhalten.

12. Verfahren nach einem der Ansprüche 1 bis 11, wobei das Bestimmen der MHK des antimikrobiellen Mittels, gegenüber dem der Mikroorganismus empfindlich ist, anhand der Datenausgabe der akustischen Durchflusszytometrie umfasst:
(a) Inkubieren in einem Kulturmedium
(i) von Zellen des Mikroorganismus aus der von dem Individuum erhaltenen Probe
und/oder
(ii) einer von dem Individuum erhaltenen Probe, wobei die Probe Zellen des Mikroorganismus umfasst,
für einen Zeitraum und unter Bedingungen, die ausreichen, damit sich die Zellen des Mikroorganismus aktiv teilen, um eine sich aktiv teilende Kultur der Zellen des Mikroorganismus zu erhalten, und
Markieren der Zellen des Mikroorganismus in oder aus der sich aktiv teilenden Kultur mit der nukleinsäurebindenden fluoreszierenden Verbindung ohne vorhergehendes Aussetzen gegenüber dem antimikrobiellen Mittel, um somit eine nicht ausgesetzte Kontrollprobe herzustellen;
(b) Messen der Zellmorphologie und der Menge der Zellen des Mikroorganismus in der nicht ausgesetzten Kontrollprobe durch akustische Durchflusszytometrie;
(c) Durchführen der Schritte (i) bis (iv), wie in Anspruch 1 definiert; und
(d) Bestimmen anhand der Datenausgabe der akustischen Durchflusszytometrie der minimalen Konzentration des antimikrobiellen Mittels, bei der die Menge oder der Anteil der dem antimikrobiellen Mittel ausgesetzten Zellen des Mikroorganismus, die eine Zellmorphologie nicht dem antimikrobiellen Mittel ausgesetzter Zellen in der nicht ausgesetzten Kontrollprobe aufweisen, gleich wie oder kleiner als eine vorgegebene Menge oder ein vorgegebener Anteil der Zellen in der nicht ausgesetzten Kontrollprobe ist, bei denen durch die akustische Durchflusszytometrie gemessen wurde, dass sie die Zellmorphologie nicht dem antimikrobiellen Mittel ausgesetzter Zellen aufweisen.

13. Verfahren nach Anspruch 12, wobei
die vorgegebene Menge oder der vorgegebene Anteil der Zellen in der nicht ausgesetzten Kontrollprobe, bei denen durch die akustische Durchflusszytometrie gemessen wurde, dass sie die Zellmorphologie nicht dem antimikrobiellen Mittel ausgesetzter Zellen aufweisen, bestimmt wird durch Bezugnahme auf bekannte minimale Hemmkonzentrationen (MHKs) des antimikrobiellen Mittels für ein Panel bekannter Isolate des Mikroorganismus; und/oder
die vorgegebene Menge oder der vorgegebene Anteil der Zellen in der nicht ausgesetzten Kontrollprobe, bei denen durch die akustische Durchflusszytometrie gemessen wurde, dass sie die Zellmorphologie nicht dem antimikrobiellen Mittel ausgesetzter Zellen aufweisen, etwa 1 % bis etwa 50 % der gesamten Zellen in der nicht ausgesetzten Kontrollprobe beträgt.

14. Verfahren nach einem der Ansprüche 1 bis 13, ferner umfassend
(a) Inkubieren von Zellen des Mikroorganismus aus der von dem Individuum erhaltenen Probe in einem Kulturmedium und/oder Inkubieren der von dem Individuum erhaltenen Probe in dem Kulturmedium, wobei die Probe die Zellen des Mikroorganismus umfasst,
für einen Zeitraum und unter Bedingungen, die ausreichen, damit sich die Zellen aktiv teilen, um eine sich aktiv teilende Kultur der Zellen des Mikroorganismus zu erhalten, und
Markieren der Zellen des Mikroorganismus in oder aus der sich aktiv teilenden Kultur mit der nukleinsäurebindenden fluoreszierenden Verbindung ohne vorhergehendes Aussetzen gegenüber dem antimikrobiellen Mittel, um somit eine nicht ausgesetzte Kontrollprobe herzustellen; und
(b) Messen der Zellmorphologie und der Menge der Zellen des Mikroorganismus in der nicht ausgesetzten Kontrollprobe durch akustische Durchflusszytometrie.

15. Verfahren nach einem der Ansprüche 1 bis 14, wobei
(a) das Inkubieren von Zellen des Mikroorganismus in und/oder aus der Probe in einem Kulturmedium für einen Zeitraum und unter Bedingungen, die ausreichen, damit sich die Zellen aktiv teilen, das Inkubieren der Zellen des Mikroorganismus für einen Zeitraum und unter Bedingungen umfasst, die ausreichen, damit etwa 1 bis etwa 5 Zellteilungen stattfinden, vorzugsweise, damit etwa 1 bis etwa 3 Zellteilungen stattfinden; und/oder
(b) das Aussetzen von Zellen des Mikroorganismus gegenüber dem antimikrobiellen Mittel für einen Zeitraum und unter Bedingungen, die ausreichen, damit sich die Zellen des Mikroorganismus aktiv teilen, das Inkubieren der Zellen des Mikroorganismus in Gegenwart des antimikrobiellen Mittels für einen Zeitraum und unter Bedingungen umfasst, die ausreichen, damit die Zellen etwa 1 bis etwa 3 Zellteilungen durchlaufen.

16. Verfahren zum Bestimmen einer Empfindlichkeit eines einzelligen oder mehrzelligen Mikroorganismus gegenüber einer Vielzahl von antimikrobiellen Mitteln, wobei das Verfahren Folgendes umfasst:
Durchführen des Verfahrens nach einem der Ansprüche 1 bis 15, wobei das Verfahren ein Aussetzen der sich aktiv teilenden Zellen des Mikroorganismus in und/oder aus der von dem Individuum erhaltenen Probe gegenüber einem oder mehreren repräsentativen antimikrobiellen Mitteln einer ersten Familie oder eines ersten Panels einer Vielzahl von antimikrobiellen Mitteln umfasst, um dadurch eine Empfindlichkeit der Zellen des Mikroorganismus gegenüber antimikrobiellen Mitteln zu bestimmen, die zu der ersten Familie oder zu dem ersten Panel der Vielzahl von antimikrobiellen Mitteln gehören.

17. Verfahren nach einem der Ansprüche 1 bis 16, wobei das Verfahren zum Hochdurchsatz-Screening der Empfindlichkeit von einem oder mehreren einzelligen oder mehrzelligen Mikroorganismus gegenüber einem oder mehreren antimikrobiellen Mitteln verwendet wird.

## Revendications

1. Procédé de détermination de la sensibilité d'un micro-organisme unicellulaire ou multicellulaire à un agent antimicrobien par cytométrie en flux acoustique, ledit procédé comprenant les étapes suivantes :
(i) incubation dans un milieu de culture
(a) de cellules du micro-organisme provenant d'un échantillon obtenu auprès d'un sujet ayant ou suspecté d'avoir une infection par ledit micro-organisme, et/ou
(b) d'un échantillon qui a été obtenu auprès du sujet ayant ou suspecté d'avoir une infection par ledit micro-organisme, ledit échantillon comprenant des cellules dudit micro-organisme, ladite incubation étant pendant une durée et dans des conditions suffisantes pour que lesdites cellules se divisent activement pour obtenir ainsi une culture se divisant activement desdites cellules dudit micro-organisme ;
(ii) exposition des cellules du micro-organisme dans et/ou à partir de la culture de cellules se divisant activement obtenue à l'étape (i) à un agent antimicrobien pendant une durée et dans des conditions suffisantes pour que lesdites cellules du micro-organisme se divisent activement ;
(iii) marquage des cellules du micro-organisme exposées à l'agent antimicrobien à l'étape (ii) avec un composé fluorescent se liant aux acides nucléiques ;
(iv) mesure de l'effet dudit agent antimicrobien sur la morphologie cellulaire desdites cellules du micro-organisme par cytométrie en flux acoustique ; et
(v) détermination de la sensibilité du micro-organisme à l'agent antimicrobien à partir des données de cytométrie en flux acoustique délivrées, l'étape (v) comprenant
(A) la détermination de la sensibilité du micro-organisme à l'agent antimicrobien de manière qualitative par détermination à partir des données de cytométrie en flux acoustique délivrées que ledit micro-organisme est sensible ou non sensible audit agent antimicrobien ; et
(B) la détermination de la sensibilité du micro-organisme à l'agent antimicrobien de manière qualitative par détermination à partir des données de cytométrie en flux acoustique délivrées de la concentration minimale inhibitrice (CMI) dudit agent antimicrobien à laquelle le micro-organisme est sensible.

2. Procédé selon la revendication 1, dans lequel
le marquage des cellules du micro-organisme exposées à l'agent antimicrobien à l'étape (ii) avec le composé fluorescent se liant aux acides nucléiques entraîne l'arrêt de la division cellulaire de cellules intactes et/ou réplicatives dudit micro-organisme.

3. Procédé selon la revendication 1 ou la revendication 2, dans lequel le micro-organisme est sensible à l'agent antimicrobien, et dans lequel la mesure de l'effet de l'agent antimicrobien sur la morphologie cellulaire des cellules du micro-organisme par cytométrie en flux acoustique comprend la mesure de l'effet dudit agent antimicrobien sur la morphologie cellulaire des cellules se divisant activement dudit micro-organisme car lesdites cellules sont compromises par ledit agent antimicrobien et avant la lyse des cellules et/ou la dégradation cellulaire complète après l'exposition desdites cellules se divisant activement dudit micro-organisme audit agent antimicrobien.

4. Procédé selon l'une quelconque des revendications 1 à 3, dans lequel ledit procédé comprend l'incubation dans le milieu de culture d'un échantillon qui a été obtenu auprès du sujet ayant ou suspecté d'avoir une infection par ledit micro-organisme, l'échantillon comprenant des cellules dudit micro-organisme, et l'échantillon qui a été obtenu auprès du sujet étant incubé directement dans ledit milieu de culture sans un premier isolement et/ou une première élimination desdites cellules dudit micro-organisme provenant dudit échantillon avant ladite incubation dudit échantillon dans ledit milieu de culture.

5. Procédé selon l'une quelconque des revendications 1 à 4, dans lequel la mesure de l'effet de l'agent antimicrobien sur la morphologie cellulaire des cellules du micro-organisme par cytométrie en flux acoustique comprend
(a) la mesure par cytométrie en flux acoustique de l'effet de l'agent antimicrobien sur
(i) la teneur en acides nucléiques en quantité dans lesdites cellules dudit micro-organisme ; et/ou
(ii) le volume cytoplasmique desdites cellules dudit micro-organisme ; et/ou
(b) la mesure d'un changement de la morphologie cellulaire des cellules se divisant activement dudit micro-organisme comme déterminé par cytométrie en flux acoustique après l'exposition desdites cellules du micro-organisme à une concentration dudit agent antimicrobien, par rapport à la morphologie cellulaire des cellules se divisant activement dudit micro-organisme dans et/ou à partir de l'échantillon qui a été obtenu auprès du sujet comme déterminé par cytométrie en flux acoustique en l'absence d'exposition desdites cellules audit agent antimicrobien.

6. Procédé selon l'une quelconque des revendications 1 à 5, dans lequel la mesure de l'effet de l'agent antimicrobien sur la morphologie cellulaire des cellules du micro-organisme par cytométrie en flux acoustique comprend la mesure d'une augmentation de la taille des cellules se divisant activement dudit micro-organisme après l'exposition desdites cellules à une concentration dudit agent antimicrobien comme déterminé par cytométrie en flux acoustique par rapport à la taille des cellules se divisant activement dudit micro-organisme dans et/ou à partir de l'échantillon obtenu auprès du sujet comme déterminé par cytométrie en flux acoustique en l'absence d'exposition desdites cellules audit agent antimicrobien,
et dans lequel la mesure d'une augmentation de la taille des cellules se divisant dudit micro-organisme comprend la mesure d'une augmentation de (i) la teneur en acides nucléiques dans lesdites cellules dudit micro-organisme et/ou (ii) le volume cytoplasmique desdites cellules dudit micro-organisme, et de préférence dans lequel la concentration de l'agent antimicrobien est une concentration inhibitrice dudit agent antimicrobien à laquelle les cellules du micro-organisme sont sensibles.

7. Procédé selon la revendication 1, dans lequel la détermination à partir des données de cytométrie en flux acoustique délivrées que le micro-organisme est sensible ou non sensible audit agent antimicrobien comprend la mesure à partir des données de cytométrie en flux acoustique délivrées de l'effet dudit agent antimicrobien sur la morphologie cellulaire des cellules se divisant activement dudit micro-organisme à différentes concentrations dudit agent antimicrobien.

8. Procédé selon la revendication 7, ledit procédé comprenant en outre la détermination que ledit micro-organisme est sensible audit agent antimicrobien lorsque :
les données de cytométrie en flux acoustique délivrées mesurent un changement de la morphologie cellulaire des cellules se divisant activement dudit micro-organisme après l'exposition desdites cellules se divisant activement dudit micro-organisme à une concentration dudit agent antimicrobien qui est égale ou inférieure à une concentration prédéterminée dudit agent antimicrobien, par rapport à la morphologie cellulaire des cellules se divisant activement dudit micro-organisme dans et/ou à partir de l'échantillon obtenu auprès du sujet en l'absence d'exposition desdites cellules audit agent antimicrobien ;
et de préférence dans lequel un changement de la morphologie cellulaire des cellules se divisant activement dudit micro-organisme comprend une augmentation de la taille des cellules se divisant activement du micro-organisme.

9. Procédé selon la revendication 7, ledit procédé comprenant en outre la détermination que ledit micro-organisme n'est pas sensible audit agent antimicrobien lorsque :
les données de cytométrie en flux acoustique délivrées échouent à mesurer un changement de la morphologie cellulaire des cellules se divisant activement dudit micro-organisme après l'exposition desdites cellules se divisant activement dudit micro-organisme à une concentration dudit agent antimicrobien qui est égale ou inférieure à une concentration prédéterminée dudit agent antimicrobien ; et/ou les données de cytométrie en flux acoustique délivrées mesurent un changement de la morphologie cellulaire des cellules se divisant activement dudit micro-organisme après l'exposition desdites cellules se divisant activement dudit micro-organisme à une concentration dudit agent antimicrobien qui est supérieure à ladite concentration prédéterminée dudit agent antimicrobien ;
et de préférence dans lequel un changement de la morphologie cellulaire des cellules se divisant activement dudit micro-organisme comprend une augmentation de la taille des cellules se divisant activement du micro-organisme.

10. Procédé selon la revendication 8 ou la revendication 9, dans lequel la concentration prédéterminée est une concentration critique de sensibilité clinique internationalement reconnue ou une CMI internationalement reconnue dudit agent antimicrobien pour ledit micro-organisme dans l'échantillon obtenu auprès du patient ou d'une espèce à laquelle appartient ledit micro-organisme ; et/ou la concentration prédéterminée est une concentration critique de sensibilité clinique ou une CMI internationalement reconnue déterminée pour l'agent antimicrobien par le Comité européen sur les tests de sensibilité aux antimicrobiens (EUCAST) ou par le Clinical & Laboratory Standards Institute (CLSI) à l'égard dudit micro-organisme dans l'échantillon obtenu auprès du patient ou pour une espèce à laquelle ledit micro-organisme obtenu auprès du patient appartient.

11. Procédé selon l'une quelconque des revendications 1 à 10, dans lequel
(a) la détermination à partir des données de cytométrie en flux acoustique délivrées que ledit micro-organisme est sensible ou non sensible audit agent antimicrobien est obtenue dans un délai d'environ 1 à 3 heures, de préférence dans un délai d'environ 1 heure, à compter du moment de l'obtention de l'échantillon comprenant les cellules du micro-organisme provenant du sujet ou d'environ 1 à 3 heures, de préférence dans un délai d'environ 1 heure, à compter du moment de la première incubation des cellules du micro-organisme à partir de ou dans ledit échantillon dans le milieu de culture pour obtenir la culture se divisant activement desdites cellules dudit micro-organisme ; et/ou
(b) la détermination de la sensibilité du micro-organisme à l'agent antimicrobien de manière qualitative par mesure à partir des données de cytométrie en flux acoustique délivrées de la concentration minimale inhibitrice dudit agent antimicrobien auquel le micro-organisme est sensible, est obtenue dans un délai d'environ 3 à 6 heures, de préférence dans un délai d'environ 3 heures, à compter du moment de l'obtention de l'échantillon comprenant les cellules du micro-organisme provenant du sujet ou d'environ 3 à 6 heures, de préférence dans un délai d'environ 3 heures, à compter du moment de la première incubation des cellules du micro-organisme à partir de ou dans ledit échantillon dans le milieu de culture pour obtenir la culture se divisant activement desdites cellules dudit micro-organisme.

12. Procédé selon l'une quelconque des revendications 1 à 11, dans lequel la détermination à partir des données de cytométrie en flux acoustique délivrées de la CMI de l'agent antimicrobien à laquelle le micro-organisme est sensible, comprend
(a) l'incubation dans un milieu de culture
(i) de cellules du micro-organisme provenant d'un échantillon qui a été obtenu auprès du sujet,
et/ou
(ii) d'un échantillon qui a été obtenu auprès du sujet, ledit
échantillon comprenant des cellules du micro-organisme, pendant une durée et dans des conditions suffisantes pour que lesdites cellules dudit micro-organisme se divisent activement pour obtenir une culture se divisant activement desdites cellules dudit micro-organisme, et
le marquage desdites cellules dudit micro-organisme dans ou à partir de ladite culture se divisant activement avec l'absence de composé fluorescent se liant aux acides nucléiques avant l'exposition à l'agent antimicrobien, pour produire ainsi un échantillon de contrôle non exposé ;
(b) la mesure par cytométrie en flux acoustique de la morphologie cellulaire et de la quantité desdites cellules dudit micro-organisme dans l'échantillon de contrôle non exposé ;
(c) la réalisation des étapes (i) à (iv) telles que définies dans la revendication 1 ; et
(d) la détermination à partir des données de cytométrie en flux acoustique délivrées de la concentration minimale dudit agent antimicrobien pour laquelle la quantité ou la proportion des cellules dudit micro-organisme exposées audit agent antimicrobien ayant une morphologie cellulaire de cellules non exposées à l'agent antimicrobien dans ledit échantillon de contrôle non exposé, est égale ou inférieur à une quantité ou une proportion prédéterminée des cellules dans ledit échantillon de contrôle non exposé mesurée par cytométrie en flux acoustique pour montrer ladite morphologie cellulaire de cellules non exposées à un agent antimicrobien.

13. Procédé selon la revendication 12, dans lequel la quantité ou la proportion prédéterminée des cellules dans ledit échantillon de contrôle non exposé mesurée par cytométrie en flux acoustique pour montrer la morphologie cellulaire de cellules non exposées à un agent antimicrobien, est déterminée par référence à des concentrations minimales inhibitrices (CMI) connues dudit agent antimicrobien à un panel d'isolats connus du micro-organisme ; et/ou la quantité ou la proportion prédéterminée des cellules dans ledit échantillon de contrôle non exposé mesurée par cytométrie en flux acoustique pour montrer ladite morphologie cellulaire de cellules non exposées à un agent antimicrobien est d'environ 1 % à environ 50 % du total des cellules dans ledit échantillon de contrôle non exposé.

14. Procédé selon l'une quelconque des revendications 1 à 13, comprenant en outre
(a) l'incubation dans un milieu de culture de cellules du micro-organisme à partir de l'échantillon qui a été obtenu auprès du sujet et/ou l'incubation dans le milieu de culture de l'échantillon qui a été obtenu auprès du sujet, l'échantillon comprenant les cellules du micro-organisme,
pendant une durée et dans des conditions suffisantes pour que lesdites cellules se divisent activement pour obtenir une culture se divisant activement desdites cellules dudit micro-organisme, et
le marquage de ladite cellule dudit micro-organisme dans ou à partir de ladite culture se divisant activement avec l'absence de composé fluorescent se liant aux acides nucléiques avant l'exposition à l'agent antimicrobien, pour produire ainsi un échantillon de contrôle non exposé ; et
(b) la mesure par cytométrie en flux acoustique de la morphologie cellulaire et de la quantité desdites cellules dudit micro-organisme dans l'échantillon de contrôle non exposé.

15. Procédé selon l'une quelconque des revendications 1 à 14, dans lequel
(a) l'incubation des cellules du micro-organisme dans et/ou à partir de l'échantillon dans un milieu de culture pendant une durée et dans des conditions suffisantes pour que lesdites cellules se divisent activement comprend l'incubation desdites cellules dudit micro-organisme pendant une durée et dans des conditions suffisantes pour qu'environ 1 à environ 5 divisions cellulaires se produisent, de préférence pour qu'environ 1 à environ 3 divisions cellulaires se produisent ; et/ou
(b) l'exposition des cellules du micro-organisme à l'agent antimicrobien pendant une durée et dans des conditions suffisantes pour que lesdites cellules du micro-organisme se divisent activement, comprend l'incubation desdites cellules dudit micro-organisme en présence de l'agent antimicrobien pendant une durée et dans des conditions suffisantes pour que lesdites cellules subissent environ 1 à environ 3 divisions cellulaires.

16. Procédé de détermination de la sensibilité d'un micro-organisme unicellulaire ou multicellulaire à une pluralité d'agents antimicrobiens, ledit procédé comprenant :
la réalisation du procédé selon l'une quelconque des revendications 1 à 15, ledit procédé comprenant l'exposition des cellules se divisant activement du micro-organisme dans et/ou à partir de l'échantillon qui a été obtenu auprès du sujet à un ou plusieurs agents antimicrobiens représentatifs d'une première famille ou d'un premier panel d'une pluralité d'agents antimicrobiens, pour ainsi déterminer la sensibilité desdites cellules dudit micro-organisme aux agents antimicrobiens appartenant à ladite première famille ou audit premier panel de la pluralité d'agents antimicrobiens.

17. Procédé selon l'une quelconque des revendications 1 à 16, dans lequel ledit procédé est utilisé pour le criblage à haut rendement de la sensibilité d'un ou de plusieurs micro-organismes unicellulaires ou multicellulaires à un ou plusieurs agents antimicrobiens.
